(19) Europäisches Patentamt
European Patent Office
Office européen des brevets

(11) EP 1 511 385 B1

(12) **EUROPEAN PATENT SPECIFICATION**

(45) Date of publication and mention of the grant of the patent:
**30.11.2005 Bulletin 2005/48**

(21) Application number: **03739104.2**

(22) Date of filing: **10.06.2003**

(51) Int Cl.$^7$: **A01N 47/38**

(86) International application number:
**PCT/US2003/018610**

(87) International publication number:
**WO 2003/103398 (18.12.2003 Gazette 2003/51)**

(54) **INSECTICIDAL AMIDES WITH NITROGEN-CONTAINING BENZO-FUSED BICYCLIC RING SYSTEMS**

INSEKTIZIDE AMIDE MIT STICKSTOFFHALTIGEN KONDENSIERTEN BIZYKLISCHEN RINGSYSTEMEN

AMIDES INSECTICIDES COMPRENANT DES SYSTEMES A NOYAU BICYCLIQUE CONDENSES AVEC UN NOYAU BENZENIQUE ET CONTENANT DE L'AZOTE

(84) Designated Contracting States:
**AT BE BG CH CY CZ DE DK EE ES FI FR GB GR HU IE IT LI LU MC NL PT RO SE SI SK TR**

(30) Priority: **11.06.2002 US 387640 P**

(43) Date of publication of application:
**09.03.2005 Bulletin 2005/10**

(73) Proprietor: **E.I. DU PONT DE NEMOURS AND COMPANY**
**Wilmington, Delaware 19898 (US)**

(72) Inventor: **STEVENSON, Thomas, Martin**
**Newark, DE 19702 (US)**

(74) Representative: **Beacham, Annabel Rose et al**
**Frank B. Dehn & Co.,**
**European Patent Attorneys,**
**179 Queen Victoria Street**
**London EC4V 4EL (GB)**

(56) References cited:
**EP-A- 0 919 542**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 511 385 B1

## Description

### FIELD OF THE INVENTION

This invention relates to certain indoline carboxamides and thiamides, their *N*-oxides, suitable salts and compositions, and a method of their use for controlling invertebrate pests in both agronomic and nonagronomic environments.

### BACKGROUND OF THE INVENTION

The control of invertebrate pests is extremely important in achieving high crop efficiency. Damage by invertebrate pests to growing and stored agronomic crops can cause significant reduction in productivity and thereby result in increased costs to the consumer. The control of invertebrate pests in forestry, greenhouse crops, ornamentals, nursery crops, stored food and fiber products, livestock, household, and public and animal health is also important. Many products are commercially available for these purposes, but the need continues for new compounds that are more effective, less costly, less toxic, environmentally safer or have different modes of action.

EP919542 discloses phthalic diamides of Formula i as insecticides

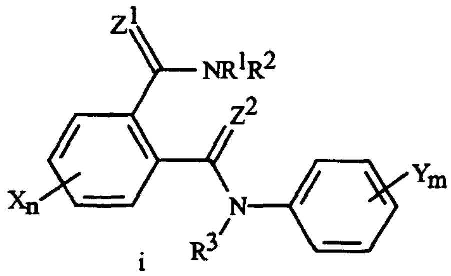

i

wherein, among others, $Z^1$ and $Z^2$ are O or S; and $R^1$, $R^2$ and $R^3$ are, among others, H, alkyl or substituted alkyl.

WO 96/23783 and US 5514690 disclose indoline carboxamide derivatives as pharmaceutical agents.

### SUMMARY OF THE INVENTION

This invention is related to compounds of Formula I and compounds of Formula II including all geometric and stereoisomers, *N*-oxides and salts thereof:

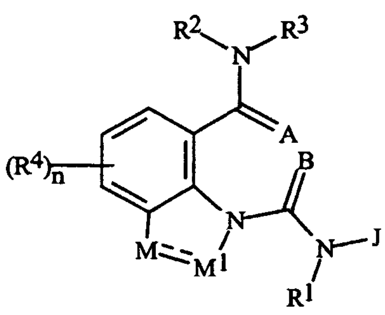

I

and

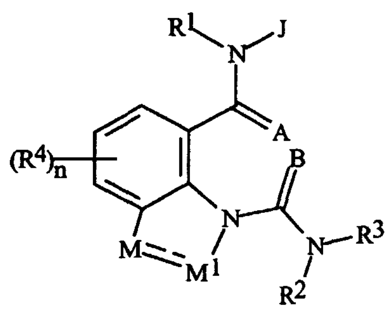

II

wherein

| | |
|---|---|
| each J | is independently a phenyl ring or a 5- or 6-membered heteroaromatic ring, each ring substituted with 1 to 4 $R^5$; |
| A and B | are independently O or S; |
| n | is 0, 1, 2 or 3; |

| | |
|---|---|
| $R^1$ | is H, $C_2$-$C_6$ alkylcarbonyl, $C_2$-$C_6$ alkoxycarbonyl, $C_2$-$C_6$ alkylaminocarbonyl or $C_3$-$C_8$ dialkylaminocarbonyl; or |
| $R^1$ | is $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $NO_2$, hydroxy, $C_1$-$C_4$ alkoxy, $C_1$-$C_4$ alkylthio, $C_1$-$C_4$ alkylsulfinyl, $C_1$-$C_4$ alkylsulfonyl, $C_2$-$C_4$ alkoxycarbonyl, $C_1$-$C_4$ alkylamino, $C_2$-$C_8$ dialkylamino and $C_3$-$C_6$ cycloalkylamino; |
| $R^2$ | is H, $C_1$-$C_6$ alkyl, $C_2$-$C_6$ alkenyl, $C_2$-$C_6$ alkynyl, $C_3$-$C_6$ cycloalkyl, $C_1$-$C_4$ alkoxy, $C_1$-$C_4$ alkylamino, $C_2$-$C_8$ dialkylamino, $C_3$-$C_6$ cycloalkylamino, $C_2$-$C_6$ alkoxycarbonyl or $C_2$-$C_6$ alkylcarbonyl; |
| $R^3$ | is H; or |
| $R^3$ | is $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $NO_2$, hydroxy, $C_1$-$C_4$ alkoxy, $C_1$-$C_4$ alkylthio, $C_1$-$C_4$ alkylsulfinyl and $C_1$-$C_4$ alkylsulfonyl; or |
| $R^2$ and $R^3$ | can be taken together with the nitrogen to which they are attached to form a ring containing 2 to 6 atoms of carbon and optionally one additional atom of nitrogen, sulfur or oxygen, said ring may be optionally substituted with 1 to 4 substituents selected from the group consisting of $C_1$-$C_2$ alkyl, halogen, CN, $NO_2$ and $C_1$-$C_2$ alkoxy; and |
| each $R^4$ and each $R^5$ | is independently H, $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_2$-$C_6$ haloalkenyl, $C_2$-$C_6$ haloalkynyl, $C_3$-$C_6$ halocycloalkyl, halogen, CN, $CO_2H$, $CONH_2$, $NO_2$, hydroxy, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl, $C_1$-$C_4$ alkylamino, $C_2$-$C_8$ dialkylamino, $C_3$-$C_6$ cycloalkylamino, $C_2$-$C_6$ alkylcarbonyl, $C_2$-$C_6$ alkoxycarbonyl, $C_2$-$C_6$ alkylaminocarbonyl, $C_3$-$C_8$ dialkylaminocarbonyl, or $C_3$-$C_6$ trialkylsilyl; or |
| each $R^4$ and each $R^5$ | is independently a phenyl, benzyl, phenoxy, or 5- or 6-membered heteroaromatic ring, each ring optionally substituted with one to three substituents independently selected from the group consisting of $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_2$-$C_4$ haloalkenyl, $C_2$-$C_4$ haloalkynyl, $C_3$-$C_6$ halocycloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ alkylamino, $C_2$-$C_8$ dialkylamino, $C_3$-$C_6$ cycloalkylamino, $C_3$-$C_6$ (alkyl)cycloalkylamino, $C_2$-$C_4$ alkylcarbonyl, $C_2$-$C_6$ alkoxycarbonyl, $C_2$-$C_6$ alkylaminocarbonyl, $C_3$-$C_8$ dialkylaminocarbonyl or $C_3$-$C_6$ trialkylsilyl; or |
| two $R^5$ | groups when attached to adjacent carbon atoms can be taken together as $-OCF_2O-$, $-CF_2CF_2O-$ or $-OCF_2CF_2O-$; |
| M and $M^1$ | are each independently $CR^6R^7$, $NR^8$, O or S when the bond between M and $M^1$ is a single bond; and are each independently $CR^6$ or N when the bond between M and $M^1$ is an aromatic bond; |
| each $R^6$ and each $R^7$ | is independently H, $C_1$-$C_4$ alkyl, halogen, CN, $C_1$-$C_4$ haloalkyl or $C_1$-$C_4$ alkoxy; and |
| each $R^8$ | is independently H or $C_1$-$C_4$ alkyl. |

This invention provides a method for controlling at least one invertebrate pest comprising contacting the invertebrate pest or its environment with a biologically effective amount of at least one compound selected from the group consisting of a compound of Formula I, an *N*-oxide or a salt thereof, and a compound of Formula II, an *N*-oxide or a salt thereof (e.g., as a composition described herein).

Of note is a method for controlling an invertebrate pest comprising contacting the invertebrate pest or its environment with a biologically effective amount of a compound of Formula I or a compound of Formula II (e.g., as a composition described herein).

This invention also provides a composition comprising at least one compound selected from the group consisting of a compound of Formula I, an *N*-oxide or a salt thereof, and a compound of Formula II, an *N*-oxide or a salt thereof; and at least one other biologically active compound or agent; and provides a method for controlling at least one invertebrate pest comprising contacting the invertebrate pest or its environment with a biologically effective amount of said composition.

Of note is a composition for controlling an invertebrate pest comprising (1) at least one compound selected from the group consisting of compounds of Formula I and compounds of Formula II, and (2) at least one additional compound or agent for controlling invertebrate pests; and provides a method for controlling an invertebrate pest comprising contacting the invertebrate pest or its environment a biologically effective amount of said composition.

This invention also provides a compound of Formula II, an *N*-oxide or a salt thereof; a composition which comprises at least one compound of Formula II, an *N*-oxide or salt thereof; and at least one additional component selected from the group consisting of a surfactant, a solid diluent, a liquid diluent and an other biologically active

compound or agent; and a method for controlling at least one invertebrate pest which comprises contacting the invertebrate pest or its environment with a biologically effective amount of said compound(s) or with a biologically effective amount of said composition.

Of note are compounds of Formula II: compositions for controlling an invertebrate pest which comprise at least one compound of Formula II and at least one additional component selected from the group consisting of surfactants, solid diluents, liquid diluents diluents and other biologically active compounds and agents; and methods for controlling an invertebrate pest which comprise contacting the invertebrate pest or its environment a biologically effective amount of said compounds or said compositions.

Of note are compounds of Formula Ic

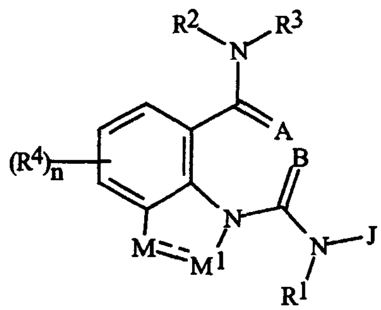

Ic

wherein

| | |
|---|---|
| M and $M^1$ | are each independently $CR^6R^7$, $NR^8$, O or S when the bond between M and $M^1$ is a single bond, provided that M and $M^1$ are not both $CR^6R^7$; and are each independently $CR^6$ or N when the bond between M and $M^1$ is an aromatic bond; and |
| A, B, J, | $R^1$ through $R^4$, $R^6$ through $R^8$ and n are defined as above. |

This invention also provides compounds of Formula Id

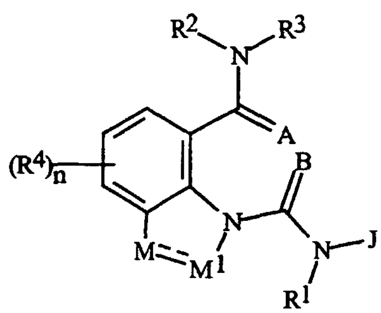

Id

wherein

| | |
|---|---|
| each $R^4$ and each $R^5$ | is independently H, $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_2$-$C_6$ haloalkenyl, $C_2$-$C_6$ haloalkynyl, $C_3$-$C_6$ halocycloalkyl, halogen, CN, $CO_2H$, $CONH_2$, $NO_2$, hydroxy, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl, $C_1$-$C_4$ alkylamino, $C_2$-$C_8$ dialkylamino, $C_3$-$C_6$ cycloalkylamino, $C_2$-$C_6$ alkylcarbonyl, $C_2$-$C_6$ alkoxycarbonyl, $C_2$-$C_6$ alkylaminocarbonyl, $C_3$-$C_8$ dialkylaminocarbonyl, or $C_3$-$C_6$ trialkylsilyl; or |
| each $R^5$ | is independently a phenyl, benzyl, phenoxy, or 5- or 6-membered heteroaromatic ring, each ring optionally substituted with one to three substituents independently selected from the |

group consisting of $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_2$-$C_4$ haloalkenyl, $C_2$-$C_4$ haloalkynyl, $C_3$-$C_6$ halocycloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ alkylamino, $C_2$-$C_8$ dialkylamino, $C_3$-$C_6$ cycloalkylamino, $C_3$-$C_6$ (alkyl)cycloalkylamino, $C_2$-$C_4$ alkylcarbonyl, $C_2$-$C_6$ alkoxycarbonyl, $C_2$-$C_6$ alkylaminocarbonyl, $C_3$-$C_8$ dialkylaminocarbonyl or $C_3$-$C_6$ trialkylsilyl; or

two $R^5$ groups when attached to adjacent carbon atoms can be taken together as $-OCF_2O-$, $-CF_2CF_2O-$ or $-OCF_2CF_2O-$; and

A, B, J, $R^1$ through $R^3$, $R^6$ through $R^8$ and n are defined as above.

This invention also provides a composition which comprises at least one compound of Formula Id, an *N*-oxide or a salt thereof; and at least one additional component selected from the group consisting of a surfactant, a solid diluent, a liquid diluent and an other biologically active compound or agent; and a method for controlling at least one invertebrate pest which comprises contacting the invertebrate pest or its environment with a biologically effective amount of said compound(s) or with a biologically effective amount of said composition.

DETAILS OF THE INVENTION

In the above recitations, the term "alkyl", used either alone or in compound words such as "alkylthio" or "haloalkyl" includes straight-chain or branched alkyl, such as, methyl, ethyl, *n*-propyl, *i*-propyl, or the different butyl, pentyl or hexyl isomers. "Alkenyl" includes straight-chain or branched alkenes such as 1-propenyl, 2-propenyl, and the different butenyl, pentenyl and hexenyl isomers. "Alkenyl" also includes polyenes such as 1,2-propadienyl and 2,4-hexadienyl. "Alkynyl" includes straight-chain or branched alkynes such as 1-propynyl, 2-propynyl and the different butynyl, pentynyl and hexynyl isomers. "Alkynyl" can also include moieties comprised of multiple triple bonds such as 2,5-hexadiynyl. "Alkoxy" includes, for example, methoxy, ethoxy, *n*-propyloxy, isopropyloxy and the different butoxy, pentoxy and hexyloxy isomers. "Alkylthio" includes branched or straight-chain alkylthio moieties such as methylthio, ethylthio, and the different propylthio, butylthio, pentylthio and hexylthio isomers. "Alkylsulfinyl" includes both enantiomers of an alkylsulfinyl group. Examples of "alkylsulfinyl" include $CH_3S(O)$, $CH_3CH_2S(O)$, $CH_3CH_2CH_2S(O)$, $(CH_3)_2CHS(O)$ and the different butylsulfinyl, pentylsulfinyl and hexylsulfinyl isomers. Examples of "alkylsulfonyl" include $CH_3S(O)_2$, $CH_3CH_2S(O)_2$, $CH_3CH_2CH_2S(O)_2$, $(CH_3)_2CHS(O)_2$ and the different butylsulfonyl, pentylsulfonyl and hexylsulfonyl isomers. "Alkylamino" includes branched or straight-chain alkylamino moieties such as methylamino, ethylamino, and the different propylamino, butylamino, pentylamino and hexylamino isomers. Examples of "alkylcarbonyl" include $C(O)CH_3$, $C(O)CH_2CH_2CH_3$ and $C(O)CH(CH_3)_2$. Examples of "alkoxycarbonyl" include $CH_3OC(=O)$, $CH_3CH_2OC(=O)$, $CH_3CH_2CH_2OC(=O)$, $(CH_3)_2CHOC(=O)$ and the different butoxy- or pentoxycarbonyl isomers. Examples of "alkylaminocarbonyl" include $CH_3NHC(=O)$, $CH_3CH_2NHC(=O)$, $CH_3CH_2CH_2NHC(=O)$, $(CH_3)_2CHNHC(=O)$ and the different butylamino- or pentylaminocarbonyl isomers. The term "dialkylamino" includes amino functions substituted with two alkyl groups that may be the same or different. "Dialkylaminocarbonyl" is defined analogously, and examples include $(CH_3)_2NC(=O)$ and $CH_3CH_2NCH_3C(=O)$.

The term "cycloalkyl", used either alone or in compound words such as "cycloalkylamino" or "halocycloalkyl" includes, for example, cyclopropyl, cyclobutyl, cyclopentyl and cyclohexyl.

The term "heteroaromatic ring" denotes fully aromatic rings in which at least one ring atom is not carbon and comprises 1 to 4 heteroatoms independently selected from the group consisting of nitrogen, oxygen and sulfur, provided that each heterocyclic ring contains no more than 4 nitrogens, no more than 2 oxygens and no more than 2 sulfurs (where aromatic indicates that the Hückel rule is satisfied). The heterocyclic ring can be attached through any available carbon or nitrogen by replacement of hydrogen on said carbon or nitrogen.

The term "halogen", either alone or in compound words such as "haloalkyl", includes fluorine, chlorine, bromine or iodine. Further, when used in compound words such as "haloalkyl", said alkyl may be partially or fully substituted with halogen atoms which may be the same or different. Examples of "haloalkyl" include $F_3C$, $ClCH_2$, $CF_3CH_2$ and $CF_3CCl_2$. The terms "haloalkenyl", "haloalkynyl", "haloalkoxy", and the like, are defined analogously to the term "haloalkyl". Examples of "haloalkenyl" include $(Cl)_2C{=}CHCH_2$ and $CF_3CH_2CH{=}CHCH_2$. Examples of "haloalkynyl" include $HC{\equiv}CCHCl$, $CF_3C{\equiv}C$, $CCl_3C{\equiv}C$ and $FCH_2C{\equiv}CCH_2$. Examples of "haloalkoxy" include $CF_3O$, $CCl_3CH_2O$, $HCF_2CH_2CH_2O$ and $CF_3CH_2O$. Examples of "haloalkylthio" include $CCl_3S$, $CF_3S$, $CCl_3CH_2S$ and $ClCH_2CH_2CH_2S$. Examples of "haloalkylsulfinyl" include $CF_3S(O)$, $CCl_3S(O)$, $CF_3CH_2S(O)$ and $CF_3CF_2S(O)$. Examples of "haloalkylsulfonyl" include $CF_3S(O)_2$, $CCl_3S(O)_2$, $CF_3CH_2S(O)_2$ and $CF_3CF_2S(O)_2$.

The combination of a broken line and an unbroken line between M and $M^1$ as shown in Formula I and Formula II designates that the bond between M and $M^1$ can be either a single bond or an aromatic bond. When the bond between M and $M^1$ is an aromatic bond then M, $M^1$ and the bond between them form a portion of an aromatic ring. For example, when the bond between M and $M^1$ is a single bond and M and $M^1$ are both $CR^6R^7$, then M and $M^1$ are both within an

indoline ring system; and when the bond between M and $M^1$ is an aromatic bond and M and $M^1$ are both $CR^6$, then M and $M^1$ are both within an indole ring system.

The total number of carbon atoms in a substituent group is indicated by the "$C_i$-$C_j$" prefix where i and j are numbers from 1 to 8. For example, $C_1$-$C_3$ alkylsulfonyl designates methylsulfonyl through propylsulfonyl; $C_2$ alkoxyalkyl designates $CH_3OCH_2$; $C_3$ alkoxyalkyl designates, for example, $CH_3CH(OCH_3)$, $CH_3OCH_2CH_2$ or $CH_3CH_2OCH_2$; and $C_4$ alkoxyalkyl designates the various isomers of an alkyl group substituted with an alkoxy group containing a total of four carbon atoms, examples including $CH_3CH_2CH_2OCH_2$ and $CH_3CH_2OCH_2CH_2$. In the above recitations, when a compound of Formula 1 contains a heteroaromatic ring, all substituents are attached to this ring through any available carbon or nitrogen by replacement of a hydrogen on said carbon or nitrogen.

When a group contains a substituent which can be hydrogen, for example $R^3$, then, when this substituent is taken as hydrogen, it is recognized that this is equivalent to said group being unsubstituted. When the number of optional substituents on a group is 0, for example when n is 0, then it is recognized that this is equivalent to said group being unsubstituted. When a bond is depicted as floating, the substituent may be attached to any of the available carbons on the ring by replacement of hydrogen. When $R^2$ and $R^3$ are taken together with the nitrogen to which they are attached to form a ring, said ring can be optionally substituted on any available carbon or optionally nitrogen in said ring.

Compounds of this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer (s) or when separated from the other stereoisomer(s). Additionally, the skilled artisan knows how to separate, enrich, and/or to selectively prepare said stereoisomers. The compounds of the invention can be present as a mixture of stereoisomers, individual stereoisomers, or as an optically active form.

The present invention involves compounds selected from Formula I or Formula II, *N*-oxides and salts thereof. One skilled in the art will appreciate that not all nitrogen containing heterocycles can form *N*-oxides since the nitrogen requires an available lone pair for oxidation to the oxide; one skilled in the art will recognize those nitrogen containing heterocycles which can form *N*-oxides. One skilled in the art will also recognize that tertiary amines can form *N*-oxides. Synthetic methods for the preparation of N-oxides of heterocycles and tertiary amines are very well known by one skilled in the art including the oxidation of heterocycles and tertiary amines with peroxy acids such as peracetic and *m*-chloroperbenzoic acid (MCPBA), hydrogen peroxide, alkyl hydroperoxides such as *t*-butyl hydroperoxide, sodium perborate, and dioxiranes such as dimethydioxirane. These methods for the preparation of *N*-oxides have been extensively described and reviewed in the literature, see for example: T. L. Gilchrist in *Comprehensive Organic Synthesis,* vol. 7, pp 748-750, S. V. Ley, Ed., Pergamon Press; M. Tisler and B. Stanovnik in *Comprehensive Heterocyclic Chemistry,* vol. 3, pp 18-19, A. J. Boulton and A. McKillop, Eds., Pergamon Press; M. R. Grimmett and B. R. T. Keene in *Advances in Heterocyclic Chemistry,* vol. 43, pp 139-151, A. R. Katritzky, Ed., Academic Press; M. Tisler and B. Stanovnik in *Advances in Heterocyclic Chemistry*, vol. 9, pp 285-291, A. R. Katritzky and A. J. Boulton, Eds., Academic Press; and G. W. H. Cheeseman and E. S. G. Werstiuk in *Advances in Heterocyclic Chemistry,* vol. 22, pp 390-392, A. R. Katritzky and A. J. Boulton, Eds., Academic Press.

The salts of the compounds of the invention include acid-addition salts with inorganic or organic acids such as hydrobromic, hydrochloric, nitric, phosphoric, sulfuric, acetic, butyric, fumaric, lactic, maleic, malonic, oxalic, propionic, salicylic, tartaric, 4-toluenesulfonic or valeric acids. In the compositions and methods of this invention, the salts of the compounds of the invention are preferably suitable for the agronomic and/or nonagronomic uses described herein.

As noted above, this invention includes a method for controlling at least one invertebrate pest comprising contacting the invertebrate pest or its environment with a biologically effective amount of at least one of a compound selected from the group consisting a compound of Formula I, an *N*-oxide or a salt thereof, and a compound of Formula II, an *N*-oxide or salt thereof. Preferred methods for reasons including greater control of invertebrate pests and/or ease of compound synthesis are:

Preferred 1. The method comprising applying a biologically effective amount of a compound of Formula I wherein

| | |
|---|---|
| A and B | are both O; |
| $R^1$ | is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; and |
| n | is 0, 1 or 2. |

Preferred 2. The method comprising applying a biologically effective amount of a compound of Formula II wherein

| | |
|---|---|
| A and B | are both O; |

$R^1$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; and

n is 0, 1 or 2.

Preferred 3. The method of Preferred 1 or Preferred 2 comprising applying a biologically effective amount of a compound of Formula I or a compound of Formula II wherein

J is a phenyl ring or a 5- or 6-membered heteroaromatic ring selected from the group consisting of J-1, J-2, J-3 and J-4

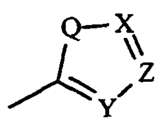 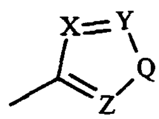 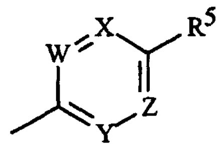 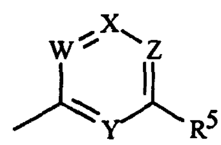

J-1 J-2 J-3 J-4 ;

Q is O, S or $NR^5$;

W, X, Y and Z are independently N or $CR^5$, provided that in J-3 and J-4 at least one of the group consisting of W, X, Y and Z is N;

$R^2$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl;

$R^3$ is H, $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $C_1$-$C_2$ alkoxy, $C_1$-$C_2$ alkylthio, $C_1$-$C_2$ alkylsulfinyl and $C_1$-$C_2$ alkylsulfonyl;

each $R^4$ is independently $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl;

each $R^5$ is independently H, $C_1$-$C_4$ alkyl, $C_1$-$C_6$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl or $C_2$-$C_4$ alkoxycarbonyl, $C_2$-$C_6$ alkylaminocarbonyl, $C_3$-$C_8$ dialkylaminocarbonyl; or

each $R^5$ is independently a phenyl, benzyl or a 5- or 6-membered heteroaromatic ring, each ring optionally substituted with $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_2$-$C_4$ haloalkenyl, $C_2$-$C_4$ haloalkynyl, $C_3$-$C_6$ halocycloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ alkylamino, $C_2$-$C_8$ dialkylamino, $C_3$-$C_6$ cycloalkylamino, $C_3$-$C_6$ (alkyl)cycloalkylamino, $C_2$-$C_4$ alkylcarbonyl, $C_2$-$C_6$ alkoxycarbonyl, $C_2$-$C_6$ alkylaminocarbonyl, $C_3$-$C_8$ dialkylaminocarbonyl or $C_3$-$C_6$ trialkylsilyl; or

two $R^5$ groups when attached to adjacent carbon atoms can be taken together as $-OCF_2O-$, $-CF_2CF_2O-$ or $-OCF_2CF_2O-$.

Preferred 4. The method of Preferred 3 comprising applying a biologically effective amount of a compound of Formula I wherein

$R^1$ is H or $C_1$-$C_4$ alkyl;

$R^2$ is H or $C_1$-$C_4$ alkyl;

$R^3$ is H, $C_1$-$C_4$ alkyl optionally substituted with halogen, CN, $OCH_3$, or $S(O)_pCH_3$;

each $R^5$ is independently H, $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl, $C_2$-$C_4$ alkoxycarbonyl or $C_3$-$C_8$ dialkylaminocarbonyl; or a phenyl, benzyl, or a 5- or 6-membered heteroaromatic ring, each ring optionally substituted with halogen, CN, $NO_2$, $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_1$-$C_4$ alkoxy or $C_1$-$C_4$ haloalkoxy;

provided that at least one $R^5$ is other than H and is attached to J at the position *ortho* to the $N(R^1)C(=B)$ moiety; and

p is 0, 1 or 2.

Preferred 5. The method of Preferred 4 comprising applying a biologically effective amount of a compound of Formula I wherein

J is a substituted phenyl, a substituted pyrazole, a substituted pyrrole, a substituted pyridine or a substituted pyrimidine.

Preferred 6. The method of Preferred 5 comprising applying a biologically effective amount of a compound of Formula I wherein

$R^1$ and $R^2$ are each H.

Preferred 7. The method of Preferred 3 comprising applying a biologically effective amount of a compound of Formula II wherein

$R^2$ is H;
$R^3$ is $C_1$-$C_4$ alkyl; and
at least one $R^5$ is other than H and is attached to J at the position *ortho* to the $N(R^1)C(=B)$ moiety.

As noted above, this invention also provides compounds of Formula Id. Preferred compounds for ease of synthesis and/or greater control of invertebrate pests are:

Preferred 8. Compounds of Formula Id wherein

A and B are both O;
$R^1$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl;
n is 0, 1 or 2.

Preferred 9. Compounds of Preferred 8 wherein

J is a phenyl ring or a 5- or 6-membered heteroaromatic ring selected from the group consisting of J-1, J-2, J-3 and J-4

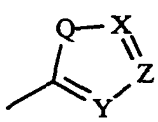
J-1

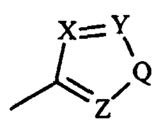
J-2

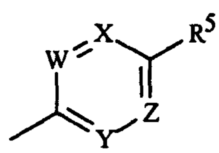
J-3

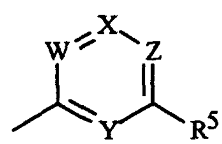
J-4

;

Q is O, S or $NR^5$;
W, X, Y and Z are independently N or $CR^5$, provided that in J-3 and J-4 at least one of the group consisting of W, X, Y and Z is N;
$R^2$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl;
$R^3$ is H, $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $C_1$-$C_2$ alkoxy, $C_1$-$C_2$ alkylthio, $C_1$-$C_2$ alkylsulfinyl and $C_1$-$C_2$ alkylsulfonyl;
each $R^4$ is independently $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl;
each $R^5$ is independently H, $C_1$-$C_4$ alkyl, $C_1$-$C_6$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$

haloalkylsulfinyl, $C_1$-$C_4$ haloalkylsulfonyl or $C_2$-$C_4$ alkoxycarbonyl, $C_2$-$C_6$ alkylaminocarbonyl, $C_3$-$C_8$ dialkylaminocarbonyl; or

each $R^5$ is independently a phenyl, benzyl or a 5- or 6-membered heteroaromatic ring, each ring optionally substituted with $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_2$-$C_4$ haloalkenyl, $C_2$-$C_4$ haloalkynyl, $C_3$-$C_6$ halocycloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ alkylamino, $C_2$-$C_8$ dialkylamino, $C_3$-$C_6$ cycloalkylamino, $C_3$-$C_6$ (alkyl)cycloalkylamino, $C_2$-$C_4$ alkylcarbonyl, $C_2$-$C_6$ alkoxycarbonyl, $C_2$-$C_6$ alkylaminocarbonyl, $C_3$-$C_8$ dialkylaminocarbonyl or $C_3$-$C_6$ trialkylsilyl; or

two $R^5$ groups when attached to adjacent carbon atoms can be taken together as $-OCF_2O-$, $-CF_2CF_2O-$ or $-OCF_2CF_2O-$.

Preferred 10. Compounds of Preferred 9 wherein

$R^1$ is H or $C_1$-$C_4$ alkyl;

$R^2$ is H or $C_1$-$C_4$ alkyl;

$R^3$ is H, $C_1$-$C_4$ alkyl optionally substituted with halogen, CN, $OCH_3$, or $S(O)_pCH_3$;

each $R^5$ is independently H, $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl, $C_2$-$C_4$ alkoxycarbonyl or $C_3$-$C_8$ dialkylaminocarbonyl; or a phenyl, benzyl, or a 5- or 6-membered heteroaromatic ring, each ring optionally substituted with halogen, CN, $NO_2$, $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_1$-$C_4$ alkoxy or $C_1$-$C_4$ haloalkoxy;

provided that at least one $R^5$ is other than H and is attached to J at the position *ortho* to the $N(R^1)C(=B)$ moiety; and

p is 0,1 or 2.

Preferred 11. Compounds of Preferred 10 wherein

J is a substituted phenyl, a substituted pyrazole, a substituted pyrrole, a substituted pyridine or a substituted pyrimidine.

Preferred 12. Compounds of Preferred 11 wherein $R^1$ and $R^2$ are each H.

Of note are compounds of Formula Ic. Preferred compounds of Formula Ic for ease of synthesis and/or greater control of invertebrate pests are:

Preferred 8a. Compounds of Formula Ic wherein

A and B are both O;

$R^1$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; and

n is 0, 1 or 2.

Preferred 9a. Compounds of Preferred 8a wherein

J is a phenyl ring or a 5- or 6-membered heteroaromatic ring selected from the group consisting of J-1, J-2, J-3 and J-4

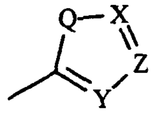
J-1

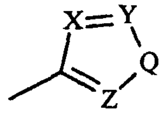
J-2

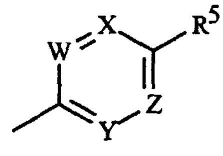
J-3

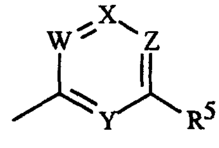
J-4

;

| | |
|---|---|
| Q | is O, S or $NR^5$; |
| W, X, Y and Z | are independently N or $CR^5$, provided that in J-3 and J-4 at least one of the group consisting of W, X, Y and Z is N; |
| $R^2$ | is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; |
| $R^3$ | is H, $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $C_1$-$C_2$ alkoxy, $C_1$-$C_2$ alkylthio, $C_1$-$C_2$ alkylsulfinyl and $C_1$-$C_2$ alkylsulfonyl; |
| each $R^4$ | is independently $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl; |
| each $R^5$ | is independently H, $C_1$-$C_4$ alkyl, $C_1$-$C_6$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl or $C_2$-$C_4$ alkoxycarbonyl, $C_2$-$C_6$ alkylaminocarbonyl, $C_3$-$C_8$ dialkylaminocarbonyl; or |
| each $R^5$ | is independently a phenyl, benzyl or a 5- or 6-membered heteroaromatic ring, each ring optionally substituted with $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_2$-$C_4$ haloalkenyl, $C_2$-$C_4$ haloalkynyl, $C_3$-$C_6$ halocycloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ alkylamino, $C_2$-$C_8$ dialkylamino, $C_3$-$C_6$ cycloalkylamino, $C_3$-$C_6$ (alkyl)cycloalkylamino, $C_2$-$C_4$ alkylcarbonyl, $C_2$-$C_6$ alkoxycarbonyl, $C_2$-$C_6$ alkylaminocarbonyl, $C_3$-$C_8$ dialkylaminocarbonyl or $C_3$-$C_6$ trialkylsilyl; or |
| two $R^5$ | groups when attached to adjacent carbon atoms can be taken together as $-OCF_2O-$, $-CF_2CF_2O-$ or $-OCF_2CF_2O-$. |

Preferred 10a. Compounds of Preferred 9a wherein

| | |
|---|---|
| $R^1$ | is H or $C_1$-$C_4$ alkyl; |
| $R^2$ | is H or $C_1$-$C_4$ alkyl; |
| $R^3$ | is H, $C_1$-$C_4$ alkyl optionally substituted with halogen, CN, $OCH_3$, or $S(O)_pCH_3$; |
| each $R^5$ | is independently H, $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl, $C_2$-$C_4$ alkoxycarbonyl or $C_3$-$C_8$ dialkylaminocarbonyl; or a phenyl, benzyl, or a 5- or 6-membered heteroaromatic ring, each ring optionally substituted with halogen, CN, $NO_2$, $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_1$-$C_4$ alkoxy or $C_1$-$C_4$ haloalkoxy; |
| | provided that at least one $R^5$ is other than H and is attached to J at the position *ortho* to the $N(R^1)C(=B)$ moiety; and |
| p | is 0, 1 or 2. |

Preferred 11a. Compounds of Preferred 10a wherein

J is a substituted phenyl, a substituted pyrazole, a substituted pyrrole, a substituted pyridine or a substituted pyrimidine.

Preferred 12a. Compounds of Preferred 11a wherein $R^1$ and $R^2$ are each H.

As noted above, this invention also provides compounds of Formula II. Preferred compounds for ease of synthesis and/or greater control of invertebrate pests are:

Preferred 13. Compounds of Formula II wherein

| | |
|---|---|
| A and B | are both O; |
| $R^1$ | is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; and |
| n | is 0, 1 or 2. |

Preferred 14. Compounds of Preferred 13 wherein

J is a phenyl ring or a 5- or 6-membered heteroaromatic ring selected from the group consisting of J-1, J-2, J-3 and J-4

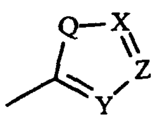

J-1

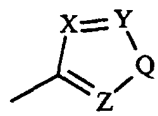

J-2

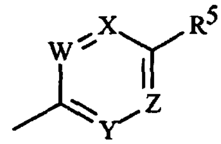

J-3

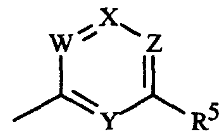

J-4

;

Q ios O, S or $NR^5$;

W, X, Y and Z are independently N or $CR^5$, provided that in J-3 and J-4 at least one of the group consisting of W, X, Y and Z is N;

$R^2$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl;

$R^3$ is H, $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $C_1$-$C_2$ alkoxy, $C_1$-$C_2$ alkylthio, $C_1$-$C_2$ alkylsulfinyl and $C_1$-$C_2$ alkylsulfonyl;

each $R^4$ is independently $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl;

each $R^5$ is independently H, $C_1$-$C_4$ alkyl, $C_1$-$C_6$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl or $C_2$-$C_4$ alkoxycarbonyl, $C_2$-$C_6$ alkylaminocarbonyl, $C_3$-$C_8$ dialkylaminocarbonyl; or

each $R^5$ is independently a phenyl, benzyl or a 5- or 6-membered heteroaromatic ring, each ring optionally substituted with $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_2$-$C_4$ haloalkenyl, $C_2$-$C_4$ haloalkynyl, $C_3$-$C_6$ halocycloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ alkylamino, $C_2$-$C_8$ dialkylamino, $C_3$-$C_6$ cycloalkylamino, $C_3$-$C_6$ (alkyl)cycloalkylamino, $C_2$-$C_4$ alkylcarbonyl, $C_2$-$C_6$ alkoxycarbonyl, $C_2$-$C_6$ alkylaminocarbonyl, $C_3$-$C_8$ dialkylaminocarbonyl or $C_3$-$C_6$ trialkylsilyl; or

two $R^5$ groups when attached to adjacent carbon atoms can be taken together as $-OCF_2O-$, $-CF_2CF_2O-$ or $-OCF_2CF_2O-$.

Preferred 15. Compounds of Formula II of Preferred 14 wherein

$R^2$ is H;

$R^3$ is $C_1$-$C_4$ alkyl; and

at least one $R^5$ is other than H and is attached to J at the position *ortho* to the $N(R^1)C(=B)$ moiety.

This invention also provides a composition comprising at least one compound selected from the group consisting of a compound of Formula I, an *N*-oxide or a salt thereof, and a compound of Formula II, an *N*-oxide or a salt thereof; and at least one other biologically active compound or agent; and also provides a method for controlling at least one invertebrate pest comprising contacting the invertebrate pest or its environment with a biologically effective amount of said composition. Preferred compositions include compositions wherein the at least one compound of Formula I, an *N*-oxide or a salt thereof and/or Formula II, an *N*-oxide or a salt thereof is selected from the compounds in Preferred 1 through 7. Also preferred is a composition further comprising an additional component selected from the group consisting of a surfactant, a solid diluent, and a liquid diluent.

Of note are compositions for controlling an invertebrate pest comprising (1) at least one compound selected from the group consisting of compounds of Formula I and compounds of Formula II, and (2) at least one additional compound or agent for controlling invertebrate pests; and also provides a method for controlling an invertebrate pest comprising the invertebrate pest or its environment a biologically effective amount of said composition. Preferred compositions include compositions wherein component (1) is selected from compounds(s) of Formula I and/or Formula II as preferred for the preferred method in Preferred 1 through 7.

This invention also provides a composition comprising at least one compound of Formula II, an *N*-oxide or a salt thereof; and at least one additional component selected from the group consisting of a surfactant, a solid diluent, a liquid diluent and an other biologically active compound or agent; and also provides a method for controlling at least one invertebrate pest comprising contacting the invertebrate pest or its environment with a biologically effective amount of said compound(s) of Formula II, its *N*-oxides and salts thereof or with a biologically effective amount of said composition. Preferred compositions and methods include compositions and methods wherein the at least one compound of Formula II, an *N*-oxides or a salt thereof, is selected from the compounds in Preferred 13 through 15.

Of note is a composition for controlling an invertebrate pest comprising at least one compound of Formula II and at least one additional component selected from the group consisting of surfactants, solid diluents, liquid diluents and other biologically active compounds and agents; and also provides a method for controlling an invertebrate pest comprising contacting the invertebrate pest or its environment a biologically effective amount of said composition. Preferred compositions include compositions wherein the compound(s) of Formula II, are selected from the compounds preferred in Preferred 13 through 15.

This invention also provides a composition comprising at least one compound of Formula Id, an *N*-oxide or a salt thereof; and at least one additional component selected from the group consisting of a surfactant, a solid diluent, a liquid diluent and an other biologically active compound or agent; and also provides a method for controlling at least one invertebrate pest comprising contacting the invertebrate pest or its environment with a biologically effective amount of said compound(s) or with a biologically effective amount of said composition. Preferred compositions and methods include compositions and methods

wherein the at least one compound of Formula Id, a *N*-oxide or a salt thereof, is selected from the compounds in Preferred 8 through 12.

Of note is a composition for controlling an invertebrate pest comprising at least one compound of Formula Ic and at least one additional component selected from the group consisting of surfactants, solid diluents, liquid diluents and other biologically active compounds or agents; and also provides a method for controlling an invertebrate pest comprising contacting the invertebrate pest or its environment a biologically effective amount of said composition. Preferred compositions include compositions wherein the compound(s) of Formula Ic are selected from the compounds preferred in Preferred 8a through 12a.

As noted above, each J is independently a phenyl ring or a 5- or 6-membered heteroaromatic ring wherein each ring is substituted with 1 to 4 $R^5$. An example of phenyl substituted with 1 to 4 $R^5$ is the ring illustrated as U-1 in Exhibit 1, wherein $R^v$ is $R^5$ and r is an integer from 1 to 4. Examples of 5- or 6-membered heteroaromatic rings optionally substituted with 1 to 4 $R^5$ include the rings U-2 through U-53 illustrated in Exhibit 1 wherein $R^v$ is $R^5$ and r is an integer from 1 to 4. Note that J-1 through J-4 above also denote 5- or 6-membered heteroaromatic rings. Note that U-2 through U-20 are examples of J-1, U-21 through U-35 and U-40 are examples of J-2, U-41 through U-48 are examples of J-3 and U-49 through U-53 are examples of J-4.

Note that when $R^v$ is H when attached to an atom, this is the same as if said atom is unsubstituted. The nitrogen atoms that require substitution to fill their valence are substituted with H or $R^v$. Note that some U groups can only be substituted with less than 4 $R^v$ groups (e.g. U-14, U-15, U-18 through U-21 and U-32 through U-34 can only be substituted with one $R^v$). Note that when the attachment point between $(R^v)_r$ and the U group is illustrated as floating, $(R^v)_r$ can be attached to any available carbon atom of the U group.

Exhibit 1

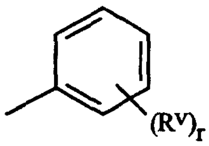,
U-1

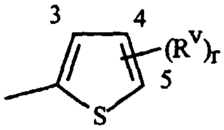,
U-2

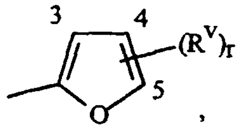,
U-3

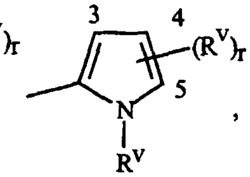,
U-4

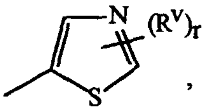,
U-5

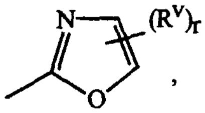,
U-6

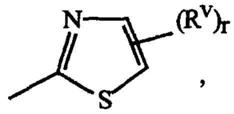,
U-7

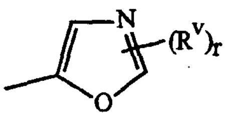,
U-8

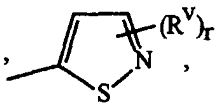,
U-9

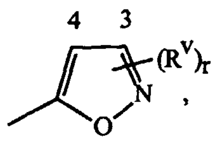,
U-10

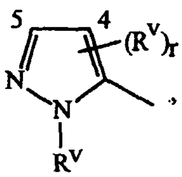,
U-11

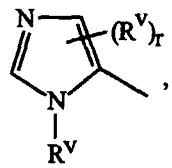,
U-12

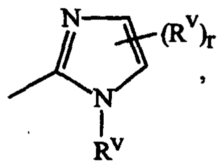,
U-13

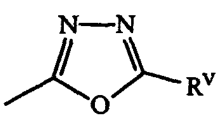,
U-14

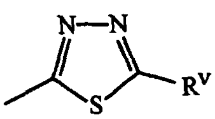,
U-15

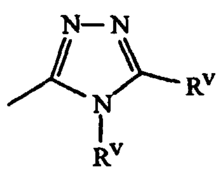,
U-16

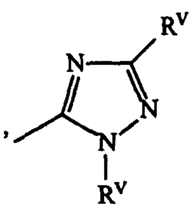,
U-17

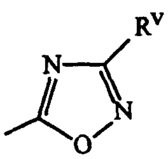,
U-18

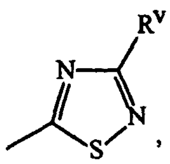,
U-19

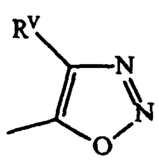,
U-20

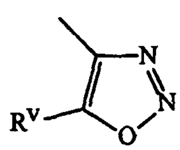,
U-21

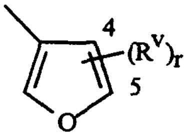
U-22 ,
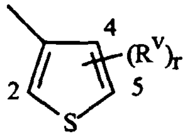
U-23 ,
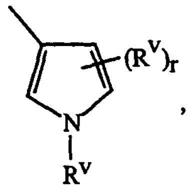
U-24 ,
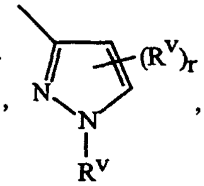
U-25 ,
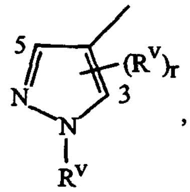
U-26 ,

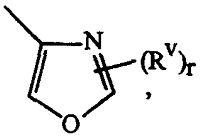
U-27 ,
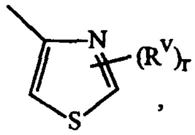
U-28 ,
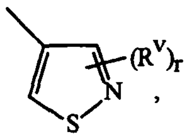
U-29 ,
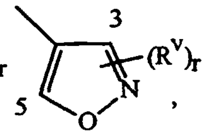
U-30 ,
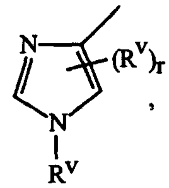
U-31 ,

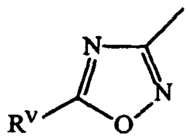
U-32 ,
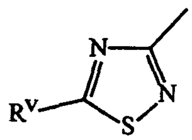
U-33 ,
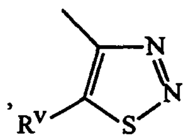
U-34 ,
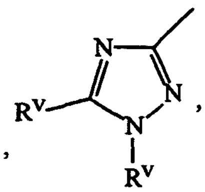
U-35 ,

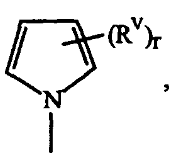
U-36 ,
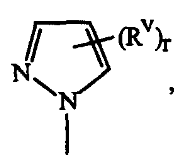
U-37 ,
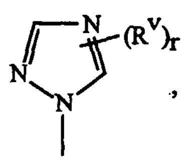
U-38 ,
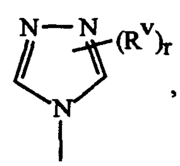
U-39 ,

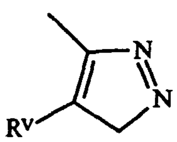
U-40 ,
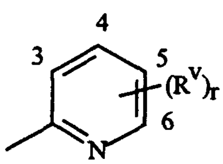
U-41 ,
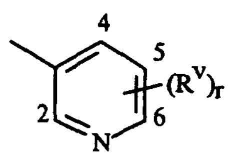
U-42 ,
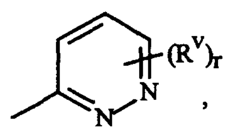
U-43 ,

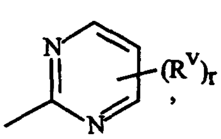
U-44 ,
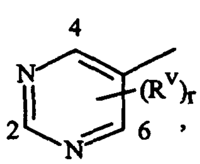
U-45 ,
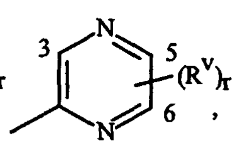
U-46 ,
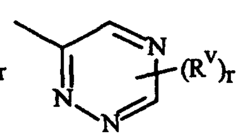
U-47 ,
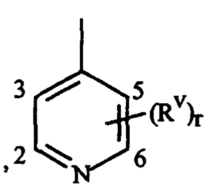
U-48 ,

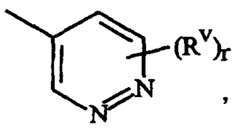, 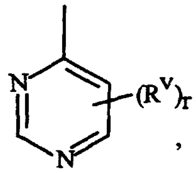, 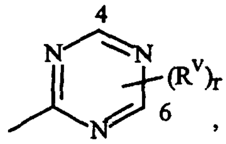, 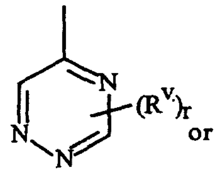 or 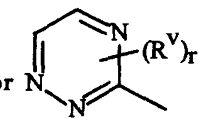

U-49 U-50 U-51 U-52 U-53

The compounds of Formula I and Formula II can be prepared by one or more of the following methods and variations as described in Schemes 1-11. The definitions of $R^1$, $R^2$, $R^3$, $R^4$, A, B, M, M $^1$ and n in the compounds described in the Schemes below are as defined above in the Summary of the Invention or their subsets unless otherwise indicated. Compounds of Formulae Ia-d are subsets of the compounds of Formula I.

As illustrated in Scheme 1, compounds of Formula I can be prepared by coupling compounds of Formula 1 with either iso(thio)cyanates of Formula 2a or (thio)carbamyl chlorides of Formula 2b. In the case of the (thio)carbamyl chloride reaction the presence of an acid acceptor is advantageous. Tertiary amines and alkali (such as lithium, sodium or potassium) carbonates and acetates are preferred as acid acceptors. Triethylamine is especially preferred. The reaction can be carried out at temperatures in the range of -30 to 120 °C. The reaction can be carried out in a variety of aprotic solvents which do not react with iso(thio)cyanates and (thio)carbamyl chlorides. Preferred solvents include, for example but not limited to, dichloromethane, toluene, ether, ethyl acetate and chloroform. General synthetic methods to prepare iso(thio)cyanates and (thio)carbamyl chlorides from their amine precursors can be found in WO 01/56962, EP 1006102 and EP 919542. The said amine precursors can be prepared by methods disclosed in WO 02/32856.

<u>Scheme 1</u>

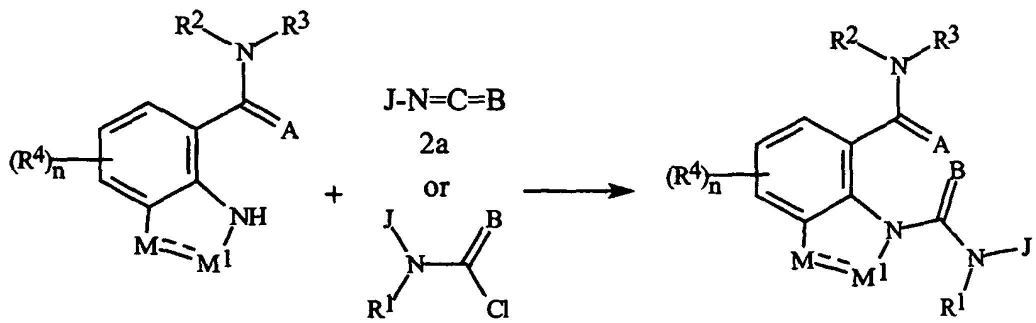


1 2b I

Compounds of Formula 1 can be synthesized by removal of the BOC (i.e. *t*-BuOOC) group found in compounds of Formula 3 as shown in Scheme 2. Reaction of a compound of Formula 3 with an acid in the presence of a suitable solvent produces a compound of Formula 1. A wide variety of acids can be used for the deprotection, including, for example but not limited to, hydrochloric acid, hydrobromic acid, triflic acid, trifluoroacetic acid, acetic acid, and methanesulfonic acid. Suitable solvents include both aqueous and organic solvents. A preferred combination of acid and solvent is trifluoroacetic acid in dichloromethane.

Scheme 2

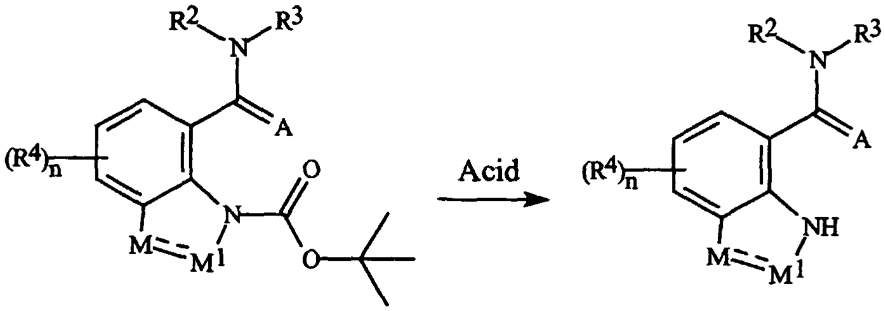


3 1

Compounds of Formula 3 can be prepared by lithiation of compounds of Formula 4 followed by quenching with either iso(thio)cyanates of Formula 5a or (thio)carbamyl chlorides of Formula 5b as shown in Scheme 3 (using iso(thio)cyanates of Formula 5a, $R^3$ in the product of Formula 3 is H). The lithiation can be carried out with an alkyl lithium reagent or a lithium dialkylamide reagent at temperatures in the range of -100 to 0 °C. The preferred conditions involve the use of *t*-butyllithium in ether at -78 °C. After addition of iso(thio)cyanates or (thio)carbamyl chlorides the reaction is allowed to warm to room temperature or is heated to temperatures between 20 and 120 °C. For a leading reference to the lithiation of indolines (M and $M^1$ are independently $CR^6R^7$ and the bond between M and $M^1$ is a single bond) see, Iwao and Kuraishi, *Heterocycles,* **1992,** *34,* 1031-1038. Iso(thio)cyanates of Formula 5a and (thio)carbamyl chlorides of Formula 5b are commercially available, well known in the chemical literature or can be made by general methods known in the chemical literature.

Scheme 3

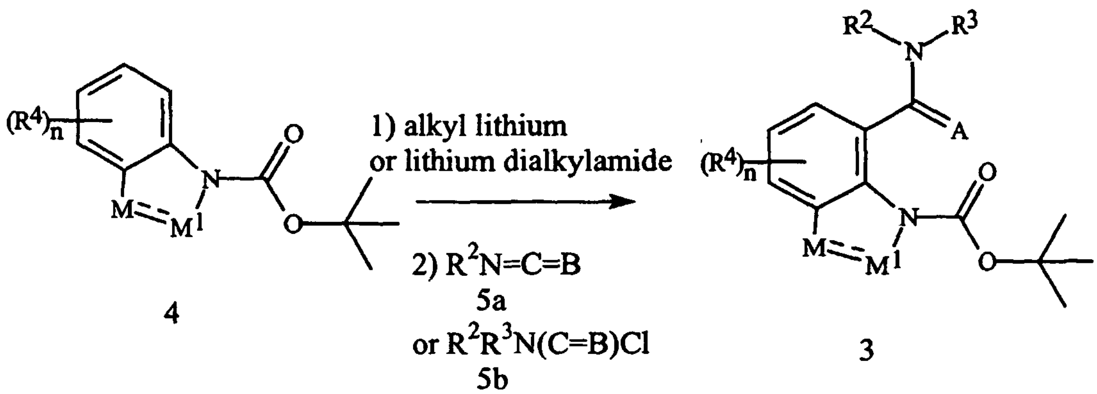


4 3

As shown in Scheme 4, a compound of Formula 4 can be prepared by reaction of a heterocycle of Formula 6 with di-*tert*-butyl dicarbonate. The reaction can be carried out at temperatures in the range of -70 to 120 °C in the presence of an acid acceptor. Tertiary amines and alkali (such as lithium, sodium or potassium) carbonates, hydroxides and acetates are preferred as acid acceptors. Triethylamine is especially preferred. The reaction is generally conducted in a solvent, such as dichloromethane, toluene, ether, ethyl acetate and chloroform.

Scheme 4

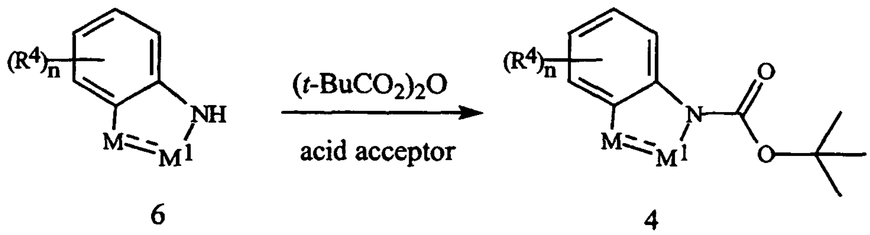


6 4

Compounds of Formula II can be prepared by reaction of compounds of Formula 7 with either iso(thio)cyanates of Formula 5a or (thio)carbamyl chlorides of Formula 5b as shown in Scheme 5. In the case of the (thio)carbamyl chloride reaction, the presence of an acid acceptor is advantageous. Tertiary amines and alkali (such as lithium, sodium or potassium) carbonates and acetates are preferred as acid acceptors. Triethylamine is especially preferred. The reaction can be carried out at temperatures in the range of -30 to 120 °C. The reaction can be carried out in a variety of aprotic solvents which do not react with iso(thio)cyanates and (thio)carbamyl chlorides. Preferred solvents include, for example, dichloromethane, toluene, ether, ethyl acetate and chloroform.

Scheme 5

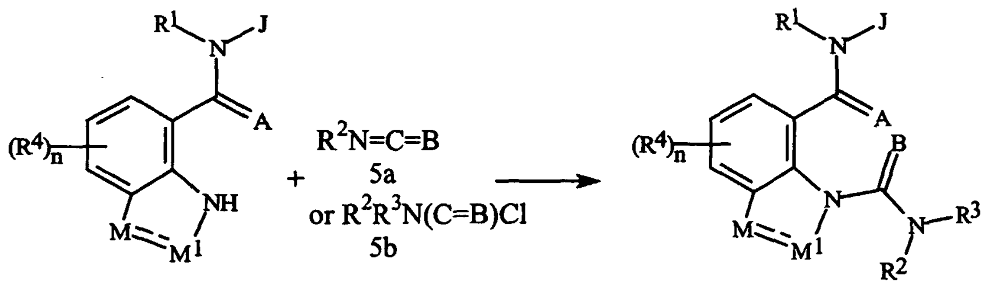


7 II

Compounds of Formula 7 can be prepared by removal the BOC (i.e. *t*-BuOOC) group found in compounds of Formula 8 as shown in Scheme 6. The reaction conditions for this deprotection are already described for the conversion of the compounds of Formula 3 to the compounds of Formula 1 in Scheme 2.

Scheme 6

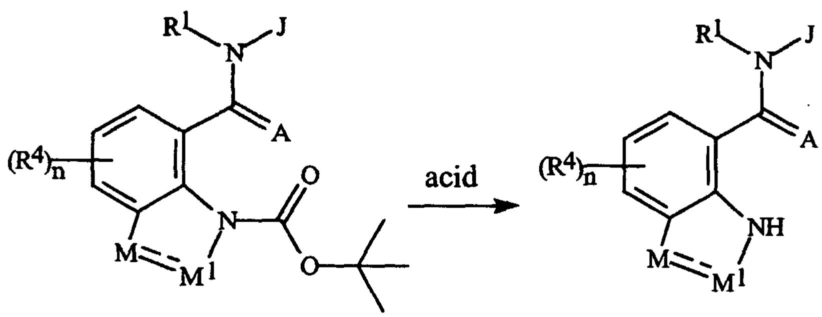


8 7

Compounds of Formula 8 can be prepared by lithiation of compounds of Formula 4 followed by quenching with either iso(thio)cyanates of Formula 2a or (thio)carbamyl chlorides of Formula 2b as shown in Scheme 7. The reaction conditions for this conversion are already described for the conversion of the compounds of Formula 4 to the compounds of Formula 3 in Scheme 3.

Scheme 7

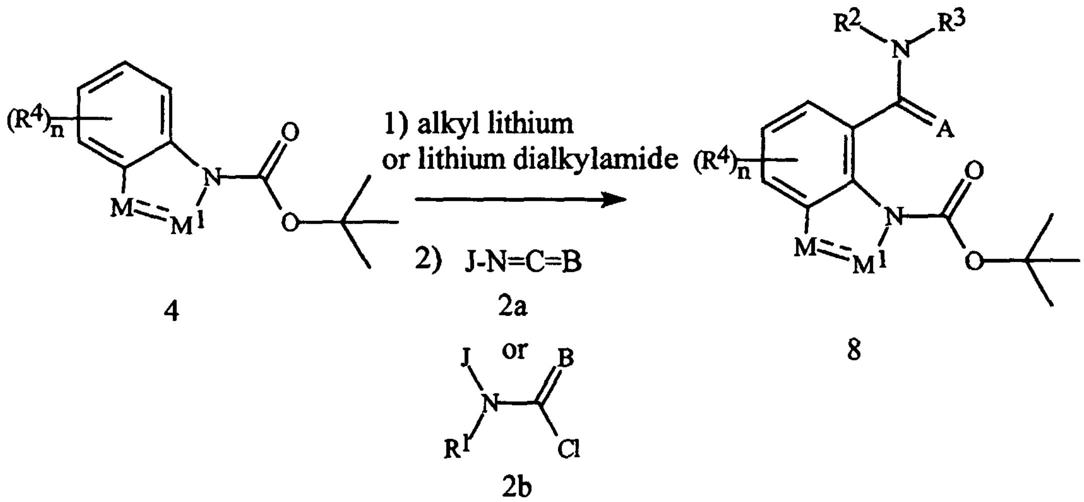


4

2a

or

2b

8

As shown in Scheme 8, alkylation of a compound of Formula 3a with an alkylating agent of Formula 9 in the presence of a base will provide a compound of Formula 3b wherein $R^3$ is other than H. Compounds of Formula 3a are a subset of compounds of Formula 3 wherein $R^3$ is H. Compounds of Formula 3b are also a subset of compounds of Formula 3 wherein $R^3$ is other than H. In the alkylating agent of Formula 9, X is a leaving group such as halogen (e.g., Br, I), $OS(O)_2CH_3$ (methanesulfonate), $OS(O)_2CF_3$, $OS(O)_2Ph$-*p*-$CH_3$ (*p*-toluenesulfonate), and the like. Examples of suitable bases include alkali (such as lithium, sodium or potassium) metal hydroxides, carbonates, hydrides and alkoxides. The reaction is generally conducted in a suitable solvent such as tetrahydrofuran, dioxane, dimethylsulfoxide and *N,N*-dimethylacetamide. Other examples of suitable solvents, particularly useful when alkali alkoxides are used as bases, include alcohols such as methanol and ethanol. Preferred conditions involve the use of sodium hydride as a base and *N,N*-dimethylformamide as a solvent.

Scheme 8

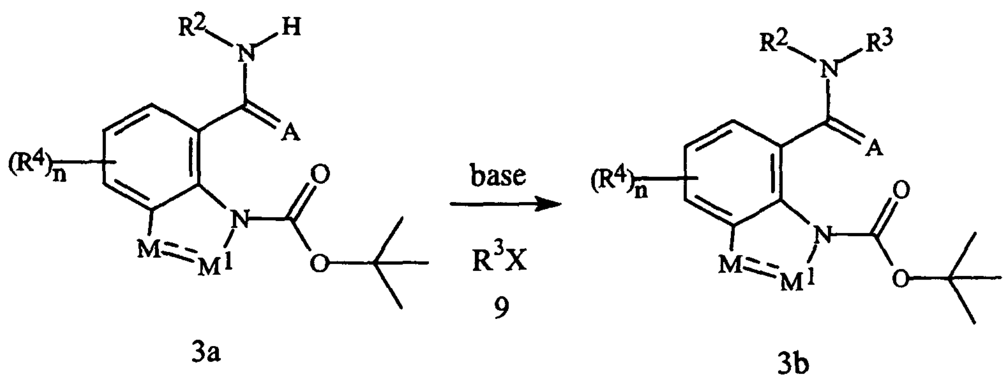


3a

3b

As an alternative method to that illustrated in Scheme 2, compounds of Formula 1 can also be prepared by amide formation of compounds of Formula 10 in the presence of an organic dehydrating reagent outlined in Scheme 9. This involves direct dehydrative coupling of a carboxylic acid of Formula 10 with an amine of Formula 11. The dehydrating reagent can be, for example, 1,3-dicyclohexylcarbodiimide (DCC), 1-[3-(dimethylamino)propyl]-3-ethylcarbodiimide hydrochloride (EDC), carbonyl diimidazole, 2-chloro-N-methylpyridinium iodide (Mukaiyama's reagent), benzotriazol-1-yloxy-tris(dimethylamino)-phosphonium hexafluorophosphate (BOP) and bis(2-oxo-3-oxazolidinyl) phosphinic chloride (BOP chloride). Various polymer-bound versions of these reagents (e.g., polystyrene-supported DCC) can be used. The amide formation is generally conducted in the presence of a base such as triethylamine and diisopropylethylamine. The preferred conditions involve the use of BOP chloride as a dehydrating reagent and triethylamine as a base in acetonitrile at temperatures in the range of 0 to 120 °C. This reaction is well known in the art, and a multitude of conditions and methods can be utilized. Benzotriazole carboxylic acids (compounds of Formula 10 where-

in M and M 1 are both N and the bond between them is an aromatic bond) are known in the art (see Japanese Kokai 11171872; *Can. J. Chem.* **1977**, *55*, 1653-1657; and *Arch. Pharm. (Weinheim, Ger.)*, **1989**, *322*, 457-459). Benzimidazole carboxylic acids (compounds of Formula 10 wherein M is N and $M^1$ is $CR^6$ and the bond between them is an aromatic bond) are also known in the art (see *Can. J. Chem.* **1977**, *55*, 1653-1657; *J. Med. Chem.* **1992**, *35*, 4595-601; *Bioorg. Med. Chem. Lett.* **1996**, *6*, 1195-1198; and *J.Med. Chem.* **1999**, *42*, 5020-5028). Indazole carboxylic acids (compounds of Formula 10 wherein M is $CR^6$ and $M^1$ is N and the bond between them is an aromatic bond) are also known in the art (see *J. Org. Chem.* **1980**, *45, 3072-7;* and *Heterocycles* **1997**, *45*, 1833-1838). Indole carboxylic acids (compounds of Formula 10 wherein M and $M^1$ are independently $CR^6$ and the bond between them is an aromatic bond) are also known in the art (see *J. Med. Chem.* **1996**, *39*, 4692-4703).

Scheme 9

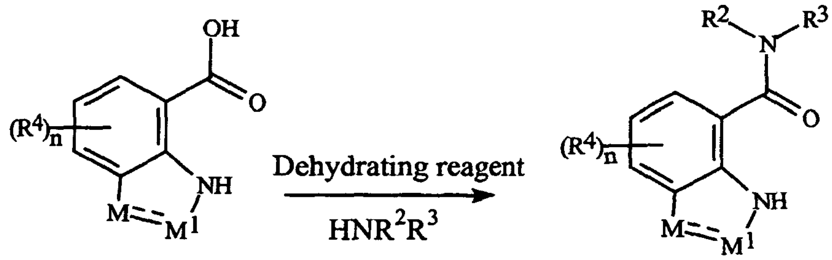


11

10

1

The carboxylic acids of Formula 10a can be prepared by the hydrolysis of thioesters of Formula 12 as shown in Scheme 10. Compounds of Formula 10a is a subset of compounds of Formula 10 where in M and $M^1$ are independently $CR^6R^7$ and the bond between them is a single bond. This hydrolysis can be carried out in $C_1$-$C_4$ alcohols (such as methanol and ethanol) with alkali (such as lithium, sodium and potassium) metal hydroxides. The preferred reaction conditions include the use of potassium or sodium hydroxide as a base and methanol or ethanol as a solvent at temperatures in the range of 20 to 120 °C. Compounds of Formula 12 are known in the art and can be prepared by the Sugasawa reaction (Sugasawa et. al. *Synthetic Communications*, **1990**, *20,* 71-84).

Scheme 10

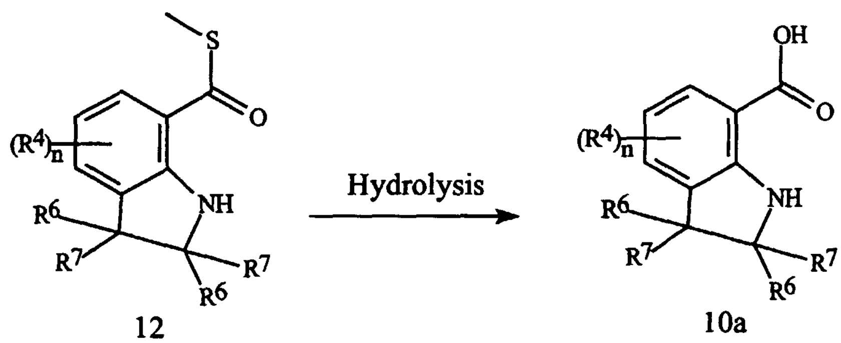


12

10a

Indolines of Formula Ia can be oxidized to indoles of Formula Ib as depicted in Scheme 11. Oxidation can be accomplished by using reagents such as 2,3-dichloro-5,6-dicyanobenzoquinone (DDQ), chloranil or manganese dioxide. Oxidations of indolines to indoles are generally well known in the art. For leading references see *Tetrahedron Lett.,* **2001**, *42(41)*, 7277-7280; and *Tetrahedron Lett.,* **2000,** *41(35),* 6721-6724.

Scheme 11

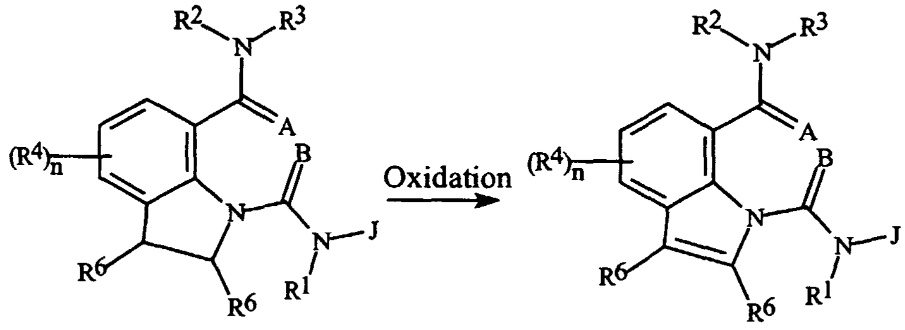


Ia

Ib

It is recognized that some reagents and reaction conditions described above for preparing compounds of Formula I and Formula II may not be compatible with certain functional groups present in the intermediates. In these instances, the incorporation of protection/deprotection sequences or functional group interconversions into the synthesis will aid in obtaining the desired products. The use and choice of the protecting groups will be apparent to one skilled in chemical synthesis (see, for example, Greene, T. W.; Wuts, P. G. M. *Protective Groups in Organic Synthesis,* 2nd ed.; Wiley: New York, 1991). One skilled in the art will recognize that, in some cases, after the introduction of a given reagent as it is depicted in any individual scheme, it may be necessary to perform additional routine synthetic steps not described in detail to complete the synthesis of compounds of Formula I and II. One skilled in the art will also recognize that it may be necessary to perform a combination of the steps illustrated in the above schemes in an order other than that implied by the particular sequence presented to prepare the compounds of Formula I and II.

One skilled in the art will also recognize that compounds of Formula I and II and the intermediates described herein can be subjected to various electrophilic, nucleophilic, radical, organometallic, oxidation, and reduction reactions to add substituents or modify existing substituents.

Without further elaboration, it is believed that one skilled in the art using the preceding description can utilize the present invention to its fullest extent. The following Examples are, therefore, to be construed as merely illustrative, and not limiting of the disclosure in any way whatsoever. $^1$H NMR spectra are reported in ppm downfield from tetramethylsilane: s is singlet, d is doublet, t is triplet, q is quartet, m is multiplet, dd is doublet of doublets, dt is doublet of triplets, br s is broad singlet. m. p. is melting point.

EXAMPLE 1

Preparation of 2,3-dihydro-*N*$^{7}$-methyl-*N*$^{1}$-[2-methyl-4-(trifluoromethyl)phenyl]-1*H*-indole-1,7-dicarboxamide

Step A: Preparation of 2,3-dihydro-1*H*-indole-7-carboxylic acid

To a solution of *S*-methyl 2,3-dihydro-1*H*-indole-7-carbothioate *(Synthetic Communications*, **1990**, *20*, 71-84, 26.88 g) in ethanol (500 mL) was added sodium hydroxide (50% aqueous solution, 50 mL) and the mixture was heated at reflux for 4 hours. Most of the ethanol was removed under reduced pressure. The residue was partitioned between water (150 mL) and dichloromethane (100 mL). The aqueous layer was acidified to pH 3 using concentrated hydrochloric acid, whereupon a solid precipitated. The solid was isolated by filtration and allowed to air dry for 2 hours. The dried solid was dissolved in 300 mL of ethyl acetate and dried over magnesium sulfate. The drying agent was removed by filtration and the filtrate was evaporated under reduced pressure to give the title compound (13.7 g) as a solid.

$^1$H NMR ($CDCl_3$) δ 3.08 (2H), 3.75 (2H), 6.59 (1H), 7.20 (1H), 7.61 (1H).

Step B: Preparation of 2,3-dihydro-*N*-methyl-1*H*-indole-7-carboxamide

To a solution of 2,3-dihydro-1*H*-indole-7-carboxylic acid (i.e. the product from step A) (1.0 g, 6.1 mmol) in acetonitrile (30 ml) was added sequentially methylamine (Aldrich, 2M in tetrahydrofuran, 6.13 mL), bis(2-oxo-3-oxazolidinyl)phosphinic chloride (i.e. BOP chloride, Aldrich, 1.56 g, 6.1 mmol), and triethylamine (1.71 ml, 12.3 mmol). The reaction mixture was allowed to stir at 25 °C for 48 hours. The mixture was partitioned between ethyl acetate (100 mL) and water (100 mL). The organic layer was washed with saturated aqueous sodium bicarbonate (50 mL), dried over magnesium sulfate, filtered and concentrated under reduced pressure to give the title compound.

$^1H$ NMR ($CDCl_3$) δ 2.95 (3H), 3.02 (2H), 3.69 (2H), 6.21 (1H), 6.3 1 (1H), 6.61 (1H), 7.1 (2H).

Step C: Preparation of 2,3-dihydro-*N*7-methyl-*N*1-[2-methyl-4-(trifluoromethyl)phenyl]-1*H*-indole-1,7-dicarboxamide

To a solution of 2,3-dihydro-*N*-methyl-1*H*-indole-7-carboxamide (i.e. the product from Step B) (300 mg) in dichloromethane was added 2-methyl-4-trifluoromethylphenyl isocyanate (0.4 g). After 3 hours the solvent was evaporated under reduced pressure. The residue was triturated with ether, filtered and washed with more ether to provide the title compound (200 mg), a compound of this invention, as a solid melting at 198 - 200 °C.

$^1H$ NMR ($CDCl_3$) δ 2.31 (3H), 2.6 (3H), 3.11 (2H), 4.12 (2H), 6.97 (1H), 7.55 (2H), 8.55 (1H).

EXAMPLE 2

Preparation of 2,3-dihydro-*N*1-(1-methylethyl)-*N*7-[2-methyl-4-[1,2,2,2-tetrafluoro-1-(trifluoromethyl)ethyl]phenyl]-1*H*-indole-1,7-dicarboxamide

Step A: Preparation of 1,1-dimethylethyl 2,3-dihydro-7-[[[2-methyl-4-[1,2,2,2-tetrafluoro-1-(trifluoromethyl)ethyl] phenyl]amino]carbonyl]-1*H*-indole-1-carboxylate

To a stirring solution of 1,1-dimethylethyl 2,3-dihydro-1*H*-indole-1-carboxylate (Aldrich, 1.0 g, 4.5 mmol), *N, N,N'N'*-tetramethylethylenediamine (0.90 mL, 690 mg, 6.0 mmol), and anhydrous $Et_2O$ (20 mL) at -78 °C, was added *sec*-butyllithium (4.2 mL, 5.4 mmol, 1.3 M in cyclohexane) dropwise over a 10 min period. The resulting solution was stirred at -78 °C for 1 hour followed by the dropwise addition of 2-methyl-4-heptafluoroisopropylphenyl isocyanate (1.4 g, 4.5 mmol) over a 4 min period. The solution was allowed to warm to room temperature over 1 hour followed by the addition of saturated aqueous $NH_4Cl$ (4 mL). The mixture was partitioned between $Et_2O$ and $H_2O$. The combined organic extract was dried ($MgSO_4$) and concentrated in vacuo. The residue was subjected to chromatography on silica gel with 30% EtOAc/hexanes as eluent to give the title compound of Step A (1.6 g, 3.1 mmol, 69% yield) as a white solid:

$^1H$ NMR ($CDCl_3$) δ 1.29 (s, 9H), 2.32 (s, 3H), 3.12 (t, 2H), 4.18 (t, 2H), 7.16 (t, 1H), 7.31-7.39 (m, 2H), 7.43 (d, 1H), 7.61 (d, 1H), 7.81 (bs, 1H), 8.59 (d, 1H).

Step B: Preparation of 2,3-dihydro-*N*-[2-methyl-4-[1,2,2,2-tetrafluoro-1-(trifluoromethyl)ethyl]phenyl]-1*H*-indole-7-carboxamide

A solution of 1,1-dimethylethyl 2,3-dihydro-7-[[[2-methyl-4-[1,2,2,2-tetrafluoro-1-(trifluoromethyl)ethyl]phenyl] amino]carbonyl]-1*H*-indole-1-carboxylate (i.e. the product from Step A) (1.1 g, 2.1 mmol), trifluoroacetic acid (15 mL), and $CH_2Cl_2$ (50 ml) was stirred at room temperature for 2 hours. The solution was carefully poured into a saturated aqueous solution of $NaHCO_3$ (500 mL). The mixture was partitioned between $CH_2Cl_2$ and $H_2O$. The combined organic extract was dried ($MgSO_4$) and concentrated in vacuo. The residue was subjected to chromatography on silica gel with 30% EtOAc/hexanes as eluent to give the title compound (800 mg, 1.9 mmol, 90% yield) as a white solid

$^1H$ NMR ($CDCl_3$) δ 2.40 (s, 3H), 3.08 (t, 2H), 3.73 (t, 2H), 6.37 (bs, 1H), 6.63(t, 1H), 7.18-7.28 (m, 3H), 7.47 (d, 1H), 7.80 (s, 1H), 8.22(d, 1H).

Step C: Preparation of 2,3-dihydro-*N*1-(1-methylethyl)-*N*7-[2-methyl-4-[1,2,2,2-tetrafluoro-1-(trifluoromethyl)ethyl] phenyl]-1*H*-indole-1,7-dicarboxamide

A solution of the product of Step B (200 mg, 0.48 mmol), isopropyl isocyanate (0.10 mL, 87 mg, 1.00 mmol), and $CH_2Cl_2$ (10 ml) was stirred at room temperature for 17 hours. The mixture was filtered and the residue was washed with a 1/1 solution of $Et_2O/CH_2Cl_2$ (3 mL) to give the title compound (1.6 g, 3.1 mmol, 69% yield), a compound of this invention, as a white solid.

$^1H$ NMR (DMSO-$d_6$) δ 0.98 (d, 6H), 2.37 (s, 3H), 3.21 (t, 2H), 3.62-3.71 (m, 1H), 4.01 (t, 1H), 6.73 (d, 1H), 7.00 (t, 1H), 7.32(d, 2H), 7.41-7.45 (m, 2H), 8.14 (d, 1H), 9.24 (s, 1H).

By the procedures described herein together with methods known in the art, the following compounds of Tables 1 to 14 can be prepared. The following abbreviations are used in the Tables: *t* is *tertiary, s* is secondary, *n* is normal, *i* is iso, *c* is cyclo, Me is methyl, Et is ethyl, Pr is propyl, *i*-Pr is isopropyl, *t*-Bu is tert butyl, Ph is phenyl, OMe is methoxy, OEt is ethoxy, SMe is methylthio, SEt is ethylthio, CN is cyano, $NO_2$ is nitro, TMS is trimethylsilyl, S(O)Me is methylsulfinyl, and $S(O)_2Me$ is methylsulfonyl.

Table 1

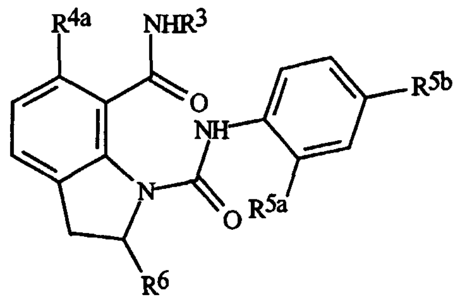

| $R^3$ | $R^{4a}$ | $R^6$ | $R^{5a}$ | $R^{5b}$ |
|---|---|---|---|---|
| Me | H | Me | H | $CF_3$ |
| Me | H | Me | H | $OCF_3$ |
| Me | H | Me | H | $OCHF_2$ |
| Me | H | Me | H | $C_2F_5$ |
| Me | H | Me | H | $OCF_2CHF_2$ |
| Me | H | Me | H | $SCF_2CHF_2$ |
| Me | H | Me | H | *n*-$C_3F_7$ |
| Me | H | Me | H | *i*-$C_3F_7$ |
| Me | H | Me | H | Br |
| Me | H | Me | H | Cl |
| Me | H | Me | H | $SCF_3$ |
| Me | H | Me | Me | $CF_3$ |
| Me | H | Me | Me | $OCF_3$ |
| Me | H | Me | Me | $OCHF_2$ |
| Me | H | Me | Me | $C_2F_5$ |
| Me | H | Me | Me | $OCF_2CHF_2$ |
| Me | H | Me | Me | $SCF_2CHF_2$ |
| Me | H | Me | Me | *n*-$C_3F_7$ |
| Me | H | Me | Me | *i*-$C_3F_7$ |
| Me | H | Me | Me | Br |

| $R^3$ | $R^{4a}$ | $R^6$ | $R^{5a}$ | $R^{5b}$ |
|---|---|---|---|---|
| Me | H | Me | Me | Cl |
| Me | H | Me | Me | $SCF_3$ |
| Me | H | Me | Me | I |
| Me | H | Me | Me | SMe |
| Me | H | Me | Me | OMe |
| Me | H | Me | Me | OEt |
| Me | H | Me | Me | Et |
| Me | H | Me | Me | $SO_2Me$ |
| Me | H | Me | Me | $SO_2CF_3$ |
| Me | H | Me | Et | $CF_3$ |
| Me | H | Me | Et | Br |
| Me | H | Me | Et | Cl |
| Me | H | Me | Ph | $CF_3$ |
| Me | H | Me | Ph | Br |
| Me | H | Me | Ph | Cl |
| Me | H | Me | 2-pyridyl | $CF_3$ |
| Me | H | Me | 2-pyridyl | Cl |
| Me | H | Me | 2-ClPh | $CF_3$ |
| Me | H | Me | 2-ClPh | $OCF_3$ |
| Me | H | Me | 2-ClPh | $SCHF_2$ |
| Me | H | Me | 2-ClPh | Br |
| Me | H | Me | 2-ClPh | Cl |
| Me | H | Me | 3-Cl-2-pyridyl | $CF_3$ |
| Me | H | Me | 3-Cl-2-pyridyl | $OCF_3$ |
| Me | H | Me | 3-Cl-2-pyridyl | $SCHF_2$ |
| Me | H | Me | 3-Cl-2-pyridyl | Br |
| Me | H | Me | 3-Cl-2-pyridyl | Cl |
| Me | H | Me | 3-Br-2-pyridyl | $CF_3$ |
| Me | H | Me | 3-Br-2-pyridyl | $OCF_3$ |
| Me | H | Me | 3-Br-2-pyridyl | Br |
| Me | H | Me | 3-Br-2-pyridyl | Cl |
| Me | H | Me | $CF_3$ | Me |
| Me | H | Me | F | $CF_3$ |
| Me | H | Me | Cl | $CF_3$ |
| Me | H | Me | *n*-Pr | $CF_3$ |
| Me | H | Me | *i*-Pr | $CF_3$ |
| Me | H | Me | $CF_3$ | $CF_3$ |

| $R^3$ | $R^{4a}$ | $R^6$ | $R^{5a}$ | $R^{5b}$ |
|---|---|---|---|---|
| Me | H | Me | OMe | $CF_3$ |
| Me | H | Me | 2-BrPh | $CF_3$ |
| Me | H | Me | 2-MePh | $CF_3$ |
| Me | H | Me | 2-CNPh | $CF_3$ |
| Me | H | Me | 2-FPh | $CF_3$ |
| Me | H | Me | 2,6-$F_2$Ph | $CF_3$ |
| Me | H | Me | 2,4-$F_2$Ph | $CF_3$ |
| Me | H | Me | 2,5-$F_2$Ph | $CF_3$ |
| Me | H | Me | 2-MeOPh | $CF_3$ |
| Me | H | Me | 3-F-2-pyridyl | $CF_3$ |
| Me | H | Me | 3-$CF_3$-2-pyridyl | $CF_3$ |
| Me | H | Me | 3-Me-2-pyridyl | $CF_3$ |
| Et | H | Me | H | $CF_3$ |
| Et | H | Me | H | $C_2F_5$ |
| Et | H | Me | H | $OCF_3$ |
| Et | H | Me | Me | $CF_3$ |
| Et | H | Me | Me | $C_2F_5$ |
| Et | H | Me | Me | $OCF_3$ |
| Me | Me | H | H | $CF_3$ |
| Me | Me | H | H | $OCF_3$ |
| Me | Me | H | H | $OCHF_2$ |
| Me | Me | H | H | $C_2F_5$ |
| Me | Me | H | H | $OCF_2CHF_2$ |
| Me | Me | H | H | $SCF_2CHF_2$ |
| Me | Me | H | H | *n*-$C_3F_7$ |
| Me | Me | H | H | *i*-$C_3F_7$ |
| Me | Me | H | H | Br |
| Me | Me | H | H | Cl |
| Me | Me | H | H | $SCF_3$ |
| Me | Me | H | H | $CF_3$ |
| Me | Me | H | H | $OCF_3$ |
| Me | Me | H | H | $OCHF_2$ |
| Me | Me | H | Me | $C_2F_5$ |
| Me | Me | H | Me | $OCF_2CHF_2$ |
| Me | Me | H | Me | $SCF_2CHF_2$ |
| Me | Me | H | Me | *n*-$C_3F_7$ |
| Me | Me | H | Me | *i*-$C_3F_7$ |

| $R^3$ | $R^{4a}$ | $R^6$ | $R^{5a}$ | $R^{5b}$ |
|---|---|---|---|---|
| Me | Me | H | Me | Br |
| Me | Me | H | Me | Cl |
| Me | Me | H | Me | $SCF_3$ |
| Me | Me | H | Me | I |
| Me | Me | H | Me | SMe |
| Me | Me | H | Me | OMe |
| Me | Me | H | Me | OEt |
| Me | Me | H | Me | Et |
| Me | Me | H | Me | $SO_2Me$ |
| Me | Me | H | Me | $SO_2CF_3$ |
| Me | Me | H | Et | $CF_3$ |
| Me | Me | H | Et | Br |
| Me | Me | H | Et | Cl |
| Me | Me | H | Ph | $CF_3$ |
| Me | Me | H | Ph | Br |
| Me | Me | H | Ph | Cl |
| Me | Me | H | 2-pyridyl | $CF_3$ |
| Me | Me | H | 2-pyridyl | Cl |
| Me | Me | H | 2-ClPh | $CF_3$ |
| Me | Me | H | 2-ClPh | $OCF_3$ |
| Me | Me | H | 2-ClPh | $SCHF_2$ |
| Me | Me | H | 2-ClPh | Br |
| Me | Me | H | 2-ClPh | Cl |
| Me | Me | H | 3-Cl-2-pyridyl | $CF_3$ |
| Me | Me | H | 3-Cl-2-pyridyl | $OCF_3$ |
| Me | Me | H | 3-Cl-2-pyridyl | $SCHF_2$ |
| Me | Me | H | 3-Cl-2-pyridyl | Br |
| Me | Me | H | 3-Cl-2-pyridyl | Cl |
| Me | Me | H | 3-Br-2-pyridyl | $CF_3$ |
| Me | Me | H | 3-Br-2-pyridyl | $OCF_3$ |
| Me | Me | H | 3-Br-2-pyridyl | Br |
| Me | Me | H | 3-Br-2-pyridyl | Cl |
| Me | Me | H | $CF_3$ | Me |
| Me | Me | H | F | $CF_3$ |
| Me | Me | H | Cl | $CF_3$ |
| Me | Me | H | *n*-Pr | $CF_3$ |
| Me | Me | H | *i*-Pr | $CF_3$ |

| $R^3$ | $R^{4a}$ | $R^6$ | $R^{5a}$ | $R^{5b}$ |
|---|---|---|---|---|
| Me | Me | H | $CF_3$ | $CF_3$ |
| Me | Me | H | OMe | $CF_3$ |
| Me | Me | H | 2-BrPh | $CF_3$ |
| Me | Me | H | 2-MePh | $CF_3$ |
| Me | Me | H | 2-CNPh | $CF_3$ |
| Me | Me | H | 2-FPh | $CF_3$ |
| Me | Me | H | 2,6-$F_2$Ph | $CF_3$ |
| Me | Me | H | 2,4-$F_2$Ph | $CF_3$ |
| Me | Me | H | 2,5-$F_2$Ph | $CF_3$ |
| Me | Me | H | 2-MeOPh | $CF_3$ |
| Me | Me | H | 3-F-2-pyridyl | $CF_3$ |
| Me | Me | H | 3-$CF_3$-2-pyridyl | $CF_3$ |
| Me | Me | H | 3-Me-2-pyridyl | $CF_3$ |
| Et | Me | H | H | $CF_3$ |
| Et | Me | H | H | $C_2F_5$ |
| Et | Me | H | H | $OCF_3$ |
| Et | Me | H | Me | $CF_3$ |
| Et | Me | H | Me | $C_2F_5$ |
| Et | Me | H | Me | $OCF_3$ |
| Me | Cl | H | H | $CF_3$ |
| Me | Cl | H | H | $OCF_3$ |
| Me | Cl | H | H | $OCHF_2$ |
| Me | Cl | H | H | $C_2F_5$ |
| Me | Cl | H | H | $OCF_2CHF_2$ |
| Me | Cl | H | H | $SCF_2CHF_2$ |
| Me | Cl | H | H | *n*-$C_3F_7$ |
| Me | Cl | H | H | *i*-$C_3F_7$ |
| Me | Cl | H | H | Br |
| Me | Cl | H | H | Cl |
| Me | Cl | H | H | $SCF_3$ |
| Me | Cl | H | Me | $CF_3$ |
| Me | Cl | H | Me | $OCF_3$ |
| Me | Cl | H | Me | $OCHF_2$ |
| Me | Cl | H | Me | $C_2F_5$ |
| Me | Cl | H | Me | $OCF_2CHF_2$ |
| Me | Cl | H | Me | $SCF_2CHF_2$ |
| Me | Cl | H | Me | *n*-$C_3F_7$ |

| $R^3$ | $R^{4a}$ | $R^6$ | $R^{5a}$ | $R^{5b}$ |
|---|---|---|---|---|
| Me | Cl | H | Me | *i*-$C_3F_7$ |
| Me | Cl | H | Me | Br |
| Me | Cl | H | Me | Cl |
| Me | Cl | H | Me | $SCF_3$ |
| Me | Cl | H | Me | I |
| Me | Cl | H | Me | SMe |
| Me | Cl | H | Me | OMe |
| Me | Cl | H | Me | OEt |
| Me | Cl | H | Me | Et |
| Me | Cl | H | Me | $SO_2Me$ |
| Me | Cl | H | Me | $SO_2CF_3$ |
| Me | Cl | H | Et | $CF_3$ |
| Me | Cl | H | Et | Br |
| Me | Cl | H | Et | Cl |
| Me | Cl | H | Ph | $CF_3$ |
| Me | Cl | H | Ph | Br |
| Me | Cl | H | Ph | Cl |
| Me | Cl | H | 2-pyridyl | $CF_3$ |
| Me | Cl | H | 2-pyridyl | Cl |
| $CH(Me)CH_2SMe$ | H | Me | H | $CF_3$ |
| $CH(Me)CH_2SMe$ | H | Me | H | $OCF_3$ |
| $CH(Me)CH_2SMe$ | H | Me | H | $OCHF_2$ |
| $CH(Me)CH_2SMe$ | H | Me | H | $C_2F_5$ |
| $CH(Me)CH_2SMe$ | H | Me | H | $OCF_2CHF_2$ |
| $CH(Me)CH_2SMe$ | H | Me | H | $SCF_2CHF_2$ |
| $CH(Me)CH_2SMe$ | H | Me | H | *n*-$C_3F_7$ |
| $CH(Me)CH_2SMe$ | H | Me | H | *i*-$C_3F_7$ |
| $CH(Me)CH_2SMe$ | H | Me | H | Br |
| $CH(Me)CH_2SMe$ | H | Me | H | Cl |
| $CH(Me)CH_2SMe$ | H | Me | H | $SCF_3$ |
| $CH(Me)CH_2SMe$ | H | Me | Me | $CF_3$ |
| $CH(Me)CH_2SMe$ | H | Me | Me | $OCF_3$ |
| $CH(Me)CH_2SMe$ | H | Me | Me | $OCHF_2$ |
| $CH(Me)CH_2SMe$ | H | Me | Me | $C_2F_5$ |
| $CH(Me)CH_2SMe$ | H | Me | Me | $OCF_2CHF_2$ |
| $CH(Me)CH_2SMe$ | H | Me | Me | $SCF_2CHF_2$ |
| $CH(Me)CH_2SMe$ | H | Me | Me | *n*-$C_3F_7$ |

| $R^3$ | $R^{4a}$ | $R^6$ | $R^{5a}$ | $R^{5b}$ |
|---|---|---|---|---|
| $CH(Me)CH_2SMe$ | H | Me | Me | *i*-$C_3F_7$ |
| $CH(Me)CH_2SMe$ | H | Me | Me | Br |
| $CH(Me)CH_2SMe$ | H | Me | Me | Cl |
| $CH(Me)CH_2SMe$ | H | Me | Me | $SCF_3$ |
| $CH(Me)CH_2SMe$ | H | Me | Me | I |
| $CH(Me)CH_2SMe$ | H | Me | Me | SMe |
| $CH(Me)CH_2SMe$ | H | Me | Me | OMe |
| $CH(Me)CH_2SMe$ | H | Me | Me | OEt |
| $CH(Me)CH_2SMe$ | H | Me | Me | Et |
| $CH(Me)CH_2SMe$ | H | Me | Me | $SO_2Me$ |
| $CH(Me)CH_2SMe$ | H | Me | Me | $SO_2CF_3$ |
| $CH(Me)CH_2SMe$ | H | Me | Et | $CF_3$ |
| $CH(Me)CH_2SMe$ | H | Me | Et | Br |
| $CH(Me)CH_2SMe$ | H | Me | Et | Cl |
| $CH(Me)CH_2SMe$ | H | Me | Ph | $CF_3$ |
| $CH(Me)CH_2SMe$ | H | Me | Ph | Br |
| $CH(Me)CH_2SMe$ | H | Me | Ph | Cl |
| $CH(Me)CH_2SMe$ | H | Me | 2-pyridyl | $CF_3$ |
| $CH(Me)CH_2SMe$ | H | Me | 2-pyridyl | Cl |
| $CH(Me)CH_2SMe$ | H | Me | 2-ClPh | $CF_3$ |
| $CH(Me)CH_2SMe$ | H | Me | 2-ClPh | $OCF_3$ |
| $CH(Me)CH_2SMe$ | H | Me | 2-ClPh | $SCHF_2$ |
| $CH(Me)CH_2SMe$ | H | Me | 2-ClPh | Br |
| $CH(Me)CH_2SMe$ | H | Me | 2-ClPh | Cl |
| $CH(Me)CH_2SMe$ | H | Me | 3-Cl-2-pyridyl | $CF_3$ |
| $CH(Me)CH_2SMe$ | H | Me | 3-Cl-2-pyridyl | $OCF_3$ |
| $CH(Me)CH_2SMe$ | H | Me | 3-Cl-2-pyridyl | $SCHF_2$ |
| $CH(Me)CH_2SMe$ | H | Me | 3-Cl-2-pyridyl | Br |
| $CH(Me)CH_2SMe$ | H | Me | 3-Cl-2-pyridyl | Cl |
| $CH(Me)CH_2SMe$ | H | Me | 3-Br-2-pyridyl | $CF_3$ |
| $CH(Me)CH_2SMe$ | H | Me | 3-Br-2-pyridyl | $OCF_3$ |
| $CH(Me)CH_2SMe$ | H | Me | 3-Br-2-pyridyl | Br |
| $CH(Me)CH_2SMe$ | H | Me | 3-Br-2-pyridyl | Cl |
| $CH(Me)CH_2SMe$ | H | Me | $CF_3$ | Me |
| $CH(Me)CH_2SMe$ | H | Me | F | $CF_3$ |
| $CH(Me)CH_2SMe$ | H | Me | Cl | $CF_3$ |
| $CH(Me)CH_2SMe$ | H | Me | *n*-Pr | $CF_3$ |

| $R^3$ | $R^{4a}$ | $R^6$ | $R^{5a}$ | $R^{5b}$ |
|---|---|---|---|---|
| $CH(Me)CH_2SMe$ | H | Me | *i*-Pr | $CF_3$ |
| $CH(Me)CH_2SMe$ | H | Me | $CF_3$ | $CF_3$ |
| $CH(Me)CH_2SMe$ | H | Me | OMe | $CF_3$ |
| $CH(Me)CH_2SMe$ | H | Me | 2-BrPh | $CF_3$ |
| $CH(Me)CH_2SMe$ | H | Me | 2-MePh | $CF_3$ |
| $CH(Me)CH_2SMe$ | H | Me | 2-CNPh | $CF_3$ |
| $CH(Me)CH_2SMe$ | H | Me | 2-FPh | $CF_3$ |
| $CH(Me)CH_2SMe$ | H | Me | 2,6-$F_2$Ph | $CF_3$ |
| $CH(Me)CH_2SMe$ | H | Me | 2,4-$F_2$Ph | $CF_3$ |
| $CH(Me)CH_2SMe$ | H | Me | 2,5-$F_2$Ph | $CF_3$ |
| $CH(Me)CH_2SMe$ | H | Me | 2-MeOPh | $CF_3$ |
| $CH(Me)CH_2SMe$ | H | Me | 3-F-2-pyridyl | $CF_3$ |
| $CH(Me)CH_2SMe$ | H | Me | 3-$CF_3$-2-pyridyl | $CF_3$ |
| $CH(Me)CH_2SMe$ | H | Me | 3-Me-2-pyridyl | $CF_3$ |
| $CH(Me)CH_2SMe$ | Me | H | H | $CF_3$ |
| $CH(Me)CH_2SMe$ | Me | H | H | $OCF_3$ |
| $CH(Me)CH_2SMe$ | Me | H | H | $OCHF_2$ |
| $CH(Me)CH_2SMe$ | Me | H | H | $C_2F_5$ |
| $CH(Me)CH_2SMe$ | Me | H | H | $OCF_2CHF_2$ |
| $CH(Me)CH_2SMe$ | Me | H | H | $SCF_2CHF_2$ |
| $CH(Me)CH_2SMe$ | Me | H | H | *n*-$C_3F_7$ |
| $CH(Me)CH_2SMe$ | Me | H | H | *i*-$C_3F_7$ |
| $CH(Me)CH_2SMe$ | Me | H | H | Br |
| $CH(Me)CH_2SMe$ | Me | H | H | Cl |
| $CH(Me)CH_2SMe$ | Me | H | H | $SCF_3$ |
| $CH(Me)CH_2SMe$ | Me | H | H | $CF_3$ |
| $CH(Me)CH_2SMe$ | Me | H | H | $OCF_3$ |
| $CH(Me)CH_2SMe$ | Me | H | H | $OCHF_2$ |
| $CH(Me)CH_2SMe$ | Me | H | Me | $C_2F_5$ |
| $CH(Me)CH_2SMe$ | Me | H | Me | $OCF_2CHF_2$ |
| $CH(Me)CH_2SMe$ | Me | H | Me | $SCF_2CHF_2$ |
| $CH(Me)CH_2SMe$ | Me | H | Me | *n*-$C_3F_7$ |
| $CH(Me)CH_2SMe$ | Me | H | Me | *i*-$C_3F_7$ |
| $CH(Me)CH_2SMe$ | Me | H | Me | Br |
| $CH(Me)CH_2SMe$ | Me | H | Me | Cl |
| $CH(Me)CH_2SMe$ | Me | H | Me | $SCF_3$ |
| $CH(Me)CH_2SMe$ | Me | H | Me | I |

| $R^3$ | $R^{4a}$ | $R^6$ | $R^{5a}$ | $R^{5b}$ |
|---|---|---|---|---|
| $CH(Me)CH_2SMe$ | Me | H | Me | SMe |
| $CH(Me)CH_2SMe$ | Me | H | Me | OMe |
| $CH(Me)CH_2SMe$ | Me | H | Me | OEt |
| $CH(Me)CH_2SMe$ | Me | H | Me | Et |
| $CH(Me)CH_2SMe$ | Me | H | Me | $SO_2Me$ |
| $CH(Me)CH_2SMe$ | Me | H | Me | $SO_2CF_3$ |
| $CH(Me)CH_2SMe$ | Me | H | Et | $CF_3$ |
| $CH(Me)CH_2SMe$ | Me | H | Et | Br |
| $CH(Me)CH_2SMe$ | Me | H | Et | Cl |
| $CH(Me)CH_2SMe$ | Me | H | Ph | $CF_3$ |
| $CH(Me)CH_2SMe$ | Me | H | Ph | Br |
| $CH(Me)CH_2SMe$ | Me | H | Ph | Cl |
| $CH(Me)CH_2SMe$ | Me | H | 2-pyridyl | $CF_3$ |
| $CH(Me)CH_2SMe$ | Me | H | 2-pyridyl | Cl |
| $CH(Me)CH_2SMe$ | Me | H | 2-ClPh | $CF_3$ |
| $CH(Me)CH_2SMe$ | Me | H | 2-ClPh | $OCF_3$ |
| $CH(Me)CH_2SMe$ | Me | H | 2-ClPh | $SCHF_2$ |
| $CH(Me)CH_2SMe$ | Me | H | 2-ClPh | Br |
| $CH(Me)CH_2SMe$ | Me | H | 2-ClPh | Cl |
| $CH(Me)CH_2SMe$ | Me | H | 3-Cl-2-pyridyl | $CF_3$ |
| $CH(Me)CH_2SMe$ | Me | H | 3-Cl-2-pyridyl | $OCF_3$ |
| $CH(Me)CH_2SMe$ | Me | H | 3-Cl-2-pyridyl | $SCHF_2$ |
| $CH(Me)CH_2SMe$ | Me | H | 3-Cl-2-pyridyl | Br |
| $CH(Me)CH_2SMe$ | Me | H | 3-Cl-2-pyridyl | Cl |
| $CH(Me)CH_2SMe$ | Me | H | 3-Br-2-pyridyl | $CF_3$ |
| $CH(Me)CH_2SMe$ | Me | H | 3-Br-2-pyridyl | $OCF_3$ |
| $CH(Me)CH_2SMe$ | Me | H | 3-Br-2-pyridyl | Br |
| $CH(Me)CH_2SMe$ | Me | H | 3-Br-2-pyridyl | Cl |
| $CH(Me)CH_2SMe$ | Me | H | $CF_3$ | Me |
| $CH(Me)CH_2SMe$ | Me | H | F | $CF_3$ |
| $CH(Me)CH_2SMe$ | Me | H | Cl | $CF_3$ |
| $CH(Me)CH_2SMe$ | Me | H | *n*-Pr | $CF_3$ |
| $CH(Me)CH_2SMe$ | Me | H | *i*-Pr | $CF_3$ |
| $CH(Me)CH_2SMe$ | Me | H | $CF_3$ | $CF_3$ |
| $CH(Me)CH_2SMe$ | Me | H | OMe | $CF_3$ |
| $CH(Me)CH_2SMe$ | Me | H | 2-BrPh | $CF_3$ |
| $CH(Me)CH_2SMe$ | Me | H | 2-MePh | $CF_3$ |

| $R^3$ | $R^{4a}$ | $R^6$ | $R^{5a}$ | $R^{5b}$ |
|---|---|---|---|---|
| $CH(Me)CH_2SMe$ | Me | H | 2-CNPh | $CF_3$ |
| $CH(Me)CH_2SMe$ | Me | H | 2-FPh | $CF_3$ |
| $CH(Me)CH_2SMe$ | Me | H | 2,6-$F_2$Ph | $CF_3$ |
| $CH(Me)CH_2SMe$ | Me | H | 2,4-$F_2$Ph | $CF_3$ |
| $CH(Me)CH_2SMe$ | Me | H | 2,5-$F_2$Ph | $CF_3$ |
| $CH(Me)CH_2SMe$ | Me | H | 2-MeOPh | $CF_3$ |
| $CH(Me)CH_2SMe$ | Me | H | 3-F-2-pyridyl | $CF_3$ |
| $CH(Me)CH_2SMe$ | Me | H | 3-$CF_3$-2-pyridyl | $CF_3$ |
| $CH(Me)CH_2SMe$ | Me | H | 3-Me-2-pyridyl | $CF_3$ |
| Me | H | H | H | $CF_3$ |
| Me | H | H | H | $OCF_3$ |
| Me | H | H | H | $OCHF_2$ |
| Me | H | H | H | $C_2F_5$ |
| Me | H | H | H | $OCF_2CHF_2$ |
| Me | H | H | H | $SCF_2CHF_2$ |
| Me | H | H | H | *n*-$C_3F_7$ |
| Me | H | H | H | *i*-$C_3F_7$ |
| Me | H | H | H | Br |
| Me | H | H | H | Cl |
| Me | H | H | H | $SCF_3$ |
| Me | H | H | Me | $CF_3$ |
| Me | H | H | Me | $OCF_3$ |
| Me | H | H | Me | $OCHF_2$ |
| Me | H | H | Me | $C_2F_5$ |
| Me | H | H | Me | $OCF_2CHF_2$ |
| Me | H | H | Me | $SCF_2CHF_2$ |
| Me | H | H | Me | *n*-$C_3F_7$ |
| Me | H | H | Me | *i*-$C_3F_7$ |
| Me | H | H | Me | Br |
| Me | H | H | Me | Cl |
| Me | H | H | Me | $SCF_3$ |
| Me | H | H | Me | I |
| Me | H | H | Me | SMe |
| Me | H | H | Me | OMe |
| Me | H | H | Me | OEt |
| Me | H | H | Me | Et |
| Me | H | H | Me | $SO_2Me$ |

| $R^3$ | $R^{4a}$ | $R^6$ | $R^{5a}$ | $R^{5b}$ |
|---|---|---|---|---|
| Me | H | H | Me | $SO_2CF_3$ |
| Me | H | H | Et | $CF_3$ |
| Me | H | H | Et | Br |
| Me | H | H | Et | Cl |
| Me | H | H | Ph | $CF_3$ |
| Me | H | H | Ph | Br |
| Me | H | H | Ph | Cl |
| Me | H | H | 2-pyridyl | $CF_3$ |
| Me | H | H | 2-pyridyl | Cl |
| Me | H | H | 2-ClPh | $CF_3$ |
| Me | H | H | 2-ClPh | $OCF_3$ |
| Me | H | H | 2-ClPh | $SCHF_2$ |
| Me | H | H | 2-ClPh | Br |
| Me | H | H | 2-ClPh | Cl |
| Me | H | H | 3-Cl-2-pyridyl | $CF_3$ |
| Me | H | H | 3-Cl-2-pyridyl | $OCF_3$ |
| Me | H | H | 3-Cl-2-pyridyl | $SCHF_2$ |
| Me | H | H | 3-Cl-2-pyridyl | Br |
| Me | H | H | 3-Cl-2-pyridyl | Cl |
| Me | H | H | 3-Br-2-pyridyl | $CF_3$ |
| Me | H | H | 3-Br-2-pyridyl | $OCF_3$ |
| Me | H | H | 3-Br-2-pyridyl | Br |
| Me | H | H | 3-Br-2-pyridyl | Cl |
| Me | H | H | $CF_3$ | Me |
| Me | H | H | F | $CF_3$ |
| Me | H | H | Cl | $CF_3$ |
| Me | H | H | *n*-Pr | $CF_3$ |
| Me | H | H | *i*-Pr | $CF_3$ |
| Me | H | H | $CF_3$ | $CF_3$ |
| Me | H | H | OMe | $CF_3$ |
| Me | H | H | 2-BrPh | $CF_3$ |
| Me | H | H | 2-MePh | $CF_3$ |
| Me | H | H | 2-CNPh | $CF_3$ |
| Me | H | H | 2-FPh | $CF_3$ |
| Me | H | H | 2,6-$F_2$Ph | $CF_3$ |
| Me | H | H | 2,4-$F_2$Ph | $CF_3$ |
| Me | H | H | 2,5-$F_2$Ph | $CF_3$ |

| $R^3$ | $R^{4a}$ | $R^6$ | $R^{5a}$ | $R^{5b}$ |
|---|---|---|---|---|
| Me | H | H | 2-MeOPh | $CF_3$ |
| Me | H | H | 3-F-2-pyridyl | $CF_3$ |
| Me | H | H | 3-$CF_3$-2-pyridyl | $CF_3$ |
| Me | H | H | 3-Me-2-pyridyl | $CF_3$ |
| Et | H | H | H | $CF_3$ |
| Et | H | H | H | $C_2F_5$ |
| Et | H | H | H | $OCF_3$ |
| Et | H | H | Me | $CF_3$ |
| Et | H | H | Me | $C_2F_5$ |
| Et | H | H | Me | $OCF_3$ |
| *i*-Pr | H | H | H | $CF_3$ |
| *i*-Pr | H | H | H | $OCF_3$ |
| *i*-Pr | H | H | H | $OCHF_2$ |
| *i*-Pr | H | H | H | $C_2F_5$ |
| *i*-Pr | H | H | H | $OCF_2CHF_2$ |
| *i*-Pr | H | H | H | $SCF_2CHF_2$ |
| *i*-Pr | H | H | H | *n*-$C_3F_7$ |
| *i*-Pr | H | H | H | *i*-$C_3F_7$ |
| *i*-Pr | H | H | H | Br |
| *i*-Pr | H | H | H | Cl |
| *i*-Pr | H | H | H | $SCF_3$ |
| *i*-Pr | H | H | Me | $CF_3$ |
| *i*-Pr | H | H | Me | $OCF_3$ |
| *i*-Pr | H | H | Me | $OCHF_2$ |
| *i*-Pr | H | H | Me | $C_2F_5$ |
| *i*-Pr | H | H | Me | $OCF_2CHF_2$ |
| *i*-Pr | H | H | Me | $SCF_2CHF_2$ |
| *i*-Pr | H | H | Me | *n*-$C_3F_7$ |
| *i*-Pr | H | H | Me | *i*-$C_3F_7$ |
| *i*-Pr | H | H | Me | Br |
| *i*-Pr | H | H | Me | Cl |
| *i*-Pr | H | H | Me | $SCF_3$ |
| *i*-Pr | H | H | Me | I |
| *i*-Pr | H | H | Me | SMe |
| *i*-Pr | H | H | Me | OMe |
| *i*-Pr | H | H | Me | OEt |
| *i*-Pr | H | H | Me | Et |

| $R^3$ | $R^{4a}$ | $R^6$ | $R^{5a}$ | $R^{5b}$ |
|---|---|---|---|---|
| *i*-Pr | H | H | Me | $SO_2Me$ |
| *i*-Pr | H | H | Me | $SO_2CF_3$ |
| *i*-Pr | H | H | Et | $CF_3$ |
| *i*-Pr | H | H | Et | Br |
| *i*-Pr | H | H | Et | Cl |
| *i*-Pr | H | H | Ph | $CF_3$ |
| *i*-Pr | H | H | Ph | Br |
| *i*-Pr | H | H | Ph | Cl |
| *i*-Pr | H | H | 2-pyridyl | $CF_3$ |
| *i*-Pr | H | H | 2-pyridyl | Cl |
| *i*-Pr | H | H | 2-ClPh | $CF_3$ |
| *i*-Pr | H | H | 2-ClPh | $OCF_3$ |
| *i*-Pr | H | H | 2-ClPh | $SCHF_2$ |
| *i*-Pr | H | H | 2-ClPh | Br |
| *i*-Pr | H | H | 2-ClPh | Cl |
| *i*-Pr | H | H | 3-Cl-2-pyridyl | $CF_3$ |
| *i*-Pr | H | H | 3-Cl-2-pyridyl | $OCF_3$ |
| *i*-Pr | H | H | 3-Cl-2-pyridyl | $SCHF_2$ |
| *i*-Pr | H | H | 3-Cl-2-pyridyl | Br |
| *i*-Pr | H | H | 3-Cl-2-pyridyl | Cl |
| *i*-Pr | H | H | 3-Br-2-pyridyl | $CF_3$ |
| *i*-Pr | H | H | 3-Br-2-pyridyl | $OCF_3$ |
| *i*-Pr | H | H | 3-Br-2-pyridyl | Br |
| *i*-Pr | H | H | 3-Br-2-pyridyl | Cl |
| *i*-Pr | H | H | $CF_3$ | Me |
| *i*-Pr | H | H | F | $CF_3$ |
| *i*-Pr | H | H | Cl | $CF_3$ |
| *i*-Pr | H | H | *n*-Pr | $CF_3$ |
| *i*-Pr | H | H | *i*-Pr | $CF_3$ |
| *i*-Pr | H | H | $CF_3$ | $CF_3$ |
| *i*-Pr | H | H | OMe | $CF_3$ |
| *i*-Pr | H | H | 2-BrPh | $CF_3$ |
| *i*-Pr | H | H | 2-MePh | $CF_3$ |
| *i*-Pr | H | H | 2-CNPh | $CF_3$ |
| *i*-Pr | H | H | 2-FPh | $CF_3$ |
| *i*-Pr | H | H | 2,6-$F_2$Ph | $CF_3$ |
| *i*-Pr | H | H | 2,4-$F_2$Ph | $CF_3$ |

| $R^3$ | $R^{4a}$ | $R^6$ | $R^{5a}$ | $R^{5b}$ |
|---|---|---|---|---|
| *i*-Pr | H | H | 2,5-$F_2$Ph | $CF_3$ |
| *i*-Pr | H | H | 2-MeOPh | $CF_3$ |
| *i*-Pr | H | H | 3-F-2-pyridyl | $CF_3$ |
| *i*-Pr | H | H | 3-$CF_3$-2-pyridyl | $CF_3$ |
| *i*-Pr | H | H | 3-Me-2-pyridyl | $CF_3$ |
| *i*-Pr | Me | H | H | $CF_3$ |
| *i*-Pr | Me | H | H | $C_2F_5$ |
| *i*-Pr | Me | H | H | $OCF_3$ |
| *i*-Pr | Me | H | Me | $CF_3$ |
| *i*-Pr | Me | H | Me | $C_2F_5$ |
| *i*-Pr | Me | H | Me | $OCF_3$ |
| Me | Cl | H | 2-ClPh | $CF_3$ |
| Me | Cl | H | 2-ClPh | $OCF_3$ |
| Me | Cl | H | 2-ClPh | $SCHF_2$ |
| Me | Cl | H | 2-ClPh | Br |
| Me | Cl | H | 2-ClPh | Cl |
| Me | Cl | H | 3-Cl-2-pyridyl | $CF_3$ |
| Me | Cl | H | 3-Cl-2-pyridyl | $OCF_3$ |
| Me | Cl | H | 3-Cl-2-pyridyl | $SCHF_2$ |
| Me | Cl | H | 3-Cl-2-pyridyl | Br |
| Me | Cl | H | 3-Cl-2-pyridyl | Cl |
| Me | Cl | H | 3-Br-2-pyridyl | $CF_3$ |
| Me | Cl | H | 3-Br-2-pyridyl | $OCF_3$ |
| Me | Cl | H | 3-Br-2-pyridyl | Br |
| Me | Cl | H | 3-Br-2-pyridyl | Cl |
| Me | Cl | H | $CF_3$ | Me |
| Me | Cl | H | F | $CF_3$ |
| Me | Cl | H | Cl | $CF_3$ |
| Me | Cl | H | *n*-Pr | $CF_3$ |
| Me | Cl | H | *i*-Pr | $CF_3$ |
| Me | Cl | H | $CF_3$ | $CF_3$ |
| Me | Cl | H | OMe | $CF_3$ |
| Me | Cl | H | 2-BrPh | $CF_3$ |
| Me | Cl | H | 2-MePh | $CF_3$ |
| Me | Cl | H | 2-CNPh | $CF_3$ |
| Me | Cl | H | 2-FPh | $CF_3$ |
| Me | Cl | H | 2,6-$F_2$Ph | $CF_3$ |

| $R^3$ | $R^{4a}$ | $R^6$ | $R^{5a}$ | $R^{5b}$ |
|---|---|---|---|---|
| Me | Cl | H | 2,4-$F_2$Ph | $CF_3$ |
| Me | Cl | H | 2,5-$F_2$Ph | $CF_3$ |
| Me | Cl | H | 2-MeOPh | $CF_3$ |
| Me | Cl | H | 3-F-2-pyridyl | $CF_3$ |
| Me | Cl | H | 3-$CF_3$-2-pyridyl | $CF_3$ |
| Me | Cl | H | 3-Me-2-pyridyl | $CF_3$ |
| Et | Cl | H | H | $OCF_3$ |
| Et | Cl | H | H | $C_2F_5$ |
| Et | Cl | H | Me | $CF_3$ |
| Et | Cl | H | Me | $C_2F_5$ |
| *i*-Pr | Cl | H | Me | $CF_3$ |
| CH(Me)$CH_2$SMe | H | H | H | $CF_3$ |
| CH(Me)$CH_2$SMe | H | H | H | $OCF_3$ |
| CH(Me)$CH_2$SMe | H | H | H | $OCHF_2$ |
| CH(Me)$CH_2$SMe | H | H | H | $C_2F_5$ |
| CH(Me)$CH_2$SMe | H | H | H | $OCF_2CHF_2$ |
| CH(Me)$CH_2$SMe | H | H | H | $SCF_2CHF_2$ |
| CH(Me)$CH_2$SMe | H | H | H | *n*-$C_3F_7$ |
| CH(Me)$CH_2$SMe | H | H | H | *i*-$C_3F_7$ |
| CH(Me)$CH_2$SMe | H | H | H | Br |
| CH(Me)$CH_2$SMe | H | H | H | Cl |
| CH(Me)$CH_2$SMe | H | H | H | $SCF_3$ |
| CH(Me)$CH_2$SMe | H | H | Me | $CF_3$ |
| CH(Me)$CH_2$SMe | H | H | Me | $OCF_3$ |
| CH(Me)$CH_2$SMe | H | H | Me | $OCHF_2$ |
| CH(Me)$CH_2$SMe | H | H | Me | $C_2F_5$ |
| CH(Me)$CH_2$SMe | H | H | Me | $OCF_2CHF_2$ |
| CH(Me)$CH_2$SMe | H | H | Me | $SCF_2CHF_2$ |
| CH(Me)$CH_2$SMe | H | H | Me | *n*-$C_3F_7$ |
| CH(Me)$CH_2$SMe | H | H | Me | *i*-$C_3F_7$ |
| CH(Me)$CH_2$SMe | H | H | Me | Br |
| CH(Me)$CH_2$SMe | H | H | Me | Cl |
| CH(Me)$CH_2$SMe | H | H | Me | $SCF_3$ |
| CH(Me)$CH_2$SMe | H | H | Me | I |
| CH(Me)$CH_2$SMe | H | H | Me | SMe |
| CH(Me)$CH_2$SMe | H | H | Me | OMe |
| CH(Me)$CH_2$SMe | H | H | Me | OEt |

| $R^3$ | $R^{4a}$ | $R^6$ | $R^{5a}$ | $R^{5b}$ |
|---|---|---|---|---|
| $CH(Me)CH_2SMe$ | H | H | Me | Et |
| $CH(Me)CH_2SMe$ | H | H | Me | $SO_2Me$ |
| $CH(Me)CH_2SMe$ | H | H | Me | $SO_2CF_3$ |
| $CH(Me)CH_2SMe$ | H | H | Et | $CF_3$ |
| $CH(Me)CH_2SMe$ | H | H | Et | Br |
| $CH(Me)CH_2SMe$ | H | H | Et | Cl |
| $CH(Me)CH_2SMe$ | H | H | Ph | $CF_3$ |
| $CH(Me)CH_2SMe$ | H | H | Ph | Br |
| $CH(Me)CH_2SMe$ | H | H | Ph | Cl |
| $CH(Me)CH_2SMe$ | H | H | 2-pyridyl | $CF_3$ |
| $CH(Me)CH_2SMe$ | H | H | 2-pyridyl | Cl |
| $CH(Me)CH_2SMe$ | H | H | 2-ClPh | $CF_3$ |
| $CH(Me)CH_2SMe$ | H | H | 2-ClPh | $OCF_3$ |
| $CH(Me)CH_2SMe$ | H | H | 2-ClPh | $SCHF_2$ |
| $CH(Me)CH_2SMe$ | H | H | 2-ClPh | Br |
| $CH(Me)CH_2SMe$ | H | H | 2-ClPh | Cl |
| $CH(Me)CH_2SMe$ | H | H | 3-Cl-2-pyridyl | $CF_3$ |
| $CH(Me)CH_2SMe$ | H | H | 3-Cl-2-pyridyl | $OCF_3$ |
| $CH(Me)CH_2SMe$ | H | H | 3-Cl-2-pyridyl | $SCHF_2$ |
| $CH(Me)CH_2SMe$ | H | H | 3-Cl-2-pyridyl | Br |
| $CH(Me)CH_2SMe$ | H | H | 3-Cl-2-pyridyl | Cl |
| $CH(Me)CH_2SMe$ | H | H | 3-Br-2-pyridyl | $CF_3$ |
| $CH(Me)CH_2SMe$ | H | H | 3-Br-2-pyridyl | $OCF_3$ |
| $CH(Me)CH_2SMe$ | H | H | 3-Br-2-pyridyl | Br |
| $CH(Me)CH_2SMe$ | H | H | 3-Br-2-pyridyl | Cl |
| $CH(Me)CH_2SMe$ | H | H | $CF_3$ | Me |
| $CH(Me)CH_2SMe$ | H | H | F | $CF_3$ |
| $CH(Me)CH_2SMe$ | H | H | Cl | $CF_3$ |
| $CH(Me)CH_2SMe$ | H | H | *n*-Pr | $CF_3$ |
| $CH(Me)CH_2SMe$ | H | H | *i*-Pr | $CF_3$ |
| $CH(Me)CH_2SMe$ | H | H | $CF_3$ | $CF_3$ |
| $CH(Me)CH_2SMe$ | H | H | OMe | $CF_3$ |
| $CH(Me)CH_2SMe$ | H | H | 2-BrPh | $CF_3$ |
| $CH(Me)CH_2SMe$ | H | H | 2-MePh | $CF_3$ |
| $CH(Me)CH_2SMe$ | H | H | 2-CNPh | $CF_3$ |
| $CH(Me)CH_2SMe$ | H | H | 2-FPh | $CF_3$ |
| $CH(Me)CH_2SMe$ | H | H | 2,6-$F_2$Ph | $CF_3$ |

| $R^3$ | $R^{4a}$ | $R^6$ | $R^{5a}$ | $R^{5b}$ |
|---|---|---|---|---|
| $CH(Me)CH_2SMe$ | H | H | 2,4-$F_2$Ph | $CF_3$ |
| $CH(Me)CH_2SMe$ | H | H | 2,5-$F_2$Ph | $CF_3$ |
| $CH(Me)CH_2SMe$ | H | H | 2-MeOPh | $CF_3$ |
| $CH(Me)CH_2SMe$ | H | H | 3-F-2-pyridyl | $CF_3$ |
| $CH(Me)CH_2SMe$ | H | H | 3-$CF_3$-2-pyridyl | $CF_3$ |
| $CH(Me)CH_2SMe$ | H | H | 3-Me-2-pyridyl | $CF_3$ |
| $CH(Me)CH_2SMe$ | Cl | H | 2-ClPh | $CF_3$ |
| $CH(Me)CH_2SMe$ | Cl | H | 2-ClPh | $OCF_3$ |
| $CH(Me)CH_2SMe$ | Cl | H | 2-ClPh | $SCHF_2$ |
| $CH(Me)CH_2SMe$ | Cl | H | 2-ClPh | Br |
| $CH(Me)CH_2SMe$ | Cl | H | 2-ClPh | Cl |
| $CH(Me)CH_2SMe$ | Cl | H | 3-Cl-2-pyridyl | $CF_3$ |
| $CH(Me)CH_2SMe$ | Cl | H | 3-Cl-2-pyridyl | $OCF_3$ |
| $CH(Me)CH_2SMe$ | Cl | H | 3-Cl-2-pyridyl | $SCHF_2$ |
| $CH(Me)CH_2SMe$ | Cl | H | 3-Cl-2-pyridyl | Br |
| $CH(Me)CH_2SMe$ | Cl | H | 3-Cl-2-pyridyl | Cl |
| $CH(Me)CH_2SMe$ | Cl | H | 3-Br-2-pyridyl | $CF_3$ |
| $CH(Me)CH_2SMe$ | Cl | H | 3-Br-2-pyridyl | $OCF_3$ |
| $CH(Me)CH_2SMe$ | Cl | H | 3-Br-2-pyridyl | Br |
| $CH(Me)CH_2SMe$ | Cl | H | 3-Br-2-pyridyl | Cl |
| $CH(Me)CH_2SMe$ | Cl | H | $CF_3$ | Me |
| $CH(Me)CH_2SMe$ | Cl | H | F | $CF_3$ |
| $CH(Me)CH_2SMe$ | Cl | H | Cl | $CF_3$ |
| $CH(Me)CH_2SMe$ | Cl | H | *n*-Pr | $CF_3$ |
| $CH(Me)CH_2SMe$ | Cl | H | *i*-Pr | $CF_3$ |
| $CH(Me)CH_2SMe$ | Cl | H | $CF_3$ | $CF_3$ |
| $CH(Me)CH_2SMe$ | Cl | H | OMe | $CF_3$ |
| $CH(Me)CH_2SMe$ | Cl | H | 2-BrPh | $CF_3$ |
| $CH(Me)CH_2SMe$ | Cl | H | 2-MePh | $CF_3$ |
| $CH(Me)CH_2SMe$ | Cl | H | 2-CNPh | $CF_3$ |
| $CH(Me)CH_2SMe$ | Cl | H | 2-FPh | $CF_3$ |
| $CH(Me)CH_2SMe$ | Cl | H | 2,6-$F_2$Ph | $CF_3$ |
| $CH(Me)CH_2SMe$ | Cl | H | 2,4-$F_2$Ph | $CF_3$ |
| $CH(Me)CH_2SMe$ | Cl | H | 2,5-$F_2$Ph | $CF_3$ |
| $CH(Me)CH_2SMe$ | Cl | H | 2-MeOPh | $CF_3$ |
| $CH(Me)CH_2SMe$ | Cl | H | 3-F-2-pyridyl | $CF_3$ |
| $CH(Me)CH_2SMe$ | Cl | H | 3-$CF_3$-2-pyridyl | $CF_3$ |

| $R^3$ | $R^{4a}$ | $R^6$ | $R^{5a}$ | $R^{5b}$ |
|---|---|---|---|---|
| $CH(Me)CH_2SMe$ | Cl | H | 3-Me-2-pyridyl | $CF_3$ |
| $CH(Me)CH_2SMe$ | Cl | H | H | $CF_3$ |
| $CH(Me)CH_2SMe$ | Cl | H | H | $OCF_3$ |
| $CH(Me)CH_2SMe$ | Cl | H | H | $OCHF_2$ |
| $CH(Me)CH_2SMe$ | Cl | H | H | $C_2F_5$ |
| $CH(Me)CH_2SMe$ | Cl | H | H | $OCF_2CHF_2$ |
| $CH(Me)CH_2SMe$ | Cl | H | H | $SCF_2CHF_2$ |
| $CH(Me)CH_2SMe$ | Cl | H | H | *n*-$C_3F_7$ |
| $CH(Me)CH_2SMe$ | Cl | H | H | *i*-$C_3F_7$ |
| $CH(Me)CH_2SMe$ | Cl | H | H | Br |
| $CH(Me)CH_2SMe$ | Cl | H | H | Cl |
| $CH(Me)CH_2SMe$ | Cl | H | H | $SCF_3$ |
| $CH(Me)CH_2SMe$ | Cl | H | Me | $CF_3$ |
| $CH(Me)CH_2SMe$ | Cl | H | Me | $OCF_3$ |
| $CH(Me)CH_2SMe$ | Cl | H | Me | $OCHF_2$ |
| $CH(Me)CH_2SMe$ | Cl | H | Me | $C_2F_5$ |
| $CH(Me)CH_2SMe$ | Cl | H | Me | $OCF_2CHF_2$ |
| $CH(Me)CH_2SMe$ | Cl | H | Me | $SCF_2CHF_2$ |
| $CH(Me)CH_2SMe$ | Cl | H | Me | *n*-$C_3F_7$ |
| $CH(Me)CH_2SMe$ | Cl | H | Me | *i*-$C_3F_7$ |
| $CH(Me)CH_2SMe$ | Cl | H | Me | Br |
| $CH(Me)CH_2SMe$ | Cl | H | Me | Cl |
| $CH(Me)CH_2SMe$ | Cl | H | Me | $SCF_3$ |
| $CH(Me)CH_2SMe$ | Cl | H | Me | I |
| $CH(Me)CH_2SMe$ | Cl | H | Me | SMe |
| $CH(Me)CH_2SMe$ | Cl | H | Me | OMe |
| $CH(Me)CH_2SMe$ | Cl | H | Me | OEt |
| $CH(Me)CH_2SMe$ | Cl | H | Me | Et |
| $CH(Me)CH_2SMe$ | Cl | H | Me | $SO_2Me$ |
| $CH(Me)CH_2SMe$ | Cl | H | Me | $SO_2CF_3$ |
| $CH(Me)CH_2SMe$ | Cl | H | Et | $CF_3$ |
| $CH(Me)CH_2SMe$ | Cl | H | Et | Br |
| $CH(Me)CH_2SMe$ | Cl | H | Et | Cl |
| $CH(Me)CH_2SMe$ | Cl | H | Ph | $CF_3$ |
| $CH(Me)CH_2SMe$ | Cl | H | Ph | Br |
| $CH(Me)CH_2SMe$ | Cl | H | Ph | Cl |
| $CH(Me)CH_2SMe$ | Cl | H | 2-pyridyl | $CF_3$ |

| $R^3$ | $R^{4a}$ | $R^6$ | $R^{5a}$ | $R^{5b}$ |
|---|---|---|---|---|
| $CH(Me)CH_2SMe$ | Cl | H | 2-pyridyl | Cl |

Table 2

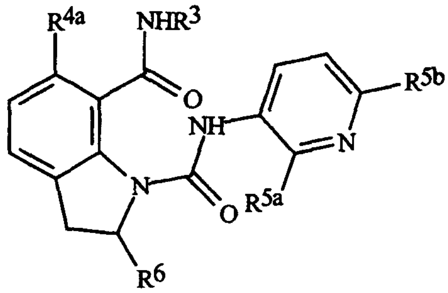

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CF_3$ | Me | H | H | H | $CF_3$ |
| Me | H | Me | H | $OCF_3$ | Me | H | H | H | $OCF_3$ |
| Me | H | Me | H | $OCHF_2$ | Me | H | H | H | $OCHF_2$ |
| Me | H | Me | H | $C_2F_5$ | Me | H | H | H | $C_2F_5$ |
| Me | H | Me | H | $OCF_2CHF_2$ | Me | H | H | H | $OCF_2CHF_2$ |
| Me | H | Me | H | $SCF_2CHF_2$ | Me | H | H | H | $SCF_2CHF_2$ |
| Me | H | Me | H | *n*-$C_3F_7$ | Me | H | H | H | *n*-$C_3F_7$ |
| Me | H | Me | H | *i*-$C_3F_7$ | Me | H | H | H | *i*-$C_3F_7$ |
| Me | H | Me | H | Br | Me | H | H | H | Br |
| Me | H | Me | H | Cl | Me | H | H | H | Cl |
| Me | H | Me | H | $SCF_3$ | Me | H | H | H | $SCF_3$ |
| Me | H | Me | Me | $CF_3$ | Me | H | H | Me | $CF_3$ |
| Me | H | Me | Me | $OCF_3$ | Me | H | H | Me | $OCF_3$ |
| Me | H | Me | Me | $OCHF_2$ | Me | H | H | Me | $OCHF_2$ |
| Me | H | Me | Me | $C_2F_5$ | Me | H | H | Me | $C_2F_5$ |
| Me | H | Me | Me | $OCF_2CHF_2$ | Me | H | H | Me | $OCF_2CHF_2$ |
| Me | H | Me | Me | $SCF_2CHF_2$ | Me | H | H | Me | $SCF_2CHF_2$ |
| Me | H | Me | Me | *n*-$C_3F_7$ | Me | H | H | Me | *n*-$C_3F_7$ |
| Me | H | Me | Me | *i*-$C_3F_7$ | Me | H | H | Me | *i*-$C_3F_7$ |
| Me | H | Me | Me | Br | Me | H | H | Me | Br |
| Me | H | Me | Me | Cl | Me | H | H | Me | Cl |
| Me | H | Me | Me | $SCF_3$ | Me | H | H | Me | $SCF_3$ |
| Me | H | Me | Me | I | Me | H | H | Me | I |
| Me | H | Me | Me | SMe | Me | H | H | Me | SMe |
| Me | H | Me | Me | OMe | Me | H | H | Me | OMe |
| Me | H | Me | Me | OEt | Me | H | H | Me | OEt |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | Me | Et | Me | H | H | Me | Et |
| Me | H | Me | Me | $SO_2Me$ | Me | H | H | Me | $SO_2Me$ |
| Me | H | Me | Me | $SO_2CF_3$ | Me | H | H | Me | $SO_2CF_3$ |
| Me | H | Me | Et | $CF_3$ | Me | H | H | Et | $CF_3$ |
| Me | H | Me | Et | Br | Me | H | H | Et | Br |
| Me | H | Me | Et | Cl | Me | H | H | Et | Cl |
| Me | H | Me | Ph | $CF_3$ | Me | H | H | Ph | $CF_3$ |
| Me | H | Me | Ph | Br | Me | H | H | Ph | Br |
| Me | H | Me | Ph | Cl | Me | H | H | Ph | Cl |
| Me | H | Me | 2-pyridyl | $CF_3$ | Me | H | H | 2-pyridyl | $CF_3$ |
| Me | H | Me | 2-pyridyl | Cl | Me | H | H | 2-pyridyl | Cl |
| Me | H | Me | 2-ClPh | $CF_3$ | Me | H | H | 2-ClPh | $CF_3$ |
| Me | H | Me | 2-ClPh | $OCF_3$ | Me | H | H | 2-ClPh | $OCF_3$ |
| Me | H | Me | 2-ClPh | $SCHF_2$ | Me | H | H | 2-ClPh | $SCHF_2$ |
| Me | H | Me | 2-ClPh | Br | Me | H | H | 2-ClPh | Br |
| Me | H | Me | 2-ClPh | Cl | Me | H | H | 2-ClPh | Cl |
| Me | H | Me | 3-Cl-2-pyridyl | $CF_3$ | Me | H | H | 3-Cl-2-pyridyl | $CF_3$ |
| Me | H | Me | 3-Cl-2-pyridyl | $OCF_3$ | Me | H | H | 3-Cl-2-pyridyl | $OCF_3$ |
| Me | H | Me | 3-Cl-2-pyridyl | $SCHF_2$ | Me | H | H | 3-Cl-2-pyridyl | $SCHF_2$ |
| Me | H | Me | 3-Cl-2-pyridyl | Br | Me | H | H | 3-Cl-2-pyridyl | Br |
| Me | H | Me | 3-Cl-2-pyridyl | Cl | Me | H | H | 3-Cl-2-pyridyl | Cl |
| Me | H | Me | 3-Br-2-pyridyl | $CF_3$ | Me | H | H | 3-Br-2-pyridyl | $CF_3$ |
| Me | H | Me | 3-Br-2-pyridyl | $OCF_3$ | Me | H | H | 3-Br-2-pyridyl | $OCF_3$ |
| Me | H | Me | 3-Br-2-pyridyl | Br | Me | H | H | 3-Br-2-pyridyl | Br |
| Me | H | Me | 3-Br-2-pyridyl | Cl | Me | H | H | 3-Br-2-pyridyl | Cl |
| Me | H | Me | $CF_3$ | Me | Me | H | H | $CF_3$ | Me |
| Me | H | Me | F | $CF_3$ | Me | H | H | F | $CF_3$ |
| Me | H | Me | Cl | $CF_3$ | Me | H | H | Cl | $CF_3$ |
| Me | H | Me | *n*-Pr | $CF_3$ | Me | H | H | *n*-Pr | $CF_3$ |
| Me | H | Me | *i*-Pr | $CF_3$ | Me | H | H | *i*-Pr | $CF_3$ |
| Me | H | Me | $CF_3$ | $CF_3$ | Me | H | H | $CF_3$ | $CF_3$ |
| Me | H | Me | OMe | $CF_3$ | Me | H | H | OMe | $CF_3$ |
| Me | H | Me | 2-BrPh | $CF_3$ | Me | H | H | 2-BrPh | $CF_3$ |
| Me | H | Me | 2-MePh | $CF_3$ | Me | H | H | 2-MePh | $CF_3$ |
| Me | H | Me | 2-CNPh | $CF_3$ | Me | H | H | 2-CNPh | $CF_3$ |
| Me | H | Me | 2-FPh | $CF_3$ | Me | H | H | 2-FPh | $CF_3$ |
| Me | H | Me | 2,6-$F_2$Ph | $CF_3$ | Me | H | H | 2,6-$F_2$Ph | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | 2,4-$F_2$Ph | $CF_3$ | Me | H | H | 2,4-$F_2$Ph | $CF_3$ |
| Me | H | Me | 2,5-$F_2$Ph | $CF_3$ | Me | H | H | 2,5-$F_2$Ph | $CF_3$ |
| Me | H | Me | 2-MeOPh | $CF_3$ | Me | H | H | 2-MeOPh | $CF_3$ |
| Me | H | Me | 3-F-2-pyridyl | $CF_3$ | Me | H | H | 3-F-2-pyridyl | $CF_3$ |
| Me | H | Me | 3-$CF_3$-2-pyridyl | $CF_3$ | Me | H | H | 3-$CF_3$-2-pyridyl | $CF_3$ |
| Me | H | Me | 3-Me-2-pyridyl | $CF_3$ | Me | H | H | 3-Me-2-pyridyl | $CF_3$ |
| Et | H | Me | H | $CF_3$ | Et | H | H | H | $CF_3$ |
| Et | H | Me | H | $C_2F_5$ | Et | H | H | H | $C_2F_5$ |
| Et | H | Me | H | $OCF_3$ | Et | H | H | H | $OCF_3$ |
| Et | H | Me | Me | $CF_3$ | Et | H | H | Me | $CF_3$ |
| Et | H | Me | Me | $C_2F_5$ | Et | H | H | Me | $C_2F_5$ |
| Et | H | Me | Me | $OCF_3$ | Et | H | H | Me | $OCF_3$ |
| Me | Me | H | H | $CF_3$ | *i*-Pr | H | H | H | $CF_3$ |
| Me | Me | H | H | $OCF_3$ | *i*-Pr | H | H | H | $OCF_3$ |
| Me | Me | H | H | $OCHF_2$ | *i*-Pr | H | H | H | $OCHF_2$ |
| Me | Me | H | H | $C_2F_5$ | *i*-Pr | H | H | H | $C_2F_5$ |
| Me | Me | H | H | $OCF_2CHF_2$ | *i*-Pr | H | H | H | $OCF_2CHF_2$ |
| Me | Me | H | H | $SCF_2CHF_2$ | *i*-Pr | H | H | H | $SCF_2CHF_2$ |
| Me | Me | H | H | *n*-$C_3F_7$ | *i*-Pr | H | H | H | *n*-$C_3F_7$ |
| Me | Me | H | H | *i*-$C_3F_7$ | *i*-Pr | H | H | H | *i*-$C_3F_7$ |
| Me | Me | H | H | Br | *i*-Pr | H | H | H | Br |
| Me | Me | H | H | Cl | *i*-Pr | H | H | H | Cl |
| Me | Me | H | H | $SCF_3$ | *i*-Pr | H | H | H | $SCF_3$ |
| Me | Me | H | H | $CF_3$ | *i*-Pr | H | H | Me | $CF_3$ |
| Me | Me | H | H | $OCF_3$ | *i*-Pr | H | H | Me | $OCF_3$ |
| Me | Me | H | H | $OCHF_2$ | *i*-Pr | H | H | Me | $OCHF_2$ |
| Me | Me | H | Me | $C_2F_5$ | *i*-Pr | H | H | Me | $C_2F_5$ |
| Me | Me | H | Me | $OCF_2CHF_2$ | *i*-Pr | H | H | Me | $OCF_2CHF_2$ |
| Me | Me | H | Me | $SCF_2CHF_2$ | *i*-Pr | H | H | Me | $SCF_2CHF_2$ |
| Me | Me | H | Me | *n*-$C_3F_7$ | *i*-Pr | H | H | Me | *n*-$C_3F_7$ |
| Me | Me | H | Me | *i*-$C_3F_7$ | *i*-Pr | H | H | Me | *i*-$C_3F_7$ |
| Me | Me | H | Me | Br | *i*-Pr | H | H | Me | Br |
| Me | Me | H | Me | Cl | *i*-Pr | H | H | Me | Cl |
| Me | Me | H | Me | $SCF_3$ | *i*-Pr | H | H | Me | $SCF_3$ |
| Me | Me | H | Me | I | *i*-Pr | H | H | Me | I |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | Me | SMe | *i*-Pr | H | H | Me | SMe |
| Me | Me | H | Me | OMe | *i*-Pr | H | H | Me | OMe |
| Me | Me | H | Me | OEt | *i*-Pr | H | H | Me | OEt |
| Me | Me | H | Me | Et | *i*-Pr | H | H | Me | Et |
| Me | Me | H | Me | $SO_2Me$ | *i*-Pr | H | H | Me | $SO_2Me$ |
| Me | Me | H | Me | $SO_2CF_3$ | *i*-Pr | H | H | Me | $SO_2CF_3$ |
| Me | Me | H | Et | $CF_3$ | *i*-Pr | H | H | Et | $CF_3$ |
| Me | Me | H | Et | Br | *i*-Pr | H | H | Et | Br |
| Me | Me | H | Et | Cl | *i*-Pr | H | H | Et | Cl |
| Me | Me | H | Ph | $CF_3$ | *i*-Pr | H | H | Ph | $CF_3$ |
| Me | Me | H | Ph | Br | *i*-Pr | H | H | Ph | Br |
| Me | Me | H | Ph | Cl | *i*-Pr | H | H | Ph | Cl |
| Me | Me | H | 2-pyridyl | $CF_3$ | *i*-Pr | H | H | 2-pyridyl | $CF_3$ |
| Me | Me | H | 2-pyridyl | Cl | *i*-Pr | H | H | 2-pyridyl | Cl |
| Me | Me | H | 2-ClPh | $CF_3$ | *i*-Pr | H | H | 2-ClPh | $CF_3$ |
| Me | Me | H | 2-ClPh | $OCF_3$ | *i*-Pr | H | H | 2-ClPh | $OCF_3$ |
| Me | Me | H | 2-ClPh | $SCHF_2$ | *i*-Pr | H | H | 2-ClPh | $SCHF_2$ |
| Me | Me | H | 2-ClPh | Br | *i*-Pr | H | H | 2-ClPh | Br |
| Me | Me | H | 2-ClPh | Cl | *i*-Pr | H | H | 2-ClPh | Cl |
| Me | Me | H | 3-Cl-2-pyridyl | $CF_3$ | *i*-Pr | H | H | 3-Cl-2-pyridyl | $CF_3$ |
| Me | Me | H | 3-Cl-2-pyridyl | $OCF_3$ | *i*-Pr | H | H | 3-Cl-2-pyridyl | $OCF_3$ |
| Me | Me | H | 3-Cl-2-pyridyl | $SCHF_2$ | *i*-Pr | H | H | 3-Cl-2-pyridyl | $SCHF_2$ |
| Me | Me | H | 3-Cl-2-pyridyl | Br | *i*-Pr | H | H | 3-Cl-2-pyridyl | Br |
| Me | Me | H | 3-Cl-2-pyridyl | Cl | *i*-Pr | H | H | 3-Cl-2-pyridyl | Cl |
| Me | Me | H | 3-Br-2-pyridyl | $CF_3$ | *i*-Pr | H | H | 3-Br-2-pyridyl | $CF_3$ |
| Me | Me | H | 3-Br-2-pyridyl | $OCF_3$ | *i*-Pr | H | H | 3-Br-2-pyridyl | $OCF_3$ |
| Me | Me | H | 3-Br-2-pyridyl | Br | *i*-Pr | H | H | 3-Br-2-pyridyl | Br |
| Me | Me | H | 3-Br-2-pyridyl | Cl | *i*-Pr | H | H | 3-Br-2-pyridyl | Cl |
| Me | Me | H | $CF_3$ | Me | *i*-Pr | H | H | $CF_3$ | Me |
| Me | Me | H | F | $CF_3$ | *i*-Pr | H | H | F | $CF_3$ |
| Me | Me | H | Cl | $CF_3$ | *i*-Pr | H | H | Cl | $CF_3$ |
| Me | Me | H | *n*-Pr | $CF_3$ | *i*-Pr | H | H | *n*-Pr | $CF_3$ |
| Me | Me | H | *i*-Pr | $CF_3$ | *i*-Pr | H | H | *i*-Pr | $CF_3$ |
| Me | Me | H | $CF_3$ | $CF_3$ | *i*-Pr | H | H | $CF_3$ | $CF_3$ |
| Me | Me | H | OMe | $CF_3$ | *i*-Pr | H | H | OMe | $CF_3$ |
| Me | Me | H | 2-BrPh | $CF_3$ | *i*-Pr | H | H | 2-BrPh | $CF_3$ |
| Me | Me | H | 2-MePh | $CF_3$ | *i*-Pr | H | H | 2-MePh | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | 2-CNPh | $CF_3$ | *i*-Pr | H | H | 2-CNPh | $CF_3$ |
| Me | Me | H | 2-FPh | $CF_3$ | *i*-Pr | H | H | 2-FPh | $CF_3$ |
| Me | Me | H | 2,6-$F_2$Ph | $CF_3$ | *i*-Pr | H | H | 2,6-$F_2$Ph | $CF_3$ |
| Me | Me | H | 2,4-$F_2$Ph | $CF_3$ | *i*-Pr | H | H | 2,4-$F_2$Ph | $CF_3$ |
| Me | Me | H | 2,5-$F_2$Ph | $CF_3$ | *i*-Pr | H | H | 2,5-$F_2$Ph | $CF_3$ |
| Me | Me | H | 2-MeOPh | $CF_3$ | *i*-Pr | H | H | 2-MeOPh | $CF_3$ |
| Me | Me | H | 3-F-2-pyridyl | $CF_3$ | *i*-Pr | H | H | 3-F-2-pyridyl | $CF_3$ |
| Me | Me | H | 3-$CF_3$-2-pyridyl | $CF_3$ | *i*-Pr | H | H | 3-$CF_3$-2-pyridyl | $CF_3$ |
| Me | Me | H | 3-Me-2-pyridyl | $CF_3$ | *i*-Pr | H | H | 3-Me-2-pyridyl | $CF_3$ |
| Et | Me | H | H | $CF_3$ | *i*-Pr | Me | H | H | $CF_3$ |
| Et | Me | H | H | $C_2F_5$ | *i*-Pr | Me | H | H | $C_2F_5$ |
| Et | Me | H | H | $OCF_3$ | *i*-Pr | Me | H | H | $OCF_3$ |
| Et | Me | H | Me | $CF_3$ | *i*-Pr | Me | H | Me | $CF_3$ |
| Et | Me | H | Me | $C_2F_5$ | *i*-Pr | Me | H | Me | $C_2F_5$ |
| Et | Me | H | Me | $OCF_3$ | *i*-Pr | Me | H | Me | $OCF_3$ |
| Me | Cl | H | H | $CF_3$ | Me | Cl | H | 2-ClPh | $CF_3$ |
| Me | Cl | H | H | $OCF_3$ | Me | Cl | H | 2-ClPh | $OCF_3$ |
| Me | Cl | H | H | $OCHF_2$ | Me | Cl | H | 2-ClPh | $SCHF_2$ |
| Me | Cl | H | H | $C_2F_5$ | Me | Cl | H | 2-ClPh | Br |
| Me | Cl | H | H | $OCF_2CHF_2$ | Me | Cl | H | 2-ClPh | Cl |
| Me | Cl | H | H | $SCF_2CHF_2$ | Me | Cl | H | 3-Cl-2-pyridyl | $CF_3$ |
| Me | Cl | H | H | *n*-$C_3F_7$ | Me | Cl | H | 3-Cl-2-pyridyl | $OCF_3$ |
| Me | Cl | H | H | *i*-$C_3F_7$ | Me | Cl | H | 3-Cl-2-pyridyl | $SCHF_2$ |
| Me | Cl | H | H | Br | Me | Cl | H | 3-Cl-2-pyridyl | Br |
| Me | Cl | H | H | Cl | Me | Cl | H | 3-Cl-2-pyridyl | Cl |
| Me | Cl | H | H | $SCF_3$ | Me | Cl | H | 3-Br-2-pyridyl | $CF_3$ |
| Me | Cl | H | Me | $CF_3$ | Me | Cl | H | 3-Br-2-pyridyl | $OCF_3$ |
| Me | Cl | H | Me | $OCF_3$ | Me | Cl | H | 3-Br-2-pyridyl | Br |
| Me | Cl | H | Me | $OCHF_2$ | Me | Cl | H | 3-Br-2-pyridyl | Cl |
| Me | Cl | H | Me | $C_2F_5$ | Me | Cl | H | $CF_3$ | Me |
| Me | Cl | H | Me | $OCF_2CHF_2$ | Me | Cl | H | F | $CF_3$ |
| Me | Cl | H | Me | $SCF_2CHF_2$ | Me | Cl | H | Cl | $CF_3$ |
| Me | Cl | H | Me | *n*-$C_3F_7$ | Me | Cl | H | *n*-Pr | $CF_3$ |
| Me | Cl | H | Me | *i*-$C_3F_7$ | Me | Cl | H | *i*-Pr | $CF_3$ |
| Me | Cl | H | Me | Br | Me | Cl | H | $CF_3$ | $CF_3$ |

| $R^3$ | $R^{4a}$ | $R^6$ | $R^{5a}$ | $R^{5b}$ |
|---|---|---|---|---|
| Me | Cl | H | Me | Cl |
| Me | Cl | H | Me | $SCF_3$ |
| Me | Cl | H | Me | I |
| Me | Cl | H | Me | SMe |
| Me | Cl | H | Me | OMe |
| Me | Cl | H | Me | OEt |
| Me | Cl | H | Me | Et |
| Me | Cl | H | Me | $SO_2Me$ |
| Me | Cl | H | Me | $SO_2CF_3$ |
| Me | Cl | H | Et | $CF_3$ |
| Me | Cl | H | Et | Br |
| Me | Cl | H | Et | Cl |
| Me | Cl | H | Ph | $CF_3$ |
| Me | Cl | H | Ph | Br |
| Me | Cl | H | Ph | Cl |
| Me | Cl | H | 2-pyridyl | $CF_3$ |
| Me | Cl | H | 2-pyridyl | Cl |

| $R^3$ | $R^{4a}$ | $R^6$ | $R^{5a}$ | $R^{5b}$ |
|---|---|---|---|---|
| Me | Cl | H | OMe | $CF_3$ |
| Me | Cl | H | 2-BrPh | $CF_3$ |
| Me | Cl | H | 2-MePh | $CF_3$ |
| Me | Cl | H | 2-CNPh | $CF_3$ |
| Me | Cl | H | 2-FPh | $CF_3$ |
| Me | Cl | H | 2,6-$F_2$Ph | $CF_3$ |
| Me | Cl | H | 2,4-$F_2$Ph | $CF_3$ |
| Me | Cl | H | 2,5-$F_2$Ph | $CF_3$ |
| Me | Cl | H | 2-MeOPh | $CF_3$ |
| Me | Cl | H | 3-F-2-pyridyl | $CF_3$ |
| Me | Cl | H | 3-$CF_3$-2-pyridyl | $CF_3$ |
| Me | Cl | H | 3-Me-2-pyridyl | $CF_3$ |
| Et | Cl | H | H | $OCF_3$ |
| Et | Cl | H | H | $C_2F_5$ |
| Et | Cl | H | Me | $CF_3$ |
| Et | Cl | H | Me | $C_2F_5$ |
| *i*-Pr | Cl | H | Me | $CF_3$ |

Table 3

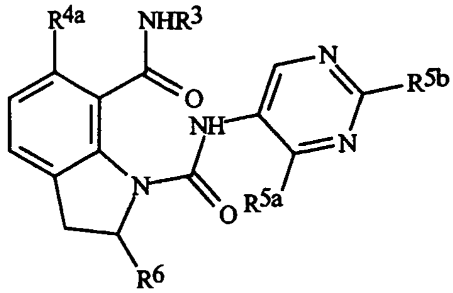

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CF_3$ | Me | H | H | H | $CF_3$ |
| Me | H | Me | H | $OCF_3$ | Me | H | H | H | $OCF_3$ |
| Me | H | Me | H | $OCHF_2$ | Me | H | H | H | $OCHF_2$ |
| Me | H | Me | H | $C_2F_5$ | Me | H | H | H | $C_2F_5$ |
| Me | H | Me | H | $OCF_2CHF_2$ | Me | H | H | H | $OCF_2CHF_2$ |
| Me | H | Me | H | $SCF_2CHF_2$ | Me | H | H | H | $SCF_2CHF_2$ |
| Me | H | Me | H | *n*-$C_3F_7$ | Me | H | H | H | *n*-$C_3F_7$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $i$-$C_3F_7$ | Me | H | H | H | $i$-$C_3F_7$ |
| Me | H | Me | H | Br | Me | H | H | H | Br |
| Me | H | Me | H | Cl | Me | H | H | H | Cl |
| Me | H | Me | H | $SCF_3$ | Me | H | H | H | $SCF_3$ |
| Me | H | Me | Me | $CF_3$ | Me | H | H | Me | $CF_3$ |
| Me | H | Me | Me | $OCF_3$ | Me | H | H | Me | $OCF_3$ |
| Me | H | Me | Me | $OCHF_2$ | Me | H | H | Me | $OCHF_2$ |
| Me | H | Me | Me | $C_2F_5$ | Me | H | H | Me | $C_2F_5$ |
| Me | H | Me | Me | $OCF_2CHF_2$ | Me | H | H | Me | $OCF_2CHF_2$ |
| Me | H | Me | Me | $SCF_2CHF_2$ | Me | H | H | Me | $SCF_2CHF_2$ |
| Me | H | Me | Me | $n$-$C_3F_7$ | Me | H | H | Me | $n$-$C_3F_7$ |
| Me | H | Me | Me | $i$-$C_3F_7$ | Me | H | H | Me | $i$-$C_3F_7$ |
| Me | H | Me | Me | Br | Me | H | H | Me | Br |
| Me | H | Me | Me | Cl | Me | H | H | Me | Cl |
| Me | H | Me | Me | $SCF_3$ | Me | H | H | Me | $SCF_3$ |
| Me | H | Me | Me | I | Me | H | H | Me | I |
| Me | H | Me | Me | SMe | Me | H | H | Me | SMe |
| Me | H | Me | Me | OMe | Me | H | H | Me | OMe |
| Me | H | Me | Me | OEt | Me | H | H | Me | OEt |
| Me | H | Me | Me | Et | Me | H | H | Me | Et |
| Me | H | Me | Me | $SO_2Me$ | Me | H | H | Me | $SO_2Me$ |
| Me | H | Me | Me | $SO_2CF_3$ | Me | H | H | Me | $SO_2CF_3$ |
| Me | H | Me | Et | $CF_3$ | Me | H | H | Et | $CF_3$ |
| Me | H | Me | Et | Br | Me | H | H | Et | Br |
| Me | H | Me | Et | Cl | Me | H | H | Et | Cl |
| Me | H | Me | Ph | $CF_3$ | Me | H | H | Ph | $CF_3$ |
| Me | H | Me | Ph | Br | Me | H | H | Ph | Br |
| Me | H | Me | Ph | Cl | Me | H | H | Ph | Cl |
| Me | H | Me | 2-pyridyl | $CF_3$ | Me | H | H | 2-pyridyl | $CF_3$ |
| Me | H | Me | 2-pyridyl | Cl | Me | H | H | 2-pyridyl | Cl |
| Me | H | Me | 2-ClPh | $CF_3$ | Me | H | H | 2-ClPh | $CF_3$ |
| Me | H | Me | 2-ClPh | $OCF_3$ | Me | H | H | 2-ClPh | $OCF_3$ |
| Me | H | Me | 2-ClPh | $SCHF_2$ | Me | H | H | 2-ClPh | $SCHF_2$ |
| Me | H | Me | 2-ClPh | Br | Me | H | H | 2-ClPh | Br |
| Me | H | Me | 2-ClPh | Cl | Me | H | H | 2-ClPh | Cl |
| Me | H | Me | 3-Cl-2-pyridyl | $CF_3$ | Me | H | H | 3-Cl-2-pyridyl | $CF_3$ |
| Me | H | Me | 3-Cl-2-pyridyl | $OCF_3$ | Me | H | H | 3-Cl-2-pyridyl | $OCF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | 3-Cl-2-pyridyl | $SCHF_2$ | Me | H | H | 3-Cl-2-pyridyl | $SCHF_2$ |
| Me | H | Me | 3-Cl-2-pyridyl | Br | Me | H | H | 3-Cl-2-pyridyl | Br |
| Me | H | Me | 3-Cl-2-pyridyl | Cl | Me | H | H | 3-Cl-2-pyridyl | Cl |
| Me | H | Me | 3-Br-2-pyridyl | $CF_3$ | Me | H | H | 3-Br-2-pyridyl | $CF_3$ |
| Me | H | Me | 3-Br-2-pyridyl | $OCF_3$ | Me | H | H | 3-Br-2-pyridyl | $OCF_3$ |
| Me | H | Me | 3-Br-2-pyridyl | Br | Me | H | H | 3-Br-2-pyridyl | Br |
| Me | H | Me | 3-Br-2-pyridyl | Cl | Me | H | H | 3-Br-2-pyridyl | Cl |
| Me | H | Me | $CF_3$ | Me | Me | H | H | $CF_3$ | Me |
| Me | H | Me | F | $CF_3$ | Me | H | H | F | $CF_3$ |
| Me | H | Me | Cl | $CF_3$ | Me | H | H | Cl | $CF_3$ |
| Me | H | Me | *n*-Pr | $CF_3$ | Me | H | H | *n*-Pr | $CF_3$ |
| Me | H | Me | *i*-Pr | $CF_3$ | Me | H | H | *i*-Pr | $CF_3$ |
| Me | H | Me | $CF_3$ | $CF_3$ | Me | H | H | $CF_3$ | $CF_3$ |
| Me | H | Me | OMe | $CF_3$ | Me | H | H | OMe | $CF_3$ |
| Me | H | Me | 2-BrPh | $CF_3$ | Me | H | H | 2-BrPh | $CF_3$ |
| Me | H | Me | 2-MePh | $CF_3$ | Me | H | H | 2-MePh | $CF_3$ |
| Me | H | Me | 2-CNPh | $CF_3$ | Me | H | H | 2-CNPh | $CF_3$ |
| Me | H | Me | 2-FPh | $CF_3$ | Me | H | H | 2-FPh | $CF_3$ |
| Me | H | Me | 2,6-$F_2$Ph | $CF_3$ | Me | H | H | 2,6-$F_2$Ph | $CF_3$ |
| Me | H | Me | 2,4-$F_2$Ph | $CF_3$ | Me | H | H | 2,4-$F_2$Ph | $CF_3$ |
| Me | H | Me | 2,5-$F_2$Ph | $CF_3$ | Me | H | H | 2,5-$F_2$Ph | $CF_3$ |
| Me | H | Me | 2-MeOPh | $CF_3$ | Me | H | H | 2-MeOPh | $CF_3$ |
| Me | H | Me | 3-F-2-pyridyl | $CF_3$ | Me | H | H | 3-F-2-pyridyl | $CF_3$ |
| Me | H | Me | 3-$CF_3$-2-pyridyl | $CF_3$ | Me | H | H | 3-$CF_3$-2-pyridyl | $CF_3$ |
| Me | H | Me | 3-Me-2-pyridyl | $CF_3$ | Me | H | H | 3-Me-2-pyridyl | $CF_3$ |
| Et | H | Me | H | $CF_3$ | Et | H | H | H | $CF_3$ |
| Et | H | Me | H | $C_2F_5$ | Et | H | H | H | $C_2F_5$ |
| Et | H | Me | H | $OCF_3$ | Et | H | H | H | $OCF_3$ |
| Et | H | Me | Me | $CF_3$ | Et | H | H | Me | $CF_3$ |
| Et | H | Me | Me | $C_2F_5$ | Et | H | H | Me | $C_2F_5$ |
| Et | H | Me | Me | $OCF_3$ | Et | H | H | Me | $OCF_3$ |
| Me | Me | H | H | $CF_3$ | *i*-Pr | H | H | H | $CF_3$ |
| Me | Me | H | H | $OCF_3$ | *i*-Pr | H | H | H | $OCF_3$ |
| Me | Me | H | H | $OCHF_2$ | *i*-Pr | H | H | H | $OCHF_2$ |
| Me | Me | H | H | $C_2F_5$ | *i*-Pr | H | H | H | $C_2F_5$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | H | $OCF_2CHF_2$ | *i*-Pr | H | H | H | $OCF_2CHF_2$ |
| Me | Me | H | H | $SCF_2CHF_2$ | *i*-Pr | H | H | H | $SCF_2CHF_2$ |
| Me | Me | H | H | *n*-$C_3F_7$ | *i*-Pr | H | H | H | *n*-$C_3F_7$ |
| Me | Me | H | H | *i*-$C_3F_7$ | *i*-Pr | H | H | H | *i*-$C_3F_7$ |
| Me | Me | H | H | Br | *i*-Pr | H | H | H | Br |
| Me | Me | H | H | Cl | *i*-Pr | H | H | H | Cl |
| Me | Me | H | H | $SCF_3$ | *i*-Pr | H | H | H | $SCF_3$ |
| Me | Me | H | H | $CF_3$ | *i*-Pr | H | H | Me | $CF_3$ |
| Me | Me | H | H | $OCF_3$ | *i*-Pr | H | H | Me | $OCF_3$ |
| Me | Me | H | H | $OCHF_2$ | *i*-Pr | H | H | Me | $OCHF_2$ |
| Me | Me | H | Me | $C_2F_5$ | *i*-Pr | H | H | Me | $C_2F_5$ |
| Me | Me | H | Me | $OCF_2CHF_2$ | *i*-Pr | H | H | Me | $OCF_2CHF_2$ |
| Me | Me | H | Me | $SCF_2CHF_2$ | *i*-Pr | H | H | Me | $SCF_2CHF_2$ |
| Me | Me | H | Me | *n*-$C_3F_7$ | *i*-Pr | H | H | Me | *n*-$C_3F_7$ |
| Me | Me | H | Me | *i*-$C_3F_7$ | *i*-Pr | H | H | Me | *i*-$C_3F_7$ |
| Me | Me | H | Me | Br | *i*-Pr | H | H | Me | Br |
| Me | Me | H | Me | Cl | *i*-Pr | H | H | Me | Cl |
| Me | Me | H | Me | $SCF_3$ | *i*-Pr | H | H | Me | $SCF_3$ |
| Me | Me | H | Me | I | *i*-Pr | H | H | Me | I |
| Me | Me | H | Me | SMe | *i*-Pr | H | H | Me | SMe |
| Me | Me | H | Me | OMe | *i*-Pr | H | H | Me | OMe |
| Me | Me | H | Me | OEt | *i*-Pr | H | H | Me | OEt |
| Me | Me | H | Me | Et | *i*-Pr | H | H | Me | Et |
| Me | Me | H | Me | $SO_2Me$ | *i*-Pr | H | H | Me | $SO_2Me$ |
| Me | Me | H | Me | $SO_2CF_3$ | *i*-Pr | H | H | Me | $SO_2CF_3$ |
| Me | Me | H | Et | $CF_3$ | *i*-Pr | H | H | Et | $CF_3$ |
| Me | Me | H | Et | Br | *i*-Pr | H | H | Et | Br |
| Me | Me | H | Et | Cl | *i*-Pr | H | H | Et | Cl |
| Me | Me | H | Ph | $CF_3$ | *i*-Pr | H | H | Ph | $CF_3$ |
| Me | Me | H | Ph | Br | *i*-Pr | H | H | Ph | Br |
| Me | Me | H | Ph | Cl | *i*-Pr | H | H | Ph | Cl |
| Me | Me | H | 2-pyridyl | $CF_3$ | *i*-Pr | H | H | 2-pyridyl | $CF_3$ |
| Me | Me | H | 2-pyridyl | Cl | *i*-Pr | H | H | 2-pyridyl | Cl |
| Me | Me | H | 2-ClPh | $CF_3$ | *i*-Pr | H | H | 2-ClPh | $CF_3$ |
| Me | Me | H | 2-ClPh | $OCF_3$ | *i*-Pr | H | H | 2-ClPh | $OCF_3$ |
| Me | Me | H | 2-ClPh | $SCHF_2$ | *i*-Pr | H | H | 2-ClPh | $SCHF_2$ |
| Me | Me | H | 2-ClPh | Br | *i*-Pr | H | H | 2-ClPh | Br |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | 2-ClPh | Cl | *i*-Pr | H | H | 2-ClPh | Cl |
| Me | Me | H | 3-Cl-2-pyridyl | $CF_3$ | *i*-Pr | H | H | 3-Cl-2-pyridyl | $CF_3$ |
| Me | Me | H | 3-Cl-2-pyridyl | $OCF_3$ | *i*-Pr | H | H | 3-Cl-2-pyridyl | $OCF_3$ |
| Me | Me | H | 3-Cl-2-pyridyl | $SCHF_2$ | *i*-Pr | H | H | 3-Cl-2-pyridyl | $SCHF_2$ |
| Me | Me | H | 3-Cl-2-pyridyl | Br | *i*-Pr | H | H | 3-Cl-2-pyridyl | Br |
| Me | Me | H | 3-Cl-2-pyridyl | Cl | *i*-Pr | H | H | 3-Cl-2-pyridyl | Cl |
| Me | Me | H | 3-Br-2-pyridyl | $CF_3$ | *i*-Pr | H | H | 3-Br-2-pyridyl | $CF_3$ |
| Me | Me | H | 3-Br-2-pyridyl | $OCF_3$ | *i*-Pr | H | H | 3-Br-2-pyridyl | $OCF_3$ |
| Me | Me | H | 3-Br-2-pyridyl | Br | *i*-Pr | H | H | 3-Br-2-pyridyl | Br |
| Me | Me | H | 3-Br-2-pyridyl | Cl | *i*-Pr | H | H | 3-Br-2-pyridyl | Cl |
| Me | Me | H | $CF_3$ | Me | *i*-Pr | H | H | $CF_3$ | Me |
| Me | Me | H | F | $CF_3$ | *i*-Pr | H | H | F | $CF_3$ |
| Me | Me | H | Cl | $CF_3$ | *i*-Pr | H | H | Cl | $CF_3$ |
| Me | Me | H | *n*-Pr | $CF_3$ | *i*-Pr | H | H | *n*-Pr | $CF_3$ |
| Me | Me | H | *i*-Pr | $CF_3$ | *i*-Pr | H | H | *i*-Pr | $CF_3$ |
| Me | Me | H | $CF_3$ | $CF_3$ | *i*-Pr | H | H | $CF_3$ | $CF_3$ |
| Me | Me | H | OMe | $CF_3$ | *i*-Pr | H | H | OMe | $CF_3$ |
| Me | Me | H | 2-BrPh | $CF_3$ | *i*-Pr | H | H | 2-BrPh | $CF_3$ |
| Me | Me | H | 2-MePh | $CF_3$ | *i*-Pr | H | H | 2-MePh | $CF_3$ |
| Me | Me | H | 2-CNPh | $CF_3$ | *i*-Pr | H | H | 2-CNPh | $CF_3$ |
| Me | Me | H | 2-FPh | $CF_3$ | *i*-Pr | H | H | 2-FPh | $CF_3$ |
| Me | Me | H | 2,6-$F_2$Ph | $CF_3$ | *i*-Pr | H | H | 2,6-$F_2$Ph | $CF_3$ |
| Me | Me | H | 2,4-$F_2$Ph | $CF_3$ | *i*-Pr | H | H | 2,4-$F_2$Ph | $CF_3$ |
| Me | Me | H | 2,5-$F_2$Ph | $CF_3$ | *i*-Pr | H | H | 2,5-$F_2$Ph | $CF_3$ |
| Me | Me | H | 2-MeOPh | $CF_3$ | *i*-Pr | H | H | 2-MeOPh | $CF_3$ |
| Me | Me | H | 3-F-2-pyridyl | $CF_3$ | *i*-Pr | H | H | 3-F-2-pyridyl | $CF_3$ |
| Me | Me | H | 3-$CF_3$-2-pyridyl | $CF_3$ | *i*-Pr | H | H | 3-$CF_3$-2-pyridyl | $CF_3$ |
| Me | Me | H | 3-Me-2-pyridyl | $CF_3$ | *i*-Pr | H | H | 3-Me-2-pyridyl | $CF_3$ |
| Et | Me | H | H | $CF_3$ | *i*-Pr | Me | H | H | $CF_3$ |
| Et | Me | H | H | $C_2F_5$ | *i*-Pr | Me | H | H | $C_2F_5$ |
| Et | Me | H | H | $OCF_3$ | *i*-Pr | Me | H | H | $OCF_3$ |
| Et | Me | H | Me | $CF_3$ | *i*-Pr | Me | H | Me | $CF_3$ |
| Et | Me | H | Me | $C_2F_5$ | *i*-Pr | Me | H | Me | $C_2F_5$ |
| Et | Me | H | Me | $OCF_3$ | *i*-Pr | Me | H | Me | $OCF_3$ |
| Me | Cl | H | H | $CF_3$ | Me | Cl | H | 2-ClPh | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Cl | H | H | $OCF_3$ | Me | Cl | H | 2-ClPh | $OCF_3$ |
| Me | Cl | H | H | $OCHF_2$ | Me | Cl | H | 2-ClPh | $SCHF_2$ |
| Me | Cl | H | H | $C_2F_5$ | Me | Cl | H | 2-ClPh | Br |
| Me | Cl | H | H | $OCF_2CHF_2$ | Me | Cl | H | 2-ClPh | Cl |
| Me | Cl | H | H | $SCF_2CHF_2$ | Me | Cl | H | 3-Cl-2-pyridyl | $CF_3$ |
| Me | Cl | H | H | *n*-$C_3F_7$ | Me | Cl | H | 3-Cl-2-pyridyl | $OCF_3$ |
| Me | Cl | H | H | *i*-$C_3F_7$ | Me | Cl | H | 3-Cl-2-pyridyl | $SCHF_2$ |
| Me | Cl | H | H | Br | Me | Cl | H | 3-Cl-2-pyridyl | Br |
| Me | Cl | H | H | Cl | Me | Cl | H | 3-Cl-2-pyridyl | Cl |
| Me | Cl | H | H | $SCF_3$ | Me | Cl | H | 3-Br-2-pyridyl | $CF_3$ |
| Me | Cl | H | Me | $CF_3$ | Me | Cl | H | 3-Br-2-pyridyl | $OCF_3$ |
| Me | Cl | H | Me | $OCF_3$ | Me | Cl | H | 3-Br-2-pyridyl | Br |
| Me | Cl | H | Me | $OCHF_2$ | Me | Cl | H | 3-Br-2-pyridyl | Cl |
| Me | Cl | H | Me | $C_2F_5$ | Me | Cl | H | $CF_3$ | Me |
| Me | Cl | H | Me | $OCF_2CHF_2$ | Me | Cl | H | F | $CF_3$ |
| Me | Cl | H | Me | $SCF_2CHF_2$ | Me | Cl | H | Cl | $CF_3$ |
| Me | Cl | H | Me | *n*-$C_3F_7$ | Me | Cl | H | *n*-Pr | $CF_3$ |
| Me | Cl | H | Me | *i*-$C_3F_7$ | Me | Cl | H | *i*-Pr | $CF_3$ |
| Me | Cl | H | Me | Br | Me | Cl | H | $CF_3$ | $CF_3$ |
| Me | Cl | H | Me | Cl | Me | Cl | H | OMe | $CF_3$ |
| Me | Cl | H | Me | $SCF_3$ | Me | Cl | H | 2-BrPh | $CF_3$ |
| Me | Cl | H | Me | I | Me | Cl | H | 2-MePh | $CF_3$ |
| Me | Cl | H | Me | SMe | Me | Cl | H | 2-CNPh | $CF_3$ |
| Me | Cl | H | Me | OMe | Me | Cl | H | 2-FPh | $CF_3$ |
| Me | Cl | H | Me | OEt | Me | Cl | H | 2,6-$F_2$Ph | $CF_3$ |
| Me | Cl | H | Me | Et | Me | Cl | H | 2,4-$F_2$Ph | $CF_3$ |
| Me | Cl | H | Me | $SO_2Me$ | Me | Cl | H | 2,5-$F_2$Ph | $CF_3$ |
| Me | Cl | H | Me | $SO_2CF_3$ | Me | Cl | H | 2-MeOPh | $CF_3$ |
| Me | Cl | H | Et | $CF_3$ | Me | Cl | H | 3-F-2-pyridyl | $CF_3$ |
| Me | Cl | H | Et | Br | Me | Cl | H | 3-$CF_3$-2-pyridyl | $CF_3$ |
| Me | Cl | H | Et | Cl | Me | Cl | H | 3-Me-2-pyridyl | $CF_3$ |
| Me | Cl | H | Ph | $CF_3$ | Et | Cl | H | H | $OCF_3$ |
| Me | Cl | H | Ph | Br | Et | Cl | H | H | $C_2F_5$ |
| Me | Cl | H | Ph | Cl | Et | Cl | H | Me | $CF_3$ |
| Me | Cl | H | 2-pyridyl | $CF_3$ | Et | Cl | H | Me | $C_2F_5$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Cl | H | 2-pyridyl | Cl | *i*-Pr | Cl | H | Me | $CF_3$ |

Table 4

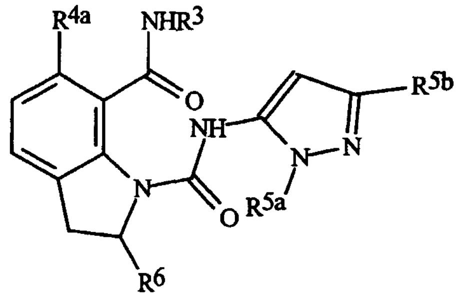

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CF_3$ | Me | H | H | H | $CF_3$ |
| Me | H | Me | H | $OCF_3$ | Me | H | H | H | $OCF_3$ |
| Me | H | Me | Me | $CF_3$ | Me | H | H | Me | $CF_3$ |
| Me | H | Me | Me | $OCF_3$ | Me | H | H | Me | $OCF_3$ |
| Me | H | Me | Me | $OCHF_2$ | Me | H | H | Me | $OCHF_2$ |
| Me | H | Me | Me | $C_2F_5$ | Me | H | H | Me | $C_2F_5$ |
| Me | H | Me | Me | $OCF_2CHF_2$ | Me | H | H | Me | $OCF_2CHF_2$ |
| Me | H | Me | Me | $SCF_2CHF_2$ | Me | H | H | Me | $SCF_2CHF_2$ |
| Me | H | Me | Me | *n*-$C_3F_7$ | Me | H | H | Me | *n*-$C_3F_7$ |
| Me | H | Me | Me | *i*-$C_3F_7$ | Me | H | H | Me | *i*-$C_3F_7$ |
| Me | H | Me | Me | Br | Me | H | H | Me | Br |
| Me | H | Me | Me | Cl | Me | H | H | Me | Cl |
| Me | H | Me | Me | $SCF_3$ | Me | H | H | Me | $SCF_3$ |
| Me | H | Me | Me | I | Me | H | H | Me | I |
| Me | H | Me | Me | SMe | Me | H | H | Me | SMe |
| Me | H | Me | Me | OMe | Me | H | H | Me | OMe |
| Me | H | Me | Me | OEt | Me | H | H | Me | OEt |
| Me | H | Me | Me | Et | Me | H | H | Me | Et |
| Me | H | Me | Me | $SO_2Me$ | Me | H | H | Me | $SO_2Me$ |
| Me | H | Me | Me | $SO_2CF_3$ | Me | H | H | Me | $SO_2CF_3$ |
| Me | H | Me | Et | $CF_3$ | Me | H | H | Et | $CF_3$ |
| Me | H | Me | Et | Br | Me | H | H | Et | Br |
| Me | H | Me | Et | Cl | Me | H | H | Et | Cl |
| Me | H | Me | Ph | $CF_3$ | Me | H | H | Ph | $CF_3$ |
| Me | H | Me | Ph | Br | Me | H | H | Ph | Br |
| Me | H | Me | Ph | Cl | Me | H | H | Ph | Cl |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | 2-pyridyl | $CF_3$ | Me | H | H | 2-pyridyl | $CF_3$ |
| Me | H | Me | 2-pyridyl | Cl | Me | H | H | 2-pyridyl | Cl |
| Me | H | Me | 2-ClPh | $CF_3$ | Me | H | H | 2-ClPh | $CF_3$ |
| Me | H | Me | 2-ClPh | $OCF_3$ | Me | H | H | 2-ClPh | $OCF_3$ |
| Me | H | Me | 2-ClPh | $SCHF_2$ | Me | H | H | 2-ClPh | $SCHF_2$ |
| Me | H | Me | 2-ClPh | Br | Me | H | H | 2-ClPh | Br |
| Me | H | Me | 2-ClPh | Cl | Me | H | H | 2-ClPh | Cl |
| Me | H | Me | 3-Cl-2-pyridyl | $CF_3$ | Me | H | H | 3-Cl-2-pyridyl | $CF_3$ |
| Me | H | Me | 3-Cl-2-pyridyl | $OCF_3$ | Me | H | H | 3-Cl-2-pyridyl | $OCF_3$ |
| Me | H | Me | 3-Cl-2-pyridyl | $SCHF_2$ | Me | H | H | 3-Cl-2-pyridyl | $SCHF_2$ |
| Me | H | Me | 3-Cl-2-pyridyl | Br | Me | H | H | 3-Cl-2-pyridyl | Br |
| Me | H | Me | 3-Cl-2-pyridyl | Cl | Me | H | H | 3-Cl-2-pyridyl | Cl |
| Me | H | Me | 3-Br-2-pyridyl | $CF_3$ | Me | H | H | 3-Br-2-pyridyl | $CF_3$ |
| Me | H | Me | 3-Br-2-pyridyl | $OCF_3$ | Me | H | H | 3-Br-2-pyridyl | $OCF_3$ |
| Me | H | Me | 3-Br-2-pyridyl | Br | Me | H | H | 3-Br-2-pyridyl | Br |
| Me | H | Me | 3-Br-2-pyridyl | Cl | Me | H | H | 3-Br-2-pyridyl | Cl |
| Me | H | Me | $CF_3$ | Me | Me | H | H | $CF_3$ | Me |
| Me | H | Me | *n*-Pr | $CF_3$ | Me | H | H | *n*-Pr | $CF_3$ |
| Me | H | Me | *i*-Pr | $CF_3$ | Me | H | H | *i*-Pr | $CF_3$ |
| Me | H | Me | $CF_3$ | $CF_3$ | Me | H | H | $CF_3$ | $CF_3$ |
| Me | H | Me | OMe | $CF_3$ | Me | H | H | OMe | $CF_3$ |
| Me | H | Me | 2-BrPh | $CF_3$ | Me | H | H | 2-BrPh | $CF_3$ |
| Me | H | Me | 2-MePh | $CF_3$ | Me | H | H | 2-MePh | $CF_3$ |
| Me | H | Me | 2-CNPh | $CF_3$ | Me | H | H | 2-CNPh | $CF_3$ |
| Me | H | Me | 2-FPh | $CF_3$ | Me | H | H | 2-FPh | $CF_3$ |
| Me | H | Me | 2,6-$F_2$Ph | $CF_3$ | Me | H | H | 2,6-$F_2$Ph | $CF_3$ |
| Me | H | Me | 2,4-$F_2$Ph | $CF_3$ | Me | H | H | 2,4-$F_2$Ph | $CF_3$ |
| Me | H | Me | 2,5-$F_2$Ph | $CF_3$ | Me | H | H | 2,5-$F_2$Ph | $CF_3$ |
| Me | H | Me | 2-MeOPh | $CF_3$ | Me | H | H | 2-MeOPh | $CF_3$ |
| Me | H | Me | 3-F-2-pyridyl | $CF_3$ | Me | H | H | 3-F-2-pyridyl | $CF_3$ |
| Me | H | Me | 3-$CF_3$-2-pyridyl | $CF_3$ | Me | H | H | 3-$CF_3$-2-pyridyl | $CF_3$ |
| Me | H | Me | 3-Me-2-pyridyl | $CF_3$ | Me | H | H | 3-Me-2-pyridyl | $CF_3$ |
| Et | H | Me | Me | $CF_3$ | Et | H | H | Me | $CF_3$ |
| Et | H | Me | Me | $C_2F_5$ | Et | H | H | Me | $C_2F_5$ |
| Et | H | Me | Me | $OCF_3$ | Et | H | H | Me | $OCF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | Me | $C_2F_5$ | *i*-Pr | H | H | Me | $C_2F_5$ |
| Me | Me | H | Me | $OCF_2CHF_2$ | *i*-Pr | H | H | Me | $OCF_2CHF_2$ |
| Me | Me | H | Me | $SCF_2CHF_2$ | *i*-Pr | H | H | Me | $SCF_2CHF_2$ |
| Me | Me | H | Me | *n*-$C_3F_7$ | *i*-Pr | H | H | Me | *n*-$C_3F_7$ |
| Me | Me | H | Me | *i*-$C_3F_7$ | *i*-Pr | H | H | Me | *i*-$C_3F_7$ |
| Me | Me | H | Me | Br | *i*-Pr | H | H | Me | Br |
| Me | Me | H | Me | Cl | *i*-Pr | H | H | Me | Cl |
| Me | Me | H | Me | $SCF_3$ | *i*-Pr | H | H | Me | $SCF_3$ |
| Me | Me | H | Me | I | *i*-Pr | H | H | Me | I |
| Me | Me | H | Me | SMe | *i*-Pr | H | H | Me | SMe |
| Me | Me | H | Me | OMe | *i*-Pr | H | H | Me | OMe |
| Me | Me | H | Me | OEt | *i*-Pr | H | H | Me | OEt |
| Me | Me | H | Me | Et | *i*-Pr | H | H | Me | Et |
| Me | Me | H | Me | $SO_2Me$ | *i*-Pr | H | H | Me | $SO_2Me$ |
| Me | Me | H | Me | $SO_2CF_3$ | *i*-Pr | H | H | Me | $SO_2CF_3$ |
| Me | Me | H | Et | $CF_3$ | *i*-Pr | H | H | Et | $CF_3$ |
| Me | Me | H | Et | Br | *i*-Pr | H | H | Et | Br |
| Me | Me | H | Et | Cl | *i*-Pr | H | H | Et | Cl |
| Me | Me | H | Ph | $CF_3$ | *i*-Pr | H | H | Ph | $CF_3$ |
| Me | Me | H | Ph | Br | *i*-Pr | H | H | Ph | Br |
| Me | Me | H | Ph | Cl | *i*-Pr | H | H | Ph | Cl |
| Me | Me | H | 2-pyridyl | $CF_3$ | *i*-Pr | H | H | 2-pyridyl | $CF_3$ |
| Me | Me | H | 2-pyridyl | Cl | *i*-Pr | H | H | 2-pyridyl | Cl |
| Me | Me | H | 2-ClPh | $CF_3$ | *i*-Pr | H | H | 2-ClPh | $CF_3$ |
| Me | Me | H | 2-ClPh | $OCF_3$ | *i*-Pr | H | H | 2-ClPh | $OCF_3$ |
| Me | Me | H | 2-ClPh | $SCHF_2$ | *i*-Pr | H | H | 2-ClPh | $SCHF_2$ |
| Me | Me | H | 2-ClPh | Br | *i*-Pr | H | H | 2-ClPh | Br |
| Me | Me | H | 2-ClPh | Cl | *i*-Pr | H | H | 2-ClPh | Cl |
| Me | Me | H | 3-Cl-2-pyridyl | $CF_3$ | *i*-Pr | H | H | 3-Cl-2-pyridyl | $CF_3$ |
| Me | Me | H | 3-Cl-2-pyridyl | $OCF_3$ | *i*-Pr | H | H | 3-Cl-2-pyridyl | $OCF_3$ |
| Me | Me | H | 3-Cl-2-pyridyl | $SCHF_2$ | *i*-Pr | H | H | 3-Cl-2-pyridyl | $SCHF_2$ |
| Me | Me | H | 3-Cl-2-pyridyl | Br | *i*-Pr | H | H | 3-Cl-2-pyridyl | Br |
| Me | Me | H | 3-Cl-2-pyridyl | Cl | *i*-Pr | H | H | 3-Cl-2-pyridyl | Cl |
| Me | Me | H | 3-Br-2-pyridyl | $CF_3$ | *i*-Pr | H | H | 3-Br-2-pyridyl | $CF_3$ |
| Me | Me | H | 3-Br-2-pyridyl | $OCF_3$ | *i*-Pr | H | H | 3-Br-2-pyridyl | $OCF_3$ |
| Me | Me | H | 3-Br-2-pyridyl | Br | *i*-Pr | H | H | 3-Br-2-pyridyl | Br |
| Me | Me | H | 3-Br-2-pyridyl | Cl | *i*-Pr | H | H | 3-Br-2-pyridyl | Cl |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | $CF_3$ | Me | *i*-Pr | H | H | $CF_3$ | Me |
| Me | Me | H | *n*-Pr | $CF_3$ | *i*-Pr | H | H | *n*-Pr | $CF_3$ |
| Me | Me | H | *i*-Pr | $CF_3$ | *i*-Pr | H | H | *i*-Pr | $CF_3$ |
| Me | Me | H | $CF_3$ | $CF_3$ | *i*-Pr | H | H | $CF_3$ | $CF_3$ |
| Me | Me | H | 2-BrPh | $CF_3$ | *i*-Pr | H | H | 2-BrPh | $CF_3$ |
| Me | Me | H | 2-MePh | $CF_3$ | *i*-Pr | H | H | 2-MePh | $CF_3$ |
| Me | Me | H | 2-CNPh | $CF_3$ | *i*-Pr | H | H | 2-CNPh | $CF_3$ |
| Me | Me | H | 2-FPh | $CF_3$ | *i*-Pr | H | H | 2-FPh | $CF_3$ |
| Me | Me | H | 2,6-$F_2$Ph | $CF_3$ | *i*-Pr | H | H | 2,6-$F_2$Ph | $CF_3$ |
| Me | Me | H | 2,4-$F_2$Ph | $CF_3$ | *i*-Pr | H | H | 2,4-$F_2$Ph | $CF_3$ |
| Me | Me | H | 2,5-$F_2$Ph | $CF_3$ | *i*-Pr | H | H | 2,5-$F_2$Ph | $CF_3$ |
| Me | Me | H | 2-MeOPh | $CF_3$ | *i*-Pr | H | H | 2-MeOPh | $CF_3$ |
| Me | Me | H | 3-F-2-pyridyl | $CF_3$ | *i*-Pr | H | H | 3-F-2-pyridyl | $CF_3$ |
| Me | Me | H | 3-$CF_3$-2-pyridyl | $CF_3$ | *i*-Pr | H | H | 3-$CF_3$-2-pyridyl | $CF_3$ |
| Me | Me | H | 3-Me-2-pyridyl | $CF_3$ | *i*-Pr | H | H | 3-Me-2-pyridyl | $CF_3$ |
| Et | Me | H | Me | $CF_3$ | *i*-Pr | Me | H | Me | $CF_3$ |
| Et | Me | H | Me | $C_2F_5$ | *i*-Pr | Me | H | Me | $C_2F_5$ |
| Et | Me | H | Me | $OCF_3$ | *i*-Pr | Me | H | Me | $OCF_3$ |
| Me | Cl | H | Me | $CF_3$ | Me | Cl | H | 2-ClPh | Br |
| Me | Cl | H | Me | $OCF_3$ | Me | Cl | H | 2-ClPh | Cl |
| Me | Cl | H | Me | $OCHF_2$ | Me | Cl | H | 3-Cl-2-pyridyl | $CF_3$ |
| Me | Cl | H | Me | $C_2F_5$ | Me | Cl | H | 3-Cl-2-pyridyl | $OCF_3$ |
| Me | Cl | H | Me | $OCF_2CHF_2$ | Me | Cl | H | 3-Cl-2-pyridyl | $SCHF_2$ |
| Me | Cl | H | Me | $SCF_2CHF_2$ | Me | Cl | H | 3-Cl-2-pyridyl | Br |
| Me | Cl | H | Me | *n*-$C_3F_7$ | Me | Cl | H | 3-Cl-2-pyridyl | Cl |
| Me | Cl | H | Me | *i*-$C_3F_7$ | Me | Cl | H | 3-Br-2-pyridyl | $CF_3$ |
| Me | Cl | H | Me | Br | Me | Cl | H | 3-Br-2-pyridyl | $OCF_3$ |
| Me | Cl | H | Me | Cl | Me | Cl | H | 3-Br-2-pyridyl | Br |
| Me | Cl | H | Me | $SCF_3$ | Me | Cl | H | 3-Br-2-pyridyl | Cl |
| Me | Cl | H | Me | I | Me | Cl | H | $CF_3$ | Me |
| Me | Cl | H | Me | SMe | Me | Cl | H | *n*-Pr | $CF_3$ |
| Me | Cl | H | Me | OMe | Me | Cl | H | *i*-Pr | $CF_3$ |
| Me | Cl | H | Me | OEt | Me | Cl | H | $CF_3$ | $CF_3$ |
| Me | Cl | H | Me | Et | Me | Cl | H | 2-BrPh | $CF_3$ |
| Me | Cl | H | Me | $SO_2Me$ | Me | Cl | H | 2-MePh | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Cl | H | Me | $SO_2CF_3$ | Me | Cl | H | 2-CNPh | $CF_3$ |
| Me | Cl | H | Et | $CF_3$ | Me | Cl | H | 2-FPh | $CF_3$ |
| Me | Cl | H | Et | Br | Me | Cl | H | 2,6-$F_2$Ph | $CF_3$ |
| Me | Cl | H | Et | Cl | Me | Cl | H | 2,4-$F_2$Ph | $CF_3$ |
| Me | Cl | H | Ph | $CF_3$ | Me | Cl | H | 2,5-$F_2$Ph | $CF_3$ |
| Me | Cl | H | Ph | Br | Me | Cl | H | 2-MeOPh | $CF_3$ |
| Me | Cl | H | Ph | Cl | Me | Cl | H | 3-F-2-pyridyl | $CF_3$ |
| Me | Cl | H | 2-pyridyl | $CF_3$ | Me | Cl | H | 3-$CF_3$-2-pyridyl | $CF_3$ |
| Me | Cl | H | 2-pyridyl | Cl | Me | Cl | H | 3-Me-2-pyridyl | $CF_3$ |
| Me | Cl | H | 2-ClPh | $CF_3$ | Et | Cl | H | Me | $CF_3$ |
| Me | Cl | H | 2-ClPh | $OCF_3$ | Et | Cl | H | Me | $C_2F_5$ |
| Me | Cl | H | 2-ClPh | $SCHF_2$ | *i*-Pr | Cl | H | Me | $CF_3$ |

Table 5

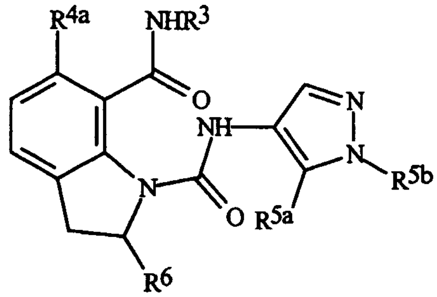

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CHF_2$ | Me | H | H | H | $CHF_2$ |
| Me | H | Me | H | $CH_2CF_3$ | Me | H | H | H | $CH_2CF_3$ |
| Et | H | Me | H | $CH_2CF_3$ | Et | H | H | H | $CH_2CF_3$ |
| Me | H | Me | Me | $CH_2CF_3$ | Me | H | H | Me | $CH_2CF_3$ |
| Me | H | Me | Et | $CH_2CF_3$ | Me | H | H | Et | $CH_2CF_3$ |
| Me | H | Me | Me | $CF_2CHF_2$ | Me | H | H | Me | $CF_2CHF_2$ |
| Me | H | Me | Et | $CHF_2$ | Me | H | H | Et | $CHF_2$ |
| Me | H | Me | Me | $CHF_2$ | Me | H | H | Me | $CHF_2$ |
| Me | H | Me | Me | $CBrF_2$ | Me | H | H | Me | $CBrF_2$ |
| Me | H | Me | Me | $CHF_2$ | Me | H | H | Me | $CHF_2$ |
| Et | H | Me | Me | $CH_2CF_3$ | Et | H | H | Me | $CH_2CF_3$ |
| Me | H | Me | Me | Et | Me | H | H | Me | Et |
| Me | H | Me | Me | *n*-Pr | Me | H | H | Me | *n*-Pr |
| Me | H | Me | Me | $CH_2C_2F_5$ | Me | H | H | Me | $CH_2C_2F_5$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| *n*-Pr | H | Me | Me | $CH_2CF_3$ | *n*-Pr | H | H | Me | $CH_2CF_3$ |
| Me | H | Me | Me | $CF_3$ | Me | H | H | Me | $CF_3$ |
| Et | H | Me | Me | $C_2F_5$ | Et | H | H | Me | $C_2F_5$ |
| Me | H | Me | Et | $CHF_2$ | Me | H | H | Et | $CHF_2$ |
| Me | H | Me | *n*-Pr | $CH_2CF_3$ | Me | H | H | *n*-Pr | $CH_2CF_3$ |
| Me | H | Me | *i*-Pr | $CHF_2$ | Me | H | H | *i*-Pr | $CHF_2$ |
| Me | H | Me | Cl | $CH_2CF_3$ | Me | H | H | Cl | $CH_2CF_3$ |
| Me | H | Me | F | $CH_2CF_3$ | Me | H | H | F | $CH_2CF_3$ |
| Me | H | Me | Me | $CH_2Cl$ | Me | H | H | Me | $CH_2Cl$ |
| Me | H | Me | Me | $CClF_2$ | Me | H | H | Me | $CClF_2$ |
| Me | H | Me | Me | $CH_2CH_2Cl$ | Me | H | H | Me | $CH_2CH_2Cl$ |
| Me | H | Me | Me | *n*-$C_3F_7$ | Me | H | H | Me | *n*-$C_3F_7$ |
| Me | H | Me | Me | *i*-$C_3F_7$ | Me | H | H | Me | *i*-$C_3F_7$ |
| Me | H | Me | Me | Allyl | Me | H | H | Me | Allyl |
| Me | H | Me | Et | $CF_2CHF_2$ | Me | H | H | Me | $CF_2CHF_2$ |
| Me | H | Me | Et | *i*-$C_3F_7$ | Me | H | H | Me | *i*-$C_3F_7$ |
| Me | H | Me | *i*-Pr | $CF_2CHF_2$ | Me | H | H | Me | $CF_2CHF_2$ |
| Me | H | Me | *n*-Pr | $CF_2CHF_2$ | Me | H | H | Me | $CF_2CHF_2$ |
| Me | H | Me | $CF_3$ | $CF_2CHF_2$ | Me | H | H | $CF_3$ | $CF_2CHF_2$ |
| Me | H | Me | $CF_3$ | Me | Me | H | H | $CF_3$ | Me |
| Me | H | Me | OMe | $CH_2CF_3$ | Me | H | H | OMe | $CH_2CF_3$ |
| Me | H | Me | H | $CH_2CF_3$ | Me | H | H | H | $CH_2CF_3$ |
| Me | H | Me | H | $CH_2CF_3$ | Me | H | H | H | $CH_2CF_3$ |
| Me | H | Me | H | $C_2F_5$ | Me | H | H | H | $C_2F_5$ |
| Et | H | Me | H | $C_2F_5$ | Et | H | H | H | $C_2F_5$ |
| Me | H | Me | H | $C_2F_5$ | Me | H | H | H | $C_2F_5$ |
| Me | H | Me | H | $CF_2CHF_2$ | Me | H | H | H | $CF_2CHF_2$ |
| Me | H | Me | *i*-Pr | $CH_2CF_3$ | Me | H | H | H | $CH_2CF_3$ |
| Me | H | Me | H | *n*-$C_3F_7$ | Me | H | H | H | *n*-$C_3F_7$ |
| Me | H | Me | H | *i*-$C_3F_7$ | Me | H | H | H | *i*-$C_3F_7$ |
| Me | H | Me | Ph | $CH_2CF_3$ | Me | H | H | H | $CH_2CF_3$ |
| Me | H | Me | Ph | $CF_2CHF_2$ | Me | H | H | H | $CF_2CHF_2$ |
| Me | H | Me | Ph | $CHF_2$ | Me | H | H | H | $CHF_2$ |
| Me | H | Me | 2-pyridyl | $CH_2CF_3$ | Me | H | H | Ph | $CH_2CF_3$ |
| Me | H | Me | 2-pyridyl | $CF_2CHF_2$ | Me | H | H | Ph | $CF_2CHF_2$ |
| Me | H | Me | 2-ClPh | $CH_2CF_3$ | Me | H | H | Ph | $CH_2CF_3$ |
| Me | H | Me | 2-ClPh | $CF_2CHF_2$ | Me | H | H | 2-pyridyl | $CF_2CHF_2$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | 2-ClPh | $CHF_2$ | Me | H | H | 2-pyridyl | $CHF_2$ |
| Me | H | Me | 2-ClPh | Et | Me | H | H | 2-ClPh | Et |
| Me | H | Me | 2-ClPh | $CBrF_2$ | Me | H | H | 2-ClPh | $CBrF_2$ |
| Me | H | Me | 2-BrPh | $CH_2CF_3$ | Me | H | H | 2-ClPh | $CH_2CF_3$ |
| Me | H | Me | 2-MePh | $CH_2CF_3$ | Me | H | H | 2-ClPh | $CH_2CF_3$ |
| Me | H | Me | 2-CNPh | $CH_2CF_3$ | Me | H | H | 2-ClPh | $CH_2CF_3$ |
| Me | H | Me | 2-FPh | $CH_2CF_3$ | Me | H | H | 2-BrPh | $CH_2CF_3$ |
| Me | H | Me | 2,6-$F_2$Ph | $CH_2CF_3$ | Me | H | H | 2-MePh | $CH_2CF_3$ |
| Me | H | Me | 2,4-$F_2$Ph | $CH_2CF_3$ | Me | H | H | 2-CNPh | $CH_2CF_3$ |
| Me | H | Me | 2,5-$F_2$Ph | $CH_2CF_3$ | Me | H | H | 2-FPh | $CH_2CF_3$ |
| Me | H | Me | 2-MeOPh | $CH_2CF_3$ | Me | H | H | 2,6-$F_2$Ph | $CH_2CF_3$ |
| Me | H | Me | 3-Cl-2-pyridyl | $CH_2CF_3$ | Me | H | H | 2,4-$F_2$Ph | $CH_2CF_3$ |
| Me | H | Me | 3-Cl-2-pyridyl | $CF_2CHF_2$ | Me | H | H | 2,5-$F_2$Ph | $CF_2CHF_2$ |
| Me | H | Me | 3-Cl-2-pyridyl | $CF_3$ | Me | H | H | 2-MeOPh | $CF_3$ |
| Me | H | Me | 3-Cl-2-pyridyl | $CHF_2$ | Me | H | H | 3-Cl-2-pyridyl | $CHF_2$ |
| Me | H | Me | 3-Cl-2-pyridyl | $CBrF_2$ | Me | H | H | 3-Cl-2-pyridyl | $CBrF_2$ |
| Me | H | Me | 3-F-2-pyridyl | $CH_2CF_3$ | Me | H | H | 3-Cl-2-pyridyl | $CH_2CF_3$ |
| Me | H | Me | 3-$CF_3$-2-pyridyl | $CH_2CF_3$ | Me | H | H | 3-$CF_3$-2-pyridyl | $CH_2CF_3$ |
| Me | H | Me | 3-Me-2-pyridyl | $CH_2CF_3$ | Me | H | H | 3-Me-2-pyridyl | $CF_2CHF_2$ |
| Me | H | Me | 3-Br-2-pyridyl | $CF_3$ | Me | H | H | 3-F-2-pyridyl | $CF_3$ |
| Me | H | Me | 3-Br-2-pyridyl | $CH_2CF_3$ | Me | H | H | 3-Cl-2-pyridyl | $CH_2CF_3$ |
| Me | H | Me | 3-Br-2-pyridyl | $CF_2CHF_2$ | Me | H | H | 3-Cl-2-pyridyl | $CH_2CF_3$ |
| Me | H | Me | 3-Br-2-pyridyl | $CClF_2$ | Me | H | H | 3-Br-2-pyridyl | $CClF_2$ |
| Me | Me | H | H | $CHF_2$ | Me | H | H | 3-Br-2-pyridyl | $CH_2CF_3$ |
| Me | Me | H | H | $CH_2CF_3$ | Me | H | H | 3-Br-2-pyridyl | $CF_3$ |
| Et | Me | H | H | $CH_2CF_3$ | Me | H | H | 3-Br-2-pyridyl | $CF_3$ |
| Me | Me | H | Me | $CH_2CF_3$ | *i*-Pr | H | H | Et | $CH_2C_2F_5$ |
| Me | Me | H | Et | $CH_2CF_3$ | *i*-Pr | H | H | *n*-Pr | $CH_2CF_3$ |
| Me | Me | H | Me | $CF_2CHF_2$ | *i*-Pr | H | H | *i*-Pr | $CF_3$ |
| Me | Me | H | Et | $CHF_2$ | *i*-Pr | H | H | Cl | $C_2F_5$ |
| Me | Me | H | Me | $CHF_2$ | *i*-Pr | H | H | F | $CHF_2$ |
| Me | Me | H | Me | $CBrF_2$ | *i*-Pr | H | H | Me | $CH_2CF_3$ |
| Me | Me | H | Me | $CHF_2$ | *i*-Pr | H | H | Me | $CHF_2$ |
| Et | Me | H | Me | $CH_2CF_3$ | *i*-Pr | H | H | Me | $CH_2CF_3$ |
| Me | Me | H | Me | Et | *i*-Pr | H | H | Me | $CH_2CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | Me | *n*-Pr | *i*-Pr | H | H | Me | $CH_2Cl$ |
| Me | Me | H | Me | $CH_2C_2F_5$ | *i*-Pr | H | H | Me | $CClF_2$ |
| *n*-Pr | Me | H | Me | $CH_2CF_3$ | *i*-Pr | H | H | Me | $CH_2CH_2Cl$ |
| Me | Me | H | Me | $CF_3$ | *i*-Pr | H | H | Me | *n*-$C_3F_7$ |
| Et | Me | H | Me | $C_2F_5$ | *i*-Pr | H | H | Me | *i*-$C_3F_7$ |
| Me | Me | H | Et | $CHF_2$ | *i*-Pr | H | H | Me | Allyl |
| Me | Me | H | *n*-Pr | $CH_2CF_3$ | *i*-Pr | H | H | $CF_3$ | $CF_2CHF_2$ |
| Me | Me | H | *i*-Pr | $CHF_2$ | *i*-Pr | H | H | $CF_3$ | *i*-$C_3F_7$ |
| Me | Me | H | Cl | $CH_2CF_3$ | *i*-Pr | H | H | OMe | $CF_2CHF_2$ |
| Me | Me | H | F | $CH_2CF_3$ | *i*-Pr | H | H | H | $CF_2CHF_2$ |
| Me | Me | H | Me | $CH_2Cl$ | *i*-Pr | H | H | H | $CF_2CHF_2$ |
| Me | Me | H | Me | $CClF_2$ | *i*-Pr | H | H | H | Me |
| Me | Me | H | Me | $CH_2CH_2Cl$ | *i*-Pr | H | H | H | $CH_2CF_3$ |
| Me | Me | H | Me | *n*-$C_3F_7$ | *i*-Pr | H | H | H | $CH_2CF_3$ |
| Me | Me | H | Me | *i*-$C_3F_7$ | *i*-Pr | H | H | H | $C_2F_5$ |
| Me | Me | H | Me | Allyl | *i*-Pr | H | H | H | $C_2F_5$ |
| Me | Me | H | Me | $CF_2CHF_2$ | *i*-Pr | H | H | H | $C_2F_5$ |
| Me | Me | H | Me | *i*-$C_3F_7$ | *i*-Pr | H | H | H | $CF_2CHF_2$ |
| Me | Me | H | Me | $CF_2CHF_2$ | *i*-Pr | H | H | H | $CH_2CF_3$ |
| Me | Me | H | Me | $CF_2CHF_2$ | *i*-Pr | H | H | H | *n*-$C_3F_7$ |
| Me | Me | H | $CF_3$ | $CF_2CHF_2$ | *i*-Pr | H | H | Ph | *i*-$C_3F_7$ |
| Me | Me | H | $CF_3$ | Me | *i*-Pr | H | H | Ph | $CH_2CF_3$ |
| Me | Me | H | OMe | $CH_2CF_3$ | *i*-Pr | H | H | Ph | $CF_2CHF_2$ |
| Me | Me | H | H | $CH_2CF_3$ | *i*-Pr | H | H | 2-pyridyl | $CHF_2$ |
| Me | Me | H | H | $CH_2CF_3$ | *i*-Pr | H | H | 2-pyridyl | $CH_2CF_3$ |
| Me | Me | H | H | $C_2F_5$ | *i*-Pr | H | H | 2-ClPh | $CF_2CHF_2$ |
| Et | Me | H | H | $C_2F_5$ | *i*-Pr | H | H | 2-ClPh | $CH_2CF_3$ |
| Me | Me | H | H | $C_2F_5$ | *i*-Pr | H | H | 2-ClPh | $CF_2CHF_2$ |
| Me | Me | H | H | $CF_2CHF_2$ | *i*-Pr | H | H | 2-ClPh | $CHF_2$ |
| Me | Me | H | H | $CH_2CF_3$ | *i*-Pr | H | H | 2-ClPh | Et |
| Me | Me | H | H | *n*-$C_3F_7$ | *i*-Pr | H | H | 2-BrPh | $CBrF_2$ |
| Me | Me | H | H | *i*-$C_3F_7$ | *i*-Pr | H | H | 2-MePh | $CH_2CF_3$ |
| Me | Me | H | H | $CH_2CF_3$ | *i*-Pr | H | H | 2-CNPh | $CH_2CF_3$ |
| Me | Me | H | H | $CF_2CHF_2$ | *i*-Pr | H | H | 2-FPh | $CH_2CF_3$ |
| Me | Me | H | H | $CHF_2$ | *i*-Pr | H | H | 2,6-$F_2$Ph | $CH_2CF_3$ |
| Me | Me | H | Ph | $CH_2CF_3$ | *i*-Pr | H | H | 2,4-$F_2$Ph | $CH_2CF_3$ |
| Me | Me | H | Ph | $CF_2CHF_2$ | *i*-Pr | H | H | 2,5-$F_2$Ph | $CH_2CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | Ph | $CH_2CF_3$ | *i*-Pr | H | H | 2-MeOPh | $CH_2CF_3$ |
| Me | Me | H | 2-pyridyl | $CF_2CHF_2$ | *i*-Pr | H | H | 3-Cl-2-pyridyl | $CH_2CF_3$ |
| Me | Me | H | 2-pyridyl | $CHF_2$ | *i*-Pr | H | H | 3-Cl-2-pyridyl | $CH_2CF_3$ |
| Me | Me | H | 2-ClPh | Et | *i*-Pr | H | H | 3-Cl-2-pyridyl | $CF_2CHF_2$ |
| Me | Me | H | 2-ClPh | $CBrF_2$ | *i*-Pr | H | H | 3-Cl-2-pyridyl | $CF_3$ |
| Me | Me | H | 2-ClPh | $CH_2CF_3$ | *i*-Pr | H | H | 3-Cl-2-pyridyl | $CHF_2$ |
| Me | Me | H | 2-ClPh | $CH_2CF_3$ | *i*-Pr | H | H | 3-F-2-pyridyl | $CBrF_2$ |
| Me | Me | H | 2-ClPh | $CH_2CF_3$ | *i*-Pr | H | H | 3-Br-2-pyridyl | $CH_2CF_3$ |
| Me | Me | H | 2-BrPh | $CH_2CF_3$ | *i*-Pr | H | H | 3-$CF_3$-2-pyridyl | $CH_2CF_3$ |
| Me | Me | H | 2-MePh | $CH_2CF_3$ | *i*-Pr | H | H | 3-Me-2-pyridyl | $CH_2CF_3$ |
| Me | Me | H | 2-CNPh | $CH_2CF_3$ | *i*-Pr | H | H | 3-Br-2-pyridyl | $CF_3$ |
| Me | Me | H | 2-FPh | $CH_2CF_3$ | *i*-Pr | H | H | 3-Br-2-pyridyl | $CH_2CF_3$ |
| Me | Me | H | 2,6-$F_2$Ph | $CH_2CF_3$ | *i*-Pr | H | H | 3-Br-2-pyridyl | $CF_2CHF_2$ |
| Me | Me | H | 2,4-$F_2$Ph | $CH_2CF_3$ | Me | Cl | H | F | $CH_2CF_3$ |
| Me | Me | H | 2,5-$F_2$Ph | $CF_2CHF_2$ | Me | Cl | H | Me | $CHF_2$ |
| Me | Me | H | 2-MeOPh | $CF_3$ | Me | Cl | H | Me | $CH_2CF_3$ |
| Me | Me | H | 3-Cl-2-pyridyl | $CHF_2$ | Me | Cl | H | Me | $CH_2CF_3$ |
| Me | Me | H | 3-Cl-2-pyridyl | $CBrF_2$ | Me | Cl | H | Me | $CH_2Cl$ |
| Me | Me | H | 3-Cl-2-pyridyl | $CH_2CF_3$ | Me | Cl | H | Me | $CClF_2$ |
| Me | Me | H | 3-Cl-2-pyridyl | $CH_2CF_3$ | Me | Cl | H | Me | $CH_2CH_2Cl$ |
| Me | Me | H | 3-Cl-2-pyridyl | $CH_2CF_3$ | Me | Cl | H | Me | *n*-$C_3F_7$ |
| Me | Me | H | 3-F-2-pyridyl | $CF_3$ | Me | Cl | H | Me | *i*-$C_3F_7$ |
| Me | Me | H | 3-$CF_3$-2-pyridyl | $CH_2CF_3$ | Me | Cl | H | Me | Allyl |
| Me | Me | H | 3-Me-2-pyridyl | $CF_2CHF_2$ | Me | Cl | H | Me | $CF_2CHF_2$ |
| Me | Me | H | 3-Br-2-pyridyl | $CClF_2$ | Me | Cl | H | $CF_3$ | *i*-$C_3F_7$ |
| Me | Me | H | 3-Br-2-pyridyl | $CH_2CF_3$ | Me | Cl | H | $CF_3$ | $CF_2CHF_2$ |
| Me | Me | H | 3-Br-2-pyridyl | $CF_3$ | Me | Cl | H | OMe | $CF_2CHF_2$ |
| Me | Me | H | 3-Br-2-pyridyl | $CF_3$ | Me | Cl | H | H | $CF_2CHF_2$ |
| Me | Cl | H | Me | $CHF_2$ | Me | Cl | H | H | Me |
| Me | Cl | H | Et | $CH_2CF_3$ | Me | Cl | H | H | $CH_2CF_3$ |
| Me | Cl | H | Me | $CH_2CF_3$ | Et | Cl | H | H | $CH_2CF_3$ |
| Me | Cl | H | Et | $CH_2CF_3$ | Me | Cl | H | H | $CH_2CF_3$ |
| Me | Cl | H | Me | $CH_2CF_3$ | Me | Cl | H | H | $C_2F_5$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Cl | H | Me | $CF_2CHF_2$ | Me | Cl | H | H | $C_2F_5$ |
| Me | Cl | H | Me | $CHF_2$ | Me | Cl | H | H | $C_2F_5$ |
| Et | Cl | H | Me | $CHF_2$ | Me | Cl | H | H | $CF_2CHF_2$ |
| Me | Cl | H | Me | $CBrF_2$ | Me | Cl | H | H | $CH_2CF_3$ |
| Me | Cl | H | Me | $CHF_2$ | Me | Cl | H | H | *n*-$C_3F_7$ |
| Me | Cl | H | Me | $CH_2CF_3$ | Me | Cl | H | 3-Me-2-pyridyl | $CH_2CF_3$ |
| *n*-Pr | Cl | H | Me | Et | Me | Cl | H | H | *i*-$C_3F_7$ |
| Me | Cl | H | Me | *n*-Pr | Me | Cl | H | Ph | $CH_2CF_3$ |
| Et | Cl | H | Me | $CH_2C_2F_5$ | Me | Cl | H | Ph | $CF_2CHF_2$ |
| Me | Cl | H | Et | $CH_2CF_3$ | Me | Cl | H | Ph | $CHF_2$ |
| Me | Cl | H | *n*-Pr | $CF_3$ | Me | Cl | H | 2-pyridyl | $CH_2CF_3$ |
| Me | Cl | H | *i*-Pr | $C_2F_5$ | Me | Cl | H | 2-pyridyl | $CF_2CHF_2$ |
| Me | Cl | H | Cl | $CHF_2$ | Me | Cl | H | 2-ClPh | $CH_2CF_3$ |
| Me | Cl | H | 3-Cl-2-pyridyl | $CH_2CF_3$ | Me | Cl | H | 2-ClPh | $CF_2CHF_2$ |
| Me | Cl | H | 3-Cl-2-pyridyl | $CF_2CHF_2$ | Me | Cl | H | 2-FPh | $CH_2CF_3$ |
| Me | Cl | H | 3-Cl-2-pyridyl | $CF_3$ | Me | Cl | H | 2,6-$F_2$Ph | $CH_2CF_3$ |
| Me | Cl | H | 3-Cl-2-pyridyl | $CHF_2$ | Me | Cl | H | 2,4-$F_2$Ph | $CH_2CF_3$ |
| Me | Cl | H | 3-Cl-2-pyridyl | $CBrF_2$ | Me | Cl | H | 2-ClPh | Et |
| Me | Cl | H | 3-F-2-pyridyl | $CH_2CF_3$ | Me | Cl | H | 2-ClPh | $CBrF_2$ |
| Me | Cl | H | 3-$CF_3$-2-pyridyl | $CH_2CF_3$ | Me | Cl | H | 2-BrPh | $CH_2CF_3$ |
| Me | Cl | H | 3-Br-2-pyridyl | $CF_3$ | Me | Cl | H | 2-MePh | $CH_2CF_3$ |
| Me | Cl | H | 3-Br-2-pyridyl | $CH_2CF_3$ | Me | Cl | H | 2-CNPh | $CH_2CF_3$ |
| Me | Cl | H | 3-Br-2-pyridyl | $CF_2CHF_2$ | Me | Cl | H | 2-MeOPh | $CH_2CF_3$ |
| Me | Cl | H | 3-Br-2-pyridyl | $CClF_2$ | Me | Cl | H | 2,5-$F_2$Ph | $CH_2CF_3$ |
| Me | Cl | H | 2-ClPh | $CHF_2$ | | | | | |

Table 6

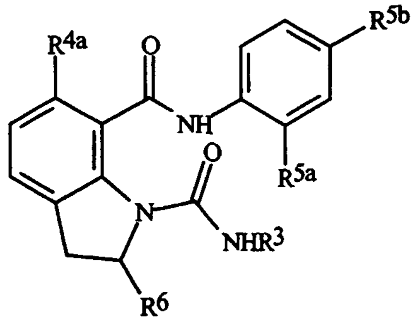

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CF_3$ | Me | H | H | H | $CF_3$ |
| Me | H | Me | H | $OCF_3$ | Me | H | H | H | $OCF_3$ |
| Me | H | Me | H | $OCHF_2$ | Me | H | H | H | $OCHF_2$ |
| Me | H | Me | H | $C_2F_5$ | Me | H | H | H | $C_2F_5$ |
| Me | H | Me | H | $OCF_2CHF_2$ | Me | H | H | H | $OCF_2CHF_2$ |
| Me | H | Me | H | $SCF_2CHF_2$ | Me | H | H | H | $SCF_2CHF_2$ |
| Me | H | Me | H | *n*-$C_3F_7$ | Me | H | H | H | *n*-$C_3F_7$ |
| Me | H | Me | H | *i*-$C_3F_7$ | Me | H | H | H | *i*-$C_3F_7$ |
| Me | H | Me | H | Br | Me | H | H | H | Br |
| Me | H | Me | H | Cl | Me | H | H | H | Cl |
| Me | H | Me | H | $SCF_3$ | Me | H | H | H | $SCF_3$ |
| Me | H | Me | Me | $CF_3$ | Me | H | H | Me | $CF_3$ |
| Me | H | Me | Me | $OCF_3$ | Me | H | H | Me | $OCF_3$ |
| Me | H | Me | Me | $OCHF_2$ | Me | H | H | Me | $OCHF_2$ |
| Me | H | Me | Me | $C_2F_5$ | Me | H | H | Me | $C_2F_5$ |
| Me | H | Me | Me | $OCF_2CHF_2$ | Me | H | H | Me | $OCF_2CHF_2$ |
| Me | H | Me | Me | $SCF_2CHF_2$ | Me | H | H | Me | $SCF_2CHF_2$ |
| Me | H | Me | Me | *n*-$C_3F_7$ | Me | H | H | Me | *n*-$C_3F_7$ |
| Me | H | Me | Me | *i*-$C_3F_7$ | Me | H | H | Me | *i*-$C_3F_7$ |
| Me | H | Me | Me | Br | Me | H | H | Me | Br |
| Me | H | Me | Me | Cl | Me | H | H | Me | Cl |
| Me | H | Me | Me | $SCF_3$ | Me | H | H | Me | $SCF_3$ |
| Me | H | Me | Me | I | Me | H | H | Me | I |
| Me | H | Me | Me | SMe | Me | H | H | Me | SMe |
| Me | H | Me | Me | OMe | Me | H | H | Me | OMe |
| Me | H | Me | Me | OEt | Me | H | H | Me | OEt |
| Me | H | Me | Me | Et | Me | H | H | Me | Et |
| Me | H | Me | Me | $SO_2Me$ | Me | H | H | Me | $SO_2Me$ |
| Me | H | Me | Me | $SO_2CF_3$ | Me | H | H | Me | $SO_2CF_3$ |
| Me | H | Me | Et | $CF_3$ | Me | H | H | Et | $CF_3$ |
| Me | H | Me | Et | Br | Me | H | H | Et | Br |
| Me | H | Me | Et | Cl | Me | H | H | Et | Cl |
| Me | H | Me | Ph | $CF_3$ | Me | H | H | Ph | $CF_3$ |
| Me | H | Me | Ph | Br | Me | H | H | Ph | Br |
| Me | H | Me | Ph | Cl | Me | H | H | Ph | Cl |
| Me | H | Me | 2-pyridyl | $CF_3$ | Me | H | H | 2-pyridyl | $CF_3$ |
| Me | H | Me | 2-pyridyl | Cl | Me | H | H | 2-pyridyl | Cl |

| R3 | R4a | R6 | R5a | R5b | R3 | R4a | R6 | R5a | R5b |
|---|---|---|---|---|---|---|---|---|---|
| Me | H | Me | 2-ClPh | $CF_3$ | Me | H | H | 2-ClPh | $CF_3$ |
| Me | H | Me | 2-ClPh | $OCF_3$ | Me | H | H | 2-ClPh | $OCF_3$ |
| Me | H | Me | 2-ClPh | $SCHF_2$ | Me | H | H | 2-ClPh | $SCHF_2$ |
| Me | H | Me | 2-ClPh | Br | Me | H | H | 2-ClPh | Br |
| Me | H | Me | 2-ClPh | Cl | Me | H | H | 2-ClPh | Cl |
| Me | H | Me | 3-Cl-2-pyridyl | $CF_3$ | Me | H | H | 3-Cl-2-pyridyl | $CF_3$ |
| Me | H | Me | 3-Cl-2-pyridyl | $OCF_3$ | Me | H | H | 3-Cl-2-pyridyl | $OCF_3$ |
| Me | H | Me | 3-Cl-2-pyridyl | $SCHF_2$ | Me | H | H | 3-Cl-2-pyridyl | $SCHF_2$ |
| Me | H | Me | 3-Cl-2-pyridyl | Br | Me | H | H | 3-Cl-2-pyridyl | Br |
| Me | H | Me | 3-Cl-2-pyridyl | Cl | Me | H | H | 3-Cl-2-pyridyl | Cl |
| Me | H | Me | 3-Br-2-pyridyl | $CF_3$ | Me | H | H | 3-Br-2-pyridyl | $CF_3$ |
| Me | H | Me | 3-Br-2-pyridyl | $OCF_3$ | Me | H | H | 3-Br-2-pyridyl | $OCF_3$ |
| Me | H | Me | 3-Br-2-pyridyl | Br | Me | H | H | 3-Br-2-pyridyl | Br |
| Me | H | Me | 3-Br-2-pyridyl | Cl | Me | H | H | 3-Br-2-pyridyl | Cl |
| Me | H | Me | $CF_3$ | Me | Me | H | H | $CF_3$ | Me |
| Me | H | Me | F | $CF_3$ | Me | H | H | F | $CF_3$ |
| Me | H | Me | Cl | $CF_3$ | Me | H | H | Cl | $CF_3$ |
| Me | H | Me | *n*-Pr | $CF_3$ | Me | H | H | *n*-Pr | $CF_3$ |
| Me | H | Me | *i*-Pr | $CF_3$ | Me | H | H | *i*-Pr | $CF_3$ |
| Me | H | Me | $CF_3$ | $CF_3$ | Me | H | H | $CF_3$ | $CF_3$ |
| Me | H | Me | OMe | $CF_3$ | Me | H | H | OMe | $CF_3$ |
| Me | H | Me | 2-BrPh | $CF_3$ | Me | H | H | 2-BrPh | $CF_3$ |
| Me | H | Me | 2-MePh | $CF_3$ | Me | H | H | 2-MePh | $CF_3$ |
| Me | H | Me | 2-CNPh | $CF_3$ | Me | H | H | 2-CNPh | $CF_3$ |
| Me | H | Me | 2-FPh | $CF_3$ | Me | H | H | 2-FPh | $CF_3$ |
| Me | H | Me | 2,6-$F_2$Ph | $CF_3$ | Me | H | H | 2,6-$F_2$Ph | $CF_3$ |
| Me | H | Me | 2,4-$F_2$Ph | $CF_3$ | Me | H | H | 2,4-$F_2$Ph | $CF_3$ |
| Me | H | Me | 2,5-$F_2$Ph | $CF_3$ | Me | H | H | 2,5-$F_2$Ph | $CF_3$ |
| Me | H | Me | 2-MeOPh | $CF_3$ | Me | H | H | 2-MeOPh | $CF_3$ |
| Me | H | Me | 3-F-2-pyridyl | $CF_3$ | Me | H | H | 3-F-2-pyridyl | $CF_3$ |
| Me | H | Me | 3-$CF_3$-2-pyridyl | $CF_3$ | Me | H | H | 3-$CF_3$-2-pyridyl | $CF_3$ |
| Me | H | Me | 3-Me-2-pyridyl | $CF_3$ | Me | H | H | 3-Me-2-pyridyl | $CF_3$ |
| Et | H | Me | H | $CF_3$ | Et | H | H | H | $CF_3$ |
| Et | H | Me | H | $C_2F_5$ | Et | H | H | H | $C_2F_5$ |
| Et | H | Me | H | $OCF_3$ | Et | H | H | H | $OCF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Et | H | Me | Me | $CF_3$ | Et | H | H | Me | $CF_3$ |
| Et | H | Me | Me | $C_2F_5$ | Et | H | H | Me | $C_2F_5$ |
| Et | H | Me | Me | $OCF_3$ | Et | H | H | Me | $OCF_3$ |
| Me | Me | H | H | $CF_3$ | *i*-Pr | H | H | H | $CF_3$ |
| Me | Me | H | H | $OCF_3$ | *i*-Pr | H | H | H | $OCF_3$ |
| Me | Me | H | H | $OCHF_2$ | *i*-Pr | H | H | H | $OCHF_2$ |
| Me | Me | H | H | $C_2F_5$ | *i*-Pr | H | H | H | $C_2F_5$ |
| Me | Me | H | H | $OCF_2CHF_2$ | *i*-Pr | H | H | H | $OCF_2CHF_2$ |
| Me | Me | H | H | $SCF_2CHF_2$ | *i*-Pr | H | H | H | $SCF_2CHF_2$ |
| Me | Me | H | H | *n*-$C_3F_7$ | *i*-Pr | H | H | H | *n*-$C_3F_7$ |
| Me | Me | H | H | *i*-$C_3F_7$ | *i*-Pr | H | H | H | *i*-$C_3F_7$ |
| Me | Me | H | H | Br | *i*-Pr | H | H | H | Br |
| Me | Me | H | H | Cl | *i*-Pr | H | H | H | Cl |
| Me | Me | H | H | $SCF_3$ | *i*-Pr | H | H | H | $SCF_3$ |
| Me | Me | H | H | $CF_3$ | *i*-Pr | H | H | Me | $CF_3$ |
| Me | Me | H | H | $OCF_3$ | *i*-Pr | H | H | Me | $OCF_3$ |
| Me | Me | H | H | $OCHF_2$ | *i*-Pr | H | H | Me | $OCHF_2$ |
| Me | Me | H | Me | $C_2F_5$ | *i*-Pr | H | H | Me | $C_2F_5$ |
| Me | Me | H | Me | $OCF_2CHF_2$ | *i*-Pr | H | H | Me | $OCF_2CHF_2$ |
| Me | Me | H | Me | $SCF_2CHF_2$ | *i*-Pr | H | H | Me | $SCF_2CHF_2$ |
| Me | Me | H | Me | *n*-$C_3F_7$ | *i*-Pr | H | H | Me | *n*-$C_3F_7$ |
| Me | Me | H | Me | *i*-$C_3F_7$ | *i*-Pr | H | H | Me | *i*-$C_3F_7$ |
| Me | Me | H | Me | Br | *i*-Pr | H | H | Me | Br |
| Me | Me | H | Me | Cl | *i*-Pr | H | H | Me | Cl |
| Me | Me | H | Me | $SCF_3$ | *i*-Pr | H | H | Me | $SCF_3$ |
| Me | Me | H | Me | I | *i*-Pr | H | H | Me | I |
| Me | Me | H | Me | SMe | *i*-Pr | H | H | Me | SMe |
| Me | Me | H | Me | OMe | *i*-Pr | H | H | Me | OMe |
| Me | Me | H | Me | OEt | *i*-Pr | H | H | Me | OEt |
| Me | Me | H | Me | Et | *i*-Pr | H | H | Me | Et |
| Me | Me | H | Me | $SO_2Me$ | *i*-Pr | H | H | Me | $SO_2Me$ |
| Me | Me | H | Me | $SO_2CF_3$ | *i*-Pr | H | H | Me | $SO_2CF_3$ |
| Me | Me | H | Et | $CF_3$ | *i*-Pr | H | H | Et | $CF_3$ |
| Me | Me | H | Et | Br | *i*-Pr | H | H | Et | Br |
| Me | Me | H | Et | Cl | *i*-Pr | H | H | Et | Cl |
| Me | Me | H | Ph | $CF_3$ | *i*-Pr | H | H | Ph | $CF_3$ |
| Me | Me | H | Ph | Br | *i*-Pr | H | H | Ph | Br |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | Ph | Cl | *i*-Pr | H | H | Ph | Cl |
| Me | Me | H | 2-pyridyl | $CF_3$ | *i*-Pr | H | H | 2-pyridyl | $CF_3$ |
| Me | Me | H | 2-pyridyl | Cl | *i*-Pr | H | H | 2-pyridyl | Cl |
| Me | Me | H | 2-ClPh | $CF_3$ | *i*-Pr | H | H | 2-ClPh | $CF_3$ |
| Me | Me | H | 2-ClPh | $OCF_3$ | *i*-Pr | H | H | 2-ClPh | $OCF_3$ |
| Me | Me | H | 2-ClPh | $SCHF_2$ | *i*-Pr | H | H | 2-ClPh | $SCHF_2$ |
| Me | Me | H | 2-ClPh | Br | *i*-Pr | H | H | 2-ClPh | Br |
| Me | Me | H | 2-ClPh | Cl | *i*-Pr | H | H | 2-ClPh | Cl |
| Me | Me | H | 3-Cl-2-pyridyl | $CF_3$ | *i*-Pr | H | H | 3-Cl-2-pyridyl | $CF_3$ |
| Me | Me | H | 3-Cl-2-pyridyl | $OCF_3$ | *i*-Pr | H | H | 3-Cl-2-pyridyl | $OCF_3$ |
| Me | Me | H | 3-Cl-2-pyridyl | $SCHF_2$ | *i*-Pr | H | H | 3-Cl-2-pyridyl | $SCHF_2$ |
| Me | Me | H | 3-Cl-2-pyridyl | Br | *i*-Pr | H | H | 3-Cl-2-pyridyl | Br |
| Me | Me | H | 3-Cl-2-pyridyl | Cl | *i*-Pr | H | H | 3-Cl-2-pyridyl | Cl |
| Me | Me | H | 3-Br-2-pyridyl | $CF_3$ | *i*-Pr | H | H | 3-Br-2-pyridyl | $CF_3$ |
| Me | Me | H | 3-Br-2-pyridyl | $OCF_3$ | *i*-Pr | H | H | 3-Br-2-pyridyl | $OCF_3$ |
| Me | Me | H | 3-Br-2-pyridyl | Br | *i*-Pr | H | H | 3-Br-2-pyridyl | Br |
| Me | Me | H | 3-Br-2-pyridyl | Cl | *i*-Pr | H | H | 3-Br-2-pyridyl | Cl |
| Me | Me | H | $CF_3$ | Me | *i*-Pr | H | H | $CF_3$ | Me |
| Me | Me | H | F | $CF_3$ | *i*-Pr | H | H | F | $CF_3$ |
| Me | Me | H | Cl | $CF_3$ | *i*-Pr | H | H | Cl | $CF_3$ |
| Me | Me | H | *n*-Pr | $CF_3$ | *i*-Pr | H | H | *n*-Pr | $CF_3$ |
| Me | Me | H | *i*-Pr | $CF_3$ | *i*-Pr | H | H | *i*-Pr | $CF_3$ |
| Me | Me | H | $CF_3$ | $CF_3$ | *i*-Pr | H | H | $CF_3$ | $CF_3$ |
| Me | Me | H | OMe | $CF_3$ | *i*-Pr | H | H | OMe | $CF_3$ |
| Me | Me | H | 2-BrPh | $CF_3$ | *i*-Pr | H | H | 2-BrPh | $CF_3$ |
| Me | Me | H | 2-MePh | $CF_3$ | *i*-Pr | H | H | 2-MePh | $CF_3$ |
| Me | Me | H | 2-CNPh | $CF_3$ | *i*-Pr | H | H | 2-CNPh | $CF_3$ |
| Me | Me | H | 2-FPh | $CF_3$ | *i*-Pr | H | H | 2-FPh | $CF_3$ |
| Me | Me | H | 2,6-$F_2$Ph | $CF_3$ | *i*-Pr | H | H | 2,6-$F_2$Ph | $CF_3$ |
| Me | Me | H | 2,4-$F_2$Ph | $CF_3$ | *i*-Pr | H | H | 2,4-$F_2$Ph | $CF_3$ |
| Me | Me | H | 2,5-$F_2$Ph | $CF_3$ | *i*-Pr | H | H | 2,5-$F_2$Ph | $CF_3$ |
| Me | Me | H | 2-MeOPh | $CF_3$ | *i*-Pr | H | H | 2-MeOPh | $CF_3$ |
| Me | Me | H | 3-F-2-pyridyl | $CF_3$ | *i*-Pr | H | H | 3-F-2-pyridyl | $CF_3$ |
| Me | Me | H | 3-$CF_3$-2-pyridyl | $CF_3$ | *i*-Pr | H | H | 3-$CF_3$-2-pyridyl | $CF_3$ |
| Me | Me | H | 3-Me-2-pyridyl | $CF_3$ | *i*-Pr | H | H | 3-Me-2-pyridyl | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Et | Me | H | H | $CF_3$ | *i*-Pr | Me | H | H | $CF_3$ |
| Et | Me | H | H | $C_2F_5$ | *i*-Pr | Me | H | H | $C_2F_5$ |
| Et | Me | H | H | $OCF_3$ | *i*-Pr | Me | H | H | $OCF_3$ |
| Et | Me | H | Me | $CF_3$ | *i*-Pr | Me | H | Me | $CF_3$ |
| Et | Me | H | Me | $C_2F_5$ | *i*-Pr | Me | H | Me | $C_2F_5$ |
| Et | Me | H | Me | $OCF_3$ | *i*-Pr | Me | H | Me | $OCF_3$ |
| Me | Cl | H | H | $CF_3$ | Me | Cl | H | 2-ClPh | $CF_3$ |
| Me | Cl | H | H | $OCF_3$ | Me | Cl | H | 2-ClPh | $OCF_3$ |
| Me | Cl | H | H | $OCHF_2$ | Me | Cl | H | 2-ClPh | $SCHF_2$ |
| Me | Cl | H | H | $C_2F_5$ | Me | Cl | H | 2-ClPh | Br |
| Me | Cl | H | H | $OCF_2CHF_2$ | Me | Cl | H | 2-ClPh | Cl |
| Me | Cl | H | H | $SCF_2CHF_2$ | Me | Cl | H | 3-Cl-2-pyridyl | $CF_3$ |
| Me | Cl | H | H | *n*-$C_3F_7$ | Me | Cl | H | 3-Cl-2-pyridyl | $OCF_3$ |
| Me | Cl | H | H | *i*-$C_3F_7$ | Me | Cl | H | 3-Cl-2-pyridyl | $SCHF_2$ |
| Me | Cl | H | H | Br | Me | Cl | H | 3-Cl-2-pyridyl | Br |
| Me | Cl | H | H | Cl | Me | Cl | H | 3-Cl-2-pyridyl | Cl |
| Me | Cl | H | H | $SCF_3$ | Me | Cl | H | 3-Br-2-pyridyl | $CF_3$ |
| Me | Cl | H | Me | $CF_3$ | Me | Cl | H | 3-Br-2-pyridyl | $OCF_3$ |
| Me | Cl | H | Me | $OCF_3$ | Me | Cl | H | 3-Br-2-pyridyl | Br |
| Me | Cl | H | Me | $OCHF_2$ | Me | Cl | H | 3-Br-2-pyridyl | Cl |
| Me | Cl | H | Me | $C_2F_5$ | Me | Cl | H | $CF_3$ | Me |
| Me | Cl | H | Me | $OCF_2CHF_2$ | Me | Cl | H | F | $CF_3$ |
| Me | Cl | H | Me | $SCF_2CHF_2$ | Me | Cl | H | Cl | $CF_3$ |
| Me | Cl | H | Me | *n*-$C_3F_7$ | Me | Cl | H | *n*-Pr | $CF_3$ |
| Me | Cl | H | Me | *i*-$C_3F_7$ | Me | Cl | H | *i*-Pr | $CF_3$ |
| Me | Cl | H | Me | Br | Me | Cl | H | $CF_3$ | $CF_3$ |
| Me | Cl | H | Me | Cl | Me | Cl | H | OMe | $CF_3$ |
| Me | Cl | H | Me | $SCF_3$ | Me | Cl | H | 2-BrPh | $CF_3$ |
| Me | Cl | H | Me | I | Me | Cl | H | 2-MePh | $CF_3$ |
| Me | Cl | H | Me | SMe | Me | Cl | H | 2-CNPh | $CF_3$ |
| Me | Cl | H | Me | OMe | Me | Cl | H | 2-FPh | $CF_3$ |
| Me | Cl | H | Me | OEt | Me | Cl | H | 2,6-$F_2$Ph | $CF_3$ |
| Me | Cl | H | Me | Et | Me | Cl | H | 2,4-$F_2$Ph | $CF_3$ |
| Me | Cl | H | Me | $SO_2Me$ | Me | Cl | H | 2,5-$F_2$Ph | $CF_3$ |
| Me | Cl | H | Me | $SO_2CF_3$ | Me | Cl | H | 2-MeOPh | $CF_3$ |
| Me | Cl | H | Et | $CF_3$ | Me | Cl | H | 3-F-2-pyridyl | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Cl | H | Et | Br | Me | Cl | H | 3-$CF_3$-2-pyridyl | $CF_3$ |
| Me | Cl | H | Et | Cl | Me | Cl | H | 3-Me-2-pyridyl | $CF_3$ |
| Me | Cl | H | Ph | $CF_3$ | Et | Cl | H | H | $OCF_3$ |
| Me | Cl | H | Ph | Br | Et | Cl | H | H | $C_2F_5$ |
| Me | Cl | H | Ph | Cl | Et | Cl | H | Me | $CF_3$ |
| Me | Cl | H | 2-pyridyl | $CF_3$ | Et | Cl | H | Me | $C_2F_5$ |
| Me | Cl | H | 2-pyridyl | Cl | *i*-Pr | Cl | H | Me | $CF_3$ |

Table 7

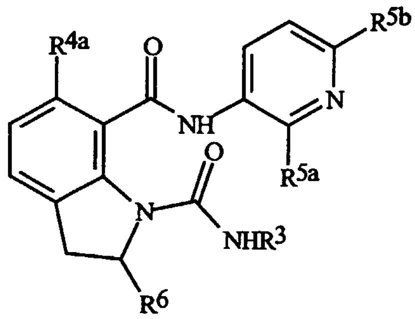

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CF_3$ | Me | H | H | H | $CF_3$ |
| Me | H | Me | H | $OCF_3$ | Me | H | H | H | $OCF_3$ |
| Me | H | Me | H | $OCHF_2$ | Me | H | H | H | $OCHF_2$ |
| Me | H | Me | H | $C_2F_5$ | Me | H | H | H | $C_2F_5$ |
| Me | H | Me | H | $OCF_2CHF_2$ | Me | H | H | H | $OCF_2CHF_2$ |
| Me | H | Me | H | $SCF_2CHF_2$ | Me | H | H | H | $SCF_2CHF_2$ |
| Me | H | Me | H | *n*-$C_3F_7$ | Me | H | H | H | *n*-$C_3F_7$ |
| Me | H | Me | H | *i*-$C_3F_7$ | Me | H | H | H | *i*-$C_3F_7$ |
| Me | H | Me | H | Br | Me | H | H | H | Br |
| Me | H | Me | H | Cl | Me | H | H | H | Cl |
| Me | H | Me | H | $SCF_3$ | Me | H | H | H | $SCF_3$ |
| Me | H | Me | Me | $CF_3$ | Me | H | H | Me | $CF_3$ |
| Me | H | Me | Me | $OCF_3$ | Me | H | H | Me | $OCF_3$ |
| Me | H | Me | Me | $OCHF_2$ | Me | H | H | Me | $OCHF_2$ |
| Me | H | Me | Me | $C_2F_5$ | Me | H | H | Me | $C_2F_5$ |
| Me | H | Me | Me | $OCF_2CHF_2$ | Me | H | H | Me | $OCF_2CHF_2$ |
| Me | H | Me | Me | $SCF_2CHF_2$ | Me | H | H | Me | $SCF_2CHF_2$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | Me | *n*-$C_3F_7$ | Me | H | H | Me | *n*-$C_3F_7$ |
| Me | H | Me | Me | *i*-$C_3F_7$ | Me | H | H | Me | *i*-$C_3F_7$ |
| Me | H | Me | Me | Br | Me | H | H | Me | Br |
| Me | H | Me | Me | Cl | Me | H | H | Me | Cl |
| Me | H | Me | Me | $SCF_3$ | Me | H | H | Me | $SCF_3$ |
| Me | H | Me | Me | I | Me | H | H | Me | I |
| Me | H | Me | Me | SMe | Me | H | H | Me | SMe |
| Me | H | Me | Me | OMe | Me | H | H | Me | OMe |
| Me | H | Me | Me | OEt | Me | H | H | Me | OEt |
| Me | H | Me | Me | Et | Me | H | H | Me | Et |
| Me | H | Me | Me | $SO_2Me$ | Me | H | H | Me | $SO_2Me$ |
| Me | H | Me | Me | $SO_2CF_3$ | Me | H | H | Me | $SO_2CF_3$ |
| Me | H | Me | Et | $CF_3$ | Me | H | H | Et | $CF_3$ |
| Me | H | Me | Et | Br | Me | H | H | Et | Br |
| Me | H | Me | Et | Cl | Me | H | H | Et | Cl |
| Me | H | Me | Ph | $CF_3$ | Me | H | H | Ph | $CF_3$ |
| Me | H | Me | Ph | Br | Me | H | H | Ph | Br |
| Me | H | Me | Ph | Cl | Me | H | H | Ph | Cl |
| Me | H | Me | 2-pyridyl | $CF_3$ | Me | H | H | 2-pyridyl | $CF_3$ |
| Me | H | Me | 2-pyridyl | Cl | Me | H | H | 2-pyridyl | Cl |
| Me | H | Me | 2-ClPh | $CF_3$ | Me | H | H | 2-ClPh | $CF_3$ |
| Me | H | Me | 2-ClPh | $OCF_3$ | Me | H | H | 2-ClPh | $OCF_3$ |
| Me | H | Me | 2-ClPh | $SCHF_2$ | Me | H | H | 2-ClPh | $SCHF_2$ |
| Me | H | Me | 2-ClPh | Br | Me | H | H | 2-ClPh | Br |
| Me | H | Me | 2-ClPh | Cl | Me | H | H | 2-ClPh | Cl |
| Me | H | Me | 3-Cl-2-pyridyl | $CF_3$ | Me | H | H | 3-Cl-2-pyridyl | $CF_3$ |
| Me | H | Me | 3-Cl-2-pyridyl | $OCF_3$ | Me | H | H | 3-Cl-2-pyridyl | $OCF_3$ |
| Me | H | Me | 3-Cl-2-pyridyl | $SCHF_2$ | Me | H | H | 3-Cl-2-pyridyl | $SCHF_2$ |
| Me | H | Me | 3-Cl-2-pyridyl | Br | Me | H | H | 3-Cl-2-pyridyl | Br |
| Me | H | Me | 3-Cl-2-pyridyl | Cl | Me | H | H | 3-Cl-2-pyridyl | Cl |
| Me | H | Me | 3-Br-2-pyridyl | $CF_3$ | Me | H | H | 3-Br-2-pyridyl | $CF_3$ |
| Me | H | Me | 3-Br-2-pyridyl | $OCF_3$ | Me | H | H | 3-Br-2-pyridyl | $OCF_3$ |
| Me | H | Me | 3-Br-2-pyridyl | Br | Me | H | H | 3-Br-2-pyridyl | Br |
| Me | H | Me | 3-Br-2-pyridyl | Cl | Me | H | H | 3-Br-2-pyridyl | Cl |
| Me | H | Me | $CF_3$ | Me | Me | H | H | $CF_3$ | Me |
| Me | H | Me | F | $CF_3$ | Me | H | H | F | $CF_3$ |
| Me | H | Me | Cl | $CF_3$ | Me | H | H | Cl | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | *n*-Pr | $CF_3$ | Me | H | H | *n*-Pr | $CF_3$ |
| Me | H | Me | *i*-Pr | $CF_3$ | Me | H | H | *i*-Pr | $CF_3$ |
| Me | H | Me | $CF_3$ | $CF_3$ | Me | H | H | $CF_3$ | $CF_3$ |
| Me | H | Me | OMe | $CF_3$ | Me | H | H | OMe | $CF_3$ |
| Me | H | Me | 2-BrPh | $CF_3$ | Me | H | H | 2-BrPh | $CF_3$ |
| Me | H | Me | 2-MePh | $CF_3$ | Me | H | H | 2-MePh | $CF_3$ |
| Me | H | Me | 2-CNPh | $CF_3$ | Me | H | H | 2-CNPh | $CF_3$ |
| Me | H | Me | 2-FPh | $CF_3$ | Me | H | H | 2-FPh | $CF_3$ |
| Me | H | Me | 2,6-$F_2$Ph | $CF_3$ | Me | H | H | 2,6-$F_2$Ph | $CF_3$ |
| Me | H | Me | 2,4-$F_2$Ph | $CF_3$ | Me | H | H | 2,4-$F_2$Ph | $CF_3$ |
| Me | H | Me | 2,5-$F_2$Ph | $CF_3$ | Me | H | H | 2,5-$F_2$Ph | $CF_3$ |
| Me | H | Me | 2-MeOPh | $CF_3$ | Me | H | H | 2-MeOPh | $CF_3$ |
| Me | H | Me | 3-F-2-pyridyl | $CF_3$ | Me | H | H | 3-F-2-pyridyl | $CF_3$ |
| Me | H | Me | 3-$CF_3$-2-pyridyl | $CF_3$ | Me | H | H | 3-$CF_3$-2-pyridyl | $CF_3$ |
| Me | H | Me | 3-Me-2-pyridyl | $CF_3$ | Me | H | H | 3-Me-2-pyridyl | $CF_3$ |
| Et | H | Me | H | $CF_3$ | Et | H | H | H | $CF_3$ |
| Et | H | Me | H | $C_2F_5$ | Et | H | H | H | $C_2F_5$ |
| Et | H | Me | H | $OCF_3$ | Et | H | H | H | $OCF_3$ |
| Et | H | Me | Me | $CF_3$ | Et | H | H | Me | $CF_3$ |
| Et | H | Me | Me | $C_2F_5$ | Et | H | H | Me | $C_2F_5$ |
| Et | H | Me | Me | $OCF_3$ | Et | H | H | Me | $OCF_3$ |
| Me | Me | H | H | $CF_3$ | *i*-Pr | H | H | H | $CF_3$ |
| Me | Me | H | H | $OCF_3$ | *i*-Pr | H | H | H | $OCF_3$ |
| Me | Me | H | H | $OCHF_2$ | *i*-Pr | H | H | H | $OCHF_2$ |
| Me | Me | H | H | $C_2F_5$ | *i*-Pr | H | H | H | $C_2F_5$ |
| Me | Me | H | H | $OCF_2CHF_2$ | *i*-Pr | H | H | H | $OCF_2CHF_2$ |
| Me | Me | H | H | $SCF_2CHF_2$ | *i*-Pr | H | H | H | $SCF_2CHF_2$ |
| Me | Me | H | H | *n*-$C_3F_7$ | *i*-Pr | H | H | H | *n*-$C_3F_7$ |
| Me | Me | H | H | *i*-$C_3F_7$ | *i*-Pr | H | H | H | *i*-$C_3F_7$ |
| Me | Me | H | H | Br | *i*-Pr | H | H | H | Br |
| Me | Me | H | H | Cl | *i*-Pr | H | H | H | Cl |
| Me | Me | H | H | $SCF_3$ | *i*-Pr | H | H | H | $SCF_3$ |
| Me | Me | H | H | $CF_3$ | *i*-Pr | H | H | Me | $CF_3$ |
| Me | Me | H | H | $OCF_3$ | *i*-Pr | H | H | Me | $OCF_3$ |
| Me | Me | H | H | $OCHF_2$ | *i*-Pr | H | H | Me | $OCHF_2$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | Me | $C_2F_5$ | *i*-Pr | H | H | Me | $C_2F_5$ |
| Me | Me | H | Me | $OCF_2CHF_2$ | *i*-Pr | H | H | Me | $OCF_2CHF_2$ |
| Me | Me | H | Me | $SCF_2CHF_2$ | *i*-Pr | H | H | Me | $SCF_2CHF_2$ |
| Me | Me | H | Me | *n*-$C_3F_7$ | *i*-Pr | H | H | Me | *n*-$C_3F_7$ |
| Me | Me | H | Me | *i*-$C_3F_7$ | *i*-Pr | H | H | Me | *i*-$C_3F_7$ |
| Me | Me | H | Me | Br | *i*-Pr | H | H | Me | Br |
| Me | Me | H | Me | Cl | *i*-Pr | H | H | Me | Cl |
| Me | Me | H | Me | $SCF_3$ | *i*-Pr | H | H | Me | $SCF_3$ |
| Me | Me | H | Me | I | *i*-Pr | H | H | Me | I |
| Me | Me | H | Me | SMe | *i*-Pr | H | H | Me | SMe |
| Me | Me | H | Me | OMe | *i*-Pr | H | H | Me | OMe |
| Me | Me | H | Me | OEt | *i*-Pr | H | H | Me | OEt |
| Me | Me | H | Me | Et | *i*-Pr | H | H | Me | Et |
| Me | Me | H | Me | $SO_2Me$ | *i*-Pr | H | H | Me | $SO_2Me$ |
| Me | Me | H | Me | $SO_2CF_3$ | *i*-Pr | H | H | Me | $SO_2CF_3$ |
| Me | Me | H | Et | $CF_3$ | *i*-Pr | H | H | Et | $CF_3$ |
| Me | Me | H | Et | Br | *i*-Pr | H | H | Et | Br |
| Me | Me | H | Et | Cl | *i*-Pr | H | H | Et | Cl |
| Me | Me | H | Ph | $CF_3$ | *i*-Pr | H | H | Ph | $CF_3$ |
| Me | Me | H | Ph | Br | *i*-Pr | H | H | Ph | Br |
| Me | Me | H | Ph | Cl | *i*-Pr | H | H | Ph | Cl |
| Me | Me | H | 2-pyridyl | $CF_3$ | *i*-Pr | H | H | 2-pyridyl | $CF_3$ |
| Me | Me | H | 2-pyridyl | Cl | *i*-Pr | H | H | 2-pyridyl | Cl |
| Me | Me | H | 2-ClPh | $CF_3$ | *i*-Pr | H | H | 2-ClPh | $CF_3$ |
| Me | Me | H | 2-ClPh | $OCF_3$ | *i*-Pr | H | H | 2-ClPh | $OCF_3$ |
| Me | Me | H | 2-ClPh | $SCHF_2$ | *i*-Pr | H | H | 2-ClPh | $SCHF_2$ |
| Me | Me | H | 2-ClPh | Br | *i*-Pr | H | H | 2-ClPh | Br |
| Me | Me | H | 2-ClPh | Cl | *i*-Pr | H | H | 2-ClPh | Cl |
| Me | Me | H | 3-Cl-2-pyridyl | $CF_3$ | *i*-Pr | H | H | 3-Cl-2-pyridyl | $CF_3$ |
| Me | Me | H | 3-Cl-2-pyridyl | $OCF_3$ | *i*-Pr | H | H | 3-Cl-2-pyridyl | $OCF_3$ |
| Me | Me | H | 3-Cl-2-pyridyl | $SCHF_2$ | *i*-Pr | H | H | 3-Cl-2-pyridyl | $SCHF_2$ |
| Me | Me | H | 3-Cl-2-pyridyl | Br | *i*-Pr | H | H | 3-Cl-2-pyridyl | Br |
| Me | Me | H | 3-Cl-2-pyridyl | Cl | *i*-Pr | H | H | 3-Cl-2-pyridyl | Cl |
| Me | Me | H | 3-Br-2-pyridyl | $CF_3$ | *i*-Pr | H | H | 3-Br-2-pyridyl | $CF_3$ |
| Me | Me | H | 3-Br-2-pyridyl | $OCF_3$ | *i*-Pr | H | H | 3-Br-2-pyridyl | $OCF_3$ |
| Me | Me | H | 3-Br-2-pyridyl | Br | *i*-Pr | H | H | 3-Br-2-pyridyl | Br |
| Me | Me | H | 3-Br-2-pyridyl | Cl | *i*-Pr | H | H | 3-Br-2-pyridyl | Cl |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | $CF_3$ | Me | *i*-Pr | H | H | $CF_3$ | Me |
| Me | Me | H | F | $CF_3$ | *i*-Pr | H | H | F | $CF_3$ |
| Me | Me | H | Cl | $CF_3$ | *i*-Pr | H | H | Cl | $CF_3$ |
| Me | Me | H | *n*-Pr | $CF_3$ | *i*-Pr | H | H | *n*-Pr | $CF_3$ |
| Me | Me | H | *i*-Pr | $CF_3$ | *i*-Pr | H | H | *i*-Pr | $CF_3$ |
| Me | Me | H | $CF_3$ | $CF_3$ | *i*-Pr | H | H | $CF_3$ | $CF_3$ |
| Me | Me | H | OMe | $CF_3$ | *i*-Pr | H | H | OMe | $CF_3$ |
| Me | Me | H | 2-BrPh | $CF_3$ | *i*-Pr | H | H | 2-BrPh | $CF_3$ |
| Me | Me | H | 2-MePh | $CF_3$ | *i*-Pr | H | H | 2-MePh | $CF_3$ |
| Me | Me | H | 2-CNPh | $CF_3$ | *i*-Pr | H | H | 2-CNPh | $CF_3$ |
| Me | Me | H | 2-FPh | $CF_3$ | *i*-Pr | H | H | 2-FPh | $CF_3$ |
| Me | Me | H | 2,6-$F_2$Ph | $CF_3$ | *i*-Pr | H | H | 2,6-$F_2$Ph | $CF_3$ |
| Me | Me | H | 2,4-$F_2$Ph | $CF_3$ | *i*-Pr | H | H | 2,4-$F_2$Ph | $CF_3$ |
| Me | Me | H | 2,5-$F_2$Ph | $CF_3$ | *i*-Pr | H | H | 2,5-$F_2$Ph | $CF_3$ |
| Me | Me | H | 2-MeOPh | $CF_3$ | *i*-Pr | H | H | 2-MeOPh | $CF_3$ |
| Me | Me | H | 3-F-2-pyridyl | $CF_3$ | *i*-Pr | H | H | 3-F-2-pyridyl | $CF_3$ |
| Me | Me | H | 3-$CF_3$-2-pyridyl | $CF_3$ | *i*-Pr | H | H | 3-$CF_3$-2-pyridyl | $CF_3$ |
| Me | Me | H | 3-Me-2-pyridyl | $CF_3$ | *i*-Pr | H | H | 3-Me-2-pyridyl | $CF_3$ |
| Et | Me | H | H | $CF_3$ | *i*-Pr | Me | H | H | $CF_3$ |
| Et | Me | H | H | $C_2F_5$ | *i*-Pr | Me | H | H | $C_2F_5$ |
| Et | Me | H | H | $OCF_3$ | *i*-Pr | Me | H | H | $OCF_3$ |
| Et | Me | H | Me | $CF_3$ | *i*-Pr | Me | H | Me | $CF_3$ |
| Et | Me | H | Me | $C_2F_5$ | *i*-Pr | Me | H | Me | $C_2F_5$ |
| Et | Me | H | Me | $OCF_3$ | *i*-Pr | Me | H | Me | $OCF_3$ |
| Me | Cl | H | H | $CF_3$ | Me | Cl | H | 2-ClPh | $CF_3$ |
| Me | Cl | H | H | $OCF_3$ | Me | Cl | H | 2-ClPh | $OCF_3$ |
| Me | Cl | H | H | $OCHF_2$ | Me | Cl | H | 2-ClPh | $SCHF_2$ |
| Me | Cl | H | H | $C_2F_5$ | Me | Cl | H | 2-ClPh | Br |
| Me | Cl | H | H | $OCF_2CHF_2$ | Me | Cl | H | 2-ClPh | Cl |
| Me | Cl | H | H | $SCF_2CHF_2$ | Me | Cl | H | 3-Cl-2-pyridyl | $CF_3$ |
| Me | Cl | H | H | *n*-$C_3F_7$ | Me | Cl | H | 3-Cl-2-pyridyl | $OCF_3$ |
| Me | Cl | H | H | *i*-$C_3F_7$ | Me | Cl | H | 3-Cl-2-pyridyl | $SCHF_2$ |
| Me | Cl | H | H | Br | Me | Cl | H | 3-Cl-2-pyridyl | Br |
| Me | Cl | H | H | Cl | Me | Cl | H | 3-Cl-2-pyridyl | Cl |
| Me | Cl | H | H | $SCF_3$ | Me | Cl | H | 3-Br-2-pyridyl | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Cl | H | Me | $CF_3$ | Me | Cl | H | 3-Br-2-pyridyl | $OCF_3$ |
| Me | Cl | H | Me | $OCF_3$ | Me | Cl | H | 3-Br-2-pyridyl | Br |
| Me | Cl | H | Me | $OCHF_2$ | Me | Cl | H | 3-Br-2-pyridyl | Cl |
| Me | Cl | H | Me | $C_2F_5$ | Me | Cl | H | $CF_3$ | Me |
| Me | Cl | H | Me | $OCF_2CHF_2$ | Me | Cl | H | F | $CF_3$ |
| Me | Cl | H | Me | $SCF_2CHF_2$ | Me | Cl | H | Cl | $CF_3$ |
| Me | Cl | H | Me | *n*-$C_3F_7$ | Me | Cl | H | *n*-Pr | $CF_3$ |
| Me | Cl | H | Me | *i*-$C_3F_7$ | Me | Cl | H | *i*-Pr | $CF_3$ |
| Me | Cl | H | Me | Br | Me | Cl | H | $CF_3$ | $CF_3$ |
| Me | Cl | H | Me | Cl | Me | Cl | H | OMe | $CF_3$ |
| Me | Cl | H | Me | $SCF_3$ | Me | Cl | H | 2-BrPh | $CF_3$ |
| Me | Cl | H | Me | I | Me | Cl | H | 2-MePh | $CF_3$ |
| Me | Cl | H | Me | SMe | Me | Cl | H | 2-CNPh | $CF_3$ |
| Me | Cl | H | Me | OMe | Me | Cl | H | 2-FPh | $CF_3$ |
| Me | Cl | H | Me | OEt | Me | Cl | H | 2,6-$F_2$Ph | $CF_3$ |
| Me | Cl | H | Me | Et | Me | Cl | H | 2,4-$F_2$Ph | $CF_3$ |
| Me | Cl | H | Me | $SO_2Me$ | Me | Cl | H | 2,5-$F_2$Ph | $CF_3$ |
| Me | Cl | H | Me | $SO_2CF_3$ | Me | Cl | H | 2-MeOPh | $CF_3$ |
| Me | Cl | H | Et | $CF_3$ | Me | Cl | H | 3-F-2-pyridyl | $CF_3$ |
| Me | Cl | H | Et | Br | Me | Cl | H | 3-$CF_3$-2-pyridyl | $CF_3$ |
| Me | Cl | H | Et | Cl | Me | Cl | H | 3-Me-2-pyridyl | $CF_3$ |
| Me | Cl | H | Ph | $CF_3$ | Et | Cl | H | H | $OCF_3$ |
| Me | Cl | H | Ph | Br | Et | Cl | H | H | $C_2F_5$ |
| Me | Cl | H | Ph | Cl | Et | Cl | H | Me | $CF_3$ |
| Me | Cl | H | 2-pyridyl | $CF_3$ | Et | Cl | H | Me | $C_2F_5$ |
| Me | Cl | H | 2-pyridyl | Cl | *i*-Pr | Cl | H | Me | $CF_3$ |

Table 8

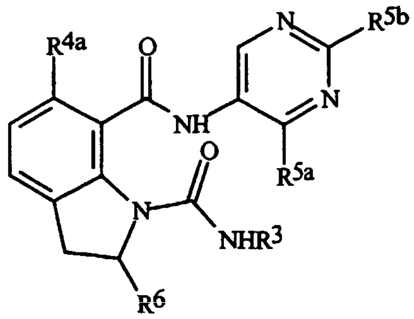

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CF_3$ | Me | H | H | H | $CF_3$ |
| Me | H | Me | H | $OCF_3$ | Me | H | H | H | $OCF_3$ |
| Me | H | Me | H | $OCHF_2$ | Me | H | H | H | $OCHF_2$ |
| Me | H | Me | H | $C_2F_5$ | Me | H | H | H | $C_2F_5$ |
| Me | H | Me | H | $OCF_2CHF_2$ | Me | H | H | H | $OCF_2CHF_2$ |
| Me | H | Me | H | $SCF_2CHF_2$ | Me | H | H | H | $SCF_2CHF_2$ |
| Me | H | Me | H | *n*-$C_3F_7$ | Me | H | H | H | *n*-$C_3F_7$ |
| Me | H | Me | H | *i*-$C_3F_7$ | Me | H | H | H | *i*-$C_3F_7$ |
| Me | H | Me | H | Br | Me | H | H | H | Br |
| Me | H | Me | H | Cl | Me | H | H | H | Cl |
| Me | H | Me | H | $SCF_3$ | Me | H | H | H | $SCF_3$ |
| Me | H | Me | Me | $CF_3$ | Me | H | H | Me | $CF_3$ |
| Me | H | Me | Me | $OCF_3$ | Me | H | H | Me | $OCF_3$ |
| Me | H | Me | Me | $OCHF_2$ | Me | H | H | Me | $OCHF_2$ |
| Me | H | Me | Me | $C_2F_5$ | Me | H | H | Me | $C_2F_5$ |
| Me | H | Me | Me | $OCF_2CHF_2$ | Me | H | H | Me | $OCF_2CHF_2$ |
| Me | H | Me | Me | $SCF_2CHF_2$ | Me | H | H | Me | $SCF_2CHF_2$ |
| Me | H | Me | Me | *n*-$C_3F_7$ | Me | H | H | Me | *n*-$C_3F_7$ |
| Me | H | Me | Me | *i*-$C_3F_7$ | Me | H | H | Me | *i*-$C_3F_7$ |
| Me | H | Me | Me | Br | Me | H | H | Me | Br |
| Me | H | Me | Me | Cl | Me | H | H | Me | Cl |
| Me | H | Me | Me | $SCF_3$ | Me | H | H | Me | $SCF_3$ |
| Me | H | Me | Me | I | Me | H | H | Me | I |
| Me | H | Me | Me | SMe | Me | H | H | Me | SMe |
| Me | H | Me | Me | OMe | Me | H | H | Me | OMe |
| Me | H | Me | Me | OEt | Me | H | H | Me | OEt |
| Me | H | Me | Me | Et | Me | H | H | Me | Et |
| Me | H | Me | Me | $SO_2Me$ | Me | H | H | Me | $SO_2Me$ |
| Me | H | Me | Me | $SO_2CF_3$ | Me | H | H | Me | $SO_2CF_3$ |
| Me | H | Me | Et | $CF_3$ | Me | H | H | Et | $CF_3$ |
| Me | H | Me | Et | Br | Me | H | H | Et | Br |
| Me | H | Me | Et | Cl | Me | H | H | Et | Cl |
| Me | H | Me | Ph | $CF_3$ | Me | H | H | Ph | $CF_3$ |
| Me | H | Me | Ph | Br | Me | H | H | Ph | Br |
| Me | H | Me | Ph | Cl | Me | H | H | Ph | Cl |
| Me | H | Me | 2-pyridyl | $CF_3$ | Me | H | H | 2-pyridyl | $CF_3$ |
| Me | H | Me | 2-pyridyl | Cl | Me | H | H | 2-pyridyl | Cl |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | 2-ClPh | $CF_3$ | Me | H | H | 2-ClPh | $CF_3$ |
| Me | H | Me | 2-ClPh | $OCF_3$ | Me | H | H | 2-ClPh | $OCF_3$ |
| Me | H | Me | 2-ClPh | $SCHF_2$ | Me | H | H | 2-ClPh | $SCHF_2$ |
| Me | H | Me | 2-ClPh | Br | Me | H | H | 2-ClPh | Br |
| Me | H | Me | 2-ClPh | Cl | Me | H | H | 2-ClPh | Cl |
| Me | H | Me | 3-Cl-2-pyridyl | $CF_3$ | Me | H | H | 3-Cl-2-pyridyl | $CF_3$ |
| Me | H | Me | 3-Cl-2-pyridyl | $OCF_3$ | Me | H | H | 3-Cl-2-pyridyl | $OCF_3$ |
| Me | H | Me | 3-Cl-2-pyridyl | $SCHF_2$ | Me | H | H | 3-Cl-2-pyridyl | $SCHF_2$ |
| Me | H | Me | 3-Cl-2-pyridyl | Br | Me | H | H | 3-Cl-2-pyridyl | Br |
| Me | H | Me | 3-Cl-2-pyridyl | Cl | Me | H | H | 3-Cl-2-pyridyl | Cl |
| Me | H | Me | 3-Br-2-pyridyl | $CF_3$ | Me | H | H | 3-Br-2-pyridyl | $CF_3$ |
| Me | H | Me | 3-Br-2-pyridyl | $OCF_3$ | Me | H | H | 3-Br-2-pyridyl | $OCF_3$ |
| Me | H | Me | 3-Br-2-pyridyl | Br | Me | H | H | 3-Br-2-pyridyl | Br |
| Me | H | Me | 3-Br-2-pyridyl | Cl | Me | H | H | 3-Br-2-pyridyl | Cl |
| Me | H | Me | $CF_3$ | Me | Me | H | H | $CF_3$ | Me |
| Me | H | Me | F | $CF_3$ | Me | H | H | F | $CF_3$ |
| Me | H | Me | Cl | $CF_3$ | Me | H | H | Cl | $CF_3$ |
| Me | H | Me | *n*-Pr | $CF_3$ | Me | H | H | *n*-Pr | $CF_3$ |
| Me | H | Me | *i*-Pr | $CF_3$ | Me | H | H | *i*-Pr | $CF_3$ |
| Me | H | Me | $CF_3$ | $CF_3$ | Me | H | H | $CF_3$ | $CF_3$ |
| Me | H | Me | OMe | $CF_3$ | Me | H | H | OMe | $CF_3$ |
| Me | H | Me | 2-BrPh | $CF_3$ | Me | H | H | 2-BrPh | $CF_3$ |
| Me | H | Me | 2-MePh | $CF_3$ | Me | H | H | 2-MePh | $CF_3$ |
| Me | H | Me | 2-CNPh | $CF_3$ | Me | H | H | 2-CNPh | $CF_3$ |
| Me | H | Me | 2-FPh | $CF_3$ | Me | H | H | 2-FPh | $CF_3$ |
| Me | H | Me | 2,6-$F_2$Ph | $CF_3$ | Me | H | H | 2,6-$F_2$Ph | $CF_3$ |
| Me | H | Me | 2,4-$F_2$Ph | $CF_3$ | Me | H | H | 2,4-$F_2$Ph | $CF_3$ |
| Me | H | Me | 2,5-$F_2$Ph | $CF_3$ | Me | H | H | 2,5-$F_2$Ph | $CF_3$ |
| Me | H | Me | 2-MeOPh | $CF_3$ | Me | H | H | 2-MeOPh | $CF_3$ |
| Me | H | Me | 3-F-2-pyridyl | $CF_3$ | Me | H | H | 3-F-2-pyridyl | $CF_3$ |
| Me | H | Me | 3-$CF_3$-2-pyridyl | $CF_3$ | Me | H | H | 3-$CF_3$-2-pyridyl | $CF_3$ |
| Me | H | Me | 3-Me-2-pyridyl | $CF_3$ | Me | H | H | 3-Me-2-pyridyl | $CF_3$ |
| Et | H | Me | H | $CF_3$ | Et | H | H | H | $CF_3$ |
| Et | H | Me | H | $C_2F_5$ | Et | H | H | H | $C_2F_5$ |
| Et | H | Me | H | $OCF_3$ | Et | H | H | H | $OCF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Et | H | Me | Me | $CF_3$ | Et | H | H | Me | $CF_3$ |
| Et | H | Me | Me | $C_2F_5$ | Et | H | H | Me | $C_2F_5$ |
| Et | H | Me | Me | $OCF_3$ | Et | H | H | Me | $OCF_3$ |
| Me | Me | H | H | $CF_3$ | *i*-Pr | H | H | H | $CF_3$ |
| Me | Me | H | H | $OCF_3$ | *i*-Pr | H | H | H | $OCF_3$ |
| Me | Me | H | H | $OCHF_2$ | *i*-Pr | H | H | H | $OCHF_2$ |
| Me | Me | H | H | $C_2F_5$ | *i*-Pr | H | H | H | $C_2F_5$ |
| Me | Me | H | H | $OCF_2CHF_2$ | *i*-Pr | H | H | H | $OCF_2CHF_2$ |
| Me | Me | H | H | $SCF_2CHF_2$ | *i*-Pr | H | H | H | $SCF_2CHF_2$ |
| Me | Me | H | H | *n*-$C_3F_7$ | *i*-Pr | H | H | H | *n*-$C_3F_7$ |
| Me | Me | H | H | *i*-$C_3F_7$ | *i*-Pr | H | H | H | *i*-$C_3F_7$ |
| Me | Me | H | H | Br | *i*-Pr | H | H | H | Br |
| Me | Me | H | H | Cl | *i*-Pr | H | H | H | Cl |
| Me | Me | H | H | $SCF_3$ | *i*-Pr | H | H | H | $SCF_3$ |
| Me | Me | H | H | $CF_3$ | *i*-Pr | H | H | Me | $CF_3$ |
| Me | Me | H | H | $OCF_3$ | *i*-Pr | H | H | Me | $OCF_3$ |
| Me | Me | H | H | $OCHF_2$ | *i*-Pr | H | H | Me | $OCHF_2$ |
| Me | Me | H | Me | $C_2F_5$ | *i*-Pr | H | H | Me | $C_2F_5$ |
| Me | Me | H | Me | $OCF_2CHF_2$ | *i*-Pr | H | H | Me | $OCF_2CHF_2$ |
| Me | Me | H | Me | $SCF_2CHF_2$ | *i*-Pr | H | H | Me | $SCF_2CHF_2$ |
| Me | Me | H | Me | *n*-$C_3F_7$ | *i*-Pr | H | H | Me | *n*-$C_3F_7$ |
| Me | Me | H | Me | *i*-$C_3F_7$ | *i*-Pr | H | H | Me | *i*-$C_3F_7$ |
| Me | Me | H | Me | Br | *i*-Pr | H | H | Me | Br |
| Me | Me | H | Me | Cl | *i*-Pr | H | H | Me | Cl |
| Me | Me | H | Me | $SCF_3$ | *i*-Pr | H | H | Me | $SCF_3$ |
| Me | Me | H | Me | I | *i*-Pr | H | H | Me | I |
| Me | Me | H | Me | SMe | *i*-Pr | H | H | Me | SMe |
| Me | Me | H | Me | OMe | *i*-Pr | H | H | Me | OMe |
| Me | Me | H | Me | OEt | *i*-Pr | H | H | Me | OEt |
| Me | Me | H | Me | Et | *i*-Pr | H | H | Me | Et |
| Me | Me | H | Me | $SO_2Me$ | *i*-Pr | H | H | Me | $SO_2Me$ |
| Me | Me | H | Me | $SO_2CF_3$ | *i*-Pr | H | H | Me | $SO_2CF_3$ |
| Me | Me | H | Et | $CF_3$ | *i*-Pr | H | H | Et | $CF_3$ |
| Me | Me | H | Et | Br | *i*-Pr | H | H | Et | Br |
| Me | Me | H | Et | Cl | *i*-Pr | H | H | Et | Cl |
| Me | Me | H | Ph | $CF_3$ | *i*-Pr | H | H | Ph | $CF_3$ |
| Me | Me | H | Ph | Br | *i*-Pr | H | H | Ph | Br |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | Ph | Cl | *i*-Pr | H | H | Ph | Cl |
| Me | Me | H | 2-pyridyl | $CF_3$ | *i*-Pr | H | H | 2-pyridyl | $CF_3$ |
| Me | Me | H | 2-pyridyl | Cl | *i*-Pr | H | H | 2-pyridyl | Cl |
| Me | Me | H | 2-ClPh | $CF_3$ | *i*-Pr | H | H | 2-ClPh | $CF_3$ |
| Me | Me | H | 2-ClPh | $OCF_3$ | *i*-Pr | H | H | 2-ClPh | $OCF_3$ |
| Me | Me | H | 2-ClPh | $SCHF_2$ | *i*-Pr | H | H | 2-ClPh | $SCHF_2$ |
| Me | Me | H | 2-ClPh | Br | *i*-Pr | H | H | 2-ClPh | Br |
| Me | Me | H | 2-ClPh | Cl | *i*-Pr | H | H | 2-ClPh | Cl |
| Me | Me | H | 3-Cl-2-pyridyl | $CF_3$ | *i*-Pr | H | H | 3-Cl-2-pyridyl | $CF_3$ |
| Me | Me | H | 3-Cl-2-pyridyl | $OCF_3$ | *i*-Pr | H | H | 3-Cl-2-pyridyl | $OCF_3$ |
| Me | Me | H | 3-Cl-2-pyridyl | $SCHF_2$ | *i*-Pr | H | H | 3-Cl-2-pyridyl | $SCHF_2$ |
| Me | Me | H | 3-Cl-2-pyridyl | Br | *i*-Pr | H | H | 3-Cl-2-pyridyl | Br |
| Me | Me | H | 3-Cl-2-pyridyl | Cl | *i*-Pr | H | H | 3-Cl-2-pyridyl | Cl |
| Me | Me | H | 3-Br-2-pyridyl | $CF_3$ | *i*-Pr | H | H | 3-Br-2-pyridyl | $CF_3$ |
| Me | Me | H | 3-Br-2-pyridyl | $OCF_3$ | *i*-Pr | H | H | 3-Br-2-pyridyl | $OCF_3$ |
| Me | Me | H | 3-Br-2-pyridyl | Br | *i*-Pr | H | H | 3-Br-2-pyridyl | Br |
| Me | Me | H | 3-Br-2-pyridyl | Cl | *i*-Pr | H | H | 3-Br-2-pyridyl | Cl |
| Me | Me | H | $CF_3$ | Me | *i*-Pr | H | H | $CF_3$ | Me |
| Me | Me | H | F | $CF_3$ | *i*-Pr | H | H | F | $CF_3$ |
| Me | Me | H | Cl | $CF_3$ | *i*-Pr | H | H | Cl | $CF_3$ |
| Me | Me | H | *n*-Pr | $CF_3$ | *i*-Pr | H | H | *n*-Pr | $CF_3$ |
| Me | Me | H | *i*-Pr | $CF_3$ | *i*-Pr | H | H | *i*-Pr | $CF_3$ |
| Me | Me | H | $CF_3$ | $CF_3$ | *i*-Pr | H | H | $CF_3$ | $CF_3$ |
| Me | Me | H | OMe | $CF_3$ | *i*-Pr | H | H | OMe | $CF_3$ |
| Me | Me | H | 2-BrPh | $CF_3$ | *i*-Pr | H | H | 2-BrPh | $CF_3$ |
| Me | Me | H | 2-MePh | $CF_3$ | *i*-Pr | H | H | 2-MePh | $CF_3$ |
| Me | Me | H | 2-CNPh | $CF_3$ | *i*-Pr | H | H | 2-CNPh | $CF_3$ |
| Me | Me | H | 2-FPh | $CF_3$ | *i*-Pr | H | H | 2-FPh | $CF_3$ |
| Me | Me | H | 2,6-$F_2$Ph | $CF_3$ | *i*-Pr | H | H | 2,6-$F_2$Ph | $CF_3$ |
| Me | Me | H | 2,4-$F_2$Ph | $CF_3$ | *i*-Pr | H | H | 2,4-$F_2$Ph | $CF_3$ |
| Me | Me | H | 2,5-$F_2$Ph | $CF_3$ | *i*-Pr | H | H | 2,5-$F_2$Ph | $CF_3$ |
| Me | Me | H | 2-MeOPh | $CF_3$ | *i*-Pr | H | H | 2-MeOPh | $CF_3$ |
| Me | Me | H | 3-F-2-pyridyl | $CF_3$ | *i*-Pr | H | H | 3-F-2-pyridyl | $CF_3$ |
| Me | Me | H | 3-$CF_3$-2-pyridyl | $CF_3$ | *i*-Pr | H | H | 3-$CF_3$-2-pyridyl | $CF_3$ |
| Me | Me | H | 3-Me-2-pyridyl | $CF_3$ | *i*-Pr | H | H | 3-Me-2-pyridyl | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Et | Me | H | H | $CF_3$ | *i*-Pr | Me | H | H | $CF_3$ |
| Et | Me | H | H | $C_2F_5$ | *i*-Pr | Me | H | H | $C_2F_5$ |
| Et | Me | H | H | $OCF_3$ | *i*-Pr | Me | H | H | $OCF_3$ |
| Et | Me | H | Me | $CF_3$ | *i*-Pr | Me | H | Me | $CF_3$ |
| Et | Me | H | Me | $C_2F_5$ | *i*-Pr | Me | H | Me | $C_2F_5$ |
| Et | Me | H | Me | $OCF_3$ | *i*-Pr | Me | H | Me | $OCF_3$ |
| Me | Cl | H | H | $CF_3$ | Me | Cl | H | 2-ClPh | $CF_3$ |
| Me | Cl | H | H | $OCF_3$ | Me | Cl | H | 2-ClPh | $OCF_3$ |
| Me | Cl | H | H | $OCHF_2$ | Me | Cl | H | 2-ClPh | $SCHF_2$ |
| Me | Cl | H | H | $C_2F_5$ | Me | Cl | H | 2-ClPh | Br |
| Me | Cl | H | H | $OCF_2CHF_2$ | Me | Cl | H | 2-ClPh | Cl |
| Me | Cl | H | H | $SCF_2CHF_2$ | Me | Cl | H | 3-Cl-2-pyridyl | $CF_3$ |
| Me | Cl | H | H | *n*-$C_3F_7$ | Me | Cl | H | 3-Cl-2-pyridyl | $OCF_3$ |
| Me | Cl | H | H | *i*-$C_3F_7$ | Me | Cl | H | 3-Cl-2-pyridyl | $SCHF_2$ |
| Me | Cl | H | H | Br | Me | Cl | H | 3-Cl-2-pyridyl | Br |
| Me | Cl | H | H | Cl | Me | Cl | H | 3-Cl-2-pyridyl | Cl |
| Me | Cl | H | H | $SCF_3$ | Me | Cl | H | 3-Br-2-pyridyl | $CF_3$ |
| Me | Cl | H | Me | $CF_3$ | Me | Cl | H | 3-Br-2-pyridyl | $OCF_3$ |
| Me | Cl | H | Me | $OCF_3$ | Me | Cl | H | 3-Br-2-pyridyl | Br |
| Me | Cl | H | Me | $OCHF_2$ | Me | Cl | H | 3-Br-2-pyridyl | Cl |
| Me | Cl | H | Me | $C_2F_5$ | Me | Cl | H | $CF_3$ | Me |
| Me | Cl | H | Me | $OCF_2CHF_2$ | Me | Cl | H | F | $CF_3$ |
| Me | Cl | H | Me | $SCF_2CHF_2$ | Me | Cl | H | Cl | $CF_3$ |
| Me | Cl | H | Me | *n*-$C_3F_7$ | Me | Cl | H | *n*-Pr | $CF_3$ |
| Me | Cl | H | Me | *i*-$C_3F_7$ | Me | Cl | H | *i*-Pr | $CF_3$ |
| Me | Cl | H | Me | Br | Me | Cl | H | $CF_3$ | $CF_3$ |
| Me | Cl | H | Me | Cl | Me | Cl | H | OMe | $CF_3$ |
| Me | Cl | H | Me | $SCF_3$ | Me | Cl | H | 2-BrPh | $CF_3$ |
| Me | Cl | H | Me | I | Me | Cl | H | 2-MePh | $CF_3$ |
| Me | Cl | H | Me | SMe | Me | Cl | H | 2-CNPh | $CF_3$ |
| Me | Cl | H | Me | OMe | Me | Cl | H | 2-FPh | $CF_3$ |
| Me | Cl | H | Me | OEt | Me | Cl | H | 2,6-$F_2$Ph | $CF_3$ |
| Me | Cl | H | Me | Et | Me | Cl | H | 2,4-$F_2$Ph | $CF_3$ |
| Me | Cl | H | Me | $SO_2Me$ | Me | Cl | H | 2,5-$F_2$Ph | $CF_3$ |
| Me | Cl | H | Me | $SO_2CF_3$ | Me | Cl | H | 2-MeOPh | $CF_3$ |
| Me | Cl | H | Et | $CF_3$ | Me | Cl | H | 3-F-2-pyridyl | $CF_3$ |
| Me | Cl | H | Et | Br | Me | Cl | H | 3-$CF_3$-2- | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| | | | | | | | | pyridyl | |
| Me | Cl | H | Et | Cl | Me | Cl | H | 3-Me-2-pyridyl | $CF_3$ |
| Me | Cl | H | Ph | $CF_3$ | Et | Cl | H | H | $OCF_3$ |
| Me | Cl | H | Ph | Br | Et | Cl | H | H | $C_2F_5$ |
| Me | Cl | H | Ph | Cl | Et | Cl | H | Me | $CF_3$ |
| Me | Cl | H | 2-pyridyl | $CF_3$ | Et | Cl | H | Me | $C_2F_5$ |
| Me | Cl | H | 2-pyridyl | Cl | *i*-Pr | Cl | H | Me | $CF_3$ |

Table 9

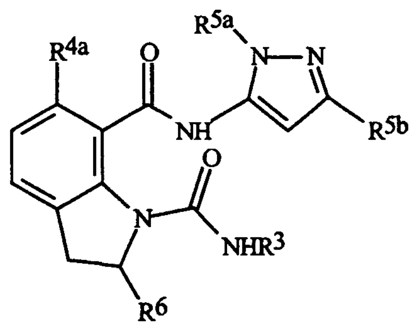

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CF_3$ | Me | H | H | H | $CF_3$ |
| Me | H | Me | H | $OCF_3$ | Me | H | H | H | $OCF_3$ |
| Me | H | Me | Me | $CF_3$ | Me | H | H | Me | $CF_3$ |
| Me | H | Me | Me | $OCF_3$ | Me | H | H | Me | $OCF_3$ |
| Me | H | Me | Me | $OCHF_2$ | Me | H | H | Me | $OCHF_2$ |
| Me | H | Me | Me | $C_2F_5$ | Me | H | H | Me | $C_2F_5$ |
| Me | H | Me | Me | $OCF_2CHF_2$ | Me | H | H | Me | $OCF_2CHF_2$ |
| Me | H | Me | Me | $SCF_2CHF_2$ | Me | H | H | Me | $SCF_2CHF_2$ |
| Me | H | Me | Me | *n*-$C_3F_7$ | Me | H | H | Me | *n*-$C_3F_7$ |
| Me | H | Me | Me | *i*-$C_3F_7$ | Me | H | H | Me | *i*-$C_3F_7$ |
| Me | H | Me | Me | Br | Me | H | H | Me | Br |
| Me | H | Me | Me | Cl | Me | H | H | Me | Cl |
| Me | H | Me | Me | $SCF_3$ | Me | H | H | Me | $SCF_3$ |
| Me | H | Me | Me | I | Me | H | H | Me | I |
| Me | H | Me | Me | SMe | Me | H | H | Me | SMe |
| Me | H | Me | Me | OMe | Me | H | H | Me | OMe |
| Me | H | Me | Me | OEt | Me | H | H | Me | OEt |
| Me | H | Me | Me | Et | Me | H | H | Me | Et |

| R3 | R4a | R6 | R5a | R5b | R3 | R4a | R6 | R5a | R5b |
|---|---|---|---|---|---|---|---|---|---|
| Me | H | Me | Me | $SO_2Me$ | Me | H | H | Me | $SO_2Me$ |
| Me | H | Me | Me | $SO_2CF_3$ | Me | H | H | Me | $SO_2CF_3$ |
| Me | H | Me | Et | $CF_3$ | Me | H | H | Et | $CF_3$ |
| Me | H | Me | Et | Br | Me | H | H | Et | Br |
| Me | H | Me | Et | Cl | Me | H | H | Et | Cl |
| Me | H | Me | Ph | $CF_3$ | Me | H | H | Ph | $CF_3$ |
| Me | H | Me | Ph | Br | Me | H | H | Ph | Br |
| Me | H | Me | Ph | Cl | Me | H | H | Ph | Cl |
| Me | H | Me | 2-pyridyl | $CF_3$ | Me | H | H | 2-pyridyl | $CF_3$ |
| Me | H | Me | 2-pyridyl | Cl | Me | H | H | 2-pyridyl | Cl |
| Me | H | Me | 2-ClPh | $CF_3$ | Me | H | H | 2-ClPh | $CF_3$ |
| Me | H | Me | 2-ClPh | $OCF_3$ | Me | H | H | 2-ClPh | $OCF_3$ |
| Me | H | Me | 2-ClPh | $SCHF_2$ | Me | H | H | 2-ClPh | $SCHF_2$ |
| Me | H | Me | 2-ClPh | Br | Me | H | H | 2-ClPh | Br |
| Me | H | Me | 2-ClPh | Cl | Me | H | H | 2-ClPh | Cl |
| Me | H | Me | 3-Cl-2-pyridyl | $CF_3$ | Me | H | H | 3-Cl-2-pyridyl | $CF_3$ |
| Me | H | Me | 3-Cl-2-pyridyl | $OCF_3$ | Me | H | H | 3-Cl-2-pyridyl | $OCF_3$ |
| Me | H | Me | 3-Cl-2-pyridyl | $SCHF_2$ | Me | H | H | 3-Cl-2-pyridyl | $SCHF_2$ |
| Me | H | Me | 3-Cl-2-pyridyl | Br | Me | H | H | 3-Cl-2-pyridyl | Br |
| Me | H | Me | 3-Cl-2-pyridyl | Cl | Me | H | H | 3-Cl-2-pyridyl | Cl |
| Me | H | Me | 3-Br-2-pyridyl | $CF_3$ | Me | H | H | 3-Br-2-pyridyl | $CF_3$ |
| Me | H | Me | 3-Br-2-pyridyl | $OCF_3$ | Me | H | H | 3-Br-2-pyridyl | $OCF_3$ |
| Me | H | Me | 3-Br-2-pyridyl | Br | Me | H | H | 3-Br-2-pyridyl | Br |
| Me | H | Me | 3-Br-2-pyridyl | Cl | Me | H | H | 3-Br-2-pyridyl | Cl |
| Me | H | Me | $CF_3$ | Me | Me | H | H | $CF_3$ | Me |
| Me | H | Me | *n*-Pr | $CF_3$ | Me | H | H | *n*-Pr | $CF_3$ |
| Me | H | Me | *i*-Pr | $CF_3$ | Me | H | H | *i*-Pr | $CF_3$ |
| Me | H | Me | $CF_3$ | $CF_3$ | Me | H | H | $CF_3$ | $CF_3$ |
| Me | H | Me | OMe | $CF_3$ | Me | H | H | OMe | $CF_3$ |
| Me | H | Me | 2-BrPh | $CF_3$ | Me | H | H | 2-BrPh | $CF_3$ |
| Me | H | Me | 2-MePh | $CF_3$ | Me | H | H | 2-MePh | $CF_3$ |
| Me | H | Me | 2-CNPh | $CF_3$ | Me | H | H | 2-CNPh | $CF_3$ |
| Me | H | Me | 2-FPh | $CF_3$ | Me | H | H | 2-FPh | $CF_3$ |
| Me | H | Me | 2,6-$F_2$Ph | $CF_3$ | Me | H | H | 2,6-$F_2$Ph | $CF_3$ |
| Me | H | Me | 2,4-$F_2$Ph | $CF_3$ | Me | H | H | 2,4-$F_2$Ph | $CF_3$ |
| Me | H | Me | 2,5-$F_2$Ph | $CF_3$ | Me | H | H | 2,5-$F_2$Ph | $CF_3$ |
| Me | H | Me | 2-MeOPh | $CF_3$ | Me | H | H | 2-MeOPh | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | 3-F-2-pyridyl | $CF_3$ | Me | H | H | 3-F-2-pyridyl | $CF_3$ |
| Me | H | Me | 3-$CF_3$-2-pyridyl | $CF_3$ | Me | H | H | 3-$CF_3$-2-pyridyl | $CF_3$ |
| Me | H | Me | 3-Me-2-pyridyl | $CF_3$ | Me | H | H | 3-Me-2-pyridyl | $CF_3$ |
| Et | H | Me | Me | $CF_3$ | Et | H | H | Me | $CF_3$ |
| Et | H | Me | Me | $C_2F_5$ | Et | H | H | Me | $C_2F_5$ |
| Et | H | Me | Me | $OCF_3$ | Et | H | H | Me | $OCF_3$ |
| Me | Me | H | Me | $C_2F_5$ | *i*-Pr | H | H | Me | $C_2F_5$ |
| Me | Me | H | Me | $OCF_2CHF_2$ | *i*-Pr | H | H | Me | $OCF_2CHF_2$ |
| Me | Me | H | Me | $SCF_2CHF_2$ | *i*-Pr | H | H | Me | $SCF_2CHF_2$ |
| Me | Me | H | Me | *n*-$C_3F_7$ | *i*-Pr | H | H | Me | *n*-$C_3F_7$ |
| Me | Me | H | Me | *i*-$C_3F_7$ | *i*-Pr | H | H | Me | *i*-$C_3F_7$ |
| Me | Me | H | Me | Br | *i*-Pr | H | H | Me | Br |
| Me | Me | H | Me | Cl | *i*-Pr | H | H | Me | Cl |
| Me | Me | H | Me | $SCF_3$ | *i*-Pr | H | H | Me | $SCF_3$ |
| Me | Me | H | Me | I | *i*-Pr | H | H | Me | I |
| Me | Me | H | Me | SMe | *i*-Pr | H | H | Me | SMe |
| Me | Me | H | Me | OMe | *i*-Pr | H | H | Me | OMe |
| Me | Me | H | Me | OEt | *i*-Pr | H | H | Me | OEt |
| Me | Me | H | Me | Et | *i*-Pr | H | H | Me | Et |
| Me | Me | H | Me | $SO_2Me$ | *i*-Pr | H | H | Me | $SO_2Me$ |
| Me | Me | H | Me | $SO_2CF_3$ | *i*-Pr | H | H | Me | $SO_2CF_3$ |
| Me | Me | H | Et | $CF_3$ | *i*-Pr | H | H | Et | $CF_3$ |
| Me | Me | H | Et | Br | *i*-Pr | H | H | Et | Br |
| Me | Me | H | Et | Cl | *i*-Pr | H | H | Et | Cl |
| Me | Me | H | Ph | $CF_3$ | *i*-Pr | H | H | Ph | $CF_3$ |
| Me | Me | H | Ph | Br | *i*-Pr | H | H | Ph | Br |
| Me | Me | H | Ph | Cl | *i*-Pr | H | H | Ph | Cl |
| Me | Me | H | 2-pyridyl | $CF_3$ | *i*-Pr | H | H | 2-pyridyl | $CF_3$ |
| Me | Me | H | 2-pyridyl | Cl | *i*-Pr | H | H | 2-pyridyl | Cl |
| Me | Me | H | 2-ClPh | $CF_3$ | *i*-Pr | H | H | 2-ClPh | $CF_3$ |
| Me | Me | H | 2-ClPh | $OCF_3$ | *i*-Pr | H | H | 2-ClPh | $OCF_3$ |
| Me | Me | H | 2-ClPh | $SCHF_2$ | *i*-Pr | H | H | 2-ClPh | $SCHF_2$ |
| Me | Me | H | 2-ClPh | Br | *i*-Pr | H | H | 2-ClPh | Br |
| Me | Me | H | 2-ClPh | Cl | *i*-Pr | H | H | 2-ClPh | Cl |
| Me | Me | H | 3-Cl-2-pyridyl | $CF_3$ | *i*-Pr | H | H | 3-Cl-2-pyridyl | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | 3-Cl-2-pyridyl | $OCF_3$ | *i*-Pr | H | H | 3-Cl-2-pyridyl | $OCF_3$ |
| Me | Me | H | 3-Cl-2-pyridyl | $SCHF_2$ | *i*-Pr | H | H | 3-Cl-2-pyridyl | $SCHF_2$ |
| Me | Me | H | 3-Cl-2-pyridyl | Br | *i*-Pr | H | H | 3-Cl-2-pyridyl | Br |
| Me | Me | H | 3-Cl-2-pyridyl | Cl | *i*-Pr | H | H | 3-Cl-2-pyridyl | Cl |
| Me | Me | H | 3-Br-2-pyridyl | $CF_3$ | *i*-Pr | H | H | 3-Br-2-pyridyl | $CF_3$ |
| Me | Me | H | 3-Br-2-pyridyl | $OCF_3$ | *i*-Pr | H | H | 3-Br-2-pyridyl | $OCF_3$ |
| Me | Me | H | 3-Br-2-pyridyl | Br | *i*-Pr | H | H | 3-Br-2-pyridyl | Br |
| Me | Me | H | 3-Br-2-pyridyl | Cl | *i*-Pr | H | H | 3-Br-2-pyridyl | Cl |
| Me | Me | H | $CF_3$ | Me | *i*-Pr | H | H | $CF_3$ | Me |
| Me | Me | H | *n*-Pr | $CF_3$ | *i*-Pr | H | H | *n*-Pr | $CF_3$ |
| Me | Me | H | *i*-Pr | $CF_3$ | *i*-Pr | H | H | *i*-Pr | $CF_3$ |
| Me | Me | H | $CF_3$ | $CF_3$ | *i*-Pr | H | H | $CF_3$ | $CF_3$ |
| Me | Me | H | 2-BrPh | $CF_3$ | *i*-Pr | H | H | 2-BrPh | $CF_3$ |
| Me | Me | H | 2-MePh | $CF_3$ | *i*-Pr | H | H | 2-MePh | $CF_3$ |
| Me | Me | H | 2-CNPh | $CF_3$ | *i*-Pr | H | H | 2-CNPh | $CF_3$ |
| Me | Me | H | 2-FPh | $CF_3$ | *i*-Pr | H | H | 2-FPh | $CF_3$ |
| Me | Me | H | 2,6-$F_2$Ph | $CF_3$ | *i*-Pr | H | H | 2,6-$F_2$Ph | $CF_3$ |
| Me | Me | H | 2,4-$F_2$Ph | $CF_3$ | *i*-Pr | H | H | 2,4-$F_2$Ph | $CF_3$ |
| Me | Me | H | 2,5-$F_2$Ph | $CF_3$ | *i*-Pr | H | H | 2,5-$F_2$Ph | $CF_3$ |
| Me | Me | H | 2-MeOPh | $CF_3$ | *i*-Pr | H | H | 2-MeOPh | $CF_3$ |
| Me | Me | H | 3-F-2-pyridyl | $CF_3$ | *i*-Pr | H | H | 3-F-2-pyridyl | $CF_3$ |
| Me | Me | H | 3-$CF_3$-2-pyridyl | $CF_3$ | *i*-Pr | H | H | 3-$CF_3$-2-pyridyl | $CF_3$ |
| Me | Me | H | 3-Me-2-pyridyl | $CF_3$ | *i*-Pr | H | H | 3-Me-2-pyridyl | $CF_3$ |
| Et | Me | H | Me | $CF_3$ | *i*-Pr | Me | H | Me | $CF_3$ |
| Et | Me | H | Me | $C_2F_5$ | *i*-Pr | Me | H | Me | $C_2F_5$ |
| Et | Me | H | Me | $OCF_3$ | *i*-Pr | Me | H | Me | $OCF_3$ |
| Me | Cl | H | Me | $CF_3$ | Me | Cl | H | 2-ClPh | Br |
| Me | Cl | H | Me | $OCF_3$ | Me | Cl | H | 2-ClPh | Cl |
| Me | Cl | H | Me | $OCHF_2$ | Me | Cl | H | 3-Cl-2-pyridyl | $CF_3$ |
| Me | Cl | H | Me | $C_2F_5$ | Me | Cl | H | 3-Cl-2-pyridyl | $OCF_3$ |
| Me | Cl | H | Me | $OCF_2CHF_2$ | Me | Cl | H | 3-Cl-2-pyridyl | $SCHF_2$ |
| Me | Cl | H | Me | $SCF_2CHF_2$ | Me | Cl | H | 3-Cl-2-pyridyl | Br |
| Me | Cl | H | Me | *n*-$C_3F_7$ | Me | Cl | H | 3-Cl-2-pyridyl | Cl |
| Me | Cl | H | Me | *i*-$C_3F_7$ | Me | Cl | H | 3-Br-2-pyridyl | $CF_3$ |
| Me | Cl | H | Me | Br | Me | Cl | H | 3-Br-2-pyridyl | $OCF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Cl | H | Me | Cl | Me | Cl | H | 3-Br-2-pyridyl | Br |
| Me | Cl | H | Me | $SCF_3$ | Me | Cl | H | 3-Br-2-pyridyl | Cl |
| Me | Cl | H | Me | I | Me | Cl | H | $CF_3$ | Me |
| Me | Cl | H | Me | SMe | Me | Cl | H | *n*-Pr | $CF_3$ |
| Me | Cl | H | Me | OMe | Me | Cl | H | *i*-Pr | $CF_3$ |
| Me | Cl | H | Me | OEt | Me | Cl | H | $CF_3$ | $CF_3$ |
| Me | Cl | H | Me | Et | Me | Cl | H | 2-BrPh | $CF_3$ |
| Me | Cl | H | Me | $SO_2Me$ | Me | Cl | H | 2-MePh | $CF_3$ |
| Me | Cl | H | Me | $SO_2CF_3$ | Me | Cl | H | 2-CNPh | $CF_3$ |
| Me | Cl | H | Et | $CF_3$ | Me | Cl | H | 2-FPh | $CF_3$ |
| Me | Cl | H | Et | Br | Me | Cl | H | 2,6-$F_2$Ph | $CF_3$ |
| Me | Cl | H | Et | Cl | Me | Cl | H | 2,4-$F_2$Ph | $CF_3$ |
| Me | Cl | H | Ph | $CF_3$ | Me | Cl | H | 2,5-$F_2$Ph | $CF_3$ |
| Me | Cl | H | Ph | Br | Me | Cl | H | 2-MeOPh | $CF_3$ |
| Me | Cl | H | Ph | Cl | Me | Cl | H | 3-F-2-pyridyl | $CF_3$ |
| Me | Cl | H | 2-pyridyl | $CF_3$ | Me | Cl | H | 3-$CF_3$-2-pyridyl | $CF_3$ |
| Me | Cl | H | 2-pyridyl | Cl | Me | Cl | H | 3-Me-2-pyridyl | $CF_3$ |
| Me | Cl | H | 2-ClPh | $CF_3$ | Et | Cl | H | Me | $CF_3$ |
| Me | Cl | H | 2-ClPh | $OCF_3$ | Et | Cl | H | Me | $C_2F_5$ |
| Me | Cl | H | 2-ClPh | $SCHF_2$ | *i*-Pr | Cl | H | Me | $CF_3$ |

Table 10

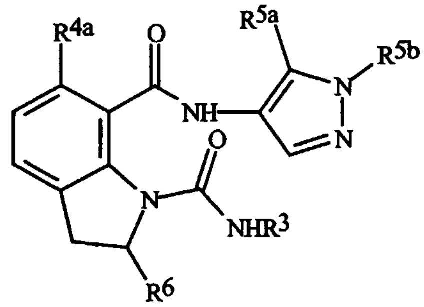

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CHF_2$ | Me | H | H | H | $CHF_2$ |
| Me | H | Me | H | $CH_2CF_3$ | Me | H | H | H | $CH_2CF_3$ |
| Et | H | Me | H | $CH_2CF_3$ | Et | H | H | H | $CH_2CF_3$ |
| Me | H | Me | Me | $CH_2CF_3$ | Me | H | H | Me | $CH_2CF_3$ |
| Me | H | Me | Et | $CH_2CF_3$ | Me | H | H | Et | $CH_2CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | Me | $CF_2CHF_2$ | Me | H | H | Me | $CF_2CHF_2$ |
| Me | H | Me | Et | $CHF_2$ | Me | H | H | Et | $CHF_2$ |
| Me | H | Me | Me | $CHF_2$ | Me | H | H | Me | $CHF_2$ |
| Me | H | Me | Me | $CBrF_2$ | Me | H | H | Me | $CBrF_2$ |
| Me | H | Me | Me | $CHF_2$ | Me | H | H | Me | $CHF_2$ |
| Et | H | Me | Me | $CH_2CF_3$ | Et | H | H | Me | $CH_2CF_3$ |
| Me | H | Me | Me | Et | Me | H | H | Me | Et |
| Me | H | Me | Me | *n*-Pr | Me | H | H | Me | *n*-Pr |
| Me | H | Me | Me | $CH_2C_2F_5$ | Me | H | H | Me | $CH_2C_2F_5$ |
| *n*-Pr | H | Me | Me | $CH_2CF_3$ | *n*-Pr | H | H | Me | $CH_2CF_3$ |
| Me | H | Me | Me | $CF_3$ | Me | H | H | Me | $CF_3$ |
| Et | H | Me | Me | $C_2F_5$ | Et | H | H | Me | $C_2F_5$ |
| Me | H | Me | Et | $CHF_2$ | Me | H | H | Et | $CHF_2$ |
| Me | H | Me | *n*-Pr | $CH_2CF_3$ | Me | H | H | *n*-Pr | $CH_2CF_3$ |
| Me | H | Me | *i*-Pr | $CHF_2$ | Me | H | H | *i*-Pr | $CHF_2$ |
| Me | H | Me | Cl | $CH_2CF_3$ | Me | H | H | Cl | $CH_2CF_3$ |
| Me | H | Me | F | $CH_2CF_3$ | Me | H | H | F | $CH_2CF_3$ |
| Me | H | Me | Me | $CH_2Cl$ | Me | H | H | Me | $CH_2Cl$ |
| Me | H | Me | Me | $CClF_2$ | Me | H | H | Me | $CClF_2$ |
| Me | H | Me | Me | $CH_2CH_2Cl$ | Me | H | H | Me | $CH_2CH_2Cl$ |
| Me | H | Me | Me | *n*-$C_3F_7$ | Me | H | H | Me | *n*-$C_3F_7$ |
| Me | H | Me | Me | *i*-$C_3F_7$ | Me | H | H | Me | *i*-$C_3F_7$ |
| Me | H | Me | Me | Allyl | Me | H | H | Me | Allyl |
| Me | H | Me | Et | $CF_2CHF_2$ | Me | H | H | Me | $CF_2CHF_2$ |
| Me | H | Me | Et | *i*-$C_3F_7$ | Me | H | H | Me | *i*-$C_3F_7$ |
| Me | H | Me | *i*-Pr | $CF_2CHF_2$ | Me | H | H | Me | $CF_2CHF_2$ |
| Me | H | Me | *n*-Pr | $CF_2CHF_2$ | Me | H | H | Me | $CF_2CHF_2$ |
| Me | H | Me | $CF_3$ | $CF_2CHF_2$ | Me | H | H | $CF_3$ | $CF_2CHF_2$ |
| Me | H | Me | $CF_3$ | Me | Me | H | H | $CF_3$ | Me |
| Me | H | Me | OMe | $CH_2CF_3$ | Me | H | H | OMe | $CH_2CF_3$ |
| Me | H | Me | H | $CH_2CF_3$ | Me | H | H | H | $CH_2CF_3$ |
| Me | H | Me | H | $CH_2CF_3$ | Me | H | H | H | $CH_2CF_3$ |
| Me | H | Me | H | $C_2F_5$ | Me | H | H | H | $C_2F_5$ |
| Et | H | Me | H | $C_2F_5$ | Et | H | H | H | $C_2F_5$ |
| Me | H | Me | H | $C_2F_5$ | Me | H | H | H | $C_2F_5$ |
| Me | H | Me | H | $CF_2CHF_2$ | Me | H | H | H | $CF_2CHF_2$ |
| Me | H | Me | *i*-Pr | $CH_2CF_3$ | Me | H | H | H | $CH_2CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $n$-$C_3F_7$ | Me | H | H | H | $n$-$C_3F_7$ |
| Me | H | Me | H | $i$-$C_3F_7$ | Me | H | H | H | $i$-$C_3F_7$ |
| Me | H | Me | Ph | $CH_2CF_3$ | Me | H | H | H | $CH_2CF_3$ |
| Me | H | Me | Ph | $CF_2CHF_2$ | Me | H | H | H | $CF_2CHF_2$ |
| Me | H | Me | Ph | $CHF_2$ | Me | H | H | H | $CHF_2$ |
| Me | H | Me | 2-pyridyl | $CH_2CF_3$ | Me | H | H | Ph | $CH_2CF_3$ |
| Me | H | Me | 2-pyridyl | $CF_2CHF_2$ | Me | H | H | Ph | $CF_2CHF_2$ |
| Me | H | Me | 2-ClPh | $CH_2CF_3$ | Me | H | H | Ph | $CH_2CF_3$ |
| Me | H | Me | 2-ClPh | $CF_2CHF_2$ | Me | H | H | 2-pyridyl | $CF_2CHF_2$ |
| Me | H | Me | 2-ClPh | $CHF_2$ | Me | H | H | 2-pyridyl | $CHF_2$ |
| Me | H | Me | 2-ClPh | Et | Me | H | H | 2-ClPh | Et |
| Me | H | Me | 2-ClPh | $CBrF_2$ | Me | H | H | 2-ClPh | $CBrF_2$ |
| Me | H | Me | 2-BrPh | $CH_2CF_3$ | Me | H | H | 2-ClPh | $CH_2CF_3$ |
| Me | H | Me | 2-MePh | $CH_2CF_3$ | Me | H | H | 2-ClPh | $CH_2CF_3$ |
| Me | H | Me | 2-CNPh | $CH_2CF_3$ | Me | H | H | 2-ClPh | $CH_2CF_3$ |
| Me | H | Me | 2-FPh | $CH_2CF_3$ | Me | H | H | 2-BrPh | $CH_2CF_3$ |
| Me | H | Me | 2,6-$F_2$Ph | $CH_2CF_3$ | Me | H | H | 2-MePh | $CH_2CF_3$ |
| Me | H | Me | 2,4-$F_2$Ph | $CH_2CF_3$ | Me | H | H | 2-CNPh | $CH_2CF_3$ |
| Me | H | Me | 2,5-$F_2$Ph | $CH_2CF_3$ | Me | H | H | 2-FPh | $CH_2CF_3$ |
| Me | H | Me | 2-MeOPh | $CH_2CF_3$ | Me | H | H | 2,6-$F_2$Ph | $CH_2CF_3$ |
| Me | H | Me | 3-Cl-2-pyridyl | $CH_2CF_3$ | Me | H | H | 2,4-$F_2$Ph | $CH_2CF_3$ |
| Me | H | Me | 3-Cl-2-pyridyl | $CF_2CHF_2$ | Me | H | H | 2,5-$F_2$Ph | $CF_2CHF_2$ |
| Me | H | Me | 3-Cl-2-pyridyl | $CF_3$ | Me | H | H | 2-MeOPh | $CF_3$ |
| Me | H | Me | 3-Cl-2-pyridyl | $CHF_2$ | Me | H | H | 3-Cl-2-pyridyl | $CHF_2$ |
| Me | H | Me | 3-Cl-2-pyridyl | $CBrF_2$ | Me | H | H | 3-Cl-2-pyridyl | $CBrF_2$ |
| Me | H | Me | 3-F-2-pyridyl | $CH_2CF_3$ | Me | H | H | 3-Cl-2-pyridyl | $CH_2CF_3$ |
| Me | H | Me | 3-$CF_3$-2-pyridyl | $CH_2CF_3$ | Me | H | H | 3-$CF_3$-2-pyridyl | $CH_2CF_3$ |
| Me | H | Me | 3-Me-2-pyridyl | $CH_2CF_3$ | Me | H | H | 3-Me-2-pyridyl | $CF_2CHF_2$ |
| Me | H | Me | 3-Br-2-pyridyl | $CF_3$ | Me | H | H | 3-F-2-pyridyl | $CF_3$ |
| Me | H | Me | 3-Br-2-pyridyl | $CH_2CF_3$ | Me | H | H | 3-Cl-2-pyridyl | $CH_2CF_3$ |
| Me | H | Me | 3-Br-2-pyridyl | $CF_2CHF_2$ | Me | H | H | 3-Cl-2-pyridyl | $CH_2CF_3$ |
| Me | H | Me | 3-Br-2-pyridyl | $CClF_2$ | Me | H | H | 3-Br-2-pyridyl | $CClF_2$ |
| Me | Me | H | H | $CHF_2$ | Me | H | H | 3-Br-2-pyridyl | $CH_2CF_3$ |
| Me | Me | H | H | $CH_2CF_3$ | Me | H | H | 3-Br-2-pyridyl | $CF_3$ |
| Et | Me | H | H | $CH_2CF_3$ | Me | H | H | 3-Br-2-pyridyl | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | Me | $CH_2CF_3$ | *i*-Pr | H | H | Et | $CH_2C_2F_5$ |
| Me | Me | H | Et | $CH_2CF_3$ | *i*-Pr | H | H | *n*-Pr | $CH_2CF_3$ |
| Me | Me | H | Me | $CF_2CHF_2$ | *i*-Pr | H | H | *i*-Pr | $CF_3$ |
| Me | Me | H | Et | $CHF_2$ | *i*-Pr | H | H | Cl | $C_2F_5$ |
| Me | Me | H | Me | $CHF_2$ | *i*-Pr | H | H | F | $CHF_2$ |
| Me | Me | H | Me | $CBrF_2$ | *i*-Pr | H | H | Me | $CH_2CF_3$ |
| Me | Me | H | Me | $CHF_2$ | *i*-Pr | H | H | Me | $CHF_2$ |
| Et | Me | H | Me | $CH_2CF_3$ | *i*-Pr | H | H | Me | $CH_2CF_3$ |
| Me | Me | H | Me | Et | *i*-Pr | H | H | Me | $CH_2CF_3$ |
| Me | Me | H | Me | *n*-Pr | *i*-Pr | H | H | Me | $CH_2Cl$ |
| Me | Me | H | Me | $CH_2C_2F_5$ | *i*-Pr | H | H | Me | $CClF_2$ |
| *n*-Pr | Me | H | Me | $CH_2CF_3$ | *i*-Pr | H | H | Me | $CH_2CH_2Cl$ |
| Me | Me | H | Me | $CF_3$ | *i*-Pr | H | H | Me | *n*-$C_3F_7$ |
| Et | Me | H | Me | $C_2F_5$ | *i*-Pr | H | H | Me | *i*-$C_3F_7$ |
| Me | Me | H | Et | $CHF_2$ | *i*-Pr | H | H | Me | Allyl |
| Me | Me | H | *n*-Pr | $CH_2CF_3$ | *i*-Pr | H | H | $CF_3$ | $CF_2CHF_2$ |
| Me | Me | H | *i*-Pr | $CHF_2$ | *i*-Pr | H | H | $CF_3$ | *i*-$C_3F_7$ |
| Me | Me | H | Cl | $CH_2CF_3$ | *i*-Pr | H | H | OMe | $CF_2CHF_2$ |
| Me | Me | H | F | $CH_2CF_3$ | *i*-Pr | H | H | H | $CF_2CHF_2$ |
| Me | Me | H | Me | $CH_2Cl$ | *i*-Pr | H | H | H | $CF_2CHF_2$ |
| Me | Me | H | Me | $CClF_2$ | *i*-Pr | H | H | H | Me |
| Me | Me | H | Me | $CH_2CH_2Cl$ | *i*-Pr | H | H | H | $CH_2CF_3$ |
| Me | Me | H | Me | *n*-$C_3F_7$ | *i*-Pr | H | H | H | $CH_2CF_3$ |
| Me | Me | H | Me | *i*-$C_3F_7$ | *i*-Pr | H | H | H | $C_2F_5$ |
| Me | Me | H | Me | Allyl | *i*-Pr | H | H | H | $C_2F_5$ |
| Me | Me | H | Me | $CF_2CHF_2$ | *i*-Pr | H | H | H | $C_2F_5$ |
| Me | Me | H | Me | *i*-$C_3F_7$ | *i*-Pr | H | H | H | $CF_2CHF_2$ |
| Me | Me | H | Me | $CF_2CHF_2$ | *i*-Pr | H | H | H | $CH_2CF_3$ |
| Me | Me | H | Me | $CF_2CHF_2$ | *i*-Pr | H | H | H | *n*-$C_3F_7$ |
| Me | Me | H | $CF_3$ | $CF_2CHF_2$ | *i*-Pr | H | H | Ph | *i*-$C_3F_7$ |
| Me | Me | H | $CF_3$ | Me | *i*-Pr | H | H | Ph | $CH_2CF_3$ |
| Me | Me | H | OMe | $CH_2CF_3$ | *i*-Pr | H | H | Ph | $CF_2CHF_2$ |
| Me | Me | H | H | $CH_2CF_3$ | *i*-Pr | H | H | 2-pyridyl | $CHF_2$ |
| Me | Me | H | H | $CH_2CF_3$ | *i*-Pr | H | H | 2-pyridyl | $CH_2CF_3$ |
| Me | Me | H | H | $C_2F_5$ | *i*-Pr | H | H | 2-ClPh | $CF_2CHF_2$ |
| Et | Me | H | H | $C_2F_5$ | *i*-Pr | H | H | 2-ClPh | $CH_2CF_3$ |
| Me | Me | H | H | $C_2F_5$ | *i*-Pr | H | H | 2-ClPh | $CF_2CHF_2$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | H | $CF_2CHF_2$ | *i*-Pr | H | H | 2-ClPh | $CHF_2$ |
| Me | Me | H | H | $CH_2CF_3$ | *i*-Pr | H | H | 2-ClPh | Et |
| Me | Me | H | H | *n*-$C_3F_7$ | *i*-Pr | H | H | 2-BrPh | $CBrF_2$ |
| Me | Me | H | H | *i*-$C_3F_7$ | *i*-Pr | H | H | 2-MePh | $CH_2CF_3$ |
| Me | Me | H | H | $CH_2CF_3$ | *i*-Pr | H | H | 2-CNPh | $CH_2CF_3$ |
| Me | Me | H | H | $CF_2CHF_2$ | *i*-Pr | H | H | 2-FPh | $CH_2CF_3$ |
| Me | Me | H | H | $CHF_2$ | *i*-Pr | H | H | 2,6-$F_2$Ph | $CH_2CF_3$ |
| Me | Me | H | Ph | $CH_2CF_3$ | *i*-Pr | H | H | 2,4-$F_2$Ph | $CH_2CF_3$ |
| Me | Me | H | Ph | $CF_2CHF_2$ | *i*-Pr | H | H | 2,5-$F_2$Ph | $CH_2CF_3$ |
| Me | Me | H | Ph | $CH_2CF_3$ | *i*-Pr | H | H | 2-MeOPh | $CH_2CF_3$ |
| Me | Me | H | 2-pyridyl | $CF_2CHF_2$ | *i*-Pr | H | H | 3-Cl-2-pyridyl | $CH_2CF_3$ |
| Me | Me | H | 2-pyridyl | $CHF_2$ | *i*-Pr | H | H | 3-Cl-2-pyridyl | $CH_2CF_3$ |
| Me | Me | H | 2-ClPh | Et | *i*-Pr | H | H | 3-Cl-2-pyridyl | $CF_2CHF_2$ |
| Me | Me | H | 2-ClPh | $CBrF_2$ | *i*-Pr | H | H | 3-Cl-2-pyridyl | $CF_3$ |
| Me | Me | H | 2-ClPh | $CH_2CF_3$ | *i*-Pr | H | H | 3-Cl-2-pyridyl | $CHF_2$ |
| Me | Me | H | 2-ClPh | $CH_2CF_3$ | *i*-Pr | H | H | 3-F-2-pyridyl | $CBrF_2$ |
| Me | Me | H | 2-ClPh | $CH_2CF_3$ | *i*-Pr | H | H | 3-Br-2-pyridyl | $CH_2CF_3$ |
| Me | Me | H | 2-BrPh | $CH_2CF_3$ | *i*-Pr | H | H | 3-$CF_3$-2-pyridyl | $CH_2CF_3$ |
| Me | Me | H | 2-MePh | $CH_2CF_3$ | *i*-Pr | H | H | 3-Me-2-pyridyl | $CH_2CF_3$ |
| Me | Me | H | 2-CNPh | $CH_2CF_3$ | *i*-Pr | H | H | 3-Br-2-pyridyl | $CF_3$ |
| Me | Me | H | 2-FPh | $CH_2CF_3$ | *i*-Pr | H | H | 3-Br-2-pyridyl | $CH_2CF_3$ |
| Me | Me | H | 2,6-$F_2$Ph | $CH_2CF_3$ | *i*-Pr | H | H | 3-Br-2-pyridyl | $CF_2CHF_2$ |
| Me | Me | H | 2,4-$F_2$Ph | $CH_2CF_3$ | Me | Cl | H | F | $CH_2CF_3$ |
| Me | Me | H | 2,5-$F_2$Ph | $CF_2CHF_2$ | Me | Cl | H | Me | $CHF_2$ |
| Me | Me | H | 2-MeOPh | $CF_3$ | Me | Cl | H | Me | $CH_2CF_3$ |
| Me | Me | H | 3-Cl-2-pyridyl | $CHF_2$ | Me | Cl | H | Me | $CH_2CF_3$ |
| Me | Me | H | 3-Cl-2-pyridyl | $CBrF_2$ | Me | Cl | H | Me | $CH_2Cl$ |
| Me | Me | H | 3-Cl-2-pyridyl | $CH_2CF_3$ | Me | Cl | H | Me | $CClF_2$ |
| Me | Me | H | 3-Cl-2-pyridyl | $CH_2CF_3$ | Me | Cl | H | Me | $CH_2CH_2Cl$ |
| Me | Me | H | 3-Cl-2-pyridyl | $CH_2CF_3$ | Me | Cl | H | Me | *n*-$C_3F_7$ |
| Me | Me | H | 3-F-2-pyridyl | $CF_3$ | Me | Cl | H | Me | *i*-$C_3F_7$ |
| Me | Me | H | 3-$CF_3$-2-pyridyl | $CH_2CF_3$ | Me | Cl | H | Me | Allyl |
| Me | Me | H | 3-Me-2-pyridyl | $CF_2CHF_2$ | Me | Cl | H | Me | $CF_2CHF_2$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | 3-Br-2-pyridyl | $CClF_2$ | Me | Cl | H | $CF_3$ | $i$-$C_3F_7$ |
| Me | Me | H | 3-Br-2-pyridyl | $CH_2CF_3$ | Me | Cl | H | $CF_3$ | $CF_2CHF_2$ |
| Me | Me | H | 3-Br-2-pyridyl | $CF_3$ | Me | Cl | H | OMe | $CF_2CHF_2$ |
| Me | Me | H | 3-Br-2-pyridyl | $CF_3$ | Me | Cl | H | H | $CF_2CHF_2$ |
| Me | Cl | H | Me | $CHF_2$ | Me | Cl | H | H | Me |
| Me | Cl | H | Et | $CH_2CF_3$ | Me | Cl | H | H | $CH_2CF_3$ |
| Me | Cl | H | Me | $CH_2CF_3$ | Et | Cl | H | H | $CH_2CF_3$ |
| Me | Cl | H | Et | $CH_2CF_3$ | Me | Cl | H | H | $CH_2CF_3$ |
| Me | Cl | H | Me | $CH_2CF_3$ | Me | Cl | H | H | $C_2F_5$ |
| Me | Cl | H | Me | $CF_2CHF_2$ | Me | Cl | H | H | $C_2F_5$ |
| Me | Cl | H | Me | $CHF_2$ | Me | Cl | H | H | $C_2F_5$ |
| Et | Cl | H | Me | $CHF_2$ | Me | Cl | H | H | $CF_2CHF_2$ |
| Me | Cl | H | Me | $CBrF_2$ | Me | Cl | H | H | $CH_2CF_3$ |
| Me | Cl | H | Me | $CHF_2$ | Me | Cl | H | H | $n$-$C_3F_7$ |
| Me | Cl | H | Me | $CH_2CF_3$ | Me | Cl | H | 3-Me-2-pyridyl | $CH_2CF_3$ |
| $n$-Pr | Cl | H | Me | Et | Me | Cl | H | H | $i$-$C_3F_7$ |
| Me | Cl | H | Me | $n$-Pr | Me | Cl | H | Ph | $CH_2CF_3$ |
| Et | Cl | H | Me | $CH_2C_2F_5$ | Me | Cl | H | Ph | $CF_2CHF_2$ |
| Me | Cl | H | Et | $CH_2CF_3$ | Me | Cl | H | Ph | $CHF_2$ |
| Me | Cl | H | $n$-Pr | $CF_3$ | Me | Cl | H | 2-pyridyl | $CH_2CF_3$ |
| Me | Cl | H | $i$-Pr | $C_2F_5$ | Me | Cl | H | 2-pyridyl | $CF_2CHF_2$ |
| Me | Cl | H | Cl | $CHF_2$ | Me | Cl | H | 2-ClPh | $CH_2CF_3$ |
| Me | Cl | H | 3-Cl-2-pyridyl | $CH_2CF_3$ | Me | Cl | H | 2-ClPh | $CF_2CHF_2$ |
| Me | Cl | H | 3-Cl-2-pyridyl | $CF_2CHF_2$ | Me | Cl | H | 2-FPh | $CH_2CF_3$ |
| Me | Cl | H | 3-Cl-2-pyridyl | $CF_3$ | Me | Cl | H | 2,6-$F_2$Ph | $CH_2CF_3$ |
| Me | Cl | H | 3-Cl-2-pyridyl | $CHF_2$ | Me | Cl | H | 2,4-$F_2$Ph | $CH_2CF_3$ |
| Me | Cl | H | 3-Cl-2-pyridyl | $CBrF_2$ | Me | Cl | H | 2-ClPh | Et |
| Me | Cl | H | 3-F-2-pyridyl | $CH_2CF_3$ | Me | Cl | H | 2-ClPh | $CBrF_2$ |
| Me | Cl | H | 3-$CF_3$-2-pyridyl | $CH_2CF_3$ | Me | Cl | H | 2-BrPh | $CH_2CF_3$ |
| Me | Cl | H | 3-Br-2-pyridyl | $CF_3$ | Me | Cl | H | 2-MePh | $CH_2CF_3$ |
| Me | Cl | H | 3-Br-2-pyridyl | $CH_2CF_3$ | Me | Cl | H | 2-CNPh | $CH_2CF_3$ |
| Me | Cl | H | 3-Br-2-pyridyl | $CF_2CHF_2$ | Me | Cl | H | 2-MeOPh | $CH_2CF_3$ |
| Me | Cl | H | 3-Br-2-pyridyl | $CClF_2$ | Me | Cl | H | 2,5-$F_2$Ph | $CH_2CF_3$ |
| Me | Cl | H | 2-ClPh | $CHF_2$ | | | | | |

Table 11

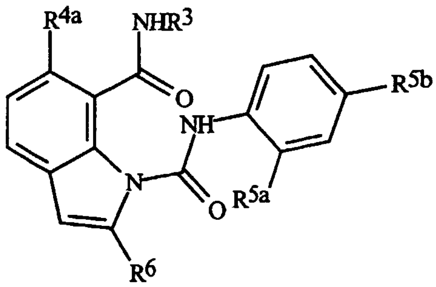

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CF_3$ | Me | H | H | H | $CF_3$ |
| Me | H | Me | H | $OCF_3$ | Me | H | H | H | $OCF_3$ |
| Me | H | Me | H | $OCHF_2$ | Me | H | H | H | $OCHF_2$ |
| Me | H | Me | H | $C_2F_5$ | Me | H | H | H | $C_2F_5$ |
| Me | H | Me | H | $OCF_2CHF_2$ | Me | H | H | H | $OCF_2CHF_2$ |
| Me | H | Me | H | $SCF_2CHF_2$ | Me | H | H | H | $SCF_2CHF_2$ |
| Me | H | Me | H | $n$-$C_3F_7$ | Me | H | H | H | $n$-$C_3F_7$ |
| Me | H | Me | H | $i$-$C_3F_7$ | Me | H | H | H | $i$-$C_3F_7$ |
| Me | H | Me | H | Br | Me | H | H | H | Br |
| Me | H | Me | H | Cl | Me | H | H | H | Cl |
| Me | H | Me | H | $SCF_3$ | Me | H | H | H | $SCF_3$ |
| Me | H | Me | Me | $CF_3$ | Me | H | H | Me | $CF_3$ |
| Me | H | Me | Me | $OCF_3$ | Me | H | H | Me | $OCF_3$ |
| Me | H | Me | Me | $OCHF_2$ | Me | H | H | Me | $OCHF_2$ |
| Me | H | Me | Me | $C_2F_5$ | Me | H | H | Me | $C_2F_5$ |
| Me | H | Me | Me | $OCF_2CHF_2$ | Me | H | H | Me | $OCF_2CHF_2$ |
| Me | H | Me | Me | $SCF_2CHF_2$ | Me | H | H | Me | $SCF_2CHF_2$ |
| Me | H | Me | Me | $n$-$C_3F_7$ | Me | H | H | Me | $n$-$C_3F_7$ |
| Me | H | Me | Me | $i$-$C_3F_7$ | Me | H | H | Me | $i$-$C_3F_7$ |
| Me | H | Me | Me | Br | Me | H | H | Me | Br |
| Me | H | Me | Me | Cl | Me | H | H | Me | Cl |
| Me | H | Me | Me | $SCF_3$ | Me | H | H | Me | $SCF_3$ |
| Me | H | Me | Me | I | Me | H | H | Me | I |
| Me | H | Me | Me | SMe | Me | H | H | Me | SMe |
| Me | H | Me | Me | OMe | Me | H | H | Me | OMe |
| Me | H | Me | Me | OEt | Me | H | H | Me | OEt |
| Me | H | Me | Me | Et | Me | H | H | Me | Et |
| Me | H | Me | Me | $SO_2Me$ | Me | H | H | Me | $SO_2Me$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | Me | $SO_2CF_3$ | Me | H | H | Me | $SO_2CF_3$ |
| Me | H | Me | Et | $CF_3$ | Me | H | H | Et | $CF_3$ |
| Me | H | Me | Et | Br | Me | H | H | Et | Br |
| Me | H | Me | Et | Cl | Me | H | H | Et | Cl |
| Me | H | Me | Ph | $CF_3$ | Me | H | H | Ph | $CF_3$ |
| Me | H | Me | Ph | Br | Me | H | H | Ph | Br |
| Me | H | Me | Ph | Cl | Me | H | H | Ph | Cl |
| Me | H | Me | 2-pyridyl | $CF_3$ | Me | H | H | 2-pyridyl | $CF_3$ |
| Me | H | Me | 2-pyridyl | Cl | Me | H | H | 2-pyridyl | Cl |
| Me | H | Me | 2-ClPh | $CF_3$ | Me | H | H | 2-ClPh | $CF_3$ |
| Me | H | Me | 2-ClPh | $OCF_3$ | Me | H | H | 2-ClPh | $OCF_3$ |
| Me | H | Me | 2-ClPh | $SCHF_2$ | Me | H | H | 2-ClPh | $SCHF_2$ |
| Me | H | Me | 2-ClPh | Br | Me | H | H | 2-ClPh | Br |
| Me | H | Me | 2-ClPh | Cl | Me | H | H | 2-ClPh | Cl |
| Me | H | Me | 3-Cl-2-pyridyl | $CF_3$ | Me | H | H | 3-Cl-2-pyridyl | $CF_3$ |
| Me | H | Me | 3-Cl-2-pyridyl | $OCF_3$ | Me | H | H | 3-Cl-2-pyridyl | $OCF_3$ |
| Me | H | Me | 3-Cl-2-pyridyl | $SCHF_2$ | Me | H | H | 3-Cl-2-pyridyl | $SCHF_2$ |
| Me | H | Me | 3-Cl-2-pyridyl | Br | Me | H | H | 3-Cl-2-pyridyl | Br |
| Me | H | Me | 3-Cl-2-pyridyl | Cl | Me | H | H | 3-Cl-2-pyridyl | Cl |
| Me | H | Me | 3-Br-2-pyridyl | $CF_3$ | Me | H | H | 3-Br-2-pyridyl | $CF_3$ |
| Me | H | Me | 3-Br-2-pyridyl | $OCF_3$ | Me | H | H | 3-Br-2-pyridyl | $OCF_3$ |
| Me | H | Me | 3-Br-2-pyridyl | Br | Me | H | H | 3-Br-2-pyridyl | Br |
| Me | H | Me | 3-Br-2-pyridyl | Cl | Me | H | H | 3-Br-2-pyridyl | Cl |
| Me | H | Me | $CF_3$ | Me | Me | H | H | $CF_3$ | Me |
| Me | H | Me | F | $CF_3$ | Me | H | H | F | $CF_3$ |
| Me | H | Me | Cl | $CF_3$ | Me | H | H | Cl | $CF_3$ |
| Me | H | Me | *n*-Pr | $CF_3$ | Me | H | H | *n*-Pr | $CF_3$ |
| Me | H | Me | *i*-Pr | $CF_3$ | Me | H | H | *i*-Pr | $CF_3$ |
| Me | H | Me | $CF_3$ | $CF_3$ | Me | H | H | $CF_3$ | $CF_3$ |
| Me | H | Me | OMe | $CF_3$ | Me | H | H | OMe | $CF_3$ |
| Me | H | Me | 2-BrPh | $CF_3$ | Me | H | H | 2-BrPh | $CF_3$ |
| Me | H | Me | 2-MePh | $CF_3$ | Me | H | H | 2-MePh | $CF_3$ |
| Me | H | Me | 2-CNPh | $CF_3$ | Me | H | H | 2-CNPh | $CF_3$ |
| Me | H | Me | 2-FPh | $CF_3$ | Me | H | H | 2-FPh | $CF_3$ |
| Me | H | Me | 2,6-$F_2$Ph | $CF_3$ | Me | H | H | 2,6-$F_2$Ph | $CF_3$ |
| Me | H | Me | 2,4-$F_2$Ph | $CF_3$ | Me | H | H | 2,4-$F_2$Ph | $CF_3$ |
| Me | H | Me | 2,5-$F_2$Ph | $CF_3$ | Me | H | H | 2,5-$F_2$Ph | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | 2-MeOPh | $CF_3$ | Me | H | H | 2-MeOPh | $CF_3$ |
| Me | H | Me | 3-F-2-pyridyl | $CF_3$ | Me | H | H | 3-F-2-pyridyl | $CF_3$ |
| Me | H | Me | 3-$CF_3$-2-pyridyl | $CF_3$ | Me | H | H | 3-$CF_3$-2-pyridyl | $CF_3$ |
| Me | H | Me | 3-Me-2-pyridyl | $CF_3$ | Me | H | H | 3-Me-2-pyridyl | $CF_3$ |
| Et | H | Me | H | $OCF_3$ | Et | H | H | H | $OCF_3$ |
| Et | H | Me | H | $C_2F_5$ | Et | H | H | H | $C_2F_5$ |
| Et | H | Me | Me | $CF_3$ | Et | H | H | Me | $CF_3$ |
| Et | H | Me | Me | $C_2F_5$ | Et | H | H | Me | $C_2F_5$ |
| *i*-Pr | H | Me | Me | $CF_3$ | *i*-Pr | H | H | Me | $CF_3$ |
| Me | Me | H | H | $CF_3$ | Me | H | Cl | H | $CF_3$ |
| Me | Me | H | H | $OCF_3$ | Me | H | Cl | H | $OCF_3$ |
| Me | Me | H | H | $OCHF_2$ | Me | H | Cl | H | $OCHF_2$ |
| Me | Me | H | H | $C_2F_5$ | Me | H | Cl | H | $C_2F_5$ |
| Me | Me | H | H | $OCF_2CHF_2$ | Me | H | Cl | H | $OCF_2CHF_2$ |
| Me | Me | H | H | $SCF_2CHF_2$ | Me | H | Cl | H | $SCF_2CHF_2$ |
| Me | Me | H | H | *n*-$C_3F_7$ | Me | H | Cl | H | *n*-$C_3F_7$ |
| Me | Me | H | H | *i*-$C_3F_7$ | Me | H | Cl | H | *i*-$C_3F_7$ |
| Me | Me | H | H | Br | Me | H | Cl | H | Br |
| Me | Me | H | H | Cl | Me | H | Cl | H | Cl |
| Me | Me | H | H | $SCF_3$ | Me | H | Cl | H | $SCF_3$ |
| Me | Me | H | H | $CF_3$ | Me | H | Cl | Me | $CF_3$ |
| Me | Me | H | H | $OCF_3$ | Me | H | Cl | Me | $OCF_3$ |
| Me | Me | H | H | $OCHF_2$ | Me | H | Cl | Me | $OCHF_2$ |
| Me | Me | H | Me | $C_2F_5$ | Me | H | Cl | Me | $C_2F_5$ |
| Me | Me | H | Me | $OCF_2CHF_2$ | Me | H | Cl | Me | $OCF_2CHF_2$ |
| Me | Me | H | Me | $SCF_2CHF_2$ | Me | H | Cl | Me | $SCF_2CHF_2$ |
| Me | Me | H | Me | *n*-$C_3F_7$ | Me | H | Cl | Me | *n*-$C_3F_7$ |
| Me | Me | H | Me | *i*-$C_3F_7$ | Me | H | Cl | Me | *i*-$C_3F_7$ |
| Me | Me | H | Me | Br | Me | H | Cl | Me | Br |
| Me | Me | H | Me | Cl | Me | H | Cl | Me | Cl |
| Me | Me | H | Me | $SCF_3$ | Me | H | Cl | Me | $SCF_3$ |
| Me | Me | H | Me | I | Me | H | Cl | Me | I |
| Me | Me | H | Me | SMe | Me | H | Cl | Me | SMe |
| Me | Me | H | Me | OMe | Me | H | Cl | Me | OMe |
| Me | Me | H | Me | OEt | Me | H | Cl | Me | OEt |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | Me | Et | Me | H | Cl | Me | Et |
| Me | Me | H | Me | $SO_2Me$ | Me | H | Cl | Me | $SO_2Me$ |
| Me | Me | H | Me | $SO_2CF_3$ | Me | H | Cl | Me | $SO_2CF_3$ |
| Me | Me | H | Et | $CF_3$ | Me | H | Cl | Et | $CF_3$ |
| Me | Me | H | Et | Br | Me | H | Cl | Et | Br |
| Me | Me | H | Et | Cl | Me | H | Cl | Et | Cl |
| Me | Me | H | Ph | $CF_3$ | Me | H | Cl | Ph | $CF_3$ |
| Me | Me | H | Ph | Br | Me | H | Cl | Ph | Br |
| Me | Me | H | Ph | Cl | Me | H | Cl | Ph | Cl |
| Me | Me | H | 2-pyridyl | $CF_3$ | Me | H | Cl | 2-pyridyl | $CF_3$ |
| Me | Me | H | 2-pyridyl | Cl | Me | H | Cl | 2-pyridyl | Cl |
| Me | Me | H | 2-ClPh | $CF_3$ | Me | H | Cl | 2-ClPh | $CF_3$ |
| Me | Me | H | 2-ClPh | $OCF_3$ | Me | H | Cl | 2-ClPh | $OCF_3$ |
| Me | Me | H | 2-ClPh | $SCHF_2$ | Me | H | Cl | 2-ClPh | $SCHF_2$ |
| Me | Me | H | 2-ClPh | Br | Me | H | Cl | 2-ClPh | Br |
| Me | Me | H | 2-ClPh | Cl | Me | H | Cl | 2-ClPh | Cl |
| Me | Me | H | 3-Cl-2-pyridyl | $CF_3$ | Me | H | Cl | 3-Cl-2-pyridyl | $CF_3$ |
| Me | Me | H | 3-Cl-2-pyridyl | $OCF_3$ | Me | H | Cl | 3-Cl-2-pyridyl | $OCF_3$ |
| Me | Me | H | 3-Cl-2-pyridyl | $SCHF_2$ | Me | H | Cl | 3-Cl-2-pyridyl | $SCHF_2$ |
| Me | Me | H | 3-Cl-2-pyridyl | Br | Me | H | Cl | 3-Cl-2-pyridyl | Br |
| Me | Me | H | 3-Cl-2-pyridyl | Cl | Me | H | Cl | 3-Cl-2-pyridyl | Cl |
| Me | Me | H | 3-Br-2-pyridyl | $CF_3$ | Me | H | Cl | 3-Br-2-pyridyl | $CF_3$ |
| Me | Me | H | 3-Br-2-pyridyl | $OCF_3$ | Me | H | Cl | 3-Br-2-pyridyl | $OCF_3$ |
| Me | Me | H | 3-Br-2-pyridyl | Br | Me | H | Cl | 3-Br-2-pyridyl | Br |
| Me | Me | H | 3-Br-2-pyridyl | Cl | Me | H | Cl | 3-Br-2-pyridyl | Cl |
| Me | Me | H | $CF_3$ | Me | Me | H | Cl | $CF_3$ | Me |
| Me | Me | H | F | $CF_3$ | Me | H | Cl | F | $CF_3$ |
| Me | Me | H | Cl | $CF_3$ | Me | H | Cl | Cl | $CF_3$ |
| Me | Me | H | *n*-Pr | $CF_3$ | Me | H | Cl | *n*-Pr | $CF_3$ |
| Me | Me | H | *i*-Pr | $CF_3$ | Me | H | Cl | *i*-Pr | $CF_3$ |
| Me | Me | H | $CF_3$ | $CF_3$ | Me | H | Cl | $CF_3$ | $CF_3$ |
| Me | Me | H | OMe | $CF_3$ | Me | H | Cl | OMe | $CF_3$ |
| Me | Me | H | 2-BrPh | $CF_3$ | Me | H | Cl | 2-BrPh | $CF_3$ |
| Me | Me | H | 2-MePh | $CF_3$ | Me | H | Cl | 2-MePh | $CF_3$ |
| Me | Me | H | 2-CNPh | $CF_3$ | Me | H | Cl | 2-CNPh | $CF_3$ |
| Me | Me | H | 2-FPh | $CF_3$ | Me | H | Cl | 2-FPh | $CF_3$ |
| Me | Me | H | 2,6-$F_2$Ph | $CF_3$ | Me | H | Cl | 2,6-$F_2$Ph | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | 2,4-$F_2$Ph | $CF_3$ | Me | H | Cl | 2,4-$F_2$Ph | $CF_3$ |
| Me | Me | H | 2,5-$F_2$Ph | $CF_3$ | Me | H | Cl | 2,5-$F_2$Ph | $CF_3$ |
| Me | Me | H | 2-MeOPh | $CF_3$ | Me | H | Cl | 2-MeOPh | $CF_3$ |
| Me | Me | H | 3-F-2-pyridyl | $CF_3$ | Me | H | Cl | 3-F-2-pyridyl | $CF_3$ |
| Me | Me | H | 3-$CF_3$-2-pyridyl | $CF_3$ | Me | H | Cl | 3-$CF_3$-2-pyridyl | $CF_3$ |
| Me | Me | H | 3-Me-2-pyridyl | $CF_3$ | Me | H | Cl | 3-Me-2-pyridyl | $CF_3$ |
| Et | Me | H | H | $OCF_3$ | Et | H | Cl | H | $OCF_3$ |
| Et | Me | H | H | $C_2F_5$ | Et | H | Cl | H | $C_2F_5$ |
| Et | Me | H | Me | $CF_3$ | Et | H | Cl | Me | $CF_3$ |
| Et | Me | H | Me | $C_2F_5$ | Et | H | Cl | Me | $C_2F_5$ |
| *i*-Pr | Me | H | Me | $CF_3$ | *i*-Pr | H | Cl | Me | $CF_3$ |
| Me | Cl | H | H | $CF_3$ | Me | Cl | H | 2-ClPh | $CF_3$ |
| Me | Cl | H | H | $OCF_3$ | Me | Cl | H | 2-ClPh | $OCF_3$ |
| Me | Cl | H | H | $OCHF_2$ | Me | Cl | H | 2-ClPh | $SCHF_2$ |
| Me | Cl | H | H | $C_2F_5$ | Me | Cl | H | 2-ClPh | Br |
| Me | Cl | H | H | $OCF_2CHF_2$ | Me | Cl | H | 2-ClPh | Cl |
| Me | Cl | H | H | $SCF_2CHF_2$ | Me | Cl | H | 3-Cl-2-pyridyl | $CF_3$ |
| Me | Cl | H | H | *n*-$C_3F_7$ | Me | Cl | H | 3-Cl-2-pyridyl | $OCF_3$ |
| Me | Cl | H | H | *i*-$C_3F_7$ | Me | Cl | H | 3-Cl-2-pyridyl | $SCHF_2$ |
| Me | Cl | H | H | Br | Me | Cl | H | 3-Cl-2-pyridyl | Br |
| Me | Cl | H | H | Cl | Me | Cl | H | 3-Cl-2-pyridyl | Cl |
| Me | Cl | H | H | $SCF_3$ | Me | Cl | H | 3-Br-2-pyridyl | $CF_3$ |
| Me | Cl | H | Me | $CF_3$ | Me | Cl | H | 3-Br-2-pyridyl | $OCF_3$ |
| Me | Cl | H | Me | $OCF_3$ | Me | Cl | H | 3-Br-2-pyridyl | Br |
| Me | Cl | H | Me | $OCHF_2$ | Me | Cl | H | 3-Br-2-pyridyl | Cl |
| Me | Cl | H | Me | $C_2F_5$ | Me | Cl | H | $CF_3$ | Me |
| Me | Cl | H | Me | $OCF_2CHF_2$ | Me | Cl | H | F | $CF_3$ |
| Me | Cl | H | Me | $SCF_2CHF_2$ | Me | Cl | H | Cl | $CF_3$ |
| Me | Cl | H | Me | *n*-$C_3F_7$ | Me | Cl | H | *n*-Pr | $CF_3$ |
| Me | Cl | H | Me | *i*-$C_3F_7$ | Me | Cl | H | *i*-Pr | $CF_3$ |
| Me | Cl | H | Me | Br | Me | Cl | H | $CF_3$ | $CF_3$ |
| Me | Cl | H | Me | Cl | Me | Cl | H | OMe | $CF_3$ |
| Me | Cl | H | Me | $SCF_3$ | Me | Cl | H | 2-BrPh | $CF_3$ |
| Me | Cl | H | Me | I | Me | Cl | H | 2-MePh | $CF_3$ |
| Me | Cl | H | Me | SMe | Me | Cl | H | 2-CNPh | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Cl | H | Me | OMe | Me | Cl | H | 2-FPh | $CF_3$ |
| Me | Cl | H | Me | OEt | Me | Cl | H | 2,6-$F_2$Ph | $CF_3$ |
| Me | Cl | H | Me | Et | Me | Cl | H | 2,4-$F_2$Ph | $CF_3$ |
| Me | Cl | H | Me | $SO_2Me$ | Me | Cl | H | 2,5-$F_2$Ph | $CF_3$ |
| Me | Cl | H | Me | $SO_2CF_3$ | Me | Cl | H | 2-MeOPh | $CF_3$ |
| Me | Cl | H | Et | $CF_3$ | Me | Cl | H | 3-F-2-pyridyl | $CF_3$ |
| Me | Cl | H | Et | Br | Me | Cl | H | 3-$CF_3$-2-pyridyl | $CF_3$ |
| Me | Cl | H | Et | Cl | Me | Cl | H | 3-Me-2-pyridyl | $CF_3$ |
| Me | Cl | H | Ph | $CF_3$ | Et | Cl | H | H | $OCF_3$ |
| Me | Cl | H | Ph | Br | Et | Cl | H | H | $C_2F_5$ |
| Me | Cl | H | Ph | Cl | Et | Cl | H | Me | $CF_3$ |
| Me | Cl | H | 2-pyridyl | $CF_3$ | Et | Cl | H | Me | $C_2F_5$ |
| Me | Cl | H | 2-pyridyl | Cl | *i*-Pr | Cl | H | Me | $CF_3$ |

Table 12

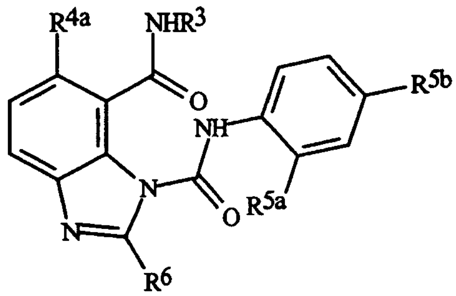

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CF_3$ | Me | H | H | H | $CF_3$ |
| Me | H | Me | H | $OCF_3$ | Me | H | H | H | $OCF_3$ |
| Me | H | Me | H | $OCHF_2$ | Me | H | H | H | $OCHF_2$ |
| Me | H | Me | H | $C_2F_5$ | Me | H | H | H | $C_2F_5$ |
| Me | H | Me | H | $OCF_2CHF_2$ | Me | H | H | H | $OCF_2CHF_2$ |
| Me | H | Me | H | $SCF_2CHF_2$ | Me | H | H | H | $SCF_2CHF_2$ |
| Me | H | Me | H | *n*-$C_3F_7$ | Me | H | H | H | *n*-$C_3F_7$ |
| Me | H | Me | H | *i*-$C_3F_7$ | Me | H | H | H | *i*-$C_3F_7$ |
| Me | H | Me | H | Br | Me | H | H | H | Br |
| Me | H | Me | H | Cl | Me | H | H | H | Cl |
| Me | H | Me | H | $SCF_3$ | Me | H | H | H | $SCF_3$ |
| Me | H | Me | Me | $CF_3$ | Me | H | H | Me | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | Me | $OCF_3$ | Me | H | H | Me | $OCF_3$ |
| Me | H | Me | Me | $OCHF_2$ | Me | H | H | Me | $OCHF_2$ |
| Me | H | Me | Me | $C_2F_5$ | Me | H | H | Me | $C_2F_5$ |
| Me | H | Me | Me | $OCF_2CHF_2$ | Me | H | H | Me | $OCF_2CHF_2$ |
| Me | H | Me | Me | $SCF_2CHF_2$ | Me | H | H | Me | $SCF_2CHF_2$ |
| Me | H | Me | Me | *n*-$C_3F_7$ | Me | H | H | Me | *n*-$C_3F_7$ |
| Me | H | Me | Me | *i*-$C_3F_7$ | Me | H | H | Me | *i*-$C_3F_7$ |
| Me | H | Me | Me | Br | Me | H | H | Me | Br |
| Me | H | Me | Me | Cl | Me | H | H | Me | Cl |
| Me | H | Me | Me | $SCF_3$ | Me | H | H | Me | $SCF_3$ |
| Me | H | Me | Me | I | Me | H | H | Me | I |
| Me | H | Me | Me | SMe | Me | H | H | Me | SMe |
| Me | H | Me | Me | OMe | Me | H | H | Me | OMe |
| Me | H | Me | Me | OEt | Me | H | H | Me | OEt |
| Me | H | Me | Me | Et | Me | H | H | Me | Et |
| Me | H | Me | Me | $SO_2Me$ | Me | H | H | Me | $SO_2Me$ |
| Me | H | Me | Me | $SO_2CF_3$ | Me | H | H | Me | $SO_2CF_3$ |
| Me | H | Me | Et | $CF_3$ | Me | H | H | Et | $CF_3$ |
| Me | H | Me | Et | Br | Me | H | H | Et | Br |
| Me | H | Me | Et | Cl | Me | H | H | Et | Cl |
| Me | H | Me | Ph | $CF_3$ | Me | H | H | Ph | $CF_3$ |
| Me | H | Me | Ph | Br | Me | H | H | Ph | Br |
| Me | H | Me | Ph | Cl | Me | H | H | Ph | Cl |
| Me | H | Me | 2-pyridyl | $CF_3$ | Me | H | H | 2-pyridyl | $CF_3$ |
| Me | H | Me | 2-pyridyl | Cl | Me | H | H | 2-pyridyl | Cl |
| Me | H | Me | 2-ClPh | $CF_3$ | Me | H | H | 2-ClPh | $CF_3$ |
| Me | H | Me | 2-ClPh | $OCF_3$ | Me | H | H | 2-ClPh | $OCF_3$ |
| Me | H | Me | 2-ClPh | $SCHF_2$ | Me | H | H | 2-ClPh | $SCHF_2$ |
| Me | H | Me | 2-ClPh | Br | Me | H | H | 2-ClPh | Br |
| Me | H | Me | 2-ClPh | Cl | Me | H | H | 2-ClPh | Cl |
| Me | H | Me | 3-Cl-2-pyridyl | $CF_3$ | Me | H | H | 3-Cl-2-pyridyl | $CF_3$ |
| Me | H | Me | 3-Cl-2-pyridyl | $OCF_3$ | Me | H | H | 3-Cl-2-pyridyl | $OCF_3$ |
| Me | H | Me | 3-Cl-2-pyridyl | $SCHF_2$ | Me | H | H | 3-Cl-2-pyridyl | $SCHF_2$ |
| Me | H | Me | 3-Cl-2-pyridyl | Br | Me | H | H | 3-Cl-2-pyridyl | Br |
| Me | H | Me | 3-Cl-2-pyridyl | Cl | Me | H | H | 3-Cl-2-pyridyl | Cl |
| Me | H | Me | 3-Br-2-pyridyl | $CF_3$ | Me | H | H | 3-Br-2-pyridyl | $CF_3$ |
| Me | H | Me | 3-Br-2-pyridyl | $OCF_3$ | Me | H | H | 3-Br-2-pyridyl | $OCF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | 3-Br-2-pyridyl | Br | Me | H | H | 3-Br-2-pyridyl | Br |
| Me | H | Me | 3-Br-2-pyridyl | Cl | Me | H | H | 3-Br-2-pyridyl | Cl |
| Me | H | Me | $CF_3$ | Me | Me | H | H | $CF_3$ | Me |
| Me | H | Me | F | $CF_3$ | Me | H | H | F | $CF_3$ |
| Me | H | Me | Cl | $CF_3$ | Me | H | H | Cl | $CF_3$ |
| Me | H | Me | *n*-Pr | $CF_3$ | Me | H | H | *n*-Pr | $CF_3$ |
| Me | H | Me | *i*-Pr | $CF_3$ | Me | H | H | *i*-Pr | $CF_3$ |
| Me | H | Me | $CF_3$ | $CF_3$ | Me | H | H | $CF_3$ | $CF_3$ |
| Me | H | Me | OMe | $CF_3$ | Me | H | H | OMe | $CF_3$ |
| Me | H | Me | 2-BrPh | $CF_3$ | Me | H | H | 2-BrPh | $CF_3$ |
| Me | H | Me | 2-MePh | $CF_3$ | Me | H | H | 2-MePh | $CF_3$ |
| Me | H | Me | 2-CNPh | $CF_3$ | Me | H | H | 2-CNPh | $CF_3$ |
| Me | H | Me | 2-FPh | $CF_3$ | Me | H | H | 2-FPh | $CF_3$ |
| Me | H | Me | 2,6-$F_2$Ph | $CF_3$ | Me | H | H | 2,6-$F_2$Ph | $CF_3$ |
| Me | H | Me | 2,4-$F_2$Ph | $CF_3$ | Me | H | H | 2,4-$F_2$Ph | $CF_3$ |
| Me | H | Me | 2,5-$F_2$Ph | $CF_3$ | Me | H | H | 2,5-$F_2$Ph | $CF_3$ |
| Me | H | Me | 2-MeOPh | $CF_3$ | Me | H | H | 2-MeOPh | $CF_3$ |
| Me | H | Me | 3-F-2-pyridyl | $CF_3$ | Me | H | H | 3-F-2-pyridyl | $CF_3$ |
| Me | H | Me | 3-$CF_3$-2-pyridyl | $CF_3$ | Me | H | H | 3-$CF_3$-2-pyridyl | $CF_3$ |
| Me | H | Me | 3-Me-2-pyridyl | $CF_3$ | Me | H | H | 3-Me-2-pyridyl | $CF_3$ |
| Et | H | Me | H | $OCF_3$ | Et | H | H | H | $OCF_3$ |
| Et | H | Me | H | $C_2F_5$ | Et | H | H | H | $C_2F_5$ |
| Et | H | Me | Me | $CF_3$ | Et | H | H | Me | $CF_3$ |
| Et | H | Me | Me | $C_2F_5$ | Et | H | H | Me | $C_2F_5$ |
| *i*-Pr | H | Me | Me | $CF_3$ | *i*-Pr | H | H | Me | $CF_3$ |
| Me | Me | H | H | $CF_3$ | Me | H | Cl | H | $CF_3$ |
| Me | Me | H | H | $OCF_3$ | Me | H | Cl | H | $OCF_3$ |
| Me | Me | H | H | $OCHF_2$ | Me | H | Cl | H | $OCHF_2$ |
| Me | Me | H | H | $C_2F_5$ | Me | H | Cl | H | $C_2F_5$ |
| Me | Me | H | H | $OCF_2CHF_2$ | Me | H | Cl | H | $OCF_2CHF_2$ |
| Me | Me | H | H | $SCF_2CHF_2$ | Me | H | Cl | H | $SCF_2CHF_2$ |
| Me | Me | H | H | *n*-$C_3F_7$ | Me | H | Cl | H | *n*-$C_3F_7$ |
| Me | Me | H | H | *i*-$C_3F_7$ | Me | H | Cl | H | *i*-$C_3F_7$ |
| Me | Me | H | H | Br | Me | H | Cl | H | Br |
| Me | Me | H | H | Cl | Me | H | Cl | H | Cl |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | H | $SCF_3$ | Me | H | Cl | H | $SCF_3$ |
| Me | Me | H | H | $CF_3$ | Me | H | Cl | Me | $CF_3$ |
| Me | Me | H | H | $OCF_3$ | Me | H | Cl | Me | $OCF_3$ |
| Me | Me | H | H | $OCHF_2$ | Me | H | Cl | Me | $OCHF_2$ |
| Me | Me | H | Me | $C_2F_5$ | Me | H | Cl | Me | $C_2F_5$ |
| Me | Me | H | Me | $OCF_2CHF_2$ | Me | H | Cl | Me | $OCF_2CHF_2$ |
| Me | Me | H | Me | $SCF_2CHF_2$ | Me | H | Cl | Me | $SCF_2CHF_2$ |
| Me | Me | H | Me | *n*-$C_3F_7$ | Me | H | Cl | Me | *n*-$C_3F_7$ |
| Me | Me | H | Me | *i*-$C_3F_7$ | Me | H | Cl | Me | *i*-$C_3F_7$ |
| Me | Me | H | Me | Br | Me | H | Cl | Me | Br |
| Me | Me | H | Me | Cl | Me | H | Cl | Me | Cl |
| Me | Me | H | Me | $SCF_3$ | Me | H | Cl | Me | $SCF_3$ |
| Me | Me | H | Me | I | Me | H | Cl | Me | I |
| Me | Me | H | Me | SMe | Me | H | Cl | Me | SMe |
| Me | Me | H | Me | OMe | Me | H | Cl | Me | OMe |
| Me | Me | H | Me | OEt | Me | H | Cl | Me | OEt |
| Me | Me | H | Me | Et | Me | H | Cl | Me | Et |
| Me | Me | H | Me | $SO_2Me$ | Me | H | Cl | Me | $SO_2Me$ |
| Me | Me | H | Me | $SO_2CF_3$ | Me | H | Cl | Me | $SO_2CF_3$ |
| Me | Me | H | Et | $CF_3$ | Me | H | Cl | Et | $CF_3$ |
| Me | Me | H | Et | Br | Me | H | Cl | Et | Br |
| Me | Me | H | Et | Cl | Me | H | Cl | Et | Cl |
| Me | Me | H | Ph | $CF_3$ | Me | H | Cl | Ph | $CF_3$ |
| Me | Me | H | Ph | Br | Me | H | Cl | Ph | Br |
| Me | Me | H | Ph | Cl | Me | H | Cl | Ph | Cl |
| Me | Me | H | 2-pyridyl | $CF_3$ | Me | H | Cl | 2-pyridyl | $CF_3$ |
| Me | Me | H | 2-pyridyl | Cl | Me | H | Cl | 2-pyridyl | Cl |
| Me | Me | H | 2-ClPh | $CF_3$ | Me | H | Cl | 2-ClPh | $CF_3$ |
| Me | Me | H | 2-ClPh | $OCF_3$ | Me | H | Cl | 2-ClPh | $OCF_3$ |
| Me | Me | H | 2-ClPh | $SCHF_2$ | Me | H | Cl | 2-ClPh | $SCHF_2$ |
| Me | Me | H | 2-ClPh | Br | Me | H | Cl | 2-ClPh | Br |
| Me | Me | H | 2-ClPh | Cl | Me | H | Cl | 2-ClPh | Cl |
| Me | Me | H | 3-Cl-2-pyridyl | $CF_3$ | Me | H | Cl | 3-Cl-2-pyridyl | $CF_3$ |
| Me | Me | H | 3-Cl-2-pyridyl | $OCF_3$ | Me | H | Cl | 3-Cl-2-pyridyl | $OCF_3$ |
| Me | Me | H | 3-Cl-2-pyridyl | $SCHF_2$ | Me | H | Cl | 3-Cl-2-pyridyl | $SCHF_2$ |
| Me | Me | H | 3-Cl-2-pyridyl | Br | Me | H | Cl | 3-Cl-2-pyridyl | Br |
| Me | Me | H | 3-Cl-2-pyridyl | Cl | Me | H | Cl | 3-Cl-2-pyridyl | Cl |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | 3-Br-2-pyridyl | $CF_3$ | Me | H | Cl | 3-Br-2-pyridyl | $CF_3$ |
| Me | Me | H | 3-Br-2-pyridyl | $OCF_3$ | Me | H | Cl | 3-Br-2-pyridyl | $OCF_3$ |
| Me | Me | H | 3-Br-2-pyridyl | Br | Me | H | Cl | 3-Br-2-pyridyl | Br |
| Me | Me | H | 3-Br-2-pyridyl | Cl | Me | H | Cl | 3-Br-2-pyridyl | Cl |
| Me | Me | H | $CF_3$ | Me | Me | H | Cl | $CF_3$ | Me |
| Me | Me | H | F | $CF_3$ | Me | H | Cl | F | $CF_3$ |
| Me | Me | H | Cl | $CF_3$ | Me | H | Cl | Cl | $CF_3$ |
| Me | Me | H | *n*-Pr | $CF_3$ | Me | H | Cl | *n*-Pr | $CF_3$ |
| Me | Me | H | *i*-Pr | $CF_3$ | Me | H | Cl | *i*-Pr | $CF_3$ |
| Me | Me | H | $CF_3$ | $CF_3$ | Me | H | Cl | $CF_3$ | $CF_3$ |
| Me | Me | H | OMe | $CF_3$ | Me | H | Cl | OMe | $CF_3$ |
| Me | Me | H | 2-BrPh | $CF_3$ | Me | H | Cl | 2-BrPh | $CF_3$ |
| Me | Me | H | 2-MePh | $CF_3$ | Me | H | Cl | 2-MePh | $CF_3$ |
| Me | Me | H | 2-CNPh | $CF_3$ | Me | H | Cl | 2-CNPh | $CF_3$ |
| Me | Me | H | 2-FPh | $CF_3$ | Me | H | Cl | 2-FPh | $CF_3$ |
| Me | Me | H | 2,6-$F_2$Ph | $CF_3$ | Me | H | Cl | 2,6-$F_2$Ph | $CF_3$ |
| Me | Me | H | 2,4-$F_2$Ph | $CF_3$ | Me | H | Cl | 2,4-$F_2$Ph | $CF_3$ |
| Me | Me | H | 2,5-$F_2$Ph | $CF_3$ | Me | H | Cl | 2,5-$F_2$Ph | $CF_3$ |
| Me | Me | H | 2-MeOPh | $CF_3$ | Me | H | Cl | 2-MeOPh | $CF_3$ |
| Me | Me | H | 3-F-2-pyridyl | $CF_3$ | Me | H | Cl | 3-F-2-pyridyl | $CF_3$ |
| Me | Me | H | 3-$CF_3$-2-pyridyl | $CF_3$ | Me | H | Cl | 3-$CF_3$-2-pyridyl | $CF_3$ |
| Me | Me | H | 3-Me-2-pyridyl | $CF_3$ | Me | H | Cl | 3-Me-2-pyridyl | $CF_3$ |
| Et | Me | H | H | $OCF_3$ | Et | H | Cl | H | $OCF_3$ |
| Et | Me | H | H | $C_2F_5$ | Et | H | Cl | H | $C_2F_5$ |
| Et | Me | H | Me | $CF_3$ | Et | H | Cl | Me | $CF_3$ |
| Et | Me | H | Me | $C_2F_5$ | Et | H | Cl | Me | $C_2F_5$ |
| *i*-Pr | Me | H | Me | $CF_3$ | *i*-Pr | H | Cl | Me | $CF_3$ |
| Me | Cl | H | H | $CF_3$ | Me | Cl | H | 2-ClPh | $CF_3$ |
| Me | Cl | H | H | $OCF_3$ | Me | Cl | H | 2-ClPh | $OCF_3$ |
| Me | Cl | H | H | $OCHF_2$ | Me | Cl | H | 2-ClPh | $SCHF_2$ |
| Me | Cl | H | H | $C_2F_5$ | Me | Cl | H | 2-ClPh | Br |
| Me | Cl | H | H | $OCF_2CHF_2$ | Me | Cl | H | 2-ClPh | Cl |
| Me | Cl | H | H | $SCF_2CHF_2$ | Me | Cl | H | 3-Cl-2-pyridyl | $CF_3$ |
| Me | Cl | H | H | *n*-$C_3F_7$ | Me | Cl | H | 3-Cl-2-pyridyl | $OCF_3$ |
| Me | Cl | H | H | *i*-$C_3F_7$ | Me | Cl | H | 3-Cl-2-pyridyl | $SCHF_2$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Cl | H | H | Br | Me | Cl | H | 3-Cl-2-pyridyl | Br |
| Me | Cl | H | H | Cl | Me | Cl | H | 3-Cl-2-pyridyl | Cl |
| Me | Cl | H | H | $SCF_3$ | Me | Cl | H | 3-Br-2-pyridyl | $CF_3$ |
| Me | Cl | H | Me | $CF_3$ | Me | Cl | H | 3-Br-2-pyridyl | $OCF_3$ |
| Me | Cl | H | Me | $OCF_3$ | Me | Cl | H | 3-Br-2-pyridyl | Br |
| Me | Cl | H | Me | $OCHF_2$ | Me | Cl | H | 3-Br-2-pyridyl | Cl |
| Me | Cl | H | Me | $C_2F_5$ | Me | Cl | H | $CF_3$ | Me |
| Me | Cl | H | Me | $OCF_2CHF_2$ | Me | Cl | H | F | $CF_3$ |
| Me | Cl | H | Me | $SCF_2CHF_2$ | Me | Cl | H | Cl | $CF_3$ |
| Me | Cl | H | Me | *n*-$C_3F_7$ | Me | Cl | H | *n*-Pr | $CF_3$ |
| Me | Cl | H | Me | *i*-$C_3F_7$ | Me | Cl | H | *i*-Pr | $CF_3$ |
| Me | Cl | H | Me | Br | Me | Cl | H | $CF_3$ | $CF_3$ |
| Me | Cl | H | Me | Cl | Me | Cl | H | OMe | $CF_3$ |
| Me | Cl | H | Me | $SCF_3$ | Me | Cl | H | 2-BrPh | $CF_3$ |
| Me | Cl | H | Me | I | Me | Cl | H | 2-MePh | $CF_3$ |
| Me | Cl | H | Me | SMe | Me | Cl | H | 2-CNPh | $CF_3$ |
| Me | Cl | H | Me | OMe | Me | Cl | H | 2-FPh | $CF_3$ |
| Me | Cl | H | Me | OEt | Me | Cl | H | 2,6-$F_2$Ph | $CF_3$ |
| Me | Cl | H | Me | Et | Me | Cl | H | 2,4-$F_2$Ph | $CF_3$ |
| Me | Cl | H | Me | $SO_2Me$ | Me | Cl | H | 2,5-$F_2$Ph | $CF_3$ |
| Me | Cl | H | Me | $SO_2CF_3$ | Me | Cl | H | 2-MeOPh | $CF_3$ |
| Me | Cl | H | Et | $CF_3$ | Me | Cl | H | 3-F-2-pyridyl | $CF_3$ |
| Me | Cl | H | Et | Br | Me | Cl | H | 3-$CF_3$-2-pyridyl | $CF_3$ |
| Me | Cl | H | Et | Cl | Me | Cl | H | 3-Me-2-pyridyl | $CF_3$ |
| Me | Cl | H | Ph | $CF_3$ | Et | Cl | H | H | $OCF_3$ |
| Me | Cl | H | Ph | Br | Et | Cl | H | H | $C_2F_5$ |
| Me | Cl | H | Ph | Cl | Et | Cl | H | Me | $CF_3$ |
| Me | Cl | H | 2-pyridyl | $CF_3$ | Et | Cl | H | Me | $C_2F_5$ |
| Me | Cl | H | 2-pyridyl | Cl | *i*-Pr | Cl | H | Me | $CF_3$ |

Table 13

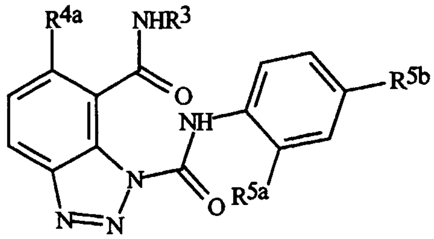

| $R^3$ | $R^{4a}$ | $R^{5a}$ | $R^{5b}$ | $R^3$ | $R^{4a}$ | $R^{5a}$ | $R^{5b}$ |
|---|---|---|---|---|---|---|---|
| Me | H | H | $CF_3$ | Me | H | H | $CF_3$ |
| Me | H | H | $OCF_3$ | Me | H | H | $OCF_3$ |
| Me | H | H | $OCHF_2$ | Me | H | H | $OCHF_2$ |
| Me | H | H | $C_2F_5$ | Me | H | H | $C_2F_5$ |
| Me | H | H | $OCF_2CHF_2$ | Me | H | H | $OCF_2CHF_2$ |
| Me | H | H | $SCF_2CHF_2$ | Me | H | H | $SCF_2CHF_2$ |
| Me | H | H | *n*-$C_3F_7$ | Me | H | H | *n*-$C_3F_7$ |
| Me | H | H | *i*-$C_3F_7$ | Me | H | H | *i*-$C_3F_7$ |
| Me | H | H | Br | Me | H | H | Br |
| Me | H | H | Cl | Me | H | H | Cl |
| Me | H | H | $SCF_3$ | Me | H | H | $SCF_3$ |
| Me | H | Me | $CF_3$ | Me | H | Me | $CF_3$ |
| Me | H | Me | $OCF_3$ | Me | H | Me | $OCF_3$ |
| Me | H | Me | $OCHF_2$ | Me | H | Me | $OCHF_2$ |
| Me | H | Me | $C_2F_5$ | Me | H | Me | $C_2F_5$ |
| Me | H | Me | $OCF_2CHF_2$ | Me | H | Me | $OCF_2CHF_2$ |
| Me | H | Me | $SCF_2CHF_2$ | Me | H | Me | $SCF_2CHF_2$ |
| Me | H | Me | *n*-$C_3F_7$ | Me | H | Me | *n*-$C_3F_7$ |
| Me | H | Me | *i*-$C_3F_7$ | Me | H | Me | *i*-$C_3F_7$ |
| Me | H | Me | Br | Me | H | Me | Br |
| Me | H | Me | Cl | Me | H | Me | Cl |
| Me | H | Me | $SCF_3$ | Me | H | Me | $SCF_3$ |
| Me | H | Me | I | Me | H | Me | I |
| Me | H | Me | SMe | Me | H | Me | SMe |
| Me | H | Me | OMe | Me | H | Me | OMe |
| Me | H | Me | OEt | Me | H | Me | OEt |
| Me | H | Me | Et | Me | H | Me | Et |
| Me | H | Me | $SO_2Me$ | Me | H | Me | $SO_2Me$ |
| Me | H | Me | $SO_2CF_3$ | Me | H | Me | $SO_2CF_3$ |
| Me | H | Et | $CF_3$ | Me | H | Et | $CF_3$ |

| $R^3$ | $R^{4a}$ | $R^{5a}$ | $R^{5b}$ | $R^3$ | $R^{4a}$ | $R^{5a}$ | $R^{5b}$ |
|---|---|---|---|---|---|---|---|
| Me | H | Et | Br | Me | H | Et | Br |
| Me | H | Et | Cl | Me | H | Et | Cl |
| Me | H | Ph | $CF_3$ | Me | H | Ph | $CF_3$ |
| Me | H | Ph | Br | Me | H | Ph | Br |
| Me | H | Ph | Cl | Me | H | Ph | Cl |
| Me | H | 2-pyridyl | $CF_3$ | Me | H | 2-pyridyl | $CF_3$ |
| Me | H | 2-pyridyl | Cl | Me | H | 2-pyridyl | Cl |
| Me | H | 2-ClPh | $CF_3$ | Me | H | 2-ClPh | $CF_3$ |
| Me | H | 2-ClPh | $OCF_3$ | Me | H | 2-ClPh | $OCF_3$ |
| Me | H | 2-ClPh | $SCHF_2$ | Me | H | 2-ClPh | $SCHF_2$ |
| Me | H | 2-ClPh | Br | Me | H | 2-ClPh | Br |
| Me | H | 2-ClPh | Cl | Me | H | 2-ClPh | Cl |
| Me | H | 3-Cl-2-pyridyl | $CF_3$ | Me | H | 3-Cl-2-pyridyl | $CF_3$ |
| Me | H | 3-Cl-2-pyridyl | $OCF_3$ | Me | H | 3-Cl-2-pyridyl | $OCF_3$ |
| Me | H | 3-Cl-2-pyridyl | $SCHF_2$ | Me | H | 3-Cl-2-pyridyl | $SCHF_2$ |
| Me | H | 3-Cl-2-pyridyl | Br | Me | H | 3-Cl-2-pyridyl | Br |
| Me | H | 3-Cl-2-pyridyl | Cl | Me | H | 3-Cl-2-pyridyl | Cl |
| Me | H | 3-Br-2-pyridyl | $CF_3$ | Me | H | 3-Br-2-pyridyl | $CF_3$ |
| Me | H | 3-Br-2-pyridyl | $OCF_3$ | Me | H | 3-Br-2-pyridyl | $OCF_3$ |
| Me | H | 3-Br-2-pyridyl | Br | Me | H | 3-Br-2-pyridyl | Br |
| Me | H | 3-Br-2-pyridyl | Cl | Me | H | 3-Br-2-pyridyl | Cl |
| Me | H | $CF_3$ | Me | Me | H | $CF_3$ | Me |
| Me | H | F | $CF_3$ | Me | H | F | $CF_3$ |
| Me | H | Cl | $CF_3$ | Me | H | Cl | $CF_3$ |
| Me | H | *n*-Pr | $CF_3$ | Me | H | *n*-Pr | $CF_3$ |
| Me | H | *i*-Pr | $CF_3$ | Me | H | *i*-Pr | $CF_3$ |
| Me | H | $CF_3$ | $CF_3$ | Me | H | $CF_3$ | $CF_3$ |
| Me | H | OMe | $CF_3$ | Me | H | OMe | $CF_3$ |
| Me | H | 2-BrPh | $CF_3$ | Me | H | 2-BrPh | $CF_3$ |
| Me | H | 2-MePh | $CF_3$ | Me | H | 2-MePh | $CF_3$ |
| Me | H | 2-CNPh | $CF_3$ | Me | H | 2-CNPh | $CF_3$ |
| Me | H | 2-FPh | $CF_3$ | Me | H | 2-FPh | $CF_3$ |
| Me | H | 2,6-$F_2$Ph | $CF_3$ | Me | H | 2,6-$F_2$Ph | $CF_3$ |
| Me | H | 2,4-$F_2$Ph | $CF_3$ | Me | H | 2,4-$F_2$Ph | $CF_3$ |
| Me | H | 2,5-$F_2$Ph | $CF_3$ | Me | H | 2,5-$F_2$Ph | $CF_3$ |
| Me | H | 2-MeOPh | $CF_3$ | Me | H | 2-MeOPh | $CF_3$ |
| Me | H | 3-F-2-pyridyl | $CF_3$ | Me | H | 3-F-2-pyridyl | $CF_3$ |

| $R^3$ | $R^{4a}$ | $R^{5a}$ | $R^{5b}$ | $R^3$ | $R^{4a}$ | $R^{5a}$ | $R^{5b}$ |
|---|---|---|---|---|---|---|---|
| Me | H | 3-$CF_3$-2-pyridyl | $CF_3$ | Me | H | 3-$CF_3$-2-pyridyl | $CF_3$ |
| Me | H | 3-Me-2-pyridyl | $CF_3$ | Me | H | 3-Me-2-pyridyl | $CF_3$ |
| Et | H | H | $OCF_3$ | Et | H | H | $OCF_3$ |
| Et | H | H | $C_2F_5$ | Et | H | H | $C_2F_5$ |
| Et | H | Me | $CF_3$ | Et | H | Me | $CF_3$ |
| Et | H | Me | $C_2F_5$ | Et | H | Me | $C_2F_5$ |
| *i*-Pr | H | Me | $CF_3$ | *i*-Pr | H | Me | $CF_3$ |
| Me | Me | H | $CF_3$ | Me | H | H | $CF_3$ |
| Me | Me | H | $OCF_3$ | Me | H | H | $OCF_3$ |
| Me | Me | H | $OCHF_2$ | Me | H | H | $OCHF_2$ |
| Me | Me | H | $C_2F_5$ | Me | H | H | $C_2F_5$ |
| Me | Me | H | $OCF_2CHF_2$ | Me | H | H | $OCF_2CHF_2$ |
| Me | Me | H | $SCF_2CHF_2$ | Me | H | H | $SCF_2CHF_2$ |
| Me | Me | H | *n*-$C_3F_7$ | Me | H | H | *n*-$C_3F_7$ |
| Me | Me | H | *i*-$C_3F_7$ | Me | H | H | *i*-$C_3F_7$ |
| Me | Me | H | Br | Me | H | H | Br |
| Me | Me | H | Cl | Me | H | H | Cl |
| Me | Me | H | $SCF_3$ | Me | H | H | $SCF_3$ |
| Me | Me | H | $CF_3$ | Me | H | Me | $CF_3$ |
| Me | Me | H | $OCF_3$ | Me | H | Me | $OCF_3$ |
| Me | Me | H | $OCHF_2$ | Me | H | Me | $OCHF_2$ |
| Me | Me | Me | $C_2F_5$ | Me | H | Me | $C_2F_5$ |
| Me | Me | Me | $OCF_2CHF_2$ | Me | H | Me | $OCF_2CHF_2$ |
| Me | Me | Me | $SCF_2CHF_2$ | Me | H | Me | $SCF_2CHF_2$ |
| Me | Me | Me | *n*-$C_3F_7$ | Me | H | Me | *n*-$C_3F_7$ |
| Me | Me | Me | *i*-$C_3F_7$ | Me | H | Me | *i*-$C_3F_7$ |
| Me | Me | Me | Br | Me | H | Me | Br |
| Me | Me | Me | Cl | Me | H | Me | Cl |
| Me | Me | Me | $SCF_3$ | Me | H | Me | $SCF_3$ |
| Me | Me | Me | I | Me | H | Me | I |
| Me | Me | Me | SMe | Me | H | Me | SMe |
| Me | Me | Me | OMe | Me | H | Me | OMe |
| Me | Me | Me | OEt | Me | H | Me | OEt |
| Me | Me | Me | Et | Me | H | Me | Et |
| Me | Me | Me | $SO_2Me$ | Me | H | Me | $SO_2Me$ |

| $R^3$ | $R^{4a}$ | $R^{5a}$ | $R^{5b}$ | $R^3$ | $R^{4a}$ | $R^{5a}$ | $R^{5b}$ |
|---|---|---|---|---|---|---|---|
| Me | Me | Me | $SO_2CF_3$ | Me | H | Me | $SO_2CF_3$ |
| Me | Me | Et | $CF_3$ | Me | H | Et | $CF_3$ |
| Me | Me | Et | Br | Me | H | Et | Br |
| Me | Me | Et | Cl | Me | H | Et | Cl |
| Me | Me | Ph | $CF_3$ | Me | H | Ph | $CF_3$ |
| Me | Me | Ph | Br | Me | H | Ph | Br |
| Me | Me | Ph | Cl | Me | H | Ph | Cl |
| Me | Me | 2-pyridyl | $CF_3$ | Me | H | 2-pyridyl | $CF_3$ |
| Me | Me | 2-pyridyl | Cl | Me | H | 2-pyridyl | Cl |
| Me | Me | 2-ClPh | $CF_3$ | Me | H | 2-ClPh | $CF_3$ |
| Me | Me | 2-ClPh | $OCF_3$ | Me | H | 2-ClPh | $OCF_3$ |
| Me | Me | 2-ClPh | $SCHF_2$ | Me | H | 2-ClPh | $SCHF_2$ |
| Me | Me | 2-ClPh | Br | Me | H | 2-ClPh | Br |
| Me | Me | 2-ClPh | Cl | Me | H | 2-ClPh | Cl |
| Me | Me | 3-Cl-2-pyridyl | $CF_3$ | Me | H | 3-Cl-2-pyridyl | $CF_3$ |
| Me | Me | 3-Cl-2-pyridyl | $OCF_3$ | Me | H | 3-Cl-2-pyridyl | $OCF_3$ |
| Me | Me | 3-Cl-2-pyridyl | $SCHF_2$ | Me | H | 3-Cl-2-pyridyl | $SCHF_2$ |
| Me | Me | 3-Cl-2-pyridyl | Br | Me | H | 3-Cl-2-pyridyl | Br |
| Me | Me | 3-Cl-2-pyridyl | Cl | Me | H | 3-Cl-2-pyridyl | Cl |
| Me | Me | 3-Br-2-pyridyl | $CF_3$ | Me | H | 3-Br-2-pyridyl | $CF_3$ |
| Me | Me | 3-Br-2-pyridyl | $OCF_3$ | Me | H | 3-Br-2-pyridyl | $OCF_3$ |
| Me | Me | 3-Br-2-pyridyl | Br | Me | H | 3-Br-2-pyridyl | Br |
| Me | Me | 3-Br-2-pyridyl | Cl | Me | H | 3-Br-2-pyridyl | Cl |
| Me | Me | $CF_3$ | Me | Me | H | $CF_3$ | Me |
| Me | Me | F | $CF_3$ | Me | H | F | $CF_3$ |
| Me | Me | Cl | $CF_3$ | Me | H | Cl | $CF_3$ |
| Me | Me | *n*-Pr | $CF_3$ | Me | H | *n*-Pr | $CF_3$ |
| Me | Me | *i*-Pr | $CF_3$ | Me | H | *i*-Pr | $CF_3$ |
| Me | Me | $CF_3$ | $CF_3$ | Me | H | $CF_3$ | $CF_3$ |
| Me | Me | OMe | $CF_3$ | Me | H | OMe | $CF_3$ |
| Me | Me | 2-BrPh | $CF_3$ | Me | H | 2-BrPh | $CF_3$ |
| Me | Me | 2-MePh | $CF_3$ | Me | H | 2-MePh | $CF_3$ |
| Me | Me | 2-CNPh | $CF_3$ | Me | H | 2-CNPh | $CF_3$ |
| Me | Me | 2-FPh | $CF_3$ | Me | H | 2-FPh | $CF_3$ |
| Me | Me | 2,6-$F_2$Ph | $CF_3$ | Me | H | 2,6-$F_2$Ph | $CF_3$ |
| Me | Me | 2,4-$F_2$Ph | $CF_3$ | Me | H | 2,4-$F_2$Ph | $CF_3$ |
| Me | Me | 2,5-$F_2$Ph | $CF_3$ | Me | H | 2,5-$F_2$Ph | $CF_3$ |

| $R^3$ | $R^{4a}$ | $R^{5a}$ | $R^{5b}$ | $R^3$ | $R^{4a}$ | $R^{5a}$ | $R^{5b}$ |
|---|---|---|---|---|---|---|---|
| Me | Me | 2-MeOPh | $CF_3$ | Me | H | 2-MeOPh | $CF_3$ |
| Me | Me | 3-F-2-pyridyl | $CF_3$ | Me | H | 3-F-2-pyridyl | $CF_3$ |
| Me | Me | 3-$CF_3$-2-pyridyl | $CF_3$ | Me | H | 3-$CF_3$-2-pyridyl | $CF_3$ |
| Me | Me | 3-Me-2-pyridyl | $CF_3$ | Me | H | 3-Me-2-pyridyl | $CF_3$ |
| Et | Me | H | $OCF_3$ | Et | H | H | $OCF_3$ |
| Et | Me | H | $C_2F_5$ | Et | H | H | $C_2F_5$ |
| Et | Me | Me | $CF_3$ | Et | H | Me | $CF_3$ |
| Et | Me | Me | $C_2F_5$ | Et | H | Me | $C_2F_5$ |
| *i*-Pr | Me | Me | $CF_3$ | *i*-Pr | H | Me | $CF_3$ |
| Me | Cl | H | $CF_3$ | Me | Cl | 2-ClPh | $CF_3$ |
| Me | Cl | H | $OCF_3$ | Me | Cl | 2-ClPh | $OCF_3$ |
| Me | Cl | H | $OCHF_2$ | Me | Cl | 2-ClPh | $SCHF_2$ |
| Me | Cl | H | $C_2F_5$ | Me | Cl | 2-ClPh | Br |
| Me | Cl | H | $OCF_2CHF_2$ | Me | Cl | 2-ClPh | Cl |
| Me | Cl | H | $SCF_2CHF_2$ | Me | Cl | 3-Cl-2-pyridyl | $CF_3$ |
| Me | Cl | H | *n*-$C_3F_7$ | Me | Cl | 3-Cl-2-pyridyl | $OCF_3$ |
| Me | Cl | H | *i*-$C_3F_7$ | Me | Cl | 3-Cl-2-pyridyl | $SCHF_2$ |
| Me | Cl | H | Br | Me | Cl | 3-Cl-2-pyridyl | Br |
| Me | Cl | H | Cl | Me | Cl | 3-Cl-2-pyridyl | Cl |
| Me | Cl | H | $SCF_3$ | Me | Cl | 3-Br-2-pyridyl | $CF_3$ |
| Me | Cl | Me | $CF_3$ | Me | Cl | 3-Br-2-pyridyl | $OCF_3$ |
| Me | Cl | Me | $OCF_3$ | Me | Cl | 3-Br-2-pyridyl | Br |
| Me | Cl | Me | $OCHF_2$ | Me | Cl | 3-Br-2-pyridyl | Cl |
| Me | Cl | Me | $C_2F_5$ | Me | Cl | $CF_3$ | Me |
| Me | Cl | Me | $OCF_2CHF_2$ | Me | Cl | F | $CF_3$ |
| Me | Cl | Me | $SCF_2CHF_2$ | Me | Cl | Cl | $CF_3$ |
| Me | Cl | Me | *n*-$C_3F_7$ | Me | Cl | *n*-Pr | $CF_3$ |
| Me | Cl | Me | *i*-$C_3F_7$ | Me | Cl | *i*-Pr | $CF_3$ |
| Me | Cl | Me | Br | Me | Cl | $CF_3$ | $CF_3$ |
| Me | Cl | Me | Cl | Me | Cl | OMe | $CF_3$ |
| Me | Cl | Me | $SCF_3$ | Me | Cl | 2-BrPh | $CF_3$ |
| Me | Cl | Me | I | Me | Cl | 2-MePh | $CF_3$ |
| Me | Cl | Me | SMe | Me | Cl | 2-CNPh | $CF_3$ |
| Me | Cl | Me | OMe | Me | Cl | 2-FPh | $CF_3$ |
| Me | Cl | Me | OEt | Me | Cl | 2,6-$F_2$Ph | $CF_3$ |

| $R^3$ | $R^{4a}$ | $R^{5a}$ | $R^{5b}$ | $R^3$ | $R^{4a}$ | $R^{5a}$ | $R^{5b}$ |
|---|---|---|---|---|---|---|---|
| Me | Cl | Me | Et | Me | Cl | 2,4-$F_2$Ph | $CF_3$ |
| Me | Cl | Me | $SO_2Me$ | Me | Cl | 2,5-$F_2$Ph | $CF_3$ |
| Me | Cl | Me | $SO_2CF_3$ | Me | Cl | 2-MeOPh | $CF_3$ |
| Me | Cl | Et | $CF_3$ | Me | Cl | 3-F-2-pyridyl | $CF_3$ |
| Me | Cl | Et | Br | Me | Cl | 3-$CF_3$-2-pyridyl | $CF_3$ |
| Me | Cl | Et | Cl | Me | Cl | 3-Me-2-pyridyl | $CF_3$ |
| Me | Cl | Ph | $CF_3$ | Et | Cl | H | $OCF_3$ |
| Me | Cl | Ph | Br | Et | Cl | H | $C_2F_5$ |
| Me | Cl | Ph | Cl | Et | Cl | Me | $CF_3$ |
| Me | Cl | 2-pyridyl | $CF_3$ | Et | Cl | Me | $C_2F_5$ |
| Me | Cl | 2-pyridyl | Cl | *i*-Pr | Cl | Me | $CF_3$ |

Table 14

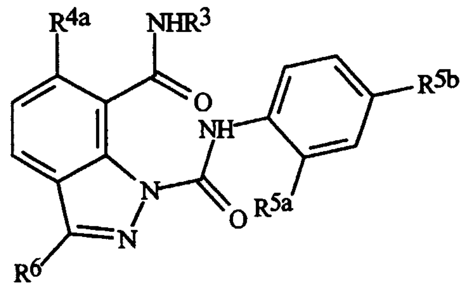

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | H | $CF_3$ | Me | H | H | H | $CF_3$ |
| Me | H | Me | H | $OCF_3$ | Me | H | H | H | $OCF_3$ |
| Me | H | Me | H | $OCHF_2$ | Me | H | H | H | $OCHF_2$ |
| Me | H | Me | H | $C_2F_5$ | Me | H | H | H | $C_2F_5$ |
| Me | H | Me | H | $OCF_2CHF_2$ | Me | H | H | H | $OCF_2CHF_2$ |
| Me | H | Me | H | $SCF_2CHF_2$ | Me | H | H | H | $SCF_2CHF_2$ |
| Me | H | Me | H | *n*-$C_3F_7$ | Me | H | H | H | *n*-$C_3F_7$ |
| Me | H | Me | H | *i*-$C_3F_7$ | Me | H | H | H | *i*-$C_3F_7$ |
| Me | H | Me | H | Br | Me | H | H | H | Br |
| Me | H | Me | H | Cl | Me | H | H | H | Cl |
| Me | H | Me | H | $SCF_3$ | Me | H | H | H | $SCF_3$ |
| Me | H | Me | Me | $CF_3$ | Me | H | H | Me | $CF_3$ |
| Me | H | Me | Me | $OCF_3$ | Me | H | H | Me | $OCF_3$ |
| Me | H | Me | Me | $OCHF_2$ | Me | H | H | Me | $OCHF_2$ |
| Me | H | Me | Me | $C_2F_5$ | Me | H | H | Me | $C_2F_5$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | Me | $OCF_2CHF_2$ | Me | H | H | Me | $OCF_2CHF_2$ |
| Me | H | Me | Me | $SCF_2CHF_2$ | Me | H | H | Me | $SCF_2CHF_2$ |
| Me | H | Me | Me | *n*-$C_3F_7$ | Me | H | H | Me | *n*-$C_3F_7$ |
| Me | H | Me | Me | *i*-$C_3F_7$ | Me | H | H | Me | *i*-$C_3F_7$ |
| Me | H | Me | Me | Br | Me | H | H | Me | Br |
| Me | H | Me | Me | Cl | Me | H | H | Me | Cl |
| Me | H | Me | Me | $SCF_3$ | Me | H | H | Me | $SCF_3$ |
| Me | H | Me | Me | I | Me | H | H | Me | I |
| Me | H | Me | Me | SMe | Me | H | H | Me | SMe |
| Me | H | Me | Me | OMe | Me | H | H | Me | OMe |
| Me | H | Me | Me | OEt | Me | H | H | Me | OEt |
| Me | H | Me | Me | Et | Me | H | H | Me | Et |
| Me | H | Me | Me | $SO_2Me$ | Me | H | H | Me | $SO_2Me$ |
| Me | H | Me | Me | $SO_2CF_3$ | Me | H | H | Me | $SO_2CF_3$ |
| Me | H | Me | Et | $CF_3$ | Me | H | H | Et | $CF_3$ |
| Me | H | Me | Et | Br | Me | H | H | Et | Br |
| Me | H | Me | Et | Cl | Me | H | H | Et | Cl |
| Me | H | Me | Ph | $CF_3$ | Me | H | H | Ph | $CF_3$ |
| Me | H | Me | Ph | Br | Me | H | H | Ph | Br |
| Me | H | Me | Ph | Cl | Me | H | H | Ph | Cl |
| Me | H | Me | 2-pyridyl | $CF_3$ | Me | H | H | 2-pyridyl | $CF_3$ |
| Me | H | Me | 2-pyridyl | Cl | Me | H | H | 2-pyridyl | Cl |
| Me | H | Me | 2-ClPh | $CF_3$ | Me | H | H | 2-ClPh | $CF_3$ |
| Me | H | Me | 2-ClPh | $OCF_3$ | Me | H | H | 2-ClPh | $OCF_3$ |
| Me | H | Me | 2-ClPh | $SCHF_2$ | Me | H | H | 2-ClPh | $SCHF_2$ |
| Me | H | Me | 2-ClPh | Br | Me | H | H | 2-ClPh | Br |
| Me | H | Me | 2-ClPh | Cl | Me | H | H | 2-ClPh | Cl |
| Me | H | Me | 3-Cl-2-pyridyl | $CF_3$ | Me | H | H | 3-Cl-2-pyridyl | $CF_3$ |
| Me | H | Me | 3-Cl-2-pyridyl | $OCF_3$ | Me | H | H | 3-Cl-2-pyridyl | $OCF_3$ |
| Me | H | Me | 3-Cl-2-pyridyl | $SCHF_2$ | Me | H | H | 3-Cl-2-pyridyl | $SCHF_2$ |
| Me | H | Me | 3-Cl-2-pyridyl | Br | Me | H | H | 3-Cl-2-pyridyl | Br |
| Me | H | Me | 3-Cl-2-pyridyl | Cl | Me | H | H | 3-Cl-2-pyridyl | Cl |
| Me | H | Me | 3-Br-2-pyridyl | $CF_3$ | Me | H | H | 3-Br-2-pyridyl | $CF_3$ |
| Me | H | Me | 3-Br-2-pyridyl | $OCF_3$ | Me | H | H | 3-Br-2-pyridyl | $OCF_3$ |
| Me | H | Me | 3-Br-2-pyridyl | Br | Me | H | H | 3-Br-2-pyridyl | Br |
| Me | H | Me | 3-Br-2-pyridyl | Cl | Me | H | H | 3-Br-2-pyridyl | Cl |
| Me | H | Me | $CF_3$ | Me | Me | H | H | $CF_3$ | Me |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | H | Me | F | $CF_3$ | Me | H | H | F | $CF_3$ |
| Me | H | Me | Cl | $CF_3$ | Me | H | H | Cl | $CF_3$ |
| Me | H | Me | *n*-Pr | $CF_3$ | Me | H | H | *n*-Pr | $CF_3$ |
| Me | H | Me | *i*-Pr | $CF_3$ | Me | H | H | *i*-Pr | $CF_3$ |
| Me | H | Me | $CF_3$ | $CF_3$ | Me | H | H | $CF_3$ | $CF_3$ |
| Me | H | Me | OMe | $CF_3$ | Me | H | H | OMe | $CF_3$ |
| Me | H | Me | 2-BrPh | $CF_3$ | Me | H | H | 2-BrPh | $CF_3$ |
| Me | H | Me | 2-MePh | $CF_3$ | Me | H | H | 2-MePh | $CF_3$ |
| Me | H | Me | 2-CNPh | $CF_3$ | Me | H | H | 2-CNPh | $CF_3$ |
| Me | H | Me | 2-FPh | $CF_3$ | Me | H | H | 2-FPh | $CF_3$ |
| Me | H | Me | 2,6-$F_2$Ph | $CF_3$ | Me | H | H | 2,6-$F_2$Ph | $CF_3$ |
| Me | H | Me | 2,4-$F_2$Ph | $CF_3$ | Me | H | H | 2,4-$F_2$Ph | $CF_3$ |
| Me | H | Me | 2,5-$F_2$Ph | $CF_3$ | Me | H | H | 2,5-$F_2$Ph | $CF_3$ |
| Me | H | Me | 2-MeOPh | $CF_3$ | Me | H | H | 2-MeOPh | $CF_3$ |
| Me | H | Me | 3-F-2-pyridyl | $CF_3$ | Me | H | H | 3-F-2-pyridyl | $CF_3$ |
| Me | H | Me | 3-$CF_3$-2-pyridyl | $CF_3$ | Me | H | H | 3-$CF_3$-2-pyridyl | $CF_3$ |
| Me | H | Me | 3-Me-2-pyridyl | $CF_3$ | Me | H | H | 3-Me-2-pyridyl | $CF_3$ |
| Et | H | Me | H | $OCF_3$ | Et | H | H | H | $OCF_3$ |
| Et | H | Me | H | $C_2F_5$ | Et | H | H | H | $C_2F_5$ |
| Et | H | Me | Me | $CF_3$ | Et | H | H | Me | $CF_3$ |
| Et | H | Me | Me | $C_2F_5$ | Et | H | H | Me | $C_2F_5$ |
| *i*-Pr | H | Me | Me | $CF_3$ | *i*-Pr | H | H | Me | $CF_3$ |
| Me | Me | H | H | $CF_3$ | Me | H | Cl | H | $CF_3$ |
| Me | Me | H | H | $OCF_3$ | Me | H | Cl | H | $OCF_3$ |
| Me | Me | H | H | $OCHF_2$ | Me | H | Cl | H | $OCHF_2$ |
| Me | Me | H | H | $C_2F_5$ | Me | H | Cl | H | $C_2F_5$ |
| Me | Me | H | H | $OCF_2CHF_2$ | Me | H | Cl | H | $OCF_2CHF_2$ |
| Me | Me | H | H | $SCF_2CHF_2$ | Me | H | Cl | H | $SCF_2CHF_2$ |
| Me | Me | H | H | *n*-$C_3F_7$ | Me | H | Cl | H | *n*-$C_3F_7$ |
| Me | Me | H | H | *i*-$C_3F_7$ | Me | H | Cl | H | *i*-$C_3F_7$ |
| Me | Me | H | H | Br | Me | H | Cl | H | Br |
| Me | Me | H | H | Cl | Me | H | Cl | H | Cl |
| Me | Me | H | H | $SCF_3$ | Me | H | Cl | H | $SCF_3$ |
| Me | Me | H | H | $CF_3$ | Me | H | Cl | Me | $CF_3$ |
| Me | Me | H | H | $OCF_3$ | Me | H | Cl | Me | $OCF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | H | $OCHF_2$ | Me | H | Cl | Me | $OCHF_2$ |
| Me | Me | H | Me | $C_2F_5$ | Me | H | Cl | Me | $C_2F_5$ |
| Me | Me | H | Me | $OCF_2CHF_2$ | Me | H | Cl | Me | $OCF_2CHF_2$ |
| Me | Me | H | Me | $SCF_2CHF_2$ | Me | H | Cl | Me | $SCF_2CHF_2$ |
| Me | Me | H | Me | $n$-$C_3F_7$ | Me | H | Cl | Me | $n$-$C_3F_7$ |
| Me | Me | H | Me | $i$-$C_3F_7$ | Me | H | Cl | Me | $i$-$C_3F_7$ |
| Me | Me | H | Me | Br | Me | H | Cl | Me | Br |
| Me | Me | H | Me | Cl | Me | H | Cl | Me | Cl |
| Me | Me | H | Me | $SCF_3$ | Me | H | Cl | Me | $SCF_3$ |
| Me | Me | H | Me | I | Me | H | Cl | Me | I |
| Me | Me | H | Me | SMe | Me | H | Cl | Me | SMe |
| Me | Me | H | Me | OMe | Me | H | Cl | Me | OMe |
| Me | Me | H | Me | OEt | Me | H | Cl | Me | OEt |
| Me | Me | H | Me | Et | Me | H | Cl | Me | Et |
| Me | Me | H | Me | $SO_2Me$ | Me | H | Cl | Me | $SO_2Me$ |
| Me | Me | H | Me | $SO_2CF_3$ | Me | H | Cl | Me | $SO_2CF_3$ |
| Me | Me | H | Et | $CF_3$ | Me | H | Cl | Et | $CF_3$ |
| Me | Me | H | Et | Br | Me | H | Cl | Et | Br |
| Me | Me | H | Et | Cl | Me | H | Cl | Et | Cl |
| Me | Me | H | Ph | $CF_3$ | Me | H | Cl | Ph | $CF_3$ |
| Me | Me | H | Ph | Br | Me | H | Cl | Ph | Br |
| Me | Me | H | Ph | Cl | Me | H | Cl | Ph | Cl |
| Me | Me | H | 2-pyridyl | $CF_3$ | Me | H | Cl | 2-pyridyl | $CF_3$ |
| Me | Me | H | 2-pyridyl | Cl | Me | H | Cl | 2-pyridyl | Cl |
| Me | Me | H | 2-ClPh | $CF_3$ | Me | H | Cl | 2-ClPh | $CF_3$ |
| Me | Me | H | 2-ClPh | $OCF_3$ | Me | H | Cl | 2-ClPh | $OCF_3$ |
| Me | Me | H | 2-ClPh | $SCHF_2$ | Me | H | Cl | 2-ClPh | $SCHF_2$ |
| Me | Me | H | 2-ClPh | Br | Me | H | Cl | 2-ClPh | Br |
| Me | Me | H | 2-ClPh | Cl | Me | H | Cl | 2-ClPh | Cl |
| Me | Me | H | 3-Cl-2-pyridyl | $CF_3$ | Me | H | Cl | 3-Cl-2-pyridyl | $CF_3$ |
| Me | Me | H | 3-Cl-2-pyridyl | $OCF_3$ | Me | H | Cl | 3-Cl-2-pyridyl | $OCF_3$ |
| Me | Me | H | 3-Cl-2-pyridyl | $SCHF_2$ | Me | H | Cl | 3-Cl-2-pyridyl | $SCHF_2$ |
| Me | Me | H | 3-Cl-2-pyridyl | Br | Me | H | Cl | 3-Cl-2-pyridyl | Br |
| Me | Me | H | 3-Cl-2-pyridyl | Cl | Me | H | Cl | 3-Cl-2-pyridyl | Cl |
| Me | Me | H | 3-Br-2-pyridyl | $CF_3$ | Me | H | Cl | 3-Br-2-pyridyl | $CF_3$ |
| Me | Me | H | 3-Br-2-pyridyl | $OCF_3$ | Me | H | Cl | 3-Br-2-pyridyl | $OCF_3$ |
| Me | Me | H | 3-Br-2-pyridyl | Br | Me | H | Cl | 3-Br-2-pyridyl | Br |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Me | H | 3-Br-2-pyridyl | Cl | Me | H | Cl | 3-Br-2-pyridyl | Cl |
| Me | Me | H | $CF_3$ | Me | Me | H | Cl | $CF_3$ | Me |
| Me | Me | H | F | $CF_3$ | Me | H | Cl | F | $CF_3$ |
| Me | Me | H | Cl | $CF_3$ | Me | H | Cl | Cl | $CF_3$ |
| Me | Me | H | *n*-Pr | $CF_3$ | Me | H | Cl | *n*-Pr | $CF_3$ |
| Me | Me | H | *i*-Pr | $CF_3$ | Me | H | Cl | *i*-Pr | $CF_3$ |
| Me | Me | H | $CF_3$ | $CF_3$ | Me | H | Cl | $CF_3$ | $CF_3$ |
| Me | Me | H | OMe | $CF_3$ | Me | H | Cl | OMe | $CF_3$ |
| Me | Me | H | 2-BrPh | $CF_3$ | Me | H | Cl | 2-BrPh | $CF_3$ |
| Me | Me | H | 2-MePh | $CF_3$ | Me | H | Cl | 2-MePh | $CF_3$ |
| Me | Me | H | 2-CNPh | $CF_3$ | Me | H | Cl | 2-CNPh | $CF_3$ |
| Me | Me | H | 2-FPh | $CF_3$ | Me | H | Cl | 2-FPh | $CF_3$ |
| Me | Me | H | 2,6-$F_2$Ph | $CF_3$ | Me | H | Cl | 2,6-$F_2$Ph | $CF_3$ |
| Me | Me | H | 2,4-$F_2$Ph | $CF_3$ | Me | H | Cl | 2,4-$F_2$Ph | $CF_3$ |
| Me | Me | H | 2,5-$F_2$Ph | $CF_3$ | Me | H | Cl | 2,5-$F_2$Ph | $CF_3$ |
| Me | Me | H | 2-MeOPh | $CF_3$ | Me | H | Cl | 2-MeOPh | $CF_3$ |
| Me | Me | H | 3-F-2-pyridyl | $CF_3$ | Me | H | Cl | 3-F-2-pyridyl | $CF_3$ |
| Me | Me | H | 3-$CF_3$-2-pyridyl | $CF_3$ | Me | H | Cl | 3-$CF_3$-2-pyridyl | $CF_3$ |
| Me | Me | H | 3-Me-2-pyridyl | $CF_3$ | Me | H | Cl | 3-Me-2-pyridyl | $CF_3$ |
| Et | Me | H | H | $OCF_3$ | Et | H | Cl | H | $OCF_3$ |
| Et | Me | H | H | $C_2F_5$ | Et | H | Cl | H | $C_2F_5$ |
| Et | Me | H | Me | $CF_3$ | Et | H | Cl | Me | $CF_3$ |
| Et | Me | H | Me | $C_2F_5$ | Et | H | Cl | Me | $C_2F_5$ |
| *i*-Pr | Me | H | Me | $CF_3$ | *i*-Pr | H | Cl | Me | $CF_3$ |
| Me | Cl | H | H | $CF_3$ | Me | Cl | H | 2-ClPh | $CF_3$ |
| Me | Cl | H | H | $OCF_3$ | Me | Cl | H | 2-ClPh | $OCF_3$ |
| Me | Cl | H | H | $OCHF_2$ | Me | Cl | H | 2-ClPh | $SCHF_2$ |
| Me | Cl | H | H | $C_2F_5$ | Me | Cl | H | 2-ClPh | Br |
| Me | Cl | H | H | $OCF_2CHF_2$ | Me | Cl | H | 2-ClPh | Cl |
| Me | Cl | H | H | $SCF_2CHF_2$ | Me | Cl | H | 3-Cl-2-pyridyl | $CF_3$ |
| Me | Cl | H | H | *n*-$C_3F_7$ | Me | Cl | H | 3-Cl-2-pyridyl | $OCF_3$ |
| Me | Cl | H | H | *i*-$C_3F_7$ | Me | Cl | H | 3-Cl-2-pyridyl | $SCHF_2$ |
| Me | Cl | H | H | Br | Me | Cl | H | 3-Cl-2-pyridyl | Br |
| Me | Cl | H | H | Cl | Me | Cl | H | 3-Cl-2-pyridyl | Cl |
| Me | Cl | H | H | $SCF_3$ | Me | Cl | H | 3-Br-2-pyridyl | $CF_3$ |

| $R^3$ | $R^{4a}$ | $R^6$ | $R^{5a}$ | $R^{5b}$ | $R^3$ | $R^{4a}$ | $R^6$ | $R^{5a}$ | $R^{5b}$ |
|---|---|---|---|---|---|---|---|---|---|
| Me | Cl | H | Me | $CF_3$ | Me | Cl | H | 3-Br-2-pyridyl | $OCF_3$ |
| Me | Cl | H | Me | $OCF_3$ | Me | Cl | H | 3-Br-2-pyridyl | Br |
| Me | Cl | H | Me | $OCHF_2$ | Me | Cl | H | 3-Br-2-pyridyl | Cl |
| Me | Cl | H | Me | $C_2F_5$ | Me | Cl | H | $CF_3$ | Me |
| Me | Cl | H | Me | $OCF_2CHF_2$ | Me | Cl | H | F | $CF_3$ |
| Me | Cl | H | Me | $SCF_2CHF_2$ | Me | Cl | H | Cl | $CF_3$ |
| Me | Cl | H | Me | *n*-$C_3F_7$ | Me | Cl | H | *n*-Pr | $CF_3$ |
| Me | Cl | H | Me | *i*-$C_3F_7$ | Me | Cl | H | *i*-Pr | $CF_3$ |
| Me | Cl | H | Me | Br | Me | Cl | H | $CF_3$ | $CF_3$ |
| Me | Cl | H | Me | Cl | Me | Cl | H | OMe | $CF_3$ |
| Me | Cl | H | Me | $SCF_3$ | Me | Cl | H | 2-BrPh | $CF_3$ |
| Me | Cl | H | Me | I | Me | Cl | H | 2-MePh | $CF_3$ |
| Me | Cl | H | Me | SMe | Me | Cl | H | 2-CNPh | $CF_3$ |
| Me | Cl | H | Me | OMe | Me | Cl | H | 2-FPh | $CF_3$ |
| Me | Cl | H | Me | OEt | Me | Cl | H | 2,6-$F_2$Ph | $CF_3$ |
| Me | Cl | H | Me | Et | Me | Cl | H | 2,4-$F_2$Ph | $CF_3$ |
| Me | Cl | H | Me | $SO_2Me$ | Me | Cl | H | 2,5-$F_2$Ph | $CF_3$ |
| Me | Cl | H | Me | $SO_2CF_3$ | Me | Cl | H | 2-MeOPh | $CF_3$ |
| Me | Cl | H | Et | $CF_3$ | Me | Cl | H | 3-F-2-pyridyl | $CF_3$ |
| Me | Cl | H | Et | Br | Me | Cl | H | 3-$CF_3$-2-pyridyl | $CF_3$ |
| Me | Cl | H | Et | Cl | Me | Cl | H | 3-Me-2-pyridyl | $CF_3$ |
| Me | Cl | H | Ph | $CF_3$ | Et | Cl | H | H | $OCF_3$ |
| Me | Cl | H | Ph | Br | Et | Cl | H | H | $C_2F_5$ |
| Me | Cl | H | Ph | Cl | Et | Cl | H | Me | $CF_3$ |
| Me | Cl | H | 2-pyridyl | $CF_3$ | Et | Cl | H | Me | $C_2F_5$ |
| Me | Cl | H | 2-pyridyl | Cl | *i*-Pr | Cl | H | Me | $CF_3$ |

Formulation/Utility

Compounds of this invention will generally be used as a formulation or composition with an agriculturally suitable carrier comprising at least one of a liquid diluent, a solid diluent or a surfactant. The formulation or composition ingredients are selected to be consistent with the physical properties of the active ingredient, mode of application and environmental factors such as soil type, moisture and temperature. Useful formulations include liquids such as solutions (including emulsifiable concentrates), suspensions, emulsions (including microemulsions and/or suspoemulsions) and the like which optionally can be thickened into gels. Useful formulations further include solids such as dusts, powders, granules, pellets, tablets, films, and the like which can be water-dispersible ("wettable") or water-soluble. Active ingredient can be (micro)encapsulated and further formed into a suspension or solid formulation; alternatively the entire formulation of active ingredient can be encapsulated (or "overcoated"). Encapsulation can control or delay release of

the active ingredient. Sprayable formulations can be extended in suitable media and used at spray volumes from about one to several hundred liters per hectare. High-strength compositions are primarily used as intermediates for further formulation.

The formulations will typically contain effective amounts of active ingredient, diluent and surfactant within the following approximate ranges that add up to 100 percent by weight.

| | Weight Percent | | |
|---|---|---|---|
| | Active Ingredient | Diluent | Surfactant |
| Water-Dispersible and Water-soluble Granules, Tablets and Powders. | 5-90 | 0-94 | 1-15 |
| Suspensions, Emulsions, Solutions (including Emulsifiable Concentrates) | 5-50 | 40-95 | 0-15 |
| Dusts | 1-25 | 70-99 | 0-5 |
| Granules and Pellets | 0.01-99 | 5-99.99 | 0-15 |
| High Strength Compositions | 90-99 | 0-10 | 0-2 |

Typical solid diluents are described in Watkins, et al., *Handbook of Insecticide Dust Diluents and Carriers,* 2nd Ed., Dorland Books, Caldwell, New Jersey. Typical liquid diluents are described in Marsden, *Solvents Guide,* 2nd Ed., Interscience, New York, 1950. *McCutcheon's Detergents and Emulsifiers Annual,* Allured Publ. Corp., Ridgewood, New Jersey, as well as Sisely and Wood, *Encyclopedia of Surface Active Agents,* Chemical Publ. Co., Inc., New York, 1964, list surfactants and recommended uses. All formulations can contain minor amounts of additives to reduce foam, caking, corrosion, microbiological growth and the like, or thickeners to increase viscosity.

Surfactants include, for example, polyethoxylated alcohols, polyethoxylated alkylphenols, polyethoxylated sorbitan fatty acid esters, dialkyl sulfosuccinates, alkyl sulfates, alkylbenzene sulfonates, organosilicones, *N,N*-dialkyltaurates, lignin sulfonates, naphthalene sulfonate formaldehyde condensates, polycarboxylates, and polyoxyethylene/polyoxypropylene block copolymers. Solid diluents include, for example, clays such as bentonite, montmorillonite, attapulgite and kaolin, starch, sugar, silica, talc, diatomaceous earth, urea, calcium carbonate, sodium carbonate and bicarbonate, and sodium sulfate. Liquid diluents include, for example, water, *N,N*-dimethylformamide, dimethyl sulfoxide, *N*-alkylpyrrolidone, ethylene glycol, polypropylene glycol, paraffins, alkylbenzenes, alkylnaphthalenes, oils of olive, castor, linseed, tung, sesame, com, peanut, cotton-seed, soybean, rape-seed and coconut, fatty acid esters, ketones such as cyclohexanone, 2-heptanone, isophorone and 4-hydroxy-4-methyl-2-pentanone, and alcohols such as methanol, cyclohexanol, decanol and tetrahydrofurfuryl alcohol.

Solutions, including emulsifiable concentrates, can be prepared by simply mixing the ingredients. Dusts and powders can be prepared by blending and, usually, grinding as in a hammer mill or fluid-energy mill. Suspensions are usually prepared by wet-milling; see, for example, U.S. 3,060,084. Granules and pellets can be prepared by spraying the active material upon preformed granular carriers or by agglomeration techniques. See Browning, "Agglomeration", *Chemical Engineering,* December 4, 1967, pp 147-48, *Perry's Chemical Engineer's Handbook*, 4th Ed., McGraw-Hill, New York, 1963, pages 8-57 and following, and PCT Publication WO 91/13546. Pellets can be prepared as described in U.S. 4,172,714. Water-dispersible and water-soluble granules can be prepared as taught in U.S. 4,144,050, U.S. 3,920,442 and DE 3,246,493. Tablets can be prepared as taught in U.S. 5,180,587, U.S. 5,232,701 and U.S. 5,208,030. Films can be prepared as taught in GB 2,095,558 and U.S. 3,299,566.

For further information regarding the art of formulation, see T. S. Woods, "The Formulator's Toolbox - Product Forms for Modem Agriculture" in *Pesticide Chemistry and Bioscience, The Food-Environment Challenge,* T. Brooks and T. R. Roberts, Eds., Proceedings of the 9th International Congress on Pesticide Chemistry, The Royal Society of Chemistry, Cambridge, 1999, pp. 120-133. See also U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, *Weed Control as a Science,* John Wiley and Sons, Inc., New York, 1961, pp 81-96; and Hance et al., *Weed Control Handbook*, 8th Ed., Blackwell Scientific Publications, Oxford, 1989.

In the following Examples, all percentages are by weight and all formulations are prepared in conventional ways. Compound numbers refer to compounds in Index Table A.

| Example A | |
|---|---|
| Wettable Powder | |
| Compound 1 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0%. |

| Example B | |
|---|---|
| Granule | |
| Compound 1 | 10.0% |
| attapulgite granules (low volatile matter, 0.71/0.30 mm; U.S.S. No. 25-50 sieves) | 90.0%. |

| Example C | |
|---|---|
| Ewxtruded Pellet | |
| Compound 1 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0%. |

| Example D | |
|---|---|
| Emulsifiable Concentrate | |
| Compound 1 | 20.0% |
| blend of oil soluble sulfonates and polyoxyethylene ethers | 10.0% |
| isophorone | 70.0%. |

| Example E | |
|---|---|
| Granule | |
| Compound 1 | 0.5% |
| cellulose | 2.5% |
| lactose | 4.0% |
| cornmeal | 93.0%. |

Compounds of this invention are characterized by favorable metabolic and/or soil residual patterns and exhibit activity controlling a spectrum of agronomic and nonagronomic invertebrate pests. (In the context of this disclosure "invertebrate pest control" means inhibition of invertebrate pest development (including mortality) that causes significant reduction in feeding or other injury or damage caused by the pest; related expressions are defined analogously.) As referred to in this disclosure, the term "invertebrate pest" includes arthropods, gastropods and nematodes of economic importance as pests. The term "arthropod" includes insects, mites, spiders, scorpions, centipedes, millipedes, pill bugs and symphylans. The term "gastropod" includes snails, slugs and other Stylommatophora. The term "nematode" includes all of the helminths, such as: roundworms, heartworms, and phytophagous nematodes (Nematoda), flukes (Tematoda), Acanthocephala, and tapeworms (Cestoda). Those skilled in the art will recognize that not all compounds are equally effective against all pests. Compounds of this invention display activity against economically important agronomic, forest, greenhouse, nursery, ornamentals, food and fiber, public and animal health, domestic and commercial structure, household, and stored product pests. These include larvae of the order Lepidoptera, such as armyworms, cutworms, loopers, and heliothines in the family Noctuidae (e.g., fall armyworm (*Spodoptera fugiperda* J. E. Smith), beet armyworm *(Spodoptera exigua* Hübner), black cutworm *(Agrotis ipsilon* Hufnagel), cabbage looper *(Trichoplusia ni* Hübner), tobacco budworm *(Heliothis virescens* Fabricius)); borers, casebearers, webworms, coneworms, cabbageworms and skeletonizers from the family Pyralidae (e.g., European corn borer *(Ostrinia nubilalis* Hübner), navel orangeworm *(Amyelois transitella* Walker), corn root webworm *(Crambus caliginosellus* Clemens), sod webworm (*Herpetogramma licarsisalis* Walker)); leafrollers, budworms, seed worms, and fruit worms in the family Tortricidae (e.g., codling moth (*Cydia pomonella* Linnaeus), grape berry moth *(Endopiza viteana* Clemens), oriental fruit moth (*Grapholita molesta* Busck)); and many other economically important lepidoptera (e.g., diamondback moth *(Plutella xylostella* Linnaeus), pink bollworm *(Pectinophora gossypiella* Saunders), gypsy moth *(Lymantria dispar* Linnaeus)); nymphs and adults of the order Blattodea including cockroaches from the families Blattellidae and Blattidae (e.g., oriental cockroach *(Blatta orientalis* Linnaeus), Asian cockroach *(Blatella asahinai* Mizukubo), German cockroach *(Blattella germanica* Linnaeus), brownbanded cockroach *(Supella longipalpa* Fabricius), American cockroach *(Periplaneta americana* Linnaeus), brown cockroach *(Periplaneta brunnea* Burmeister), Madeira cockroach *(Leucophaea maderae* Fabricius)); foliar feeding larvae and adults of the order Coleoptera including weevils from the families Anthribidae, Bruchidae, and Curculionidae (e.g., boll weevil *(Anthonomus grandis* Boheman), rice water weevil *(Lissorhoptrus oryzophilus* Kuschel), granary weevil *(Sitophilus granarius* Linnaeus), rice weevil *(Sitophilus oryzae* Linnaeus)); flea beetles, cucumber beetles, rootworms, leaf beetles, potato beetles, and leafminers in the family Chrysomelidae (e.g., Colorado potato beetle *(Leptinotarsa decemlineata* Say), western corn rootworm *(Diabrotica virgifera virgifera* LeConte)); chafers and other beetles from the family Scaribaeidae (e.g., Japanese beetle *(Popillia japonica* Newman) and European chafer *(Rhizotrogus majalis* Razoumowsky)); carpet beetles from the family Dermestidae; wireworms from the family Elateridae; bark beetles from the family Scolytidae and flour beetles from the family Tenebrionidae. In addition it includes: adults and larvae of the order Dermaptera including earwigs from the family Forficulidae (e.g., European earwig (*Forficula auricularia* Linnaeus), black earwig *(Chelisoches morio* Fabricius)); adults and nymphs of the orders Hemiptera and Homoptera such as, plant bugs from the family Miridae, cicadas from the family Cicadidae, leafhoppers (e.g. *Empoasca* spp.) from the family Cicadellidae, planthoppers from the families Fulgoroidae and Delphacidae, treehoppers from the family Membracidae, psyllids from the family Psyllidae, whiteflies from the family Aleyrodidae, aphids from the family Aphididae, phylloxera from the family Phylloxeridae, mealybugs from the family Pseudococcidae, scales from the families Coccidae, Diaspididae and Margarodidae, lace bugs from the family Tingidae, stink bugs from the family Pentatomidae, cinch bugs (e.g., *Blissus* spp.) and other seed bugs from the family Lygaeidae, spittlebugs from the family Cercopidae squash bugs from the family Coreidae, and red bugs and cotton stainers from the family Pyrrhocoridae. Also included are adults and larvae of the order Acari (mites) such as spider mites and red mites in the family Tetranychidae (e.g., European red mite *(Panonychus ulmi* Koch), two spotted spider mite *(Tetranychus urticae* Koch), McDaniel mite *(Tetranychus mcdanieli* McGregor)), flat mites in the family Tenuipalpidae (e.g., citrus flat mite *(Brevipalpus lewisi* McGregor)), rust and bud mites in the family Eriophyidae and other foliar feeding mites and mites important in human and animal health, i.e. dust mites in the family Epidermoptidae, follicle mites in the family Demodicidae, grain mites in the family Glycyphagidae, ticks in the order Ixodidae (e.g., deer tick (*Ixodes scapularis* Say), Australian paralysis tick *(Ixodes holocyclus* Neumann), American dog tick *(Dermacentor variabilis* Say), lone star tick *(Amblyomma americanum* Linnaeus) and scab and itch mites in the families Psoroptidae, Pyemotidae, and Sarcoptidae; adults and immatures of the order Orthoptera including grasshoppers, locusts and crickets (e.g., migratory grasshoppers (e.g., *Melanoplus sanguinipes* Fabricius, *M differentialis* Thomas), American grasshoppers (e.g., *Schistocerca americana* Drury), desert locust *(Schistocerca gregaria* Forskal), migratory locust *(Locusta migratoria* Linnaeus), house cricket *(Acheta domesticus* Linnaeus), mole crickets *(Gryllotalpa* spp.)); adults and immatures of the order Diptera including leafminers, midges, fruit flies (Tephritidae), frit flies (e.g., *Oscinella frit* Linnaeus), soil maggots, house flies (e.g., *Musca domestica* Linnaeus), lesser house flies (e.g., *Fannia canicularis* Linnaeus, *F. femoralis* Stein), stable flies (e.g., *Stomoxys calcitrans* Linnaeus), face flies, horn flies, blow flies (e.g., *Chrysomya* spp., *Phormia* spp.), and other muscoid fly pests, horse flies (e.g., *Tabanus* spp.), bot flies (e.g., *Gastrophilus* spp., *Oestrus* spp.), cattle grubs (e.g., *Hypoderma* spp.), deer flies (e.g., *Chrysops* spp.), keds (e.g., *Melophagus ovinus* Linnaeus) and other Brachycera, mosquitoes (e.g., *Aedes* spp., *Anopheles* spp., *Culex* spp.), black flies (e.g., *Prosimulium* spp., *Simulium* spp.), biting midges, sand flies, sciarids, and other Nematocera; adults and immatures of the order Thysanoptera including onion thrips *(Thrips tabaci* Lindeman) and other foliar feeding thrips; insect pests of the order Hymenoptera including ants (e.g., red carpenter ant *(Camponotus ferrugineus* Fabricius), black carpenter ant (*Camponotus pennsylvanicus* De Geer), Pharaoh ant *(Monomorium pharaonis* Linnaeus), little fire ant *(Wasmannia auropunctata* Roger), fire ant *(Solenopsis geminata* Fabricius), red imported fire ant *(Solenopsis invicta* Buren), Argentine ant *(Iridomyrmex humilis* Mayr), crazy ant *(Paratrechina longicornis* Latreille), pavement ant *(Tetramorium caespitum* Linnaeus), cornfield ant *(Lasius alienus* Förster), odorous house ant *(Tapinoma sessile* Say)), bees (including carpenter bees), hornets, yellow

jackets and wasps; insect pests of the order Isoptera including the eastern subterranean termite (*Reticulitermes flavipes* Kollar), western subterranean termite *(Reticulitermes hesperus* Banks), Formosan subterranean termite (*Coptotermes formosanus* Shiraki), West Indian drywood termite *(Incisitermes immigrans* Snyder) and other termites of economic importance; insect pests of the order Thysanura such as silverfish *(Lepisma saccharina* Linnaeus) and firebrat *(Thermobia domestica* Packard); insect pests of the order Mallophaga and including the head louse *(Pediculus humanus capitis* De Geer), body louse *(Pediculus humanus humanus* Linnaeus), chicken body louse *(Menacanthus stramineus* Nitszch), dog biting louse *(Trichodectes canis* De Geer), fluff louse *(Goniocotes gallinae* De Geer), sheep body louse *(Bovicola ovis* Schrank), short-nosed cattle louse *(Haematopinus eurysternus* Nitzsch), long-nosed cattle louse *(Linognathus vituli* Linnaeus) and other sucking and chewing parasitic lice that attack man and animals; insect pests of the order Siphonoptera including the oriental rat flea (*Xenopsylla cheopis* Rothschild), cat flea (*Ctenocephalides felis* Bouche), dog flea (*Ctenocephalides canis* Curtis), hen flea *(Ceratophyllus gallinae* Schrank), sticktight flea *(Echidnophaga gallinacea* Westwood), human flea *(Pulex irritans* Linnaeus) and other fleas afflicting mammals and birds. Additional arthropod pests covered include: spiders in the order Araneae such as the brown recluse spider *(Loxosceles reclusa* Gertsch & Mulaik) and the black widow spider *(Latrodectus mactans* Fabricius), and centipedes in the order Scutigeromorpha such as the house centipede *(Scutigera coleoptrata* Linnaeus). Activity also includes members of the Classes Nematoda, Cestoda, Trematoda, and Acanthocephala including economically important members of the orders Strongylida, Ascaridida, Oxyurida, Rhabditida, Spirurida, and Enoplida such as but not limited to economically important agricultural pests (i.e. root knot nematodes in the genus *Meloidogyne,* lesion nematodes in the genus *Pratylenchus*, stubby root nematodes in the genus *Trichodorus,* etc.) and animal and human health pests (i.e. all economically important flukes, tapeworms, and roundworms, such as *Strongylus vulgaris* in horses, *Toxocara canis* in dogs, *Haemonchus contortus* in sheep, *Dirofilaria immitis* Leidy in dogs, *Anoplocephala perfoliata* in horses, *Fasciola hepatica* Linnaeus in ruminants, etc.).

Compounds of the invention show particularly high activity against pests in the order Lepidoptera (e.g., *Alabama argillacea* Hübner (cotton leaf worm), *Archips argyrospila* Walker (fruit tree leaf roller), *A. rosana* Linnaeus (European leaf roller) and other *Archips* species, *Chilo suppressalis* Walker (rice stem borer), *Cnaphalocrosis medinalis* Guenee (rice leaf roller), *Crambus caliginosellus* Clemens (corn root webworm), *Crambus teterrellus* Zincken (bluegrass webworm), *Cydia pomonella* Linnaeus (codling moth), *Earias insulana* Boisduval (spiny bollworm), *Earias vittella* Fabricius (spotted bollworm), *Helicoverpa armigera* Hübner (American bollworm), *Helicoverpa zea* Boddie (com earworm), *Heliothis virescens* Fabricius (tobacco budworm), *Herpetogramma licarsisalis* Walker (sod webworm), *Lobesia botrana* Denis & Schiffermüller (grape berry moth), *Pectinophora gossypiella* Saunders (pink bollworm), *Phyllocnistis citrella* Stainton (citrus leafminer), *Pieris brassicae* Linnaeus (large white butterfly), *Pieris rapae* Linnaeus (small white butterfly), *Plutella xylostella* Linnaeus (diamondback moth), *Spodoptera exigua* Hübner (beet armyworm), *Spodoptera litura* Fabricius (tobacco cutworm, cluster caterpillar), *Spodoptera frugiperda* J. E. Smith (fall armyworm), *Trichoplusia ni* Hübner (cabbage looper) and *Tuta absoluta* Meyrick (tomato leafminer)). Compounds of the invention also have commercially significant activity on members from the order Homoptera including: *Acyrthisiphon pisum* Harris (pea aphid), *Aphis craccivora* Koch (cowpea aphid), *Aphis fabae* Scopoli (black bean aphid), *Aphis gossypii* Glover (cotton aphid, melon aphid), *Aphis pomi* De Geer (apple aphid), *Aphis spiraecola* Patch (spirea aphid), *Aulacorthum solani* Kaltenbach (foxglove aphid), *Chaetosiphon fragaefolii* Cockerell (strawberry aphid), *Diuraphis noxia* Kurdjumov/Mordvilko (Russian wheat aphid), *Dysaphis plantaginea* Paaserini (rosy apple aphid), *Eriosoma lanigerum* Hausmann (woolly apple aphid), *Hyalopterus pruni* Geoffroy (mealy plum aphid), *Lipaphis erysimi* Kaltenbach (turnip aphid), *Metopolophium dirrhodum* Walker (cereal aphid), *Macrosipum euphorbiae* Thomas (potato aphid), *Myzus persicae* Sulzer (peach-potato aphid, green peach aphid), *Nasonovia ribisnigri* Mosley (lettuce aphid), *Pemphigus* spp. (root aphids and gall aphids), *Rhopalosiphum maidis* Fitch (com leaf aphid), *Rhopalosiphum padi* Linnaeus (bird cherryoat aphid), *Schizaphis graminum* Rondani (greenbug), *Sitobion avenae* Fabricius (English grain aphid), *Therioaphis maculata* Buckton (spotted alfalfa aphid), *Toxoptera aurantii* Boyer de Fonscolombe (black citrus aphid), and *Toxoptera citricida* Kirkaldy (brown citrus aphid); *Adelges* spp. (adelgids); *Phylloxera devastatrix* Pergande (pecan phylloxera); *Bemisia tabaci* Gennadius (tobacco whitefly, sweetpotato whitefly), *Bemisia argentifolii* Bellows & Perring (silverleaf whitefly), *Dialeurodes citri* Ashmead (citrus whitefly) and *Trialeurodes vaporariorum* Westwood (greenhouse whitefly); *Empoasca fabae* Harris (potato leafhopper), *Laodelphax striatellus* Fallen (smaller brown planthopper), *Macrolestes quadrilineatus* Forbes (aster leafhopper), *Nephotettix cinticeps* Uhler (green leafhopper), *Nephotettix nigropictus* Stål (rice leafhopper), *Nilaparvata lugens* Stål (brown planthopper), *Peregrinus maidis* Ashmead (com planthopper), *Sogatella furcifera* Horvath (white-backed planthopper), *Sogatodes orizicola* Muir (rice delphacid), *Typhlocyba pomaria* McAtee white apple leafhopper, *Erythroneoura* spp. (grape leafhoppers); *Magicidada septendecim* Linnaeus (periodical cicada); *Icerya purchasi* Maskell (cottony cushion scale), *Quadraspidiotus perniciosus* Comstock (San Jose scale); *Planococcus citri* Risso (citrus mealybug); *Pseudococcus* spp. (other mealybug complex); *Cacopsylla pyricola* Foerster (pear psylla), *Trioza diospyri* Ashmead (persimmon psylla). These compounds also have activity on members from the order Hemiptera including: *Acrosternum hilare* Say (green stink bug), *Anasa tristis* De Geer (squash bug), *Blissus leucopterus leucopterus* Say (chinch bug), *Corythuca gossypii* Fabricius (cotton lace bug), *Cyrtopeltis modesta* Distant (tomato

bug), *Dysdercus suturellus* Herrich-Schäffer (cotton stainer), *Euchistus servus* Say (brown stink bug), *Euchistus variolarius* Palisot de Beauvois (one-spotted stink bug), *Graptosthetus* spp. (complex of seed bugs), *Leptoglossus corculus* Say (leaf-footed pine seed bug), *Lygus lineolaris* Palisot de Beauvois (tarnished plant bug), *Nezara viridula* Linnaeus (southern green stink bug), *Oebalus pugnax* Fabricius (rice stink bug), *Oncopeltus fasciatus* Dallas (large milkweed bug), *Pseudatomoscelis seriatus* Reuter (cotton fleahopper). Other insect orders controlled by compounds of the invention include Thysanoptera (e.g., *Frankliniella occidentalis* Pergande (western flower thrip), *Scirthothrips citri* Moulton (citrus thrip), *Sericothrips variabilis* Beach (soybean thrip), and *Thrips tabaci* Lindeman (onion thrip); and the order Coleoptera (e.g., *Leptinotarsa decemlineata* Say (Colorado potato beetle), *Epilachna varivestis* Mulsant (Mexican bean beetle) and wireworms of the genera *Agriotes, Athous* or *Limonius).*

In accordance with this invention, compounds of Formula I, their *N*-oxides or salts thereof, and/or compounds of Formula II, their *N*-oxides or salts thereof, can be mixed with one or more other biologically active compounds or agents, e.g. a compound different from a compound of Formula I and/or Formula II and their *N*-oxides and salts, as the case may be, including insecticides, fungicides, nematocides, bactericides, acaricides, growth regulators such as rooting stimulants, chemosterilants, semiochemicals, repellents, attractants, pheromones, feeding stimulants, other biologically active compounds or entomopathogenic bacteria, virus or fungi to form a multi-component pesticide giving an even broader spectrum of agricultural utility. Thus, compositions of the present invention comprising at least one Formula I compound and/or Formula II compound can also comprise at least one other biologically active compound or agent selected from the group consisting of an other insecticide, a fungicide, a nematocide, a bactericide, an acaricide, a growth regulator, a rooting stimulant, a chemosteilant, a semiochemical, a repellent, an attractant, a pheromone, a feeding stimulant, and an entomopathogenic bacterium, virus or fungus. Such compositions can further comprise at least one additional component selected from the group consisting of a surfactant, a solid diluent, and a liquid diluent.

Examples of other biologically active compounds or agents useful in this invention include: insecticides such as abamectin, acephate, acetamiprid, avermectin, azadirachtin, azinphos-methyl, bifenthrin, binfenazate, buprofezin, carbofuran, chlorfenapyr, chlorfluazuron, chlorpyrifos, chlorpyrifos-methyl, chromafenozide, clothianidin, cyfluthrin, beta-cyfluthrin, cyhalothrin, lambda-cyhalothrin, cypermethrin, cyromazine, deltamethrin, diafenthiuron, diazinon, diflubenzuron, dimethoate, diofenolan, emamectin, endosulfan, esfenvalerate, ethiprole, fenothicarb, fenoxycarb, fenpropathrin, fenproximate, fenvalerate, fipronil, flonicamid, flucythrinate, tau-fluvalinate, flufenoxuron, fonophos, halofenozide, hexaflumuron, imidacloprid, indoxacarb, isofenphos, lufenuron, malathion, metaldehyde, methamidophos, methidathion, methomyl, methoprene, methoxychlor, monocrotophos, methoxyfenozide, nithiazin, novaluron, oxamyl, parathion, parathion-methyl, permethrin, phorate, phosalone, phosmet, phosphamidon, pirimicarb, profenofos, pymetrozine, pyridalyl, pyriproxyfen, rotenone, spinosad, sulprofos, tebufenozide, teflubenzuron, tefluthrin, terbufos, tetrachlorvinphos, thiacloprid, thiamethoxam, thiodicarb, thiosultap-sodium, tralomethrin, trichlorfon and triflumuron; fungicides such as acibenzolar, azoxystrobin, benomyl, blasticidin-S, Bordeaux mixture (tribasic copper sulfate), bromuconazole, carpropamid, captafol, captan, carbendazim, chloroneb, chlorothalonil, copper oxychloride, copper salts, cyflufenamid, cymoxanil, cyproconazole, cyprodinil, (*S*)-3,5-dichloro-*N*-(3-chloro-1-ethyl-1-methyl-2-oxopropyl)-4-methylbenzamide (RH 7281), diclocymet (S-2900), diclomezine, dicloran, difenoconazole, (*S*)-3,5-dihydro-5-methyl-2-(methylthio)-5-phenyl-3-(phenylamino)-4*H*-imidazol-4-one (RP 407213), dimethomorph, dimoxystrobin, diniconazole, diniconazole-M, dodine, edifenphos, epoxiconazole, famoxadone, fenamidone, fenarimol, fenbuconazole, fencaramid (SZX0722), fenpiclonil, fenpropidin, fenpropimorph, fentin acetate, fentin hydroxide, fluazinam, fludioxonil, flumetover (RPA 403397), fluquinconazole, flusilazole, flutolanil, flutriafol, folpet, fosetyl-aluminum, furalaxyl, furametapyr (S-82658), hexaconazole, ipconazole, iprobenfos, iprodione, isoprothiolane, kasugamycin, kresoxim-methyl, mancozeb, maneb, mefenoxam, mepronil, metalaxyl, metconazole, metominostrobin/fenominostrobin (SSF-126), myclobutanil, neo-asozin (ferric methanearsonate), oxadixyl, penconazole, pencycuron, probenazole, prochloraz, propamocarb, propiconazole, pyrifenox, pyraclostrobin, pyrimethanil, pyroquilon, quinoxyfen, spiroxamine, sulfur, tebuconazole, tetraconazole, thiabendazole, thifluzamide, thiophanate-methyl, thiram, tiadinil, triadimefon, triadimenol, tricyclazole, trifloxystrobin, triticonazole, validamycin and vinclozolin; nematocides such as aldicarb, oxamyl and fenamiphos; bactericides such as streptomycin; acaricides such as amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben and tebufenpyrad; and biological agents such as *Bacillus thuringiensis* including ssp. *aizawai* and *kurstaki, Bacillus thuringiensis* delta endotoxin, baculovirus, and entomopathogenic bacteria, virus and fungi.

A general reference for these agricultural protectants is *The Pesticide Manual, 12th Edition,* C. D. S. Tomlin, Ed., British Crop Protection Council, Farnham, Surrey, U.K., 2000.

Of note are compositions which comprise at least one compound selected from the group consisting of a compound of Formula I, an *N*-oxide or a salt thereof, and a compound of Formula II, an *N*-oxide or a salt thereof; and at least one other biologically active compound or agent selected from the group consisting of abamectin, acephate, acetamiprid, avermectin, azadirachtin, azinphos-methyl, bifenthrin, binfenazate, buprofezin, carbofuran, chlorfenapyr, chlorfluazuron, chlorpyrifos, chlorpyrifos-methyl, chromafenozide, clothianidin, cyfluthrin, beta-cyfluthrin, cyhalothrin, lambda-cyhalothrin, cypermethrin, cyromazine, deltamethrin, diafenthiuron, diazinon, diflubenzuron, dimethoate, diofenolan, emamectin, endosulfan, esfenvalerate, ethiprole, fenothicarb, fenoxycarb, fenpropathrin, fenproximate, fenvalerate, fipronil, flonicamid, flucythrinate, tau-fluvalinate, flufenoxuron, fonophos, halofenozide, hexaflumuron, imidacloprid, indoxacarb, isofenphos, lufenuron, malathion, metaldehyde, methamidophos, methidathion, methomyl, methoprene, methoxychlor, monocrotophos, methoxyfenozide, nithiazin, novaluron, oxamyl, parathion, parathion-methyl, permethrin, phorate, phosalone, phosmet, phosphamidon, pirimicarb, profenofos, pymetrozine, pyridalyl, pyriproxyfen, rotenone, spinosad, sulprofos, tebufenozide, teflubenzuron, tefluthrin, terbufos, tetrachlorvinphos, thiacloprid, thiamethoxam, thiodicarb, thiosultap-sodium, tralomethrin, trichlorfon and triflumuron, aldicarb, oxamyl, fenamiphos, amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben, tebufenpyrad, *Bacillus thuringiensis, Bacillus thuringiensis* delta endotoxin, baculovirus, and entomopathogenic bacteria, virus and fungi.

Preferred compositions include compositions which comprise at least one compound selected from the group consisting of a compound of Formula I, an *N*-oxide or a salt thereof and a compound of Formula II, an *N*-oxide or a salt thereof; and at least one other biologically active compound or agent selected from the group consisting of a pyrethroid (e.g. cypermethrin, cyhalothrin, cyfluthrin, beta-cyfluthrin, esfenvalerate, fenvalerate and tralomethrin); a carbamate (e.g. fenothicarb, methomyl, oxamyl and thiodicarb); a neonicotinoid (e.g. clothianidin, imidacloprid and thiacloprid); a neuronal sodium channel blocker (e.g. indoxacarb); an insecticidal macrocyclic lactone (e.g. spinosad, abamectin, avermectin and emamectin); a γ-aminobutyric acid (GABA) antagonist (e.g. endosulfan, ethiprole and fipronil); an insecticidal urea (e.g. flufenoxuron and triflumuron); a juvenile hormone mimic (e.g. diofenolan and pyriproxyfen); pymetrozine; and amitraz. Preferred other biologically active agents useful in compositions of this invention include *Bacillus thuringiensis* and *Bacillus thuringiensis* delta endotoxin as well as naturally occurring and genetically modified viral insecticides including members of the family Baculoviridae as well as entomophagous fungi.

Of note are preferred compositions which comprise at least one compound selected from the group consisting of a compound of Formula I, an *N*-oxide or a salt thereof, and a compound of Formula II, an *N*-oxide or a salt thereof; and at least one biologically active compound or agent selected from the group consisting of cypermethrin, cyhalothrin, cyfluthrin, beta-cyfluthrin, esfenvalerate, fenvalerate, tralomethrin, fenothicarb, methomyl, oxamyl, thiodicarb, clothianidin, imidacloprid, thiacloprid, indoxacarb, spinosad, abamectin, avermectin, emamectin, endosulfan, ethiprole, fipronil, flufenoxuron, triflumuron, diofenolan, pyriproxyfen, pymetrozine, amitraz, *Bacillus thuringiensis, Bacillus thuringiensis* delta endotoxin and entomophagous fungi.

Most preferred mixtures useful in the compositions and methods of this invention include a mixture of a compound of this invention with cyhalothrin; a mixture of a compound of this invention with cyfluthrin; a mixture of a compound of this invention with beta-cyfluthrin; a mixture of a compound of this invention with esfenvalerate; a mixture of a compound of this invention with methomyl; a mixture of a compound of this invention with imidacloprid; a mixture of a compound of this invention with thiacloprid; a mixture of a compound of this invention with indoxacarb; a mixture of a compound of this invention with abamectin; a mixture of a compound of this invention with endosulfan; a mixture of a compound of this invention with ethiprole; a mixture of a compound of this invention with fipronil; a mixture of a compound of this invention with flufenoxuron; a mixture of a compound of this invention with pyriproxyfen; a mixture of a compound of this invention with pymetrozine; a mixture of a compound of this invention with amitraz; a mixture of a compound of this invention with *Bacillus thuringiensis* and a mixture of a compound of this invention with *Bacillus thuringiensis* delta endotoxin.

In certain instances, combinations with other invertebrate pest control compounds or agents having a similar spectrum of control but a different mode of action will be particularly advantageous for resistance management. Thus, compositions and methods of the present invention can further comprise or utilize a biologically effective amount of at least one other invertebrate pest control compound or agent having a similar spectrum of control but a different mode of action from the compound of the invention. Contacting a plant genetically modified to express a plant protection compound (e.g., protein) or the locus of the plant with a biologically effective amount of a compound of this invention can also provide a broader spectrum of plant protection and be advantageous for resistance management.

At least one invertebrate pest is controlled and protection of agronomic, horticultural and specialty crops, animal and human health is achieved by applying, one or more of the compounds or compositions of this invention, in a biologically effective amount, to the environment of the pest including the agronomic and/or nonagronomic locus of infestation, to the area to be protected, or directly on the pest to be controlled. Thus, the present invention further comprises a method for the control of at least one foliar- or soil-inhabiting invertebrate and/or protection of agronomic and/or nonagronomic environments, comprising contacting the invertebrate pest or its environment with a biologically effective amount of one or more of the compounds of the invention, or with a biologically effective amount of a composition comprising at least one such compound, or with a composition comprising at least one such compound and a biologically effective amount of at least biologically effective amount of a one other biologically active compound or agent. Examples of suitable compositions comprising at least one compound of the invention and at least one other biologically active compound or agent include granular compositions wherein the other biologically active compound is present on the same granule as the compound of the invention or on a granule that is separate from the granule

where the compound of this invention is present.

A preferred method of contact is by spraying. Alternatively, a granular composition comprising a compound of the invention can be applied to the plant foliage or the soil. Compounds of this invention are effective in delivery through plant uptake by contacting the plant with a composition comprising a compound of this invention applied as a soil drench of a liquid formulation, a granular formulation to the soil, a nursery box treatment or a dip of transplants. Other methods of contact include application of a compound or a composition of the invention by direct and residual sprays, aerial sprays, seed coats, microencapsulations, systemic uptake, baits, eartags, boluses, foggers, fumigants, aerosols, dusts and many others.

The compounds of this invention can be incorporated into baits that are consumed by the invertebrates or within devices such as traps and the like. Granules or baits comprising between 0.01-5% active ingredient, 0.05-10% moisture retaining agent(s) and 40-99% vegetable flour are effective in controlling soil insects at very low application rates, particularly at doses of active ingredient that are lethal by ingestion rather than by direct contact.

The compounds of this invention can be applied in their pure state, but most often application will be of a formulation comprising one or more compounds with suitable carriers, diluents, and surfactants and possibly in combination with a food depending on the contemplated end use. A preferred method of application involves spraying a water dispersion or refined oil solution of the compounds. Combinations with spray oils, spray oil concentrations, spreader stickers, adjuvants, other solvents, and synergists such as piperonyl butoxide often enhance compound efficacy.

The rate of application required for effective control (i.e. "biologically effective amount") will depend on such factors as the species of invertebrate to be controlled, the pest's life cycle, life stage, its size, location, time of year, host crop or animal, feeding behavior, mating behavior, ambient moisture, temperature, and the like. Under normal circumstances, application rates of about 0.01 to 2 kg of active ingredient per hectare are sufficient to control pests in agronomic ecosystems, but as little as 0.0001 kg/hectare may be sufficient or as much as 8 kg/hectare may be required. For nonagronomic applications, effective use rates will range from about 1.0 to 50 mg/square meter but as little as 0.1 mg/square meter may be sufficient or as much as 150 mg/square meter may be required. One skilled in the art can easily determine the biologically effective amount necessary for the desired level of invertebrate pest control.

The following Tests in the Biological Examples of the Invention demonstrate the control efficacy of compounds of this invention on specific pests. "Control efficacy" represents inhibition of arthropod development (including mortality) that causes significantly reduced feeding. The pest control protection afforded by the compounds is not limited, however, to these species. See Index Tables A-E for compound descriptions. The following abbreviations are used in the Index Tables that follow: *t* is tertiary, *n* is normal, *i* is iso, *s* is secondary, Me is methyl, Et is ethyl, Pr is propyl and Bu is butyl; accordingly *i*-Pr is isopropyl, *s*-Bu is secondary butyl, etc. Ac is $COCH_3$. The abbreviation "Ex." stands for "Example" and is followed by a number indicating in which example the compound is prepared.

## INDEX TABLE A

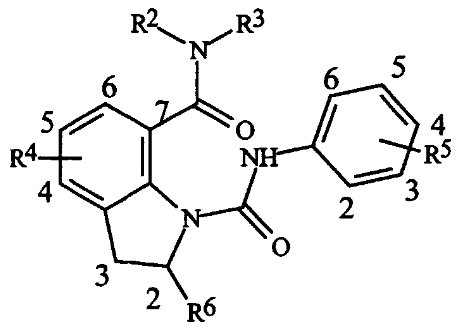

| Compound | $R^2$ | $R^3$ | $R^4$ | $R^5$ | $R^6$ | m.p. °C |
|---|---|---|---|---|---|---|
| 1 | *i*-Pr | H | H | 4-$CF_3$ | H | 200-202 |
| 2 (Ex. 1) | Me | H | H | 4-$CF_3$ | H | 198-200 |
| 3 | *i*-Pr | H | 5-Br | 4-$CF_3$ | H | * |
| 4 | *i*-Pr | H | H | 4-*i*-$C_3F_7$ | H | * |
| 5 | Me | H | H | 4-*i*-$C_3F_7$ | H | 202-204 |
| 12 | Et | H | 5-Cl | 2-Me-4-$CF_3$ | H | 239-240 |
| 13 | *i*-Pr | H | 5-Cl | 2-Me-4-$CF_3$ | H | 238-239 |
| 14 | Et | H | 6-Cl | 2-Me-4-$CF_3$ | H | 228-230 |
| 15 | *i*-Pr | H | H | 2-Me-4-$CF_3$ | Me | 179-180 |
| 16 | *i*-Pr | H | H | 2-Me-4-OMe | H | 214-215 |
| 17 | *i*-Pr | H | H | 3-$CF_3$-5-$CF_3$ | H | 235-137 |
| 18 | *i*-Pr | H | H | 2,3-di-F | H | 237-238 |
| 19 | *i*-Pr | H | H | 2-Me-4-Cl | H | 216-217 |
| 20 | Me | H | H | 2-Me-4-$CF_3$ | Me | 218-219 |
| 21 | Me | H | 6-Cl | 2-Me-4-$CF_3$ | H | 225-226 |
| 22 | *i*-Pr | H | H | 2,3-di-Cl | H | 239-240 |
| 23 | *i*-Pr | H | H | 2,4-di-Cl | H | 209-210 |
| 24 | *i*-Pr | H | H | 2-Cl-4-$CF_3$ | H | 196-197 |
| 25 | *i*-Pr | H | H | 4-$OCF_3$ | H | 223-224 |

| | | | | | | |
|---|---|---|---|---|---|---|
| 26 | *i*-Pr | H | H | 2-$CF_3$ | H | 241-242 |
| 27 | *i*-Pr | H | H | 2-Cl-4-$CF_3$ | Me | 189-190 |
| 28 | Et | H | 5-Cl | 2-Cl-4-$CF_3$ | H | 225-227 |
| 29 | *i*-Pr | H | 5-Cl | 2-Cl-4-$CF_3$ | H | 224-226 |
| 30 | Me | H | 5-Cl | 2-Cl-4-$CF_3$ | H | 245-247 |
| 31 | *i*-Pr | H | 6-Cl | 2-Me-4-$CF_3$ | H | 215-217 |
| 32 | *i*-Pr | H | H | 2-Me-4-Br | H | 212-213 |
| 33 | Me | H | 6-Cl | 2-Me-4-*i*-$C_3F_7$ | H | 213-214 |

*See Index Table D for NMR data.

## INDEX TABLE B

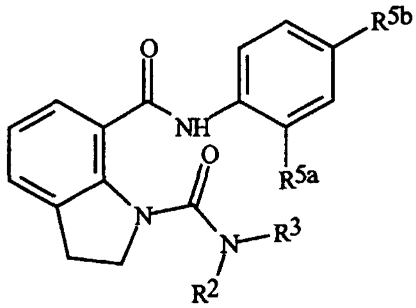

| Compound | $R^2$ | $R^3$ | $R^{5a}$ | $R^{5b}$ | m.p. °C |
|---|---|---|---|---|---|
| 6 | *i*-Pr | H | Me | $CF_3$ | 231-233 |
| 7 | Me | H | Me | $CF_3$ | 261-263 |
| 8 | Et | H | Me | $CF_3$ | 259-260 |
| 9 | Me | H | Me | *i*-$C_3F_7$ | * |
| 10 | Et | H | Me | *i*-$C_3F_7$ | * |
| 11 (Ex. 2) | *i*-Pr | H | Me | *i*-$C_3F_7$ | * |

## INDEX TABLE C

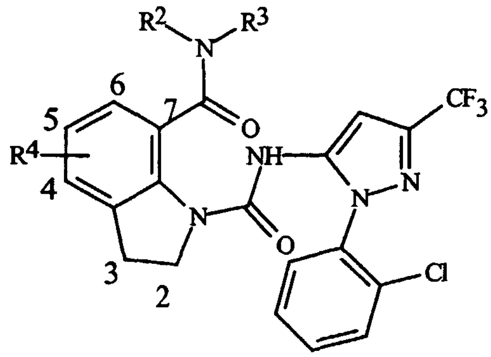

| Compound | $R^2$ | $R^3$ | $R^4$ | m.p. °C |
|---|---|---|---|---|
| 34 | *i*-Pr | H | H | 74-75 |

## INDEX TABLE D

| Cmpd No. | $^1$H NMR Data ($CDCl_3$ solution unless indicated otherwise)[a] |
|---|---|
| 3 | δ 1.1 (6H), 2.3 (3H), 3.1 (2H), 3.9 (1H), 4.1 (2H), 7.3 (1H), 7.5 (3H), 7.7 (1H), 7.9 (1H), 8.7 (1H). |
| 4 | δ 1.14 (d, 6H), 2.20 (s, 3H), 3.13 (t, 2H), 4.03-4.18 (m, 1H), 4.25 (t, 2H), 6.00 (d, 1H), 6.95 (s, 1H), 7.14 (t, 1H), 7.30-7.37 (m, 2H), 7.40 (d, 1H), 7.49 (d, 1H), 8.20 (d, 1H). |
| 9 | (DMSO-$d_6$) δ: 2.36 (s, 3H), 2.59 (d, 3H), 3.11( t, 2H), 4.00 (t, 2H), 6.84 (d, 1H), 6.99 (t, 1H), 7.31 (d, 1H), 7.42-7.48 (m, 2H), 7.97 (d, 1H), 9.39 (s, 1H). |
| 10 | (DMSO-$d_6$) δ: 0.93 (t, 3H), 2.37 (s, 3H), 3.03 (q, 2H), 3.13 (t, 2H), 4.00 (t, 2H), 6.98 (t, 1H), 7.14 (d, 1H), 7.33 (d, 1H), 7.42-7.46 (m, 2H), 8.04 (d, 1H), 9.29 (s, 1H). |
| 11 | (DMSO-$d_6$) δ: 0.98 (d, 6H), 2.37 (s, 3H), 3.21 (t, 2H), 3.62-3.71 (m, 1H), 4.01 (t, 1H), 6.73 (d, 1H), 7.00 (t, 1H), 7.32(d, 2H), 7.41-7.45 (m, 2H), 8.14 (d, 1H), 9.24 (s, 1H). |

[a] $^1$H NMR data are in ppm downfield from tetramethylsilane. Couplings are designated by (s)-singlet, (d)-doublet, (t)-triplet, (q)-quartet, (m)-multiplet, (dd)-doublet of doublets, (dt)-doublet of triplets, (br s)-broad singlet, (bs q)-broad quartet.

BIOLOGICAL EXAMPLES OF THE INVENTION

TEST A

For evaluating control of fall armyworm (*Spodoptera frugiperda*) the test unit consisted of a small open container with a 4-5-day-old corn (maize) plant inside. This was pre-infested with 10-15 1-day-old larvae on a piece of insect diet by use of a core sampler to remove a plug from a sheet of hardened insect diet having many larvae growing on it and transfer the plug containing larvae and diet to the test unit. The larvae moved onto the test plant as the diet plug dried out.

Test compounds were formulated using a solution containing 10% acetone, 90% water and 300 ppm X-77® Spreader Lo-Foam Formula non-ionic surfactant containing alkylarylpolyoxyethylene, free fatty acids, glycols and isopropanol (Loveland Industries, Inc. Greeley, Colorado, USA), unless otherwise indicated. The formulated compounds were applied in 1 mL of liquid through a SUJ2 atomizer nozzle with 1/8 JJ custom body (Spraying Systems Co. Wheaton, Illinois, USA) positioned 1.27 cm (0.5 inches) above the top of each test unit. All experimental compounds in this screen were sprayed at 50 ppm and replicated three times. After spraying of the formulated test compound, each test unit was allowed to dry for 1 hour and then a black, screened cap was placed on top. The test units were held for 6 days in a growth chamber at 25 °C and 70% relative humidity. Plant feeding damage was then visually assessed.

Of the compounds tested, the following provided excellent levels of plant protection (10% or less feeding damage): 1, 2, 4, 5, 21 and 33.

TEST B

For evaluating control of tobacco budworm *(Heliothis virescens)* the test unit consisted of a small open container with a 6-7 day old cotton plant inside. This was pre-infested with 8 2-day-old larvae on a piece of insect diet by use of a core sampler as described for Test A.

Test compounds were formulated and sprayed at 50 ppm as described for Test A. The applications were replicated three times. After spraying, the test units were maintained in a growth chamber and then visually rated as described for Test A.

Of the compounds tested, the following provided excellent levels of plant protection (10% or less feeding damage): 1 and 2.

TEST C

For evaluating control of diamondback moth *(Plutella xylostella)* the test unit consisted of a small open container with a 12-14-day-old radish plant inside. This was pre-infested with 10-15 neonate larvae on a piece of insect diet by use of a core sampler as described for Test A.

Test compounds were formulated and sprayed at 50 ppm as described for Test A. The applications were replicated three times. After spraying, the test units were maintained in a growth chamber and then visually rated as described for Test A.

Of the compounds tested, the following provided excellent levels of plant protection (10% or less feeding damage): 1, 2, 4, 5, 21, 25 and 33.

TEST D

For evaluating control of beet armyworm *(Spodoptera exigua)* the test unit consisted of a small open container with a 4-5-day-old corn plant inside. This was pre-infested with 10-15 1-day-old larvae on a piece of insect diet by use of a core sampler as described for Test A.

Test compounds were formulated and sprayed at 50 ppm as described for Test A. The applications were replicated three times. After spraying, the test units were maintained in a growth chamber and then visually rated as described for Test A.

Of the compounds tested, the following provided excellent levels of plant protection (10% or less feeding damage): 4.

## Claims

**1.** A method for controlling at least one invertebrate pest, comprising: contacting the invertebrate pest or its environment with a biologically effective amount of at least one compound selected from the group consisting of a compound of Formula I, an *N*-oxide or a salt thereof, and a compound of Formula II, an *N*-oxide or a salt thereof

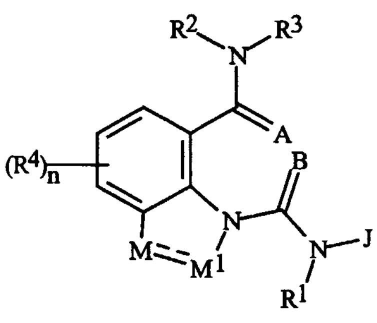

I

and

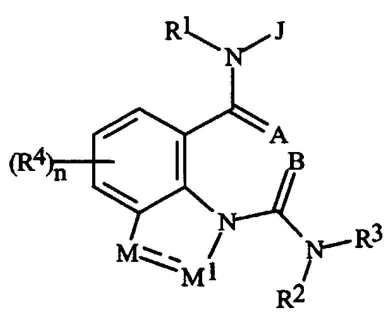

II

wherein

each J is independently a phenyl ring or a 5- or 6-membered heteroaromatic ring, each ring substituted with 1 to 4 $R^5$;

| | |
|---|---|
| A and B | are independently O or S; |
| n | is 0, 1, 2 or 3; |
| $R^1$ | is H, $C_2$-$C_6$ alkylcarbonyl, $C_2$-$C_6$ alkoxycarbonyl, $C_2$-$C_6$ alkylaminocarbonyl or $C_3$-$C_8$ dialkylaminocarbonyl; or |
| $R^1$ | is $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $NO_2$, hydroxy, $C_1$-$C_4$ alkoxy, $C_1$-$C_4$ alkylthio, $C_1$-$C_4$ alkylsulfinyl, $C_1$-$C_4$ alkylsulfonyl, $C_2$-$C_4$ alkoxycarbonyl, $C_1$-$C_4$ alkylamino, $C_2$-$C_8$ dialkylamino and $C_3$-$C_6$ cycloalkylamino; |
| $R^2$ | is H, $C_1$-$C_6$ alkyl, $C_2$-$C_6$ alkenyl, $C_2$-$C_6$ alkynyl, $C_3$-$C_6$ cycloalkyl, $C_1$-$C_4$ alkoxy, $C_1$-$C_4$ alkylamino, $C_2$-$C_8$ dialkylamino, $C_3$-$C_6$ cycloalkylamino, $C_2$-$C_6$ alkoxycarbonyl or $C_2$-$C_6$ alkylcarbonyl; |
| $R^3$ | is H; or |
| $R^3$ | is $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $NO_2$, hydroxy, $C_1$-$C_4$ alkoxy, $C_1$-$C_4$ alkylthio, $C_1$-$C_4$ alkylsulfinyl and $C_1$-$C_4$ alkylsulfonyl; or |
| $R^2$ | and $R^3$ can be taken together with the nitrogen to which they are attached to form a ring containing 2 to 6 atoms of carbon and optionally one additional atom of nitrogen, sulfur or oxygen, said ring may be optionally substituted with 1 to 4 substituents selected from the group consisting of $C_1$-$C_2$ alkyl, halogen, CN, $NO_2$ and $C_1$-$C_2$ alkoxy; and |
| each $R^4$ and each $R^5$ | is independently H, $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_2$-$C_6$ haloalkenyl, $C_2$-$C_6$ haloalkynyl, $C_3$-$C_6$ halocycloalkyl, halogen, CN, $CO_2H$, $CONH_2$, $NO_2$, hydroxy, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl, $C_1$-$C_4$ alkylamino, $C_2$-$C_8$ dialkylamino, $C_3$-$C_6$ cycloalkylamino, $C_2$-$C_6$ alkylcarbonyl, $C_2$-$C_6$ alkoxycarbonyl, $C_2$-$C_6$ alkylaminocarbonyl, $C_3$-$C_8$ dialkylaminocarbonyl, or $C_3$-$C_6$ trialkylsilyl; or |
| each $R^4$ and each $R^5$ | is independently a phenyl, benzyl, phenoxy, or 5- or 6-membered heteroaromatic ring, each ring optionally substituted with one to three substituents independently selected from the group consisting of $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_2$-$C_4$ haloalkenyl, $C_2$-$C_4$ haloalkynyl, $C_3$-$C_6$ halocycloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ alkylamino, $C_2$-$C_8$ dialkylamino, $C_3$-$C_6$ cycloalkylamino, $C_3$-$C_6$ (alkyl)cycloalkylamino, $C_2$-$C_4$ alkylcarbonyl, $C_2$-$C_6$ alkoxycarbonyl, $C_2$-$C_6$ alkylaminocarbonyl, $C_3$-$C_8$ dialkylaminocarbonyl or $C_3$-$C_6$ trialkylsilyl; or |
| two $R^5$ | groups when attached to adjacent carbon atoms can be taken together as $-OCF_2O-$, $-CF_2CF_2O-$ or $-OCF_2CF_2O-$; |
| M and $M^1$ | are each independently $CR^6R^7$, $NR^8$, O or S when the bond between M and $M^1$ is a single bond; and are each independently $CR^6$ or N when the bond between M and $M^1$ is an aromatic bond; |
| each $R^6$ and each $R^7$ | is independently H, $C_1$-$C_4$ alkyl, halogen, CN, $C_1$-$C_4$ haloalkyl or $C_1$-$C_4$ alkoxy; and |
| each $R^8$ | is independently H or $C_1$-$C_4$ alkyl. |

**2.** The method of Claim 1 comprising applying a biologically effective amount of a compound of Formula I wherein

| | |
|---|---|
| A and B | are both O; |
| $R^1$ | is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; and |
| n | is 0, 1 or 2. |

**3.** The method of Claim 1 comprising applying a biologically effective amount of a compound of Formula II wherein

| | |
|---|---|
| A and B | are both O; |
| $R^1$ | is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; and |
| n | is 0, 1 or 2. |

**4.** A composition comprising: at least one compound selected from the group consisting of a compound of Formula I as defined in Claim 1, an N-oxide or a salt thereof, and a compound of Formula II as defined in Claim 1, an N-oxide or a salt thereof; and at least one other biologically active compound or agent.

**5.** The composition of Claim 4 further comprising an additional component selected from the group consisting of a surfactant, a solid diluent, and a liquid diluent.

**6.** The composition of Claim 4 wherein the other biologically active compound or agent is selected from the group consisting of an other insecticide, a fungicide, a nematocide, a bactericide, an acaricide, a growth regulator, a rooting stimulant, a chemosterilant, a semiochemical, a repellent, an attractant, a pheromone, a feeding stimulant, and a entomopathogenic bacterium, virus or fungus.

**7.** The composition of Claim 6 wherein the other insecticide is selected from the group consisting of a pyrethroid, a carbamate, a neonicotinoid, a neuronal sodium channel blocker, an insecticidal macrocyclic lactone, a γ-aminobutyric acid (GABA) antagonist, an insecticidal urea, a juvenile hormone mimic, pymetrozine, and amitraz.

**8.** The composition of Claim 6 wherein the other insecticide is selected from the group consisting of abamectin, acephate, acetamiprid, avermectin, azadirachtin, azinphos-methyl, bifenthrin, binfenazate, buprofezin, carbofuran, chlorfenapyr, chlorfluazuron, chlorpyrifos, chlorpyrifos-methyl, chromafenozide, clothianidin, cyfluthrin, beta-cyfluthrin, cyhalothrin, lambda-cyhalothrin, cypermethrin, cyromazine, deltamethrin, diafenthiuron, diazinon, diflubenzuron, dimethoate, diofenolan, emamectin, endosulfan, esfenvalerate, ethiprole, fenothicarb, fenoxycarb, fenpropathrin, fenproximate, fenvalerate, fipronil, flonicamid, flucythrinate, tau-fluvalinate, flufenoxuron, fonophos, halofenozide, hexaflumuron, imidacloprid, indoxacarb, isofenphos, lufenuron, malathion, metaldehyde, methamidophos, methidathion, methomyl, methoprene, methoxychlor, monocrotophos, methoxyfenozide, nithiazin, novaluron, oxamyl, parathion, parathion-methyl, permethrin, phorate, phosalone, phosmet, phosphamidon, pirimicarb, profenofos, pymetrozine, pyridalyl, pyriproxyfen, rotenone, spinosad, sulprofos, tebufenozide, teflubenzuron, tefluthrin, terbufos, tetrachlorvinphos, thiacloprid, thiamethoxam, thiodicarb, thiosultap-sodium, tralomethrin, trichlorfon and triflumuron, aldicarb, oxamyl, fenamiphos, amitraz, chinomethionat, chlorobenzilate, cyhexatin, dicofol, dienochlor, etoxazole, fenazaquin, fenbutatin oxide, fenpropathrin, fenpyroximate, hexythiazox, propargite, pyridaben, tebufenpyrad, *Bacillus thuringiensis, Bacillus thuringiensis* delta endotoxin, baculovirus, and entomopathogenic bacteria, virus and fungi.

**9.** The composition of Claim 6 wherein the other insecticide is selected from the group consisting of cypermethrin, cyhalothrin, cyfluthrin, beta-cyfluthrin, esfenvalerate, fenvalerate, tralomethrin, fenothicarb, methomyl, oxamyl, thiodicarb, clothianidin, imidacloprid, thiacloprid, indoxacarb, spinosad, abamectin, avermectin, emamectin, endosulfan, ethiprole, fipronil, flufenoxuron, triflumuron, diofenolan, pyriproxyfen, pymetrozine, amitraz, *Bacillus thuringiensis, Bacillus thuringiensis* delta endotoxin and entomophagous fungi.

**10.** A method for controlling at least one invertebrate pest, comprising:

contacting the invertebrate pest or its environment with a biologically effective amount of a composition of Claim 4 or Claim 5.

**11.** A compound of Formula II as defined in Claim 1, an *N*-oxide thereof or a salt thereof.

**12.** The compound of Claim 11 wherein

A and B are both O;
$R^1$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; and
n is 0, 1 or 2.

**13.** The compound of Claim 12 wherein

J is a phenyl ring or a 5- or 6-membered heteroaromatic ring selected from the group consisting of J-1, J-2, J-3 and J-4, each J ring optionally substituted with 1 to 3 $R^5$

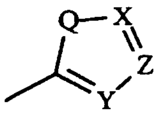
J-1

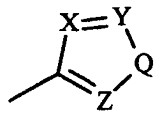
J-2

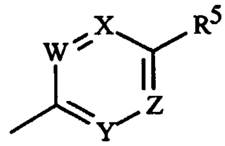
J-3

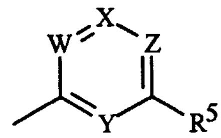
J-4

;

Q is O, S or $NR^5$;
W, X, Y and Z are independently N or $CR^5$, provided that in J-3 and J-4 at least one of the group consisting of W, X, Y and Z is N;
$R^2$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl;
$R^3$ is H, $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $C_1$-$C_2$ alkoxy, $C_1$-$C_2$ alkylthio, $C_1$-$C_2$ alkylsulfinyl and $C_1$-$C_2$ alkylsulfonyl;
each $R^4$ is independently $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl;
each $R^5$ is independently H, $C_1$-$C_4$ alkyl, $C_1$-$C_6$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl or $C_2$-$C_4$ alkoxycarbonyl, $C_2$-$C_6$ alkylaminocarbonyl, $C_3$-$C_8$ dialkylaminocarbonyl; or
each $R^5$ is independently a phenyl, benzyl or a 5- or 6-membered heteroaromatic ring, each ring optionally substituted with $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_2$-$C_4$ haloalkenyl, $C_2$-$C_4$ haloalkynyl, $C_3$-$C_6$ halocycloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ alkylamino, $C_2$-$C_8$ dialkylamino, $C_3$-$C_6$ cycloalkylamino, $C_3$-$C_6$ (alkyl)cycloalkylamino, $C_2$-$C_4$ alkylcarbonyl, $C_2$-$C_6$ alkoxycarbonyl, $C_2$-$C_6$ alkylaminocarbonyl, $C_3$-$C_8$ dialkylaminocarbonyl or $C_3$-$C_6$ trialkylsilyl; or
two $R^5$ groups when attached to adjacent carbon atoms can be taken together as $-OCF_2O-$, $-CF_2CF_2O-$ or $-OCF_2CF_2O-$.

**14.** The compound of Claim 13 wherein

$R^2$ is H;
$R^3$ is $C_1$-$C_4$ alkyl; and

at least one $R^5$ is other than H and is attached to J at the position *ortho* to the $N(R^1)C(=B)$ moiety.

**15.** A composition comprising:

at least one compound of Claim 11; and
at least one additional component selected from the group consisting of a surfactant, a solid diluent, a liquid diluent and an other biologically active compound or agent.

**16.** A method for controlling at least one invertebrate pest, comprising: contacting the invertebrate pest or its environment with a biologically effective amount of a compound of Claim 11 or with a biologically effective amount of a composition of Claim 15.

**17.** A compound of Formula Id, an *N*-oxide or a salt thereof,

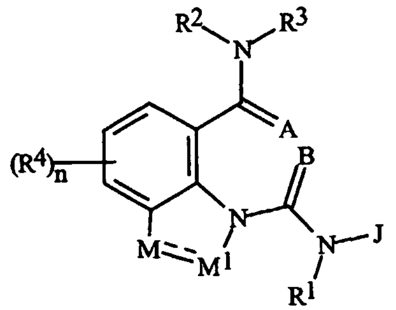

Id

wherein

| | |
|---|---|
| each J | is independently a phenyl ring or a 5- or 6-membered heteroaromatic ring, each ring optionally substituted with 1 to 4 $R^5$; |
| A and B | are independently O or S; |
| n | is 0, 1, 2 or 3; |
| $R^1$ | is H, $C_2$-$C_6$ alkylcarbonyl, $C_2$-$C_6$ alkoxycarbonyl, $C_2$-$C_6$ alkylaminocarbonyl or $C_3$-$C_8$ dialkylaminocarbonyl; or |
| $R^1$ | is $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $NO_2$, hydroxy, $C_1$-$C_4$ alkoxy, $C_1$-$C_4$ alkylthio, $C_1$-$C_4$ alkylsulfinyl, $C_1$-$C_4$ alkylsulfonyl, $C_2$-$C_4$ alkoxycarbonyl, $C_1$-$C_4$ alkylamino, $C_2$-$C_8$ dialkylamino and $C_3$-$C_6$ cycloalkylamino; |
| $R^2$ | is H, $C_1$-$C_6$ alkyl, $C_2$-$C_6$ alkenyl, $C_2$-$C_6$ alkynyl, $C_3$-$C_6$ cycloalkyl, $C_1$-$C_4$ alkoxy, $C_1$-$C_4$ alkylamino, $C_2$-$C_8$ dialkylamino, $C_3$-$C_6$ cycloalkylamino, $C_2$-$C_6$ alkoxycarbonyl or $C_2$-$C_6$ alkylcarbonyl; |
| $R^3$ | is H; or |
| $R^3$ | is $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $NO_2$, hydroxy, $C_1$-$C_4$ alkoxy, $C_1$-$C_4$ alkylthio, $C_1$-$C_4$ alkylsulfinyl and $C_1$-$C_4$ alkylsulfonyl; or |
| $R^2$ and $R^3$ | can be taken together with the nitrogen to which they are attached to form a ring containing 2 to 6 atoms of carbon and optionally one additional atom of nitrogen, sulfur or oxygen, said ring may be optionally substituted with 1 to 4 substituents selected from the group consisting of $C_1$-$C_2$ alkyl, halogen, CN, $NO_2$ and $C_1$-$C_2$ alkoxy; and |
| each $R^4$ and each $R^5$ | is independently H, $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_2$-$C_6$ haloalkenyl, $C_2$-$C_6$ haloalkynyl, $C_3$-$C_6$ halocycloalkyl, halogen, CN, $CO_2H$, $CONH_2$, $NO_2$, hydroxy, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl, $C_1$-$C_4$ alkylamino, $C_2$-$C_8$ dialkylamino, $C_3$-$C_6$ cycloalkylamino, $C_2$-$C_6$ alkylcarbonyl, $C_2$-$C_6$ alkoxycarbonyl, $C_2$-$C_6$ alkylaminocarbonyl, $C_3$-$C_8$ dialkylaminocarbonyl, or $C_3$-$C_6$ trialkylsilyl; or |
| each $R^5$ | is independently a phenyl, benzyl, phenoxy, or 5- or 6-membered heteroaromatic ring, each ring optionally substituted with one to three substituents independently selected from the group consisting of $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_2$-$C_4$ haloalkenyl, $C_2$-$C_4$ haloalkynyl, $C_3$-$C_6$ halocycloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ alkylamino, $C_2$-$C_8$ dialkylamino, $C_3$-$C_6$ cycloalkylamino, $C_3$-$C_6$ (alkyl)cycloalkylamino, $C_2$-$C_4$ alkylcarbonyl, $C_2$-$C_6$ alkoxycarbonyl, $C_2$-$C_6$ alkylaminocarbonyl, $C_3$-$C_8$ dialkylaminocarbonyl or $C_3$-$C_6$ trialkylsilyl; or |
| two $R^5$ | groups when attached to adjacent carbon atoms can be taken together as $-OCF_2O-$, $-CF_2CF_2O-$ or $-OCF_2CF_2O-$; |
| M and $M^1$ | are each independently $CR^6R^7$, $NR^8$, O or S when the bond between M and $M^1$ is a single bond; and are each independently $CR^6$ or N when the bond between M and $M^1$ is an aromatic bond; |
| each $R^6$ and each $R^7$ | is independently H, $C_1$-$C_4$ alkyl, halogen, CN, $C_1$-$C_4$ haloalkyl or $C_1$-$C_4$ alkoxy; and |

each $R^8$ is independently H or $C_1$-$C_4$ alkyl.

**18.** The compound of Claim 17 wherein

A and B are both O;
$R^1$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl; and
n is 0, 1 or 2.

**19.** The compound of Claim 18 wherein

J is a phenyl ring or a 5- or 6-membered heteroaromatic ring selected from the group consisting of J-1, J-2, J-3 and J-4

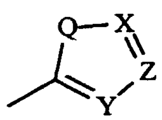
J-1

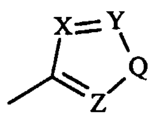
J-2

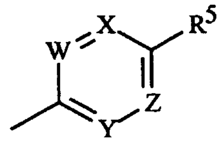
J-3

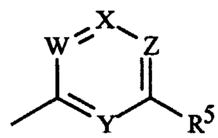
J-4

;

Q is O, S or $NR^5$;

W, X, Y and Z are independently N or $CR^5$, provided that in J-3 and J-4 at least one of the group consisting of W, X, Y and Z is N;
$R^2$ is H, $C_1$-$C_4$ alkyl, $C_2$-$C_4$ alkenyl, $C_2$-$C_4$ alkynyl, $C_3$-$C_6$ cycloalkyl, $C_2$-$C_6$ alkylcarbonyl or $C_2$-$C_6$ alkoxycarbonyl;
$R^3$ is H, $C_1$-$C_6$ alkyl, $C_2$-$C_6$ alkenyl, $C_2$-$C_6$ alkynyl or $C_3$-$C_6$ cycloalkyl each optionally substituted with one or more substituents selected from the group consisting of halogen, CN, $C_1$-$C_2$ alkoxy, $C_1$-$C_2$ alkylthio, $C_1$-$C_2$ alkylsulfinyl and $C_1$-$C_2$ alkylsulfonyl;
each $R^4$ is independently $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl or $C_1$-$C_4$ haloalkylsulfonyl;
each $R^5$ is independently H, $C_1$-$C_4$ alkyl, $C_1$-$C_6$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl or $C_2$-$C_4$ alkoxycarbonyl, $C_2$-$C_6$ alkylaminocarbonyl, $C_3$-$C_8$ dialkylaminocarbonyl; or
each $R^5$ is independently a phenyl, benzyl or a 5- or 6-membered heteroaromatic ring, each ring optionally substituted with $C_1$-$C_4$ alkyl, $C_2$-$C_4$ alkenyl, $C_2$-$C_4$ alkynyl, $C_3$-$C_6$ cycloalkyl, $C_1$-$C_4$ haloalkyl, $C_2$-$C_4$ haloalkenyl, $C_2$-$C_4$ haloalkynyl, $C_3$-$C_6$ halocycloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ alkylamino, $C_2$-$C_8$ dialkylamino, $C_3$-$C_6$ cycloalkylamino, $C_3$-$C_6$ (alkyl)cycloalkylamino, $C_2$-$C_4$ alkylcarbonyl, $C_2$-$C_6$ alkoxycarbonyl, $C_2$-$C_6$ alkylaminocarbonyl, $C_3$-$C_8$ dialkylaminocarbonyl or $C_3$-$C_6$ trialkylsilyl; or
two $R^5$ groups when attached to adjacent carbon atoms can be taken together as $-OCF_2O-$, $-CF_2CF_2O-$ or $-OCF_2CF_2O-$.

**20.** The compound of Claim 19 wherein

$R^1$ is H or $C_1$-$C_4$ alkyl;
$R^2$ is H or $C_1$-$C_4$ alkyl;
$R^3$ is H, $C_1$-$C_4$ alkyl optionally substituted with halogen, CN, $OCH_3$, or $S(O)_pCH_3$;
each $R^5$ is independently H, $C_1$-$C_4$ alkyl, $C_1$-$C_4$ haloalkyl, halogen, CN, $NO_2$, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, $C_1$-$C_4$ haloalkylthio, $C_1$-$C_4$ haloalkylsulfinyl, $C_1$-$C_4$ haloalkylsulfonyl, $C_2$-$C_4$ alkoxycarbonyl or $C_3$-$C_8$ dialkylaminocarbonyl; or a phenyl, benzyl, or a 5- or 6-membered heteroaromatic ring, each ring optionally substituted with halogen, CN, $NO_2$, $C_1$-$C_4$

alkyl, $C_2$-$C_4$ alkenyl, $C_2$-$C_4$ alkynyl, $C_3$-$C_6$ cycloalkyl, $C_1$-$C_4$ haloalkyl, $C_1$-$C_4$ alkoxy or $C_1$-$C_4$ haloalkoxy;
provided that at least one $R^5$ is other than H and is attached to J at the position *ortho* to the $N(R^1)C(=B)$ moiety; and

p is 0, 1 or 2.

**21.** The compound of Claim 20 wherein

J is a substituted phenyl, a substituted pyrazole, a substituted pyrrole, a substituted pyridine or a substituted pyrimidine.

**22.** The compound of Claim 21 wherein $R^1$ and $R^2$ are each H.

**23.** A composition comprising: at least one compound of Claim 17; and at least one additional component selected from the group consisting of a surfactant, a solid diluent, a liquid diluent and an other biologically active compound or agent.

**24.** A method for controlling at least one invertebrate pest, comprising: contacting the invertebrate pest or its environment with a biologically effective amount of a compound of Claim 17 or with a biologically effective amount of a composition of Claim 23.

**Patentansprüche**

**1.** Verfahren zur Kontrolle von mindestens einem wirbellosen Schädling, umfassend: Kontaktieren des wirbellosen Schädlings oder seiner Umgebung mit einer biologisch wirksamen Menge von mindestens einer Verbindung, die aus der Gruppe ausgewählt ist, bestehend aus einer Verbindung der Formel I, einem *N*-Oxid oder einem Salz davon und einer Verbindung der Formel II, einem *N*-Oxid oder einem Salz davon

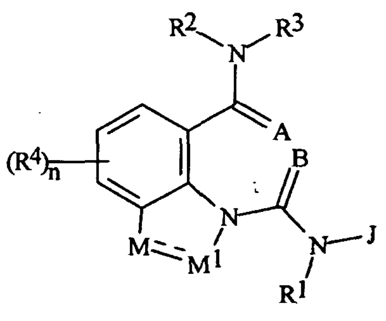

I

und

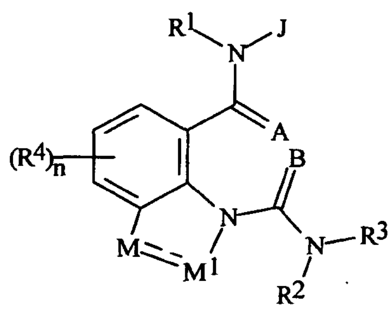

II

worin

jedes J unabhängig einen Phenylring oder einen 5- oder 6-gliedrigen heteroaromatischen Ring darstellt, wobei jeder Ring mit 1 bis 4 $R^5$ substituiert ist;
A und B unabhängig O oder S darstellen;
n für 0, für 1, für 2 oder für 3 steht;
$R^1$ für H, $C_2$-$C_6$-Alkylcarbonyl, $C_2$-$C_6$-Alkoxycarbonyl, $C_2$-$C_6$-Alkylaminocarbonyl oder $C_3$-$C_8$-Dialkylaminocarbonyl steht; oder
$R^1$ für $C_1$-$C_6$-Alkyl, $C_2$-$C_6$-Alkenyl, $C_2$-$C_6$-Alkinyl oder $C_3$-$C_6$-Cycloalkyl steht, wobei jedes optional mit einem oder mehr Substituenten substituiert ist, die aus der Gruppe ausgewählt sind, bestehend aus Halogen, CN, $NO_2$, Hydroxy, $C_1$-$C_4$-Alkoxy, $C_1$-$C_4$-Alkylthio, $C_1$-$C_4$-Alkylsulfinyl, $C_1$-$C_4$-Alkylsulfonyl, $C_2$-$C_4$-Alkoxycarbonyl, $C_1$-$C_4$-Alkylamino, $C_2$-$C_8$-Dialkylamino und $C_3$-$C_6$-Cycloalkylamino;
$R^2$ für H, $C_1$-$C_6$-Alkyl, $C_2$-$C_6$-Alkenyl, $C_2$-$C_6$-Alkinyl, $C_3$-$C_6$-Cycloalkyl, $C_1$-$C_4$-Alkoxy, $C_1$-$C_4$-Alkylamino, $C_2$-$C_8$-Dialkylamino, $C_3$-$C_6$-Cycloalkylamino, $C_2$-$C_6$-Alkoxycarbonyl oder $C_2$-$C_6$-Alkylcarbonyl steht;
$R^3$ für H steht; oder

$R^3$ für $C_1$-$C_6$-Alkyl, $C_2$-$C_6$-Alkenyl, $C_2$-$C_6$-Alkinyl oder $C_3$-$C_6$-Cycloalkyl steht, wobei jedes optional mit einem oder mehr Substituenten substituiert ist, die aus der Gruppe ausgewählt sind, bestehend aus Halogen, CN, $NO_2$, Hydroxy, $C_1$-$C_4$-Alkoxy, $C_1$-$C_4$-Alkylthio, $C_1$-$C_4$-Alkylsulfinyl und $C_1$-$C_4$-Alkylsulfonyl; oder
$R^2$ und $R^3$ mit dem Stickstoff zusammen genommen werden können, an den sie zur Bildung eines Rings, enthaltend 2 bis 6 Kohlenstoffatome und optional ein zusätzliches Stickstoff-, Schwefel- oder Sauerstoffatom, gebunden sind, wobei der Ring optional mit 1 bis 4 Substituenten substituiert sein kann, die aus der Gruppe ausgewählt sind, bestehend aus $C_1$-$C_2$-Alkyl, Halogen, CN, $NO_2$ und $C_1$-$C_2$-Alkoxy; und
jedes $R^4$ und jedes $R^5$ unabhängig H, $C_1$-$C_6$-Alkyl, $C_2$-$C_6$-Alkenyl, $C_2$-$C_6$-Alkinyl, $C_3$-$C_6$-Cycloalkyl, $C_1$-$C_6$-Haloalkyl, $C_2$-$C_6$-Haloalkenyl, $C_2$-$C_6$-Haloalkinyl, $C_3$-$C_6$-Halocycloalkyl, Halogen, CN, $CO_2H$, $CONH_2$, $NO_2$, Hydroxy, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Haloalkylthio, $C_1$-$C_4$-Haloalkylsulfinyl, $C_1$-$C_4$-Haloalkylsulfonyl, $C_1$-$C_4$-Alkylamino, $C_2$-$C_8$-Dialkylamino, $C_3$-$C_6$-Cycloalkylamino, $C_2$-$C_6$-Alkylcarbonyl, $C_2$-$C_6$-Alkoxycarbonyl, $C_2$-$C_6$-Alkylaminocarbonyl, $C_3$-$C_8$-Dialkylaminocarbonyl oder $C_3$-$C_6$-Trialkylsilyl darstellt; oder
jedes $R^4$ und jedes $R^5$ unabhängig ein Phenyl, Benzyl, Phenoxy oder einen 5- oder 6-gliedrigen heteroaromatischen Ring darstellt, wobei jeder Ring optional mit einem bis drei Substituenten substituiert ist, die unabhängig aus der Gruppe ausgewählt sind, bestehend aus $C_1$-$C_4$-Alkyl, $C_2$-$C_4$-Alkenyl, $C_2$-$C_4$-Alkinyl, $C_3$-$C_6$-Cycloalkyl, $C_1$-$C_4$-Haloalkyl, $C_2$-$C_4$-Haloalkenyl, $C_2$-$C_4$-Haloalkinyl, $C_3$-$C_6$-Halocycloalkyl, Halogen, CN, $NO_2$, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Alkylamino, $C_2$-$C_8$-Dialkylamino, $C_3$-$C_6$-Cycloalkylamino, $C_3$-$C_6$-(Alkyl)cycloalkylamino, $C_2$-$C_4$-Alkylcarbonyl, $C_2$-$C_6$-Alkoxycarbonyl, $C_2$-$C_6$-Alkylaminocarbonyl, $C_3$-$C_8$-Dialkylaminocarbonyl oder $C_3$-$C_6$-Trialkylsilyl; oder
zwei $R^5$-Gruppen, wenn sie an benachbarte Kohlenstoffatome gebunden sind, zusammen genommen werden können als $-OCF_2O-$, $-CF_2CF_2O-$ oder $-OCF_2CF_2O-$;
M und $M^1$ jeweils unabhängig $CR^6R^7$, $NR^8$, O oder S darstellen, wenn die Bindung zwischen M und $M^1$ eine Einfachbindung darstellt; und jeweils unabhängig $CR^6$ oder N darstellen, wenn die Bindung zwischen M und $M^1$ eine aromatische Bindung darstellt;
jedes $R^6$ und jedes $R^7$ unabhängig H, $C_1$-$C_4$-Alkyl, Halogen, CN, $C_1$-$C_4$-Haloalkyl oder $C_1$-$C_4$-Alkoxy darstellt; und
jedes $R^8$ unabhängig H oder $C_1$-$C_4$-Alkyl darstellt.

**2.** Verfahren nach Anspruch 1, umfassend die Applikation einer biologisch wirksamen Menge einer Verbindung der Formel I, worin

A und B beide O darstellen;
$R^1$ für H, $C_1$-$C_4$-Alkyl, $C_2$-$C_4$-Alkenyl, $C_2$-$C_4$-Alkinyl, $C_3$-$C_6$-Cycloalkyl, $C_2$-$C_6$-Alkylcarbonyl oder $C_2$-$C_6$-Alkoxycarbonyl steht; und
n für 0, für 1 oder für 2 steht.

**3.** Verfahren nach Anspruch 1, umfassend die Applikation einer biologisch wirksamen Menge einer Verbindung der Formula II, worin

A und B beide O darstellen;
$R^1$ für H, $C_1$-$C_4$-Alkyl, $C_2$-$C_4$-Alkenyl, $C_2$-$C_4$-Alkinyl, $C_3$-$C_6$-Cycloalkyl, $C_2$-$C_6$-Alkylcarbonyl oder $C_2$-$C_6$-Alkoxycarbonyl steht; und
n für 0, für 1 oder für 2 steht.

**4.** Zusammensetzung, umfassend: mindestens eine Verbindung, die aus der Gruppe ausgewählt ist, bestehend aus einer Verbindung der Formel I wie nach Anspruch 1 definiert, einem *N*-Oxid oder einem Salz davon, und einer Verbindung der Formel II wie nach Anspruch 1 definiert, einem *N*-Oxid oder einem Salz davon; und mindestens einer anderen biologisch aktiven Verbindung oder einem anderen biologisch aktiven Mittel.

**5.** Zusammensetzung nach Anspruch 4 weiter umfassend eine zusätzliche Komponente, die aus der Gruppe ausgewählt ist, bestehend aus einem Tensid, einem festen Verdünnungsmittel und einem flüssigen Verdünnungsmittel.

**6.** Zusammensetzung nach Anspruch 4, worin die andere biologisch aktive Verbindung oder das andere biologisch aktive Mittel aus der Gruppe ausgewählt ist, bestehend aus einem anderen Insektizid, einem Fungizid, einem Nematizid, einem Bakterizid, einem Akarizid, einem Wachstumsregulator, einem Wurzelstimulans, einem Chemosterilans, einer Semiochemikalie, einem Repellens, einem Attraktans, einem Pheromon, einem Fraßstimulans und einem entomopathogenen Bakterium, Virus oder Pilz.

**7.** Zusammensetzung nach Anspruch 6, worin das andere Insektizid aus der Gruppe ausgewählt ist, bestehend aus einem Pyrethroid, einem Carbamat, einem Neonicotinoid, einem neuronalen Natriumkanalblocker, einem insektiziden makrocyclischen Lacton, einem γ-Aminobuttersäure-Antagonist (GABA-Antagonist), einem insektiziden Harnstoff, einem Juvenilhormon-Mimik, Pymetrozin und Amitraz.

**8.** Zusammensetzung nach Anspruch 6, worin das andere Insektizid aus der Gruppe ausgewählt ist, bestehend aus Abamectin, Acephat, Acetamiprid, Avermectin, Azadirachtin, Azinphos-Methyl, Bifenthrin, Binfenazat, Buprofezin, Carbofuran, Chlorfenapyr, Chlorfluazuron, Chlorpyrifos, Chlorpyrifos-Methyl, Chromafenozid, Clothianidin, Cyfluthrin, β-Cyfluthrin, Cyhalothrin, λ-Cyhalothrin, Cypermethrin, Cyromazin, Deltamethrin, Diafenthiuron, Diazinon, Diflubenzuron, Dimethoat, Diofenolan, Emamectin, Endosulfan, Esfenvalerat, Ethiprol, Fenothicarb, Fenoxycarb, Fenpropathrin, Fenproximat, Fenvalerat, Fipronil, Flonicamid, Flucythrinat, τ-Fluvalinat, Flufenoxuron, Fonophos, Halofenozid, Hexaflumuron, Imidacloprid, Indoxacarb, Isofenphos, Lufenuron, Malathion, Metaldehyd, Methamidophos, Methidathion, Methomyl, Methopren, Methoxychlor, Monocrotophos, Methoxyfenozid, Nithiazin, Novaluron, Oxamyl, Parathion, Parathion-Methyl, Permethrin, Phorat, Phosalon, Phosmet, Phosphamidon, Pirimicarb, Profenofos, Pymetrozin, Pyridalyl, Pyriproxyfen, Rotenon, Spinosad, Sulprofos, Tebufenozid, Teflubenzuron, Tefluthrin, Terbufos, Tetrachlorvinphos, Thiacloprid, Thiamethoxam, Thiodicarb, Thiosultap-Natrium, Tralomethrin, Trichlorfon und Triflumuron, Aldicarb, Oxamyl, Fenamiphos, Amitraz, Chinomethionat, Chlorobenzilat, Cyhexatin, Dicofol, Dienochlor, Etoxazol, Fenazaquin, Fenbutatinoxid, Fenpropathrin, Fenpyroximat, Hexythiazox, Propargit, Pyridaben, Tebufenpyrad, *Bacillus thuringiensis,* δ-Endotoxin von *Bacillus thuringiensis,* Baculovirus und entomopathogenen Bakterien, Viren und Pilzen.

**9.** Zusammensetzung nach Anspruch 6, worin das andere Insektizid aus der Gruppe ausgewählt ist, bestehend aus Cypermethrin, Cyhalothrin, Cyfluthrin, β-Cyfluthrin, Esfenvalerat, Fenvalerat, Tralomethrin, Fenothicarb, Methomyl, Oxamyl, Thiodicarb, Clothianidin, Imidacloprid, Thiacloprid, Indoxacarb, Spinosad, Abamectin, Avermectin, Emamectin, Endosulfan, Ethiprol, Fipronil, Flufenoxuron, Triflumuron, Diofenolan, Pyriproxyfen, Pymetrozin, Amitraz, *Bacillus thuringiensis,* δ-Endotoxin von *Bacillus thuringiensis* und entomophagen Pilzen.

**10.** Verfahren zur Kontrolle von mindestens einem wirbellosen Schädling, umfassend:

Kontaktieren des wirbellosen Schädlings oder seiner Umgebung mit einer biologisch wirksamen Menge einer Zusammensetzung nach Anspruch 4 oder 5.

**11.** Verbindung der Formel II wie nach Anspruch 1 definiert, ein *N*-Oxid davon oder ein Salz davon.

**12.** Verbindung nach Anspruch 11, worin

A und B beide O darstellen;
$R^1$ für H, $C_1$-$C_4$-Alkyl, $C_2$-$C_4$-Alkenyl, $C_2$-$C_4$-Alkinyl, $C_3$-$C_6$-Cycloalkyl, $C_2$-$C_6$-Alkylcarbonyl oder $C_2$-$C_6$-Alkoxycarbonyl steht; und
n für 0, für 1 oder für 2 steht.

**13.** Verbindung nach Anspruch 12, worin

J einen Phenylring oder einen 5- oder 6-gliedrigen heteroaromatischen Ring darstellt, der aus der Gruppe ausgewählt ist, bestehend aus J-1, J-2, J-3 und J-4, wobei jeder J-Ring optional mit 1 bis 3 $R^5$ substituiert ist

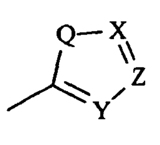
J-1

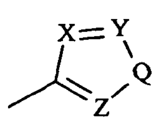
J-2

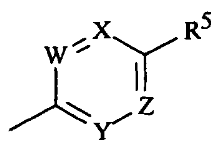
J-3

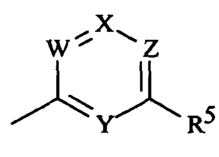
J-4

;

Q für O, S oder $NR^5$ steht;
W, X, Y und Z unabhängig N oder $CR^5$ darstellen, vorausgesetzt, dass in J-3 und J-4 mindestens eine der Gruppen aus W, X, Y besteht und Z für N steht;

$R^2$ für H, $C_1$-$C_4$-Alkyl, $C_2$-$C_4$-Alkenyl, $C_2$-$C_4$-Alkinyl, $C_3$-$C_6$-Cycloalkyl, $C_2$-$C_6$-Alkylcarbonyl oder $C_2$-$C_6$-Alkoxycarbonyl steht;
$R^3$ für H, $C_1$-$C_6$-Alkyl, $C_2$-$C_6$-Alkenyl, $C_2$-$C_6$-Alkinyl oder $C_3$-$C_6$-Cycloalkyl steht, wobei jedes optional mit einem oder mehr Substituenten substituiert ist, die aus der Gruppe ausgewählt sind, bestehend aus Halogen, CN, $C_1$-$C_2$-Alkoxy, $C_1$-$C_2$-Alkylthio, $C_1$-$C_2$-Alkylsulfinyl und $C_1$-$C_2$-Alkylsulfonyl;
jedes $R^4$ unabhängig $C_1$-$C_4$-Alkyl, $C_1$-$C_4$-Haloalkyl, Halogen, CN, $NO_2$, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Haloalkylthio, $C_1$-$C_4$-Haloalkylsulfinyl oder $C_1$-$C_4$-Haloalkylsulfonyl darstellt;
jedes $R^5$ unabhängig H, $C_1$-$C_4$-Alkyl, $C_1$-$C_6$-Haloalkyl, Halogen, CN, $NO_2$, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Haloalkylthio; $C_1$-$C_4$-Haloalkylsulfinyl, $C_1$-$C_4$-Haloalkylsulfonyl oder $C_2$-$C_4$-Alkoxycarbonyl, $C_2$-$C_6$-Alkylaminocarbonyl, $C_3$-$C_8$-Dialkylaminocarbonyl darstellt; oder
jedes $R^5$ unabhängig ein Phenyl, Benzyl oder einen 5- oder 6-gliedrigen heteroaromatischen Ring darstellt, wobei jeder Ring optional substituiert ist mit: $C_1$-$C_4$-Alkyl, $C_2$-$C_4$-Alkenyl, $C_2$-$C_4$-Alkinyl, $C_3$-$C_6$-Cycloalkyl, $C_1$-$C_4$-Haloalkyl, $C_2$-$C_4$-Haloalkenyl, $C_2$-$C_4$-Haloalkinyl, $C_3$-$C_6$-Halocycloalkyl, Halogen, CN, $NO_2$, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Alkylamino, $C_2$-$C_8$-Dialkylamino, $C_3$-$C_6$-Cycloalkylamino, $C_3$-$C_6$-(Alkyl)cycloalkylamino, $C_2$-$C_4$-Alkylcarbonyl, $C_2$-$C_6$-Alkoxycarbonyl, $C_2$-$C_6$-Alkylaminocarbonyl, $C_3$-$C_8$-Dialkylaminocarbonyl oder $C_3$-$C_6$-Trialkylsilyl; oder
zwei $R^5$-Gruppen, wenn sie an benachbarte Kohlenstoffatome gebunden sind, zusammengenommen werden können als $-OCF_2O-$, $-CF_2CF_2O-$ oder $-OCF_2CF_2O-$.

**14.** Verbindung nach Anspruch 13, worin

R2 für H steht;
$R^3$ für $C_1$-$C_4$-Alkyl steht; und
mindestens ein $R^5$ anders als H ist und an J an der *ortho*-Stellumg an die $N(R^1)C(=B)$-Komponente gebunden ist.

**15.** Zusammensetzung, umfassend:

mindestens eine Verbindung nach Anspruch 11; und
mindestens eine zusätzliche Komponente, die aus der Gruppe ausgewählt ist, bestehend aus einem Tensid, einem festen Verdünnungsmittel, einem flüssigen Verdünnungsmittel und einer anderen biologisch aktiven Verbindung oder einem anderen biologisch aktiven Mittel.

**16.** Verfahren zur Kontrolle von mindestens einem wirbellosen Schädling, umfassend: Kontaktieren des wirbellosen Schädlings oder seiner Umgebung mit einer biologisch wirksamen Menge einer Verbindung nach Anspruch 11 oder mit einer biologisch wirksamen Menge einer Zusammensetzung nach Anspruch 15.

**17.** Verbindung der Formula Id, eines *N*-Oxids oder eines Salzes davon,

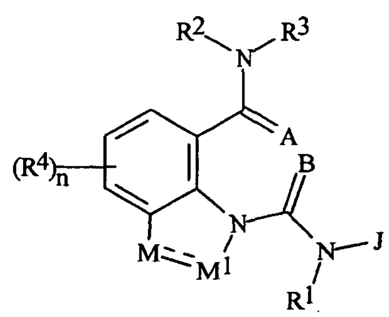

Id

worin

jedes J unabhängig einen Phenylring oder einen 5- oder 6-gliedrigen heteroaromatischen Ring darstellt, wobei jeder Ring optional mit 1 bis 4 $R^5$ substituiert ist;

A und B unabhängig O oder S darstellen;
n für 0, für 1, für 2 oder für 3 steht;
$R^1$ für H, $C_2$-$C_6$-Alkylcarbonyl, $C_2$-$C_6$-Alkoxycarbonyl, $C_2$-$C_6$-Akylaminocarbonyl oder $C_3$-$C_8$-Dialkylaminocarbonyl steht; oder
$R^1$ für $C_1$-$C_6$-Alkyl, $C_2$-$C_6$-Alkenyl, $C_2$-$C_6$-Alkinyl oder $C_3$-$C_6$-Cycloalkyl steht, wobei jedes optional mit einem oder mehr Substituenten substituiert ist, die aus der Gruppe ausgewählt sind, bestehend aus Halogen, CN, $NO_2$, Hydroxy, $C_1$-$C_4$-Alkoxy, $C_1$-$C_4$-Alkylthio, $C_1$-$C_4$-Alkylsulfinyl, $C_1$-$C_4$-Alkylsulfonyl, $C_2$-$C_4$-Alkoxycarbonyl, $C_1$-$C_4$-Alkylamino, $C_2$-$C_8$-Dialkylamino und $C_3$-$C_6$-Cycloalkylamino;
$R^2$ für H, $C_1$-$C_6$-Alkyl, $C_2$-$C_6$-Alkenyl, $C_2$-$C_6$-Alkinyl, $C_3$-$C_6$-Cycloalkyl, $C_1$-$C_4$-Alkoxy, $C_1$-$C_4$-Alkylamino, $C_2$-$C_8$-Dialkylamino, $C_3$-$C_6$-Cycloalkylamino, $C_2$-$C_6$-Alkoxycarbonyl oder $C_2$-$C_6$-Alkylcarbonyl steht;
$R^3$ für H steht; oder
$R^3$ für $C_1$-$C_6$-Alkyl, $C_2$-$C_6$-Alkenyl, $C_2$-$C_6$-Alkinyl oder $C_3$-$C_6$-Cycloalkyl steht, wobei jedes optional mit einem oder mehr Substituenten substituiert ist, die aus der Gruppe ausgewählt sind, bestehend aus Halogen, CN, $NO_2$, Hydroxy, $C_1$-$C_4$-Alkoxy, $C_1$-$C_4$-Alkylthio, $C_1$-$C_4$-Alkylsulfinyl und $C_1$-$C_4$-Alkylsulfonyl; oder
$R^2$ und $R^3$ mit dem Stickstoff zusammengenommen werden können, an den sie zur Bildung eines Rings, enthaltend 2 bis 6 Kohlenstoffatome und optional ein zusätzliches Stickstoff-, Schwefel- oder Sauerstoffatom gebunden sind, wobei der Ring optional mit 1 bis 4 Substituenten substituiert sein kann, die aus der Gruppe ausgewählt sind, bestehend aus $C_1$-$C_2$-Alkyl, Halogen, CN, $NO_2$ und $C_1$-$C_2$-Alkoxy; und
jedes $R^4$ und jedes $R^5$ unabhängig H, $C_1$-$C_6$-Alkyl, $C_2$-$C_6$-Alkenyl, $C_2$-$C_6$-Alkinyl, $C_3$-$C_6$-Cycloalkyl, $C_1$-$C_6$-Haloalkyl, $C_2$-$C_6$-Haloalkenyl, $C_2$-$C_6$-Haloalkinyl, $C_3$-$C_6$-Halocycloalkyl, Halogen, CN, $CO_2H$, $CONH_2$, $NO_2$, Hydroxy, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Haloalkylthio, $C_1$-$C_4$-Haloalkylsulfinyl, $C_1$-$C_4$-Haloalkylsulfonyl, $C_1$-$C_4$-Alkylamino, $C_2$-$C_8$-Dialkylamino, $C_3$-$C_6$-Cycloalkylamino, $C_2$-$C_6$-Alkylcarbonyl, $C_2$-$C_6$-Alkoxycarbonyl, $C_2$-$C_6$-Alkylaminocarbonyl, $C_3$-$C_8$-Dialkylaminocarbonyl oder $C_3$-$C_6$-Trialkylsilyl darstellt; oder
jedes $R^5$ unabhängig ein Phenyl, Benzyl, Phenoxy oder einen 5- oder 6-gliedrigen heteroaromatischen Ring darstellt, wobei jeder Ring optional mit einem bis drei Substituenten optional substituiert ist, die unabhängig aus der Gruppe ausgewählt sind, bestehend aus $C_1$-$C_4$-Alkyl, $C_2$-$C_4$-Alkenyl, $C_2$-$C_4$-Alkinyl, $C_3$-$C_6$-Cycloalkyl, $C_1$-$C_4$-Haloalkyl, $C_2$-$C_4$-Haloalkenyl, $C_2$-$C_4$-Haloalkinyl, $C_3$-$C_6$-Halocycloalkyl, Halogen, CN, $NO_2$, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Alkylamino, $C_2$-$C_8$-Dialkylamino, $C_3$-$C_6$-Cycloalkylamino, $C_3$-$C_6$-(Alkyl)cycloalkylamino, $C_2$-$C_4$-Alkylcarbonyl, $C_2$-$C_6$-Alkoxycarbonyl, $C_2$-$C_6$-Alkylaminocarbonyl, $C_3$-$C_8$-Dialkylaminocarbonyl oder $C_3$-$C_6$-Trialkylsilyl; oder
zwei $R^5$-Gruppen, wenn sie an benachbarte Kohlenstoffatome gebunden sind, zusammengenommen werden können als $-OCF_2O-$, $-CF_2CF_2O-$ oder $-OCF_2CF_2O-$;
M und $M^1$ jeweils unabhängig $CR^6R^7$, $NR^8$, O oder S darstellen, wenn die Bindung zwischen M und $M^1$ eine Einfachbindung darstellt; und jeweils unabhängig $CR^6$ oder N darstellen, wenn die Bindung zwischen M und $M^1$ eine aromatische Bindung darstellt;
jedes $R^6$ und jedes $R^7$ unabhängig H, $C_1$-$C_4$-Alkyl, Halogen, CN, $C_1$-$C_4$-Haloalkyl oder $C_1$-$C_4$-Alkoxy darstellt; und
jedes $R^8$ unabhängig H oder $C_1$-$C_4$-Alkyl darstellt.

**18.** Verbindung nach Anspruch 17, worin

A und B beide O darstellen;
$R^1$ für H, $C_1$-$C_4$-Alkyl, $C_2$-$C_4$-Alkenyl, $C_2$-$C_4$-Alkinyl, $C_3$-$C_6$-Cycloalkyl, $C_2$-$C_6$-Alkylcarbonyl oder $C_2$-$C_6$-Alkoxycarbonyl steht; und
n für 0, für 1 oder für 2 steht.

**19.** Verbindung nach Anspruch 18, worin

J einen Phenylring oder einen 5- oder 6-gliedrigen heteroaromatischen Ring darstellt, der aus der Gruppe ausgewählt ist, bestehend aus J-1, J-2, J-3 und J-4

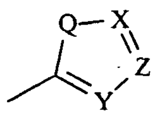 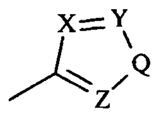 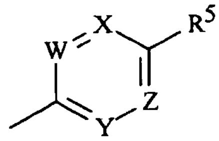 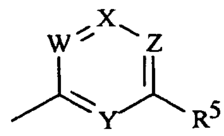

J-1 J-2 J-3 J-4

Q für O, S oder $NR^5$ steht;
W, X, Y und Z unabhängig N oder $CR^5$ darstellen, vorausgesetzt, dass in J-3 und J-4 mindestens eine der Gruppen aus W, X, Y besteht und Z für N steht;
$R^2$ für H, $C_1$-$C_4$-Alkyl, $C_2$-$C_4$-Alkenyl, $C_2$-$C_4$-Alkinyl, $C_3$-$C_6$-Cycloalkyl, $C_2$-$C_6$-Alkylcarbonyl oder $C_2$-$C_6$-Alkoxycarbonyl steht;
$R^3$ für H, $C_1$-$C_6$-Alkyl, $C_2$-$C_6$-Alkenyl, $C_2$-$C_6$-Alkinyl oder $C_3$-$C_6$-Cycloalkyl steht, wobei jedes optional mit einem oder mehr Substituenten substituiert ist, die aus der Gruppe ausgewählt sind, bestehend aus Halogen, CN, $C_1$-$C_2$-Alkoxy, $C_1$-$C_2$-Alkylthio, $C_1$-$C_2$-Alkylsulfinyl und $C_1$-$C_2$-Alkylsulfonyl;
jedes $R^4$ unabhängig $C_1$-$C_4$-Alkyl, $C_1$-$C_4$-Haloalkyl, Halogen, CN, $NO_2$, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Haloalkylthio, $C_1$-$C_4$-Haloalkylsulfinyl oder $C_1$-$C_4$-Haloalkylsulfonyl darstellt;
jedes $R^5$ unabhängig H, $C_1$-$C_4$-Alkyl, $C_1$-$C_6$-Haloalkyl, Halogen, CN, $NO_2$, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Haloalkylthio, $C_1$-$C_4$-Haloalkylsulfinyl, $C_1$-$C_4$-Haloalkylsulfonyl oder $C_2$-$C_4$-Alkoxycarbonyl, $C_2$-$C_6$-Alkylaminocarbonyl, $C_3$-$C_8$-Dialkylaminocarbonyl darstellt; oder
jedes $R^5$ unabhängig ein Phenyl, Benzyl oder einen 5- oder 6-gliedrigen heteroaromatischen Ring darstellt, wobei jeder Ring optional substituiert ist mit: $C_1$-$C_4$-Alkyl, $C_2$-$C_4$-Alkenyl, $C_2$-$C_4$-Alkinyl, $C_3$-$C_6$-Cycloalkyl, $C_1$-$C_4$-Haloalkyl, $C_2$-$C_4$-Haloalkenyl, $C_2$-$C_4$-Haloalkinyl, $C_3$-$C_6$-Halocycloalkyl, Halogen, CN, $NO_2$, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Alkylamino, $C_2$-$C_8$-Dialkylamino, $C_3$-$C_6$-Cycloalkylamino, $C_3$-$C_6$-(Alkyl)cycloalkylamino, $C_2$-$C_4$-Alkylcarbonyl, $C_2$-$C_6$-Alkoxycarbonyl, $C_2$-$C_6$-Alkylaminocarbonyl, $C_3$-$C_8$-Dialkylaminocarbonyl oder $C_3$-$C_6$-Trialkylsilyl; oder
zwei $R^5$-Gruppen, wenn sie an benachbarte Kohlenstoffatome gebunden sind, zusammengenommen werden können als $-OCF_2O-$, $-CF_2CF_2O-$ oder $-OCF_2CF_2O-$.

**20.** Verbindung nach Anspruch 19, worin

$R^1$ für H oder $C_1$-$C_4$-Alkyl steht;
$R^2$ für H oder $C_1$-$C_4$-Alkyl steht;
$R^3$ für H, $C_1$-$C_4$-Alkyl optional substituiert mit Halogen, CN, $OCH_3$, oder $S(O)_pCH_3$ steht;
jedes $R^5$ unabhängig H, $C_1$-$C_4$-Alkyl, $C_1$-$C_4$-Haloalkyl, Halogen, CN, $NO_2$, $C_1$-$C_4$-Alkoxy, $C_1$-$C_4$-Haloalkoxy, $C_1$-$C_4$-Alkylthio, $C_1$-$C_4$-Alkylsulfinyl, $C_1$-$C_4$-Alkylsulfonyl, $C_1$-$C_4$-Haloalkylthio, $C_1$-$C_4$-Haloalkylsulfinyl, $C_1$-$C_4$-Haloalkylsulfonyl, $C_2$-$C_4$-Alkoxycarbonyl oder $C_3$-$C_8$-Dialkylaminocarbonyl; oder ein Phenyl, Benzyl, oder einen 5- oder 6-gliedrigen heteroaromatischen Ring darstellt, wobei jeder Ring optional substituiert ist mit Halogen, CN, $NO_2$, $C_1$-$C_4$-Alkyl, $C_2$-$C_4$-Alkenyl, $C_2$-$C_4$-Alkinyl, $C_3$-$C_6$-Cycloalkyl, $C_1$-$C_4$-Haloalkyl, $C_1$-$C_4$-Alkoxy oder $C_1$-$C_4$-Haloalkoxy;
vorausgesetzt, dass mindestens ein $R^5$ anders als H ist und an J an der ortho-Stellung an die $N(R^1)C(=B)$ -Komponente gebunden ist; und
p für 0, für 1 oder für 2 steht.

**21.** Verbindung nach Anspruch 20, worin

J ein substituiertes Phenyl, ein substituiertes Pyrazol, ein substituiertes Pyrrol, ein substituiertes Pyridin oder ein substituiertes Pyrimidin darstellt.

**22.** Verbindung nach Anspruch 21, worin $R^1$ und $R^2$ jeweils H darstellen.

**23.** Zusammensetzung, umfassend: mindestens eine Verbindung nach Anspruch 17; und mindestens eine zusätzliche Komponente, die aus der Gruppe ausgewählt ist, bestehend aus einem Tensid, einem festen Verdünnungsmittel, einem flüssigen Verdünnungsmittel und einer anderen biologisch aktiven Verbindung oder einem anderen biologisch aktiven Mittel.

**24.** Verfahren zur Kontrolle von mindestens einem wirbellosen Schädling, umfassend: Kontaktieren des wirbellosen Schädlings oder seiner Umgebung mit einer biologisch wirksamen Menge einer Verbindung nach Anspruch 17 oder mit einer biologisch wirksamen Menge einer Zusammensetzung nach Anspruch 23.

## Revendications

**1.** Procédé de contrôle d'au moins un animal nuisible invertébré, comprenant: la mise en contact de l'animal nuisible invertébré ou de son environnement avec une quantité biologiquement efficace d'au moins un composé choisi parmi le groupe constitué d'un composé de formule I, un *N*-oxyde ou un sel de celui-ci, et un composé de formule II, un *N*-oxyde ou un sel de celui-ci

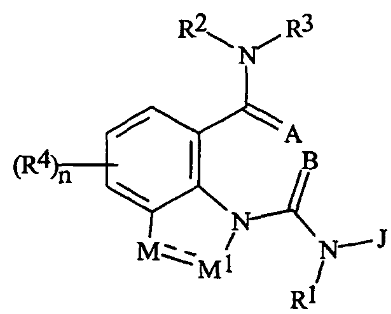

I

et

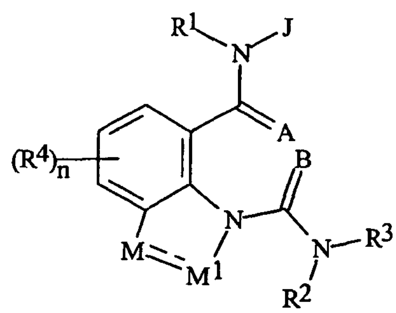

II

dans lesquelles

chaque J représente indépendamment un cycle phényle ou un noyau hétéroaromatique à 5 ou 6 chaînons, chaque cycle substitué avec 1 à 4 $R^5$;
A et B représentent indépendamment O ou S;
n a la valeur de 0, de 1, de 2 ou de 3;
R' représente un H, alkylcarbonyle en $C_2$-$C_6$, alcoxycarbonyle en $C_2$-$C_6$, alkylaminocarbonyle en $C_2$-$C_6$ ou dialkylaminocarbonyle en $C_3$-$C_8$; ou
R' représente un alkyle en $C_1$-$C_6$, un alcényle en $C_2$-$C_6$, un alcynyle en $C_2$-$C_6$ ou un cycloalkyle en $C_3$-$C_6$ chacun éventuellement substitué avec un ou plusieurs substituant(s) choisi(s) parmi le groupe constitué d'un halogène, CN, $NO_2$, un hydroxy, un alcoxy en $C_1$-$C_4$, un alkylthio en $C_1$-$C_4$, un alkylsulfinyle en $C_1$-$C_4$, un alkylsulfonyle en $C_1$-$C_4$, un alcoxycarbonyle en $C_2$-$C_4$, un alkylamino en $C_1$-$C_4$, un dialkylamino en $C_2$-$C_8$ et un cycloalkylamino en $C_3$-$C_6$;
$R^2$ représente H, un alkyle en $C_1$-$C_6$, un alcényle en $C_2$-$C_6$, un alcynyle en $C_2$-$C_6$, un cycloalkyle en $C_3$-$C_6$, un alcoxy en $C_1$-$C_4$, un alkylamino en $C_1$-$C_4$, un dialkylamino en $C_2$-$C_8$, un cycloalkylamino en $C_3$-$C_6$, un alcoxycarbonyle en $C_2$-$C_6$, ou un alkylcarbonyle en $C_2$-$C_6$;
$R^3$ représente H; ou
$R^3$ représente un alkyle en $C_1$-$C_6$, un alcényle en $C_2$-$C_6$, un alcynyle en $C_2$-$C_6$ ou un cycloalkyle en $C_3$-$C_6$ chacun éventuellement substitué avec un ou plusieurs substituant(s) choisi(s) parmi le groupe constitué d'un halogène, CN, $NO_2$, un hydroxy, un alcoxy en $C_1$-$C_4$, un alkylthio en $C_1$-$C_4$, un alkylsulfinyle en $C_1$-$C_4$ et un alkylsulfonyle en $C_1$-$C_4$; ou
$R^2$ et $R^3$ peuvent être pris conjointement avec l'azote auquel ils sont fixés pour former un cycle contenant 2 à 6 atomes de carbone et éventuellement un atome supplémentaire d'azote, de soufre ou d'oxygène, ledit cycle peut être éventuellement substitué avec 1 à 4 substituant(s) choisi(s) parmi le groupe constitué d'un alkyle en $C_1$-$C_2$, un halogène, CN, $NO_2$ et un alcoxy en $C_1$-$C_2$; et
chaque $R^4$ et chaque $R^5$ représentent indépendamment H, un alkyle en $C_1$-$C_6$, un alcényle en $C_2$-$C_6$, un alcynyle en $C_2$-$C_6$, un cycloalkyle en $C_3$-$C_6$, un alkyle en $C_1$-$C_6$ halogéné, un alcényle en $C_2$-$C_6$ halogéné, un alcynyle en $C_2$-$C_6$ halogéné, un cycloalkyle en $C_3$-$C_6$ halogéné, un halogène, CN, $CO_2H$, $CONH_2$, $NO_2$, un hydroxy, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylthio en $C_1$-$C_4$ halogéné, un alkylsulfinyle en $C_1$-$C_4$ halogéné, un alkylsul-

fonyle en $C_1$-$C_4$ halogéné, un alkylamino en $C_1$-$C_4$, un dialkylamino en $C_2$-$C_8$, un cycloalkylamino en $C_3$-$C_6$, un alkylcarbonyle en $C_2$-$C_6$, un alcoxycarbonyle en $C_2$-$C_6$, un alkylaminocarbonyle en $C_2$-$C_6$, un dialkylaminocarbonyle en $C_3$-$C_8$, ou un trialkylsilyle en $C_3$-$C_6$; ou

chaque $R^4$ et chaque $R^5$ représentent indépendamment un phényle, un benzyle, un phénoxy ou un cycle hétéroaromatique à 5 ou 6 chaînons, chaque cycle éventuellement substitué avec un à trois substituant(s) indépendamment choisi(s) parmi le groupe constitué d'un alkyle en $C_1$-$C_4$, un alcényle en $C_2$-$C_4$, un alcynyle en $C_2$-$C_4$, un cycloalkyle en $C_3$-$C_6$, un alkyle en $C_1$-$C_4$ halogéné, un alcényle en $C_2$-$C_4$ halogéné, un alcynyle en $C_2$-$C_4$ halogéné, un cycloalkyle en $C_3$-$C_6$ halogéné, un halogène, CN, $NO_2$, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylamino en $C_1$-$C_4$, un dialkylamino en $C_2$-$C_8$, un cycloalkylamino en $C_3$-$C_6$, un (alkyl)cycloalkylamino en $C_3$-$C_6$, un alkylcarbonyle en $C_2$-$C_4$, un alcoxycarbonyle en $C_2$-$C_6$, un alkylaminocarbonyle en $C_2$-$C_6$, un dialkylaminocarbonyle en $C_3$-$C_8$, ou un trialkylsilyle en $C_3$-$C_6$;

ou

deux groupes $R^5$ lorsque fixés à des atomes de carbone adjacents peuvent être pris conjointement comme

$$-OCF_2O-, -CF_2CF_2O- \text{ ou } -OCF_2CF_2O-;$$

M et $M^1$ représentent chacun indépendamment $CR^6R^7$, $NR^8$, O ou S lorsque la liaison entre M et $M^1$ est une liaison simple; et représentent chacun indépendamment $CR^6$ ou N lorsque la liaison entre M et $M^1$ est une liaison aromatique;

chaque $R^6$ et chaque $R^7$ représentent indépendamment H, un alkyle en $C_1$-$C_4$, un halogène, CN, un alkyle en $C_1$-$C_4$ halogéné ou un alcoxy en $C_1$-$C_4$; et

chaque $R^8$ représente indépendamment un atome d'hydrogène ou un groupe alkyle en $C_1$-$C_4$.

**2.** Procédé selon la revendication 1, comprenant l'application d'une quantité biologiquement efficace d'un composé de formule I, dans laquelle

A et B représentent tous deux un atome O;
$R^1$ représente un atome H, un groupe alkyle en $C_1$-$C_4$, alcényle en $C_2$-$C_4$, alcynyle en $C_2$-$C_4$, cycloalkyle en $C_3$-$C_6$, alkylcarbonyle en $C_2$-$C_6$ ou alcoxycarbonyle en $C_2$-$C_6$; et
n a la valeur de 0, de 1 ou de 2.

**3.** Procédé selon la revendication 1, comprenant l'application d'une quantité biologiquement efficace d'un composé de formule II, dans laquelle

A et B représentent tous deux un atome O;
$R^1$ représente un atome H, un groupe alkyle en $C_1$-$C_4$, alcényle en $C_2$-$C_4$, alcynyle en $C_2$-$C_4$, cycloalkyle en $C_3$-$C_6$, alkylcarbonyle en $C_2$-$C_6$ ou alcoxycarbonyle en $C_2$-$C_6$; et
n a la valeur de 0, de 1 ou de 2.

**4.** Composition comprenant: au moins un composé choisi parmi le groupe constitué d'un composé de formule I tel que défini dans la revendication 1, un *N*-oxyde ou un sel de celui-ci, et un composé de formule II tel que défini dans la revendication 1, un *N*-oxyde ou un sel de celui-ci; et au moins un autre composé ou agent biologiquement actif.

**5.** Composition selon la revendication 4, comprenant en outre un composant supplémentaire choisi parmi le groupe constitué d'un agent de surface, d'un diluant solide, et d'un diluant liquide.

**6.** Composition selon la revendication 4, dans laquelle l'autre composé ou agent biologiquement actif est choisi parmi le groupe constitué d'un autre insecticide, d'un fongicide, d'un nématicide, d'un bactéricide, d'un acaricide, d'un régulateur de croissance, d'un stimulant racinaire, d'un chimiostérilisant, d'une substance sémiochimique, d'un répulsif, d'un attractif, d'une phéromone, d'un phagostimulant, et d'une bactérie, d'un virus ou d'un champignon entomopathogènes.

**7.** Composition selon la revendication 6, dans laquelle l'autre insecticide est choisi parmi le groupe constitué d'un pyréthroïde, d'un carbamate, d'un néonicotinoïde, d'un agent bloquant du canal sodique neuronal, d'une lactone macrocyclique insecticide, d'un antagoniste de l'acide $\gamma$-aminobutyrique (GABA), d'une urée insecticide, d'un agent

mimétique de l'hormone juvénile, d'une pymétrozine, et d'un amitraz.

**8.** Composition selon la revendication 6, dans laquelle l'autre insecticide est choisi parmi le groupe constitué de l'abamectine, de l'acéphate, de l'acétamipride, de l'avermectine, de l'azadirachtine, de l'azinphos-méthyle, de la bifenthrine, du binfénazate, de la buprofézine, du carbofurane, du chlorfénapyr, du chlorfluazuron, du chlorpyrifos, du chlorpyrifos-méthyle, du chromafénozide, de la clothianidine, de la cyfluthrine, de la bêta-cyfluthrine, de la cyhalothrine, de la lambda-cyhalothrine, de la cyperméthrine, de la cyromazine, de la deltaméthrine, du diafenthiuron, du diazinon, du diflubenzuron, du diméthoate, du diofénolan, de l'émamectine, de l'endosulfan, de l'esfenvalérate, de l'éthiprole, du fénothicarb, du fénoxycarb, de la fenpropathrine, du fenproximate, de la fenvalérate, du fipronil, du flonicamide, du flucythrinate, du tau-fluvalinate, du flufénoxuron, du fonophos, de l'halofénozide, de l'hexaflumuron, de l'imidaclopride, de l'indoxacarb, de l'isofenphos, du lufénuron, du malathion, du métaldéhyde, du méthamidophos, du méthidathion, du méthomyl, du méthoprène, du méthoxychlor, du monocrotophos, du méthoxyfénozide, de la nithiazine, du novaluron, de l'oxamyle, du parathion, du parathion-méthyle, de la perméthrine, du phorate, de la phosalone, du phosmet, du phosphamidon, du pirimicarb, du profénofos, de la pymétrozine, du pyridalyle, du pyriproxyfène, de la roténone, du spinosad, du sulprofos, du tébufénozide, du téflubenzuron, de la téfluthrine, du terbufos, du tétrachlorvinphos, du thiaclopride, du thiaméthoxame, du thiodicarb, du thiosultap-sodium, de la tralométhrine, du trichlorfon et du triflumuron, de l'aldicarb, de l'oxamyl, du fénamiphos, de l'amitraz, du chinométhionate, du chlorobenziate, de la cyhexatine, du dicofol, du diénochlor, de l'étoxazole, du fénazaquin, de l'oxyde de fenbutatine, de la fenpropathrine, du fenpyroximate, de l'hexythiazox, du propargite, du pyridabène et le tébufenpyrad, du *Bacillus thuringiensis,* de la delta endotoxine de *Bacillus thuringiensis,* et des bactéries, virus et champignons entomopathogènes.

**9.** Composition selon la revendication 6, dans laquelle l'autre insecticide est choisi parmi le groupe constitué de la cyperméthrine, de la cyhalothrine, de la cyfluthrine, de la bêta-cyfluthrine, de l'esfenvalérate, du fenvalérate, de la tralométhrine, du fénothicarb, du méthomyl, de l'oxamyle, du thiodicarb, de la clothianidine, de l'imidaclopride, du thiaclopride, de l'indoxacarb, du spinosad, de l'abamectine, de l'avermectine, de l'émamectine, de l'endosulfan, de l'éthiprole, du fipronil, du flufénoxuron, du triflumuron, du diofénolan, du pyriproxyfène, de la pymétrozine, de l'amitraz, du *Bacillus thuringiensis,* de l'endotoxine delta de *Bacillus thuringiensis* et champignons entomophages.

**10.** Procédé de contrôle d'au moins un animal nuisible invertébré, comprenant:

la mise en contact de l'animal nuisible invertébré ou de son environnement avec une quantité biologiquement efficace d'une composition selon la revendication 4 ou la revendication 5.

**11.** Composé selon la formule II tel que défini dans la revendication 1, un *N*-oxyde de celui-ci ou un sel de celui-ci.

**12.** Composé selon la revendication 11, dans lequel

A et B représentent tous deux O;
$R^1$ représente un atome H, un groupe alkyle en $C_1$-$C_4$, alcényle en $C_2$-$C_4$, alcynyle en $C_2$-$C_4$, cycloalkyle en $C_3$-$C_6$, alkylcarbonyle en $C_2$-$C_6$ ou alcoxycarbonyle en $C_2$-$C_6$; et
n a la valeur de 0, de 1 ou de 2.

**13.** Composé selon la revendication 12, dans lequel

J représente un cycle phényle ou un noyau hétéroaromatique à 5 ou 6 chaînons choisi parmi le groupe constitué de J-1, J-2, J-3 et J-4, chaque cycle J éventuellement substitué avec 1 à 3 $R^5$

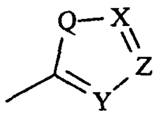
J-1

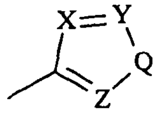
J-2

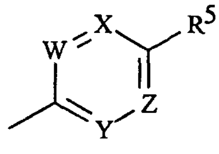
J-3

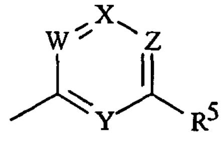
J-4

;

Q représente O, S ou $NR^5$;

W, X, Y et Z représentent indépendamment N ou $CR^5$, à condition que dans J-3 et J-4 au moins un élément parmi le groupe constitué de W, X, Y et Z représente un atome N;

$R^2$ représente H, un alkyle en $C_1$-$C_4$, un alcényle en $C_2$-$C_4$, un alcynyle en $C_2$-$C_4$, un cycloalkyle en $C_3$-$C_6$, un alkylcarbonyle en $C_2$-$C_6$ ou un alcoxycarbonyle en $C_2$-$C_6$;

$R^3$ représente H, un alkyle en $C_1$-$C_6$, un alcényle en $C_2$-$C_6$, un alcynyle en $C_2$-$C_6$ ou un cycloalkyle en $C_3$-$C_6$ chacun éventuellement substitué avec un ou plusieurs substituant(s) choisi(s) parmi le groupe constitué d'un halogène, CN, un alcoxy en $C_1$-$C_2$, un alkylthio en $C_1$-$C_2$, un alkylsulfinyle en $C_1$-$C_2$ et un alkylsulfonyle en $C_1$-$C_2$;

chaque $R^4$ représente indépendamment un alkyle en $C_1$-$C_4$, un alkyle en $C_1$-$C_4$ halogéné, un halogène, CN, $NO_2$, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylthio en $C_1$-$C_4$ halogéné, un alkylsulfinyle en $C_1$-$C_4$ halogéné ou un alkylsulfonyle en $C_1$-$C_4$ halogéné;

chaque $R^5$ représente indépendamment H, un alkyle en $C_1$-$C_4$, un alkyle en $C_1$-$C_6$ halogéné, un halogène, CN, $NO_2$, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylthio en $C_1$-$C_4$ halogéné, un alkylsulfinyle en $C_1$-$C_4$ halogéné, un alkylsulfonyle en $C_1$-$C_4$ halogéné ou un alcoxycarbonyle en $C_2$-$C_4$, un alkylaminocarbonyle en $C_2$-$C_6$, un dialkylaminocarbonyle en $C_3$-$C_8$; ou

chaque $R^5$ représente indépendamment un phényle, un benzyle ou un noyau hétéroaromatique à 5 ou 6 chaînons, chaque cycle éventuellement substitué avec un alkyle en $C_1$-$C_4$, un alcényle en $C_2$-$C_4$, un alcynyle en $C_2$-$C_4$, un cycloalkyle en $C_3$-$C_6$, un alkyle en $C_1$-$C_4$ halogéné, un alcényle en $C_2$-$C_4$ halogéné, un alcynyle en $C_2$-$C_4$ halogéné, un cycloalkyle en $C_3$-$C_6$ halogéné, un halogène, CN, $NO_2$, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylamino en $C_1$-$C_4$, un dialkylamino en $C_2$-$C_8$, un cycloalkylamino en $C_3$-$C_6$, un (alkyl)cycloalkylamino en $C_3$-$C_6$, un alkylcarbonyle en $C_2$-$C_4$, un alcoxycarbonyle en $C_2$-$C_6$, un alkylaminocarbonyle en $C_2$-$C_6$, un dialkylaminocarbonyle en $C_3$-$C_8$, ou un trialkylsilyle en $C_3$-$C_6$; ou

deux groupes $R^5$ lorsque fixés à des atomes de carbone adjacents peuvent être pris conjointement comme

$$-OCF_2O-,\ -CF_2CF_2O-\ \text{ou}\ -OCF_2CF_2O-.$$

**14.** Composé selon la revendication 13, dans lequel

$R^2$ représente H;

$R^3$ représente un groupe alkyle en $C_1$-$C_4$; et

au moins un $R^5$ est autre que H et est fixé à J en position *ortho* par rapport à la fraction $N(R^1)C(=B)$.

**15.** Composition comprenant:

au moins un composé selon la revendication 11; et

au moins un composant supplémentaire choisi parmi le groupe constitué d'un agent de surface, d'un diluant solide, d'un diluant liquide et d'un autre composé ou agent biologiquement actif.

**16.** Procédé de contrôle d'au moins un animal nuisible invertébré, comprenant: la mise en contact de l'animal nuisible invertébré ou de son environnement avec une quantité biologiquement efficace d'un composé selon la revendication 11 ou avec une quantité biologiquement efficace d'une composition selon la revendication 15.

**17.** Composé de la formule Id, un *N*-oxyde ou un sel de celui-ci,

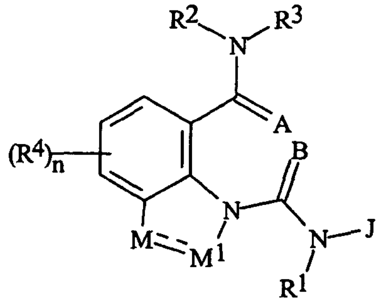

Id

dans laquelle

chaque J représente indépendamment un cycle phényle ou un noyau hétéroaromatique à 5 ou 6 chaînons, chaque cycle éventuellement substitué avec 1 à 4 $R^5$;
A et B représentent indépendamment O ou S;
n a la valeur de 0, de 1, de 2 ou de 3;
$R^1$ représente un H, un alkylcarbonyle en $C_2$-$C_6$, alcoxycarbonyle en $C_2$-$C_6$, alkylaminocarbonyle en $C_2$-$C_6$ ou dialkylaminocarbonyle en $C_3$-$C_8$; ou
$R^1$ représente un alkyle en $C_1$-$C_6$, un alcényle en $C_2$-$C_6$, un alcynyle en $C_2$-$C_6$ ou un cycloalkyle en $C_3$-$C_6$ chacun éventuellement substitué avec un ou plusieurs substituant(s) choisi(s) parmi le groupe constitué d'un halogène, CN, $NO_2$, un hydroxy, un alcoxy en $C_1$-$C_4$, un alkylthio en $C_1$-$C_4$, un alkylsulfinyle en $C_1$-$C_4$, un alkylsulfonyle en $C_1$-$C_4$, un alcoxycarbonyle en $C_2$-$C_4$, un alkylamino en $C_1$-$C_4$, un dialkylamino en $C_2$-$C_8$ et un cycloalkylamino en $C_3$-$C_6$;
$R^2$ représente H, un alkyle en $C_1$-$C_6$, un alcényle en $C_2$-$C_6$, un alcynyle en $C_2$-$C_6$, un cycloalkyle en $C_3$-$C_6$, un alcoxy en $C_1$-$C_4$, un alkylamino en $C_1$-$C_4$, un dialkylamino en $C_2$-$C_8$, un cycloalkylamino en $C_3$-$C_6$, un alcoxycarbonyle en $C_2$-$C_6$, ou un alkylcarbonyle en $C_2$-$C_6$;
$R^3$ représente H; ou
$R^3$ représente un alkyle en $C_1$-$C_6$, un alcényle en $C_2$-$C_6$, un alcynyle en $C_2$-$C_6$ ou un cycloalkyle en $C_3$-$C_6$ chacun éventuellement substitué avec un ou plusieurs substituant(s) choisi(s) parmi le groupe constitué d'un halogène, CN, $NO_2$, un hydroxy, un alcoxy en $C_1$-$C_4$, un alkylthio en $C_1$-$C_4$, un alkylsulfinyle en $C_1$-$C_4$ et un alkylsulfonyle en $C_1$-$C_4$; ou
$R^2$ et $R^3$ peuvent être pris conjointement avec l'azote auquel ils sont fixés pour former un cycle contenant 2 à 6 atomes de carbone et éventuellement un atome supplémentaire d'azote, de soufre ou d'oxygène, ledit cycle peut être éventuellement substitué avec 1 à 4 substituant(s) choisi(s) parmi le groupe constitué d'un alkyle en $C_1$-$C_2$, un halogène, CN, $NO_2$ et un alcoxy en $C_1$-$C_2$; et
chaque $R^4$ et chaque $R^5$ représentent indépendamment H, un alkyle en $C_1$-$C_6$, un alcényle en $C_2$-$C_6$, un alcynyle en $C_2$-$C_6$, un cycloalkyle en $C_3$-$C_6$, un alkyle en $C_1$-$C_6$ halogéné, un alcényle en $C_2$-$C_6$ halogéné, un alcynyle en $C_2$-$C_6$ halogéné, un cycloalkyle en $C_3$-$C_6$ halogéné, un halogène, CN, $CO_2H$, $CONH_2$, $NO_2$, un hydroxy, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylthio en $C_1$-$C_4$ halogéné, un alkylsulfinyle en $C_1$-$C_4$ halogéné, un alkylsulfonyle en $C_1$-$C_4$ halogéné, un alkylamino en $C_1$-$C_4$, un dialkylamino en $C_2$-$C_8$, un cycloalkylamino en $C_3$-$C_6$, un alkylcarbonyle en $C_2$-$C_6$, un alcoxycarbonyle en $C_2$-$C_6$, un alkylaminocarbonyle en $C_2$-$C_6$, un dialkylaminocarbonyle en $C_3$-$C_8$ ou un trialkylsilyle en $C_3$-$C_6$; ou
chaque $R^5$ représente indépendamment un phényle, un benzyle, un phénoxy ou un noyau hétéroaromatique à 5 ou 6 chaînons, chaque cycle éventuellement substitué avec un à trois substituant(s) indépendamment choisi(s) parmi le groupe constitué d'un alkyle en $C_1$-$C_4$, un alcényle en $C_2$-$C_4$, un alcynyle en $C_2$-$C_4$, un cycloalkyle en $C_3$-$C_6$, un alkyle en $C_1$-$C_4$ halogéné, un alcényle en $C_2$-$C_4$ halogéné, un alcynyle en $C_2$-$C_4$ halogéné, un cycloalkyle en $C_3$-$C_6$ halogéné, un halogène, CN, $NO_2$, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylamino en $C_1$-$C_4$, un dialkylamino en $C_2$-$C_8$, un cycloalkylamino en $C_3$-$C_6$, un (alkyl)cycloalkylamino en $C_3$-$C_6$, un alkylcarbonyle en $C_2$-$C_4$, un alcoxycarbonyle en $C_2$-$C_6$, un alkylaminocarbonyle en $C_2$-$C_6$, un dialkylaminocarbonyle en $C_3$-$C_8$, ou un trialkylsilyle en $C_3$-$C_6$; ou
deux groupes $R^5$, lorsque fixés à des atomes de carbone adjacents, peuvent être pris conjointement comme

-$OCF_2O$-, -$CF_2CF_2O$- ou -$OCF_2CF_2O$-;

M et $M^1$ représentent chacun indépendamment $CR^6R^7$, $NR^8$, O ou S lorsque la liaison entre M et $M^1$ est une simple liaison; et représentent chacun indépendamment $CR^6$ ou N lorsque la liaison entre M et $M^1$ est une liaison aromatique;
chaque $R^6$ et chaque $R^7$ représentent indépendamment H, un groupe alkyle en $C_1$-$C_4$, un halogène, CN, un groupe alkyle en $C_1$-$C_4$ halogéné ou alcoxy en $C_1$-$C_4$; et
chaque $R^8$ représente indépendamment H ou un groupe alkyle en $C_1$-$C_4$.

**18.** Composé selon la revendication 17, dans lequel

A et B représentent tous deux O;
$R^1$ représente H, un groupe alkyle en $C_1$-$C_4$, alcényle en $C_2$-$C_4$, alcynyle en $C_2$-$C_4$, cycloalkyle en $C_3$-$C_6$, alkycarbonyle en $C_2$-$C_6$ ou alcoxycarbonyle en $C_2$-$C_6$; et
n a la valeur de 0, de 1 ou de 2.

**19.** Composé selon la revendication 18, dans lequel

J représente un cycle phényle ou un noyau hétéroaromatique de 5 ou 6 chaînons choisi parmi le groupe constitué de J-1, J-2, J-3 et J-4

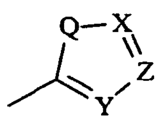
J-1

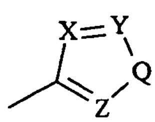
J-2

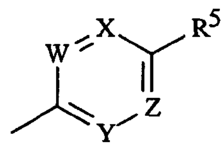
J-3

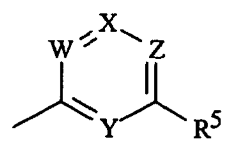
J-4
;

Q représente O, S ou $NR^5$;
W, X, Y et Z représentent indépendamment N ou $CR^5$, à condition que dans J-3 et J-4 au moins un élément parmi le groupe constitué de W, X, Y et Z soit N;
$R^2$ représente H, un alkyle en $C_1$-$C_4$, un alcényle en $C_2$-$C_4$, un alcynyle en $C_2$-$C_4$, un cycloalkyle en $C_3$-$C_6$, un alkylcarbonyle en $C_2$-$C_6$ ou un alcoxycarbonyle en $C_2$-$C_6$;
$R^3$ représente H, un alkyle en $C_1$-$C_6$, un alcényle en $C_2$-$C_6$, un alcynyle en $C_2$-$C_6$ ou un cycloalkyle en $C_3$-$C_6$ chacun éventuellement substitué avec un ou plusieurs substituant(s) choisi(s) parmi le groupe constitué d'un halogène, CN, un alcoxy en $C_1$-$C_2$, un alkylthio en $C_1$-$C_2$, un alkylsulfinyle en $C_1$-$C_2$ et un alkylsulfonyle en $C_1$-$C_2$;
chaque $R^4$ représente indépendamment un alkyle en $C_1$-$C_4$, un alkyle en $C_1$-$C_4$ halogéné, un halogène, CN, $NO_2$, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylthio en $C_1$-$C_4$ halogéné, un alkylsulfinyle en $C_1$-$C_4$ halogéné ou un alkylsulfonyle en $C_1$-$C_4$ halogéné;
chaque $R^5$ représente indépendamment H, un alkyle en $C_1$-$C_4$, un alkyle en $C_1$-$C_6$ halogéné, un halogène, CN, $NO_2$, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylthio en $C_1$-$C_4$ halogéné, un alkylsulfinyle en $C_1$-$C_4$ halogéné, un alkylsulfonyle en $C_1$-$C_4$ halogéné ou un alcoxycarbonyle en $C_2$-$C_4$, un alkylaminocarbonyle en $C_2$-$C_6$, un dialkylaminocarbonyle en $C_3$-$C_8$; ou
chaque $R^5$ représente indépendamment un phényle, un benzyle ou un noyau hétéroaromatique à 5 ou 6 chaînons, chaque cycle éventuellement substitué avec un alkyle en $C_1$-$C_4$, un alcényle en $C_2$-$C_4$, un alcynyle en $C_2$-$C_4$, un cycloalkyle en $C_3$-$C_6$, un alkyle en $C_1$-$C_4$ halogéné, un alcényle en $C_2$-$C_4$ halogéné, un alcynyle en $C_2$-$C_4$ halogéné, un cycloalkyle en $C_3$-$C_6$ halogéné, un halogène, CN, $NO_2$, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylamino en $C_1$-$C_4$, un dialkylamino en $C_2$-$C_8$, un cycloalkylamino en $C_3$-$C_6$, un (alkyl)cycloalkylamino en $C_3$-$C_6$, un alkylcarbonyle en $C_2$-$C_4$, un alcoxycarbonyle en $C_2$-$C_6$, un alkylaminocarbonyle en $C_2$-$C_6$, un dialkylaminocarbonyle en $C_3$-$C_8$, ou un trialkylsilyle en $C_3$-$C_6$; ou

deux groupes $R^5$ lorsque fixés à des atomes de carbone adjacents peuvent être pris conjointement comme

$$-OCF_2O-, -CF_2CF_2O- \text{ ou } -OCF_2CF_2O-.$$

**20.** Composé selon la revendication 19, dans lequel

$R^1$ représente H ou un alkyle en $C_1$-$C_4$;
$R^2$ représente H ou un alkyle en $C_1$-$C_4$;
$R^3$ représente H, un alkyle en $C_1$-$C_4$ éventuellement substitué avec un halogène, CN, $OCH_3$, ou $S(O)_pCH_3$;
chaque $R^5$ représente indépendamment un atome H, un alkyle en $C_1$-$C_4$, un alkyle en $C_1$-$C_4$ halogéné, un halogène, CN, $NO_2$, un alcoxy en $C_1$-$C_4$, un alcoxy en $C_1$-$C_4$ halogéné, un alkylthio en $C_1$-$C_4$, un alkylsulfinyle en $C_1$-$C_4$, un alkylsulfonyle en $C_1$-$C_4$, un alkylthio en $C_1$-$C_4$ halogéné, un alkylsulfinyle en $C_1$-$C_4$ halogéné, un alkylsulfonyle en $C_1$-$C_4$ halogéné, un alcoxycarbonyle en $C_2$-$C_4$ ou un dialkylaminocarbonyle en $C_3$-$C_8$; ou un phényle, un benzyle, ou un noyau hétéroaromatique à 5 ou 6 chaînons, chaque cycle éventuellement substitué avec un halogène, CN, $NO_2$, un alkyle en $C_1$-$C_4$, un alcényle en $C_2$-$C_4$, un alcynyle en $C_2$-$C_4$, un cycloalkyle en $C_3$-$C_6$, un alkyle en $C_1$-$C_4$ halogéné, un alcoxy en $C_1$-$C_4$ ou un alcoxy en $C_1$-$C_4$ halogéné;
à condition qu'au moins un $R^5$ soit autre que H et soit fixé à J en position *ortho* par rapport à la fraction $N(R^1)C(=B)$; et
p a la valeur de 0, de 1 ou de 2.

**21.** Composé selon la revendication 20, dans lequel

J représente un groupe phényle substitué, un groupe pyrazole substitué, un groupe pyrrole substitué, un groupe pyridine substitué ou un groupe pyrimidine substitué.

**22.** Composé selon la revendication 21, dans lequel $R^1$ et $R^2$ représentent chacun un atome H.

**23.** Composition comprenant au moins un composé selon la revendication 17, et au moins un composant supplémentaire choisi parmi le groupe constitué d'un agent de surface, d'un diluant solide, d'un diluant liquide et d'un autre composé ou agent biologiquement actif.

**24.** Procédé de contrôle d'au moins un animal nuisible invertébré, comprenant: la mise en contact de l'animal nuisible invertébré ou de son environnement avec une quantité biologiquement efficace d'un composé selon la revendication 17 ou avec une quantité biologiquement efficace d'une composition selon la revendication 23.